# EUROPEAN PATENT APPLICATION

(11) **EP 4 248 905 A2**
(43) Date of publication of application: **27.09.2023**
(21) Application number: 23183618.0
(22) Date of filing: 14.01.2014
(51) Int. Cl.: A61B 90/40, A61B 17/34, A61B 42/00, A61B 46/27, A61B 90/00

(54) **SURGICAL ASSISTING DEVICE**

(30) Priority: 14.01.2013 SE 1350042
(62) Divisional of application: 14706347.3
(71) Applicant: MedicalTree Patent Ltd., 223 70 Lund (SE)
(72) Inventor: Forsell, Peter, 223 70 Lund (SE)
(74) Representative: AWA Sweden AB

(57) **Abstract**

A medical device adapted to be positioned inside a patient's body via an incision made in the skin of the patient. The medical device comprises an internal sealing device, comprising an internal sealing member adapted to be positioned in connection to and seal against at least a part of at least one human organ or human tissue related to and in contact with said human organ, for creating a seal between the medical device and at least the part of the at least one human organ or human tissue related to and in contact with said human organ. The medical device further comprises a tubular wall adapted to create a medical device chamber sealingly connected to the internal sealing device, such that a portion of at least one human organ or tissue is placed inside of the medical device chamber. The internal sealing member (2605oa) has an adjustable circumference, for enclosing or encircling a portion of the at least one human organ or human tissue.

## Description

### Technical field

The present invention relates to the field of endoscopic and/or open surgery.

### Background art

Surgical procedures have to some extent been performed as long as the human being have existed, however the techniques that started looking like the surgery being used today started its development during the 16:th century. Minimally invasive surgery is a concept in which the surgeon aims to minimize the scarring and post-operative hospital stay by performing the procedure through a small incision in the skin. The first minimally invasive procedure was performed in the beginning of the 20:th century and has developed into the most preferred technique in the industrialized world for procedures that allow a laparoscopic approach. Laparoscopic surgery is usually performed through 1 - 4 trocars placed in small incisions in the skin. For creating a cavity in the body of the patient for enabling the surgeon to view inside of the body CO2 gas is usually inserted through one of the trocars inflating the body. The use of CO2 gas requires that the trocars seal properly against the skin of the patient, since to large leaking would reduce the inflation of the cavity, thus not creating a large enough cavity for the surgeon to see the surgical field well enough. The use of vacuum for creating an airtight seal between the body of the patient and a medical device is efficient as the use of vacuum promotes full contact between the medical device and the skin of the patient. The use of vacuum also enables direct feedback of the level of sealing, as the level of sealing can be determined by measuring the leakage from a vacuum sealing member. When vacuum is applied to the skin of the patient, superficial capillaries under the skin may be ruptured creating a hematoma, which alters the skin tone. The use of vacuum on one portion of the skin of the patient may also affect the circulation to another portion of the skin of the patient, which in severe cases may cause ischemia of skin portions.

Even if laparoscopic techniques are widely used today complications do occur, which in some instances leads to conversion into open surgery. The reasons for having to convert a laparoscopic procedure to open surgery could for example be accidental invasion of the adjacent tissue, the size of for example a tumor being removed exceeding the expected size or due to inflammatory disease. Normal conversion rates for laparoscopic procedures range between 5 - 36%. Since the conversions are done without proper preparation and usually as response to a problem, patients that have undergone converted surgery require longer postoperative hospital stay than scheduled open surgical patients. Complications with converted patients are also a lot higher than with scheduled open surgical patients, as much as 70% higher. In for example laparoscopic Cholecystectomy, 10% is converted to open surgery, due to bleedings, abscess and inflammations and of the converted patients the complications leading to the death of the patient is as much as twice as high. A general drawback with all minimally invasive procedure is that there is a size limitation of the objects possible to transfer between the inside of the patient's body and the outside thereof. Usually the removing of larger specimens from the body is performed by means of making a larger incision at the end of the surgery while releasing the CO2 gas, which has occupied the cavity in which the laparoscopic surgery is performed. Examples of surgical procedure where relatively large specimens are removed from the body are: appendectomy, cholecystectomy, nephrectomy, hysterectomy, oophorectomy, adrenalectomy, splenectomy, colon resection, small intestine resection, stomach resection, prostatectomy, urinary bladder resection, liver resection and a huge number of different cancer extirpation.

Arthroscopy is a surgical discipline similar to laparoscopic surgery but focuses on the joints of the patient, which in arthroscopic surgery can be operated without being fully opened. Usually, a plurality of smaller incisions are made so as to enable a surgeon to insert at least one optical instrument (arthroscope) for optical inspection of the joint, and at least one manipulating instrument for performing a manipulating operation within the joint. A clear liquid is circled in the joint to expand the joint and enable vision within the joint. The advantage of arthroscopy over traditional open surgery is that it reduces recovery time and increases the rate of surgical success, since the procedure imposes less traumatization to the tissue. Arthroscopy is especially useful for professional athletes since they require fast healing time.

Since normal arthroscopy is performed with a plurality of small incisions it has the same drawbacks as laparoscopic surgery in that it imposes a limitation on the size of objects that can be transferred into the joint, for example can most arthroplastic surgery normally not be performed using arthroscopy since typically all implants have a size far exceeding that of the typical incisions made in the normal arthroscopic procedures. Arthroplastic procedures are therefore confined to the use of open surgery in which incisions, and thus the opening into the area of the joints needs to be of considerable size creating a large surface exposed to the environment of the operating room. The risk of the patient getting an infectious disease following orthopedic surgery is considerably larger than in abdominal surgery, which has been a focus area of leading orthopedic surgeons the last decades. Generally, the effect of bacterial infection if connection with orthopedic surgery includes: prolong rehabilitation, morbidity and mortality. Disease-carrying bacteria, viruses, and parasites that get into the body can destroy healthy tissue, multiply and spread through the blood. Even if the infection occurs superficially in the skin and other soft tissue, the infection can lead to infection of bones (osteomyelitis) and joints (septic arthritis). Orthopedic infections can, if not promptly treated become chronic and lead to paralysis or morbidity of infected extremities, as well as destroy an otherwise healthy joint.

The increased awareness of the high risk of infections in connection with orthopedic surgery has increased precaution during and after orthopedic surgery. Important factors for maintaining a low incidence of infection is typically: operating theatre design, meticulous surgical technique and aseptic discipline within all involved areas of the operation. Specific precautions during orthopedic surgery includes: use of especially sterilized operating rooms, for example requiring special air filters in the air conditioning systems, limiting of the amount of staff present in the operating room, imposing special requirements on clothing to be worn in the operating rooms, including the use of full body protective suits. Complex instruments and parts of machinery are generally very difficult to sterilize. The most commonly occurring solution to this problem is that the robots are fully covered with disposable sheets, a process being both time-consuming and costly.

The operating environments of the industrialized world are generally both very clean and all measures are taken to reduce the risk that infectious disease should affect the post-operative care. However, environments where surgery is performed around the globe is far from always at the operating theatres of the industrialized world, which in some cases make the proper sterilization of the operating environment or the tools to be used in that environment difficult to perform. In any instance, surgery is still needed in remote places and transportable sterile environments and pre-sterilized instruments is therefore of importance.

### Summary

A medical device adapted to be positioned in relation to an incision made in the skin of the patient is provided. The medical device comprises a vacuum sealing device comprising an internal vacuum sealing member adapted to be positioned inside the body of the patient, for creating a vacuum seal between the medical device and human tissue inside of the skin of the patient. By providing vacuum sealing inside of the body of the patient the surface of the skin can be kept unaffected, which means that no hematomas or discoloring of the skin will occur, also the internal vacuum sealing member makes it possible to seal further inside the body of the patient and thereby isolate certain areas in the body of the patient. The vacuum sealing device could for example be adapted to provide sealing against muscle tissue, muscle fascia, fat tissue, the inside of the patient's skin, subcutaneous tissue, the surface of a section made in the body of the patient, a bodily organ, or fibrotic tissue.

According to one embodiment, the internal vacuum sealing device or internal vacuum sealing member is adapted to be positioned in the incision made in the skin of the patient and/or on the inside of the incision. The

The medical device may have a device chamber adapted to communicate with a cavity in the patient's body and the internal vacuum sealing device may be adapted to be positioned inside the body to seal the total chamber formed by the cavity and the medical device towards the outside thereof.

According to one embodiment, the first vacuum sealing member comprises a vacuum groove creating a vacuum chamber together with the human tissue inside of the skin of the patient.

According to one embodiment, the vacuum sealing device comprises a second vacuum sealing member comprising a second vacuum groove creating a second vacuum chamber together with another part of the human tissue inside of the skin of the patient. At least one of the vacuum sealing members may be a loop shaped vacuum sealing member adapted to encircle the incision made in the skin of the patient on the inside of the incision in the skin and further adapted to connect to human tissue of the patient inside the body, sealing towards a muscle fascia and/or the outside of a muscle fascia and/or the inside of a muscle fascia and/or the muscle fascia of the rectus abdominis in the abdominal wall and/or the muscle fascia of the peritoneum and/or fat tissue and/or fibrotic tissue and/or muscle tissue, and/or a body organ, all positioned inside the body. The vacuum sealing member is adapted to be placed to seal with vacuum towards the human tissue, when an incision has been made in the skin of the patient.

The medical device may further comprise a holding device adapted to hold the medical device positioned in relation to the skin of the patient and further adapted to be holding the medical device from inside the patient's body, and/or be holding the medical device from both the inside of the patient's body and the skin on the outside of the body, and/or be holding by clamping on both side of at least one of; the abdominal wall, the thoraxial wall and the wall of a created body cavity, and/or be integrated with the vacuum sealing device.

According to one embodiment, at least one of the vacuum sealing members is a circular vacuum sealing member.

The vacuum sealing member of the medical device according to any one of the embodiments may further comprise an external vacuum sealing member adapted to seal against the skin of the patient, on the outside thereof. The external sealing member may function as a holding member for holding the medical device from the outside of the body of the patient. The external vacuum sealing member may be a loop shaped external vacuum sealing member adapted to encircle the incision made in the skin of the patient and connect to the skin of the patient, on the outside thereof.

The medical device may further comprise a closable body port adapted to enable transfer from the outside of the patient's body to the inside of the patient's body, through the incision made in the skin of the patient. According to one embodiment, the medical device may comprise at least two closable body ports adapted to enable transfer from the outside of the patient's body to the inside of the patient's body, through the incision made in the skin of the patient.

The medical device may also comprise a wall adapted to enclose a chamber adapted to be in fluid connection with a cavity in the body of the patient, in which a portion of the surgical procedure can be performed, the chamber is. The chamber may be placed entirely external to the body of the patient, partially internally and partially externally, or fully internally in the body of the patient. The chamber may have a volume larger than 500 000 mm3, such that a step of the surgical procedure can be performed inside of the chamber.

The wall could according to one embodiment comprise a closable wall port adapted to enable transfer from the ambient environment to the inside of the chamber. The wall may be flexible and/or elastic and/or transparent and could for example be made from PVC with a plasticizer additive.

The wall may further comprise at least one integrated glove enabling manual manipulation within the chamber of the medical device and/or inside of the cavity of the patient.

According to one embodiment, the medical device further comprises a coupling adapted to connect an inset to the medical device, the coupling may be adapted to be placed in at least one of: the wall of the medical device, and the incision made in the patient's skin. The inset could for example be an inset selected from a glove inset, a hand access inset, a port inset, a multiport inset, and a gel port inset.

The closable wall port and the closable body port in any of the embodiments herein may additionally comprise connecting means for connecting the closable wall port to the closable body port. The closable body port may comprise a first penetrable self sealing gel and the closable wall port may comprise a second penetrable self sealing gel, and the first and second penetrable self sealing gels may be adapted to be placed tightly together by the wall and body ports being connected, such that they act as a single penetrable self sealing gel adapted to, in a non-compressed state, provide sealing, and in a compressed state enable a hand or an object to be inserted through the self sealing gel while maintaining the seal.

The wall forming the chamber may in one embodiment be adapted to hold a pressure within the chamber exceeding atmospheric pressure.

The medical device according to any of the embodiments herein may be part of a medical device system further comprising a pressure adjustment device connected to the vacuum sealing device, for creating a pressure below atmospheric pressure in at least one vacuum sealing member. The medical device system may additionally comprise a control unit for controlling the pressure adjustment device.

According to one embodiment, the medical device may further comprise at least one sensor, and the control unit may be adapted to control the pump on the basis of input from the at least one sensor. The sensor may be adapted to sense a physiological parameter of the body of the patient, which could be a physiological parameter selected from: the blood flow of the patient, the saturation of the blood of the patient, an ischemia marker of the patient, the temperature at the skin of the patient, and the skin tone of the patient. In alternative embodiments, the sensor may be adapted to sense a physical parameter of the medical device. The physical parameter may be a parameter selected from: the pressure in the sealing device, the pressure in the chamber, and the direct or indirect leakage of fluid from the chamber. In any of the embodiments of the medical device system, the control unit may be adapted to control the pressure of the vacuum sealing device, such that the pressure of the vacuum sealing device does not substantially affect the blood flow of the patient.

The internal vacuum sealing member in the medical device according to any of the embodiments may have an adjustable circumference, such that the size of the area enclosed or encircled can be adjusted.

A medical device adapted to be positioned inside a patient's body via an incision made in the skin of the patient is provided. The medical device comprises an internal sealing device, comprising an internal sealing member adapted to be positioned in connection to and seal against at least a part of at least one human organ or human tissue related to and in contact with said human organ, for creating a seal between the medical device and at least the part of the at least one human organ or human tissue related to and in contact with said human organ. The medical device further comprises a wall adapted to create a medical device chamber sealingly connected to the internal sealing device, such that the medical device chamber is placed in connection with at least one of; the at least one human organ, and at least one surgically opened organ. A sealed space is created, by the wall enclosing the medical device chamber and at least one of; at least a part of the at least one human organ, and a cavity in the at least one surgically opened organ, when the medical device is implanted during the surgical procedure.

According to one embodiment, the internal sealing device comprises an internal vacuum sealing device, sealing by using a vacuum towards the human organ or human tissue related to and in contact with said human organ, and/or an internal pressure sealing device, sealing using a pressure towards the human organ or human tissue related to and in contact with said human organ, and/or an internal adhesive sealing device, sealing by adhesively contacting the human organ or human tissue related to and in contact with said human organ.

The internal sealing device may be adapted to be positioned, on the human organ, and/or on human tissue related to and in contact with said human organ, and/or in an incision made in the at least one human organ of the patient, and/or on the inside of the at least one human organ or human tissue related to and in contact with the inside of said human organ, and/or on the outside of the at least one human organ or human tissue related to and in contact with the outside of said human organ.

The organ could for example be; an esophagus, a stomach, a small intestine, a large intestine, an anus, a gallbladder, a bile duct, a kidney, a renal pelvis, an ureter, a urine bladder, an urethra, a blood vessel, a heart, a lung, a bronchus, an orifice of the body, muscle fascia, peritoneum, fat tissue, fibrotic tissue, muscle tissue, and a body organ, all positioned inside the body, and the internal sealing device may be adapted to be placed to seal towards the at least one human organ or human tissue related to and in contact with said human organ, when an incision has been made in the patient's body.

The medical device chamber in any of the embodiments may be adapted to communicate with the cavity inside the organ in the patient's body and/or a port placed in the incision of the body, wherein the medical device chamber is extending from the at least one human organ, the cavity in said organ, or human tissue related to and in contact with said human organ, to reach and connect to the port in the incision area of the human body, and/or communicate with a part of the medical chamber placed outside the body, and/or a part of the medical chamber placed outside the body, wherein a port placed in the incision of the body and connected to the wall of the chamber, is adapted to be opened or closed to allow communication to the part of the chamber placed outside the body when said port being open. The internal sealing device is adapted to be positioned inside the body to seal the total chamber formed by the alternatives above, towards the outside thereof.

The medical device may further be adapted to seal fluid or other bodily matter from the inside of the at least one human organ, when an incision is made in the human organ, to avoid the spreading of such fluid or other bodily matter outside the sealed space.

The medical device may be adapted to seal the sealed space during a surgical procedure involving the opening of an organ with bodily matter containing bacteria, and/or the extirpation of at least a part of the at least one organ or human tissue related to and contact with said organ, and/or extirpation of any bodily matter from inside or outside the at least one organ, and/or extirpation of any bodily tissue from inside or outside the at least one organ or relating to the at least one organ, and/or connecting different parts of the at least one organ, and/or connecting different parts of different organs, and/or placing at least one medical device in relation to the at least one organ, and/or replacing at least one bodily function, and/or placing at least one medical device in relation to the at least one organ, and/or replacing at least one bodily function by placing at least one medical device in relation to the at least one organ.

The medical device in any of the embodiments may be adapted to receive within the medical device chamber, at least one trocar and/or at least one inset and/or at least one port and/or at least one camera and/or at least one tube or channel for injecting fluid into the chamber and/or at least one suction instrument and/or at least one diathermy instrument and/or at least one instrument or tool, to be able to perform the surgical procedure in the sealed space.

According to one embodiment, the internal vacuum sealing member of the medical device may comprise a vacuum groove adapted to create a vacuum chamber together with the human organ or human tissue.

The medical device according to any of the embodiments herein may comprise a second or more vacuum sealing member comprising at least a second vacuum groove creating a second vacuum chamber together with another part of the human body and/or a part of the same medical device chamber having a different shape, wherein the chamber is enclosed by the two or more internal vacuum sealing members, adapted to contact different body parts.

At least one of the internal vacuum sealing members herein may be a loop shaped vacuum sealing member adapted to at least one of; encircle the incision made in the at least one organ of the patient, and encircling the human organ. The at least one vacuum sealing member may be adapted to be placed to seal by means of vacuum towards the at least one human organ to create the sealed space for performing a surgical procedure, when an incision has been made in the organ of the patient.

The medical device may further comprise a holding member adapted to hold the medical device positioned in relation to the incision and/or the at least one organ of the patient and further be adapted to be holding the medical device from inside the patient's body and/or be holding the medical device from both the inside of the patient's body and the skin on the outside of the body and/or be holding by clamping on both side of the abdominal wall and/or the thoraxial wall and/or the wall of a created body cavity, and/or be integrated with the vacuum sealing device and/or be holding the medical device connected to a body port placed in relation the incision in the body, and/or be holding in different separate positions.

According to one embodiment, the internal vacuum sealing member is a circular vacuum sealing member.

The medical device according to any one of the embodiments may additionally comprise an additional vacuum sealing member adapted to seal the incision in the body, in the incision and/or tissue connected to the cut part of the incision, on the outside thereof and/or on the inside thereof and/or on the both the inside and outside of the incised wall of the cavity.

According to one embodiment, the medical device further comprises at least one holding member and/or at least one a pressure sealing member and/or an additional vacuum sealing member, adapted to be placed on the inside and/or the outside and/or both inside and outside of the incision in the body or the tissue related to the incision.

The additional vacuum sealing member may in any of the embodiments be a loop shaped additional vacuum sealing member adapted to encircle the incision made in the skin of the patient, and be adapted to connect to the skin of the patient on the outside thereof and/or human tissue of the patient inside the body, sealing towards at least one of a muscle fascia and/or the outside of a muscle fascia and/or the inside of a muscle fascia and/or the muscle fascia of the rectus abdominis in the abdominal wall and/or the peritoneum and/or fat tissue and/or fibrotic tissue and/or muscle tissue and/or a body organ, all positioned inside the body. The at least one vacuum additional sealing member may be adapted to be placed to seal with vacuum towards the human tissue in close relation to the incision, when an incision has been made in the skin of the patient.

The medical device according to any of the embodiments may further comprise a closable body port adapted to enable transfer from the outside of the patient's body to the inside of the patient's body through the incision made in the skin of the patient, and/or a closable wall port placed in another part of portion of the wall of the chamber, adapted to form a part of portion of the chamber placed outside the incision, and wherein the closable wall port is adapted to enable transfer from the outside of the medical device to the inside of the medical device chamber through the wall of the medical device in which the wall port is mounted.

According to one embodiment, the medical device further comprises a multiport and a portion of the chamber formed outside the incision is adapted to change from a first to a second shape during the surgical procedure. In the first shape, the medical device allows the multiport to be positioned in close relation to the incision in the skin of the patient to allow key hole surgery through the multiport placed in close relation to the incision ion the skin, and in the second shape allows the multiport to be positioned further from the incision in the skin of the patient, such that the portion of the chamber placed outside the skin is adapted to be formed with enough space to receive at least one of; a hand placed inside an inset comprising a glove, an apparatus, a non-body part, a body part, an instrument and a part used for the surgical procedure.

The medical device according to any one of the embodiments herein may be adapted to create a sealed tunnel between a body port connected to the wall of the medical device, the body port adapted to be placed in close in proximity to the incision in the skin of the patient and the human organ or human tissue related to and in contact with said human organ sealed towards the medical device by the internal sealing device, thereby containing operational matter inside the sealed space. The tissue in proximity to the incision in the skin of the patient could be the skin of the patient on the outside thereof and/or human tissue of the patient inside the body in the incision.

The multiport in any of the embodiments herein may comprises a two part port adapted to be disconnectably connected, and wherein the two parts are connected to the wall to create a portion of the sealed chamber placed outside the body.

The medical device may further comprise at least two closable body ports and at least two closable multiports comprising two, three or more ports adapted to enable transfer from the outside of the patient's body to the inside of the patient's body, through the incision made in the skin of the patient.

According to one embodiment, the medical device wall is adapted to enclose a chamber in which a portion of the surgical procedure can be performed, and the chamber may be adapted to be in fluid connection with a cavity having both a first external part in adapted to be placed on the outside of the body and a second internal part being adapted to be placed inside the body of the patient, wherein the medical device is adapted to have the external and internal parts of the chamber adapted to be at least one of; forming together one chamber, and allowing a closable body port adapted to be placed in close proximity to the incision of the body between the external and internal part of portion of the chamber, to have the chamber to be divided in two separate closed parts of the chamber.

In one of the embodiments herein, the medical device may comprise a tube or channel adapted to be in fluid connection with and reaching from the outside to the inside of the body for allowing injection of an disinfection agent or injecting fluid to clean and/or disinfect remaining body parts inside the sealed space, when the surgical procedure are performed.

In some of the embodiments herein, the medical device may be adapted to at least one of; loosen the at least one internal sealing device from the human organ or human tissue related to and in contact with said human organ, and use the at least one internal sealing device to keep the sealed space closed after the first internal sealing device has been loosened.

According to any one of the embodiments herein, the medical device may further comprise a closing sealing device, adapted to keep the sealed space closed when finishing the surgical procedure to avoid leakage of bodily matter from the sealed space.

The sealed tunnel of any of the embodiments herein will reduce infection rate, when performing at least one of; appendectomy, cholecystectomy, nephrectomy, hysterectomy, oophorectomy, adrenalectomy, gastric bypass, Nissen fundoplication, intestinal shunts, hernia repair, splenectomy, colon or small intestine resection, liver resection, cecostomy, colostomy, duodenostomy, ileostomy, jejunostomy, appendicostomy, esophagostomy, gastrostomy, urostomy, nephrostomy, ureterostomy, vesicostomy, endoscopic surgery, open surgery, combined open and endoscopic surgery, arthroscopic surgery, open j oint surgery, combined open j oint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of another medical device and other foreign parts using combined open and laparoscopic or arthroscopic surgery, the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ, and replacing at least one bodily function by placing at least one medical device in relation to the at least one organ. A medical implant for insertion could for example comprise at least one of; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant.

The medical device according to any one of the embodiments herein may be adapted to be used together with at least one of: a surgical instrument, an endoscopic instrument, a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an instrument, and a machine.

The medical device according to any one of embodiments herein may have a volume larger than 500 000 mm3, such that steps of a surgical procedure can be performed in the chamber.

The wall of the medical device may in some embodiments comprise a closable wall port adapted to enable transfer from the ambient environment to the inside of the chamber. The wall may be flexible and/or elastic and/or transparent, such as a wall made from PVC with a plasticizer additive.

The wall of the medical device may additionally comprise at least one integrated glove r inset comprising an integrated glove enabling manual manipulation within the chamber of the medical device and/or inside the cavity of the patient.

The medical device according to any one of embodiments may further comprise a coupling adapted to connect an inset or port to the medical device. The coupling may be placed in at least one of: the wall of the medical device, and the incision made in the patient's skin.

The inset may be an inset selected from a glove inset, a hand access inset, a port inset, a multiport inset, and a gel port inset.

The medical device according to any one of the embodiments herein may comprise a closable wall port/multiport and/or a closable body port/multiport adapted to be placed in or in close relation to the incision of the patient. The closable wall port/multiport and the closable body port/multiport may comprise connecting means for connecting the closable wall port/multiport to the closable body port/multiport, and wherein the multiport comprise two, three, four or more or more ports placed in one multiport casing.

The closable body port may comprise a first penetrable self sealing gel and the closable wall port may comprise a second penetrable self sealing gel, and the first and second penetrable self sealing gels may be adapted to be placed tightly together by the wall and body ports being connected, such that they act as a single penetrable self sealing gel adapted to, in a non-compressed state, provide sealing, and in a compressed state enable a hand or an object to be inserted through the self sealing gel while maintaining the seal.

In any of the embodiments described, the wall forming the chamber may be adapted to hold a pressure within the chamber exceeding atmospheric pressure, such that a laparoscopic procedure can be performed.

The internal or external vacuum sealing members in any of the embodiments herein may be loop shaped vacuum sealing members adapted to at least one of; encircle the incision made, encircle two or more positions on or around the at least one organ of the patient, and encircling two or more human organs to enclose a larger surgical area in the sealed space. The at least one vacuum sealing member is adapted to be placed to seal by means of vacuum towards the at least one human organ or related tissue, when an incision has been made in the organ of the patient.

The closing sealing device may be adapted to seal with vacuum to keep the sealed space closed when finishing the surgical procedure to avoid leakage of bodily matter from the sealed space.

The medical device may additionally comprise a closable wall multiport, and a closable body multiport adapted to be placed in or in close relation to the incision of the patient. The closable wall port and the closable body port may comprise connecting means for connecting the closable wall port to the closable body port.

The medical device may additionally comprise one or two or more fluid conduits leading from outside the body to inside the body, inside the chamber, adapted to allow sucking and injecting fluid into the chamber from outside the body, wherein the one or two or more fluid conduits is adapted to be at least one of; integrated in at least one of; in the medical device, the wall and a body port placed in close proximity to the incision, placed as a separate tube and having the two ends of the fluid conduit displaced as a separate tubes and there in between adapted to be integrated in at least one of; in the medical device, the wall and the body port placed in close proximity to the incision.

The medical device chamber may be adapted to encompass part of the surgical procedure to be performed, and the medical device may further comprise a closeable wall multiport placed in a portion of the wall, and a closeable body multiport placed in a second portion of the wall and adapted to be placed in or closely related to an incision in the patient's body. A sealing device may be adapted to be at least one of; sealingly connected to at least one of; the wall and the closable body port, and an integrated part of at least one of; the wall and he closable body port, and the sealing device may further be adapted to seal against at least one of the skin and tissue of the patient. The wall, wall multiport and body multiport are adapted to together form an airlock sluice between a cavity in the body of the patient and the ambient environment, when the surgical procedure is performed.

The wall and body ports of the medical device may be positioned relative to each other such that an elongated object is longitudinally displaceable through the wall and body ports.

In any of the embodiments herein, the wall port of the medical device may be displaceable between a first position and a second position, wherein the second position is situated more remote from the body port. The wall multiport may be adapted to be at least one of: connected to the body multiport, abutting the body multiport, locked to the body multiport, formed into an integrated port together with the body multiport.

At least one of; the closable wall multiport and the closable body multiport may comprise a penetratable self sealing gel, such that an object or hand can be inserted through the self sealing gel, when both the wall multiport and the body multiport comprises such penetratable self sealing gel, allowing at least one of; an object, apparatus, instrument or hand to be inserted through the wall multiport and further through the body multiport into the body of the patient, and a body part to be moved from inside the body through both the body multiport and thereafter the wall multiport to the outside of the chamber, while maintaining the sealed environment during the surgical procedure.

The medical device may further comprise at least one of; an additional body port, an additional body multiport, an additional body inset, an additional wall port, an additional wall multiport and an additional wall inset. At least one of the wall port, wall multiport, body port, body multiport, wall inset, body inset may be exchangeable by at least one of; the additional body port, the additional body multiport, the additional body inset, the additional wall port, the additional wall multiport and the additional wall inset.

In one embodiment, the medical device a comprises a sealing member adapted to seal the wall towards human tissue and the internal sealing member comprising at least one of; a vacuum sealing member adapted to hold a pressure less than atmospheric pressure, and a pressure sealing member adapted to hold a pressure above atmospheric pressure to seal between the wall and said human tissue.

The medical device may additionally comprise a closable body port and closable multiport. The closeable body port may comprise a closeable body multi-port adapted to be placed from inside the chamber in an incision to be performed in the skin of the patient, and the body multiport may be adapted to be connected to at least one of; the wall port, and a wall multiport, when the medical device is applied on the patient's body for performing a surgical procedure. The closable wall port may comprise a closeable wall multiport adapted to be connected to at least one of; the body port, and a body multiport placed in an incision in the patient's body, when the medical device is applied on the patient's body for performing a surgical procedure.

According to one embodiment, the wall is a flexible wall comprising a first inset and an interconnectable port, the interconnectable port may comprise a first port and a second coupling adapted be interconnected with at least one of; the first port/multiport to form the interconnectable port, and the first inset, wherein the second coupling is disconnectable from the first port/multiport or first inset. The first port/multiport may be connected to a first portion of the flexible wall and the first inset may be connected to a third portion of the flexible wall and the second coupling may be connected to a second portion of the flexible wall and the flexible wall may be adapted to form a chamber together with the first port and/or first inset and/or the second coupling. The second coupling may have a closable opening, the chamber may be adapted to allow communication through the coupling into a cavity inside the patient's body, after an incision has been made in the skin of the patient, when a surgical procedure is performed. The first port and first inset may in a first position be adapted to connect to the second coupling in close relation to the patient's skin wherein the chamber may has a first smaller volume, and at least one of; the first port and first inset in a second position may be adapted to be disconnected from the second coupling, being separated from the coupling in a more remote position in relation to the patient's skin wherein the chamber has a second larger volume.

According to one embodiment, the first port forms a closable wall port/multiport when disconnected from the second coupling.

According to one embodiment, the second coupling forms a closable body port/multiport by itself when the first port has been disconnected from the second coupling.

The first port in any of the embodiments herein may comprise a multiport, comprising two, three, four or more individual ports.

The first inset may comprise at least one of: a surgical glove and a device or instrument mount for assisting with in the surgical procedure.

The medical device may comprise a first coupling and the first port and/or multiport and/or first inset may be connected to the second coupling, and the second coupling may be adapted to be connected to the first coupling. The first coupling is adapted to be reversibly directly connected to the second coupling.

One of the first and second coupling may comprise a protruding member and at least one of the first and second coupling may comprise a recess, and the protruding member may be adapted to engage the recess for locking the first coupling to the second coupling. The recess may be a groove in at least one of the first and second coupling.

The interconnectable port in any of the embodiments herein may be adapted to, in a first state, when the first port or first coupling is interconnected with the second coupling, enable a surgeon to operate using endoscopic surgery through the interconnected interconnectable port, and in a second state, when the second coupling is disconnected from the first port or first coupling, enable the surgeon to perform steps of the procedure within the chamber formed by the flexible wall between the first port and second coupling of the interconnectable port. The interconnectable port may be adapted to, in the second state, enable the surgeon to remove a specimen from the body of the patient through the second coupling and into the chamber, and to perform one of: placing the specimen within the chamber, and removing the specimen from the chamber through the first port.

According to one embodiment of the medical device a second port (body port) may also be adapted to be placed in the second coupling. One of the first port, the second body port and the first coupling may be adapted to be reconnected to the second coupling after having been disconnected from the second coupling, such that the surgeon can continue operating through the interconnected port after the specimen has been removed.

The medical device according to any one of the embodiments herein may further comprise a retracting system, comprising a flexible connecting member, an inside retracting member and/or a holding member and/or a mechanical sealing member, adapted to be positioned at the inside of the patient's tissue wall around an incision to be performed in the skin and be connected to the flexible connecting member, and an outside retracting member and/or an external holding member and/or an external mechanical member connected to at least one of; the first flexible wall, the flexible connecting member, and the second coupling and/or a body port and/or a body multiport, placed in close proximity to the incision, or at least one of the outside retracting member, the external holding member and the external mechanical member, being an integrated part of at least one of; the second coupling, the body port and the body multiport. The second coupling is adapted to be positioned in at least one of; close proximity to the incision, in the incision, and at the outside of the patient's skin around the incision being an integrated part of the second coupling adapted to be positioned at the outside of the patient's skin around the incision. The flexible connecting member is interconnecting the inside and outside retracting members, whereby the inside and outside retracting members may be adapted to be larger than the incision allowing the flexible connecting member to retract the skin and tissue in the incision opening substantially parallely to the skin. thus, the flexible connecting member is direct or indirect interconnecting the at least one of; inside retracting member, holding member and mechanical sealing member, and the at least one of; outside retracting members, external holding member, and external mechanical sealing member, whereby at least one of; the inside and outside retracting members, the holding or external holding member, and mechanical sealing member and the external mechanical sealing member, are adapted to be larger than the incision allowing the flexible connecting member to retract the skin and tissue in the incision opening substantially parallely with the skin. The flexible connecting member is adapted to be placed in connection with at least one of; the first flexible wall and the second coupling or is an integrated part of the first flexible wall adapted to be placed through the incision into the body cavity and adapted to together with the inside and outside retracting members retract, substantially parallely with the skin, the skin and tissue in close relation to the incision, to enlarge the incision passage from the chamber into the body cavity, during the surgical procedure.

The medical device according to any one of the embodiments herein may further comprising a sealing device, comprising a flexible connecting member, a first sealing member, adapted to be positioned at the inside of the patient's skin around an incision to be performed in the skin and being sealingly connected to the flexible connecting member, and a second sealing member sealingly connected to at least one of; the first flexible wall, the flexible connecting member, and the second coupling or being an integrated part of the second coupling, adapted to be positioned at the outside of the patient's skin around the incision, wherein the flexible connecting member is sealingly interconnecting the first and second sealing members, whereby the first and second sealing members are adapted to be larger than the incision to seal between at least one of: the second sealing member and the skin or tissue of the patient, and the first sealing member and tissue of the patient, and wherein the flexible connecting member is adapted to be placed sealingly in connection with at least one of; the first flexible wall and the coupling or being an integrated part of the first flexible wall, adapted to be involved in sealing the communicating combined volume of the chamber and the body cavity in the body against the ambient environment, when at least one of; the second coupling, the body port or the body multiport, is open, during the surgical procedure.

According to one embodiment, the first port forms a closable wall port when disconnected from the second coupling. The second coupling may form a closable body port by itself when the first port has been disconnected from the second coupling.

The medical device in any of the embodiments herein may be adapted to have the chamber connected to a pressurized fluid supply, and wherein the medical device is adapted to receive pressure and the pressurized fluid is adapted to hold the pressure, according to at least one of; holding the same pressure as a pressurized fluid filling a body cavity outside the chamber, holding a lower pressure than a pressurized fluid filling a body cavity outside the chamber, and holding a higher pressure than a pressurized fluid filling a body cavity outside the chamber.

The medical device may further comprise at least one of; a nipple for fluid supply placed outside the body, and a nipple for fluid supply placed outside the body in combination with a second nipple adapted to receive flow of fluid leaving the chamber to allow circulation of fluid.

The wall may be adapted to be at least partially applied on the patient's body to form the medical device chamber together with the patient's body, in which a sealed environment can be maintained for encompassing part of the surgical procedure to be performed. The medical device may further comprise at least one of; a closeable wall port/multiport and inset placed in a portion of the wall and being adapted to be detachably connected to a closeable body port placed in an incision in the patient's body connecting to a body cavity, when the medical device is applied on the patient's body for performing the surgical procedure, at least one of; a closeable body port/multiport and an inset adapted to be placed in an incision in the patient's body and further adapted to be detachably connected to the closeable wall port/multiport, when the medical device is applied on the patient's body for performing the surgical procedure, a sealing device connected to the wall and adapted to seal against the incision of the patient, when the medical device is applied on the patient's body. The closable wall port/multiport, the wall and the sealing device is adapted to the closable body port and the closable wall port and the closable body port together, with the mentioned closable body port or multiport are adapted to form an airlock sluice (18) between a cavitythe chamber in the body of the patient and the ambient environment, when the medical device is applied on the patient's body for performing the surgical procedure, and wherein a multiport comprising at least one of; two, three, four, or five or more integrated ports.

At least one of; the closable wall port/multiport, the closable body port/multiport and the closable wall port/multiport and the closable body port/multiport together may be adapted to form an airlock sluice between a cavity in the body of the patient and the ambient environment, when the medical device is applied on the patient's body for performing the surgical procedure.

The closeable wall port and closeable body port may be adapted to be positioned relative to each other such that an elongated object is longitudinally displaceable through the closeable wall port/multiport and closeable and body port/multiport.

In any one of the embodiments herein at least one of; the closeable wall port and the inset may be displaceable between a first position, in which the closeable wall port or inset is situated relatively close to the incision and at least one of the closeable body port/multiport and the inset, and a second position, in which at least one of; the closeable wall port/multiport and the inset is situated more remote from the closeable body port/multiport or the inset.

The closeable wall port or inset may in any of the embodiments comprising the closable body port/multiport or inset, may be adapted to be at least one of; connected to the closeable body port and locked to the closeable body port, when the medical device is applied on the patient's body for performing a surgical procedure.

At least one of the closable body port and the closeable wall port may comprise a penetratable self sealing gel such that an object or hand can be inserted through the closeable wall port while maintaining the sealed environment.

According to one embodiment, the medical device further comprises a coupling comprising at least one of; a coupling part adapted to be placed in the incision and connect to at least one of; the closable body port, the multiport and the inset, and a second coupling part adapted to be placed in the wall and connect to at least one of; the closable wall port, the multiport and the wall inset, wherein the coupling is adapted to at least one of; detachably attach directly or indirectly at least one of; the closable body port, the multiport and the inset to at least one of; the closable wall port, the multiport or the inset, and attach the coupling parts.

The medical device may further comprise at least one of; an additional port and an additional inset and at least one of the closeable wall port or inset and the closeable body port or inset may be exchangeable by the additional port.

The medical device according to any one of the embodiments herein may comprise an airlock sluice which is least one of: placed separate from the wall port, partly using the wall port in the airlock sluice and fully integrated in the wall port for allowing passage of objects between the chamber and the outside of the chamber.

The wall of the medical device according to any one of the embodiments wherein a second separate portion of the wall may be adapted to be at least partially applied on the patient's body to form an external medical device chamber together with the patient's body, placed outside the body, in which a sealed environment can be maintained for encompassing part of the surgical procedure to be performed. The medical device may further comprise a closeable wall port placed in a portion of the wall and being adapted to be connected to a port placed in an incision in the patient's body, when the medical device is applied on the patient's body for performing the surgical procedure, a sealing device connected to the wall and adapted to seal against the incision of the patient. The closable wall port may be adapted to form at least a part of an airlock sluice together with a closable port placed in the skin, the airlock sluice between the medical device chamber placed in the patient's body and the ambient environment, when the medical device is applied on the patient's body for performing the surgical procedure.

According to one embodiment, the closeable wall port may be adapted to be positioned relative to an openable port placed in an incision in the patient's body, such that an elongated object is longitudinally displaceable through the ports when the medical device is applied on the patient's body for performing the surgical procedure.

In one embodiment, the closeable wall port may be displaceable between a first position, in which the closeable wall port is situated relatively close to at least one of, the skin of the patient and a skin port placed in a skin opening, and a second position, in which the closeable wall port is situated more remote from the skin of the patient, when the medical device is applied on the patient's body for performing the surgical procedure.

The closeable wall port in any of the embodiments herein, may when the medical device is applied on the patient's body for performing a surgical procedure, be adapted to be at least one of; connected to a port connecting to a cavity in the patient's body, and locked to a port connecting to a cavity in the patient's body.

The closeable wall port in any of the embodiments herein may comprise a penetratable self sealing gel, such that an object or hand can be inserted through the closeable wall port while maintaining the sealed environment in the chamber.

The medical device according to any one of the embodiments herein may further comprise at least one of; an additional port, an inset, and a coupling wherein the closeable wall port is adapted to be replaced by at least one of; the additional port, the inset and the coupling, when the medical device is applied on the patient's body for performing the surgical procedure.

According to one embodiment, the medical device comprises an internal part of the chamber placed inside the body and an external part of the chamber placed outside the body. The wall of the external portion of the medical device chamber comprises a second separate wall adapted to enclose a sealed environment can to be maintained for encompassing part of the surgical procedure to be performed, wherein the second wall is adapted to form the external chamber direct or indirect reversible connectable to the internal chamber. The medical device further comprises a closable wall port or multiport placed in a portion of the second wall, at least one of; a closable body port/multiport and a coupling placed in a second portion of the second wall, and further adapted to be placed directly in an incision in the body of the patient or indirectly connected to the internal part of the chamber placed inside the body, connected to at least one of; an internal body port, a second coupling, a holding member, and an inside or outside retracting member, being further connected to the internal part of the chamber. The medical device further comprising at least one of; an additional sealing device connected to the wall or second wall and adapted to seal against the skin and/or body tissue in close proximity to or in the incision of the patient, and at least one of; the closable body port or multiport and the coupling, adapted to seal against the skin and/or body tissue of the patient. The wall port/multiport is adapted to connect to at least one of; the internal body port or multiport, the second coupling, the holding member, and the inside or outside retracting member to form the sealed space including the internal and external part of the chamber.

The wall in any of the embodiments herein may be adapted to form the medical device chamber in which a sealed environment can be maintained for encompassing part of the surgical procedure to be performed. The medical device may further comprise a closeable wall port placed in a portion of the wall, a closeable body port adapted to be placed in a portion of the wall and further placed an incision in the body of the patient, and a sealing device connected to the wall and adapted to seal against the skin and/or body of the patient. The wall port and body port together may form an airlock sluice between a cavity in the body of the patient and the ambient environment and at least one of; the closable wall port, closable body port and the closable wall port and closable body port together are adapted to form an airlock sluice between a cavity in the body of the patient and the ambient environment, when the medical device is applied on the patient's body for performing the surgical procedure.

In one embodiment, the closable wall port/multiport is adapted to be at least one of; attached, mounted together in a predefined position and locked to at least one of; the closable body port or multiport, the second coupling, the holding member, and the inside or outside retracting member.

The medical device according to any one of the embodiments herein may further comprise an additional port or additional inset, and at least one of the closeable wall port/multiport and closeable body port/multiport may be exchangeable by the additional port/multiport or inset.

According to one embodiment, the closeable body port comprises a closeable body multi-port adapted to be placed from inside the chamber in an incision to be performed in the skin of the patient, and the closeable body multi-port comprises at least two closeable body ports adapted to enable passage of an object between the chamber and the inside of the patient's body. The closable body multiport may be adapted to be connected to at least one of; a wall port and a wall multiport together placed in an incision in the patient's body connecting to the chamber inside the body, when the medical device is applied on the patient's body for performing a surgical procedure, and the closable wall port comprises a wall multiport, comprising at least two closable wall ports, wherein the wall multiport is adapted to be connected to at least one of; a closable body port and a closable body multiport placed in an incision in the patient's body connecting to the chamber inside the body, when the medical device is applied on the patient's body for performing a surgical procedure.

The closeable wall port and/or closable body port may comprise at least one of an elastic membrane and a flexible membrane, and the membrane may be adapted to, in a non-compressed state, seal between the chamber and the environment outside the chamber, and in a compressed state enable a hand or an object to be inserted through the membrane.

The medical device may further comprise a sealing device having a first sealing member adapted to be positioned at the inside of the patient's skin around an incision to be performed in the skin, and a second sealing member adapted to be positioned at outside of the patient's skin around the incision, and a spring-loaded or elastic connecting member sealingly interconnecting the first and second sealing members. The spring-loaded or elastic connecting member may seal between at least one of: (a) the second sealing member and the skin of the patient, (b) the first sealing member and tissue of the patient, and c) the connecting member and tissue in relation to the incision.

The medical device according to any one of the embodiments herein may comprise at least one of: a filtering unit for filtering fluid supplied to the chamber, a sterilization unit for directly or indirectly sterilizing fluid supplied to the chamber, a fluid tempering unit adapted change the temperature of fluid supplied to the chamber, a filtering unit for filtering fluid and a sterilization unit for directly or indirectly sterilizing fluid supplied to the chamber, a filtering unit for filtering fluid supplied to the chamber and a fluid tempering unit adapted change the temperature of fluid supplied to the chamber, a sterilization unit for directly or indirectly sterilizing fluid supplied to the chamber and a fluid tempering unit adapted change the temperature of fluid supplied to the chamber, and a filtering unit for filtering fluid supplied to the chamber, a sterilization unit for directly or indirectly sterilizing fluid supplied to the chamber and a fluid tempering unit adapted change the temperature of fluid supplied to the chamber.

The medical device according to any one of the embodiments herein may further comprise at least one of: at least one inlet provided in at least one of: the wall of the medical device, and the skin of the patient for supplying a fluid to at least one of the chamber, and the cavity in the patient's body, and at least one outlet provided in at least one of: the wall of the medical device, and the skin of the patient for discharging the fluid from at least one of: the chamber, and the body cavity.

The medical device according to any one of the embodiments herein may further comprise a pump adapted to circulate the fluid from the outlet to the inlet.

At least one of the wall and body port may comprise a protruding member, and at least one of the wall and body port comprises a recess. The protruding member may be adapted to engage the recess for locking the wall port to the body port. The recess may be a groove in at least one of the wall port and body port.

According to one embodiment the wall port is adapted to be fixated to a first coupling and the body port is adapted to be fixated to a second coupling, and the first and second couplings are adapted to be connected for connecting the wall port and body port. At least one of the first and second coupling may comprise a magnet or a first magnetic material, and at least one of the first and second coupling may comprise a magnet or a magnetic material, and the magnet may be adapted to engage a magnet or magnetic material for locking the first coupling to the second coupling.

At least one of the first and second coupling comprises a protruding member, and at least one of the first and second coupling comprises a recess, and the protruding member may be adapted to engage the recess for locking the first coupling to the second coupling.

According to one embodiment, the recess is a groove in at least one of the coupling and the second coupling.

The medical device according to any one of the preceding claims may comprise an internal portion of the chamber placed inside the body and external portion of the chamber placed outside the body, wherein an external portion of the wall is adapted to form the external portion of the medical device chamber, directly or indirectly connected to an internal portion of the wall. The medical device further comprising an interconnectable port adapted to be at least partially placed in an incision in a patient's body. The interconnectable port may comprise a first and second port adapted be interconnected to form the interconnectable port, wherein the second port is disconnectable from the first port, wherein the first port is connected to a first portion of the wall being flexible, and the second port is connected to a second portion of the flexible wall, and wherein the flexible wall is adapted to form the medical device chamber. The first port may form a closable wall port when disconnected from the second port.

The second port may form a closable body port by itself when the first port has been disconnected from the second port.

The chamber may have a first volume when the first and second ports are interconnected and a second volume when the second port is disconnected from the first port, and the second volume may be larger than the first volume.

The first volume may be smaller than 100 000 mm3, and the second volume may be larger than 500 000 mm3. The flexible wall may comprise a pleated portion such that the flexible wall can be expanded.

The medical device according to any one of the embodiments herein may comprises a first and second coupling, and the first port may be connected to the first coupling, and the second port is connected to the second coupling. At least one of the first and second coupling may comprise a protruding member, and at least one of the first and second coupling may comprise a recess, and the protruding member may be adapted to engage the recess for locking the first coupling to the second coupling. The recess may be a groove in at least one of the first and second coupling.

The chamber formed by the flexible wall may in any of the embodiments herein be adapted to hold a pressure exceeding atmospheric pressure.

The medical device according to any one of the embodiments herein may comprise an interconnectable port adapted to in a first state, when the first and second ports are interconnected, enable a surgeon to operate with endoscopic surgery through the interconnected interconnectable port, and in a second state, when the second port is disconnected from the first part enable the surgeon to perform steps of the procedure within the chamber formed by the external portion of the flexible wall between the first and second ports of the interconnectable port.

The medical device according to any one of the embodiments herein may comprise an interconnectable port adapted to, in the second state, enable the surgeon to remove a specimen from the body of the patient through the second port and into the chamber, and to perform one of: placing the specimen within the chamber, and removing the specimen from the chamber through the second port. The chamber may further comprise a pouch for holding the specimen within the chamber. The pouch can be closed for creating a pouch-chamber within the chamber for isolating the removed specimen within the chamber.

The first and second ports of the interconnectable port may be adapted to be reconnected after having been disconnected, such that the surgeon can continue operating through the interconnected port after the specimen has been removed.

The medical device may further comprise a pressure adjustment device connected to the vacuum sealing device, for creating a pressure below atmospheric pressure in at least one vacuum sealing member.

The medical device may further comprise a control unit for controlling at least one of a pressure adjustment device of and the pressure inside the chamber.

The medical device may further comprise a sensor, a motor and a pump, and the control unit may be adapted to control the motor and pump on the basis of input from the at least one sensor.

The medical device may further comprise at least one sensor adapted to sense a physiological or physical parameter of the patient. The physiological or physical parameter may be a parameter selected from: the blood flow of the patient, the saturation of the blood of the patient, an ischemia marker of the patient, the temperature at the skin of the patient, and the patient's skin tone, a pressure, a force, a time, a movement, a stretching, a distance, and a volume. The sensor may in alternative embodiments be adapted to sense a functional or physical parameter of the medical device. The functional or physical parameter may be a parameter selected from: the pressure in the sealing device, the pressure in the chamber, and the direct or indirect leakage of fluid from the chamber, a force, a time, a movement, a stretching, a distance, a volume, an electrical parameter, energy, energy balance, voltage, current, and temperature.

The control unit may be adapted to control the pressure of the vacuum sealing device, such that the vacuum does not substantially affect the blood flow of the patient.

According to one embodiment, the medical device comprises at least one holding member comprising at least one of; a ring shape, a circular bendable shape, a flexible structure, two circular bendable shaped structures, whereof at least one is adapted to hold inside the incision, one, two or more ring shaped structures adapted to have a size larger than the incision in the body, at least one ring shaped structure adapted to be bendable for introducing through the incision, adapted to hold inside the incision, two or more holding member parts adapted to be placed on at least one of; the inside of the incision, outside the incision, and against the tissue related to the incisional cut, wherein two or more parts is adapted to be placed a s separate parts in the selected places described, at least two parts connected to each other, at least two parts connected to each other, the connection being elongated and being substantially longer than the closest distance between the parts, a construction adapted to allow an expansion of the incisional area or circumference of the incision to allow a hand or other parts to pass through the incision.

According to one embodiment, at least one holding member comprising an adaption to at least one of; be sealingly attached to the wall, be sealingly attached to a separate body port or body multiport, the port free from connection to the wall, be sealingly attached to a body port or body multiport, the port connected to the wall, be placed free from the separate body port or body multiport, be placed inside the chamber contacting the wall and non-attached to the wall, be placed outside the chamber towards tissue in close proximity to or in the incision, contacting the wall and non-attached to the wall, and be attached to a comprised adhesive to the skin of the patient.

The body multiport in any of the embodiments may further be adapted to be mounted to the skin by an adhesive.

The body multiport may further be adapted have a second coupling placed in the periphery of the body multiport, adapted to allow the body multiport and the second coupling to be reversible connectable, the body multiport further adapted to be placed outside the skin, the second coupling adapted to have an inner peripheral size larger than the incision in the skin to allow passage of a hand placed in a glove inside the coupling, when the body multiport has been disconnected, and wherein the skin being elastic enough to allow larger incisional size for the hand.

According to one embodiment, the medical device comprises at least one holding member adapted to at least one of, hold the medical device and hold the medical device and simultaneously seal the incision towards the inside of the body, wherein the first internal holding member circumferentially is adapted to have a size larger than the incision, and further adapted to be bendable and bent and introduced inside the incision wall, when performing the surgical procedure. The holding member is further adapted to hold the medical device from inside the incision, and adapted to; have a inner peripheral size allowing passage of said hand, and regaining the circumferential shape after the introduction through the smaller incision, the medical device further comprises a second external holding member adapted to be placed outside the incision, wherein said first internal and second external holding members are connected by a flexible connecting member, and wherein said flexible connecting member is at least one of; an integrated part of the medical device wall, a separate flexible connecting wall adapted to be placed outside the chamber, and a separate flexible connecting wall adapted to be placed inside the chamber.

According to one embodiment, at least one of; the second coupling is an integrated part of the second external holding member, and the flexible connecting member is an integrated part of the medical device wall.

The medical device in any of the embodiments herein may further comprise a body multiport being adapted to seal the chamber towards the incision in the skin of the patient.

The medical device may further comprise an internal portion of the chamber placed inside the body and an external portion of the chamber placed outside the body. The internal and external portion of the chamber may be divided by a closable body port or body multiport comprising two, three, four or more ports. Both the internal and external chamber may be adapted to have at least one of; at least one fluid inlet, and at least one fluid outlet to increase the pressure above atmospheric pressure inside both the internal and external chamber.

The sealing member or internal sealing member may be deformable during introduction through the incision and to regain its shape after said introduction. The wall enclosing the chamber may also be adapted to keep its shape by means of a loop shaped sealing member reinforcing the wall, and the medical device may comprise a plurality of ring or loop shaped reinforcements placed in the wall perpendicular to the axis from the incision to the sealing member placed on the organ or related tissue.

The chamber may be adapted to substantially keep its shape, when the pressure outside the chamber is higher than inside the chamber. The chamber may be adapted to have a pressure above atmospheric pressure and the body cavity outside the chamber may be adapted to have a different pressure.

The medical device may comprise an internal portion of the chamber placed inside the body and an external portion of the chamber placed outside the body, the internal and external portion of the chamber is divided by a closable body port or body multiport comprising two, three, four or more ports, wherein both the internal and external chamber is adapted to have a pressure above atmospheric pressure, wherein the internal and external chamber is adapted to have different pressures.

A method, using a medical device according to any one of the embodiments herein is provided. The method comprises the steps of; performing an incision in the skin of the patient, dissecting a human organ or tissue related to the organ, placing a medical device comprising a wall adapted to create a medical device chamber sealingly connected to an internal sealing device, placing the at least one internal sealing device on the human organ or tissue related to the organ, or more sealing devices on several different organs or related tissue, sealing by the first sealing device comprising a first sealing member against at least a part of at least one human organ or human tissue related to or in contact with said human organ, for creating a seal between the medical device and at least the part of; the at least one human organ or the human tissue, sealing the medical device chamber, by at least one of a closable wall port placed in the wall of the chamber, a closable body port placed in close proximity to the incision and a second sealing member placed close to the incision on tissue relating to the cut incision and/or on the in and/or outside thereof, filling the chamber with a pressurized fluid, placing the medical device chamber in connection with at least one of; the at least one human organ, and at least one during the surgical procedure surgically opened organ, creating a sealed space by the wall or the wall and one or more ports enclosing the medical device chamber and at least one of; at least a part of the at least one human organ, and a cavity in the at least one surgically opened organ, and performing a surgical procedure using instruments.

The method may further comprises at least one of the following steps; placing the closable body port being multiport, in the incision or in close relation to the incision, comprising allowing transfer from the outside of the patient's body to the inside of the patient's body, through the incision made in the skin of the patient, and performing the surgical procedure through said multiport comprising two, three, four or more ports placed together.

The method may further comprises at least one of the following steps; a. placing the medical device comprising the step of placing an external portion of the chamber outside the body, b. changing in a predefined manner the shape of the medical device during the surgical procedure, c. the step of placing the body port or body multiport and the step of placing a chamber outside the body, comprising wall port or wall multiport placed in the wall in outside part of the chamber, the body port and wall port being reversible connectable, interconnecting the body port or multiport with the wall port or wall multiport according to point c, thereby changing the shape according to point b, whereby the volume of the chamber is smaller when the ports are interconnected and larger when the ports are separated, e. operating in an endoscopic surgical procedure, when the interconnected ports are connected according to point d, through the interconnected body and wall port or multiport, using any instrument and when the ports are separated, transferring at least one of; an object, an apparatus or implant, a glove, a hand placed in a glove, a body part and a surgical instrument from the outside environment into the external portion of the chamber and further into the internal portion of the chamber, f. operating with an implant according to point e, g. sealing, by means of the second external sealing member, the incision in the skin of the patient by sealing on the inside or outside or both inside and outside of the skin incision, creating a sealed tunnel between one of; the tissue related to the incision and the medical device, and at least a part of at least one human organ or human tissue and the medical device, preventing operational matter reaching outside the sealed space, and reducing infection rate by creating a sealed tunnel.

The method may further comprises at least one of the following surgical procedures performed within the sealed space; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hernia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open j oint surgery, combined open j oint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

The method may further comprises placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant, and/or placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

Please note that all of the embodiments described above can be combined in any way in general terms.

A medical device adapted to be positioned inside a patient's body via an incision made in the skin of the patient is provided. The medical device comprises a first internal sealing device, comprising a first internal sealing member adapted to be positioned in connection to and seal against at least a part of at least one human organ or human tissue for creating a seal between the medical device and at least the part of the at least one human organ or human tissue. The medical device may further comprise a second internal sealing device, comprising a second internal sealing member adapted to be positioned in connection to and seal against at least a part of at least one human organ or human tissue, for creating a seal between the medical device and at least the part of the at least one human organ or human tissue, and a wall adapted to create a medical device chamber sealingly connected to the first and second internal sealing device, such that a portion of at least one human organ is placed inside of the medical device chamber between the first and second internal sealing members.

According to one embodiment, the medical device may further comprise a body port placed in an incision in the skin of the patient, and wherein the wall is connected to the port, such that the medical device chamber is created between the first internal sealing member, the second internal sealing member and the body port.

At least one of the first and second sealing members may be adapted to encircle a tubular organ.

According to one embodiment, the wall of the medical device may comprise a closing portion adapted to be opened and closed for enclosing a human organ or human tissue in the medical device chamber. The closing portion may comprise a zipper. The closing portion may be placed between the first and second internal sealing device.

According to one embodiment, the first internal sealing device is adapted to encircle and seal against an intestine of the patient and the second internal sealing device is adapted to encircle and seal against the esophagus of the patient, and the wall may be adapted to encapsulate the stomach of the patient, such that the stomach of the patient is placed in the medical device chamber between the first sealing member, the second sealing member, and the wall.

According to one embodiment the first sealing member of the medical device has a smaller circumference than the second sealing member.

The internal sealing device may comprise at least one of; an internal vacuum sealing device, sealing by using a vacuum towards the human organ or human tissue related to and in contact with said human organ, an internal pressure sealing device, sealing using a pressure towards the human organ or human tissue related to and in contact with said human organ, and an internal adhesive sealing device, sealing by adhesively contacting the human organ or human tissue related to and in contact with said human organ.

The internal sealing device of any of the embodiments herein may be adapted to be positioned, at least one of; on the human organ, on human tissue related to and in contact with said human organ, in an incision made in the at least one human organ of the patient, on the inside of the at least one human organ or human tissue related to and in contact with the inside of said human organ, and on the outside of the at least one human organ or human tissue related to and in contact with the outside of said human organ.

The medical device according to any one of the preceding embodiments may be adapted to communicate with at least one of; the cavity inside the organ in the patient's body, a portion of the medical chamber placed outside the body, and a portion of the medical chamber placed outside the body, wherein a port placed in the incision of the body and connected to the wall, is adapted to be opened or closed to allow communication to the portion of the chamber placed outside the body when said port being open.

The medical device according to any one of the preceding embodiments may be adapted to seal fluid or other bodily matter from the inside of the at least one human organ, when an incision is made in the human organ, to avoid the spreading of such fluid or other bodily matter outside the sealed space.

The medical device according to any one of the preceding embodiments may be adapted to seal the sealed space during a surgical procedure involving at least one of the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ, and replacing at least one bodily function by placing at least one medical device in relation to the at least one organ.

According to one embodiment of the medical device, the medical device is adapted to receive within the medical device chamber, at least one of; at least one trocar, at least one inset, at least one port, at least one camera, at least one tube or channel for injecting fluid into the chamber, at least one suction instrument, at least one diathermy instrument, and at least one instrument or tool, to be able to perform the surgical procedure in the sealed space.

According to one embodiment, the internal vacuum sealing member comprises a vacuum groove adapted to create a vacuum chamber together with the human organ or human tissue.

According to one embodiment, the internal vacuum sealing device comprises a second or more vacuum sealing member comprising at least a second vacuum groove creating a second vacuum chamber together with another part of the human body.

The internal vacuum sealing members be a loop shaped vacuum sealing member adapted to at least one of; encircle the incision made in the at least one organ of the patient, and encircling the human organ, wherein at least one of the vacuum sealing members is adapted to be placed to seal by means of vacuum towards the at least one human organ, to create the sealed space for performing a surgical procedure when an incision has been made in the skin of the patient.

According to one embodiment, further comprises at least one holding member adapted to hold the medical device positioned in relation to at least one of; the incision, and the at least one organ of the patient, at least one the holding member further be adapted to at least one of; be holding the medical device from inside the patient's body, be holding the medical device from both the inside of the patient's body and the skin on the outside of the body, be holding by clamping on both sides of at least one of; the abdominal wall, the thoraxial wall and the wall of a created body cavity, and be integrated with the vacuum sealing device, be holding the medical device connected to a body port placed in relation the incision in the body, and be holding in different separate positions.

According to one embodiment, the at least one internal vacuum sealing member is a circular vacuum sealing member.

According to one embodiment, the medical device further comprises an additional vacuum sealing member adapted to seal the incision in the body, adapted to seal against at least one of; the inside of the incision, and tissue connected to the cut part of the incision.

The medical device may further comprising an additional vacuum sealing member adapted to seal the incision in the skin of the patient, on the outside thereof.

According to one embodiment, the medical device further comprises least one of; at least one holding member, and at least one a pressure sealing member, adapted to be placed on the inside, the outside or both inside and outside of the incision in the body, the medical device further comprising an additional vacuum sealing member, adapted to seal the incision in the patient, adapted to be placed on, the inside, the outside or both inside and outside of the incision in the body.

According to one embodiment, the medical device further comprises the additional vacuum sealing member is a loop shaped vacuum sealing member adapted to encircle the incision made in the skin of the patient, and wherein the additional vacuum sealing member is further adapted to connect to at least one of; the skin of the patient on the outside thereof, and human tissue of the patient inside the body, sealing towards at least one of; a muscle fascia, the outside of a muscle fascia, the inside of a muscle fascia, the muscle fascia of the rectus abdominis in the abdominal wall, the peritoneum, fat tissue, fibrotic tissue, muscle tissue, and a body organ, all positioned inside the body, wherein the at least one additional vacuum sealing member is adapted to be placed to seal with vacuum towards the human tissue in close relation to the incision, when an incision has been made in the skin of the patient.

According to one embodiment, the medical device further comprises at least one of; a closable body port adapted to enable transfer from the outside of the patient's body to the inside of the patient's body through the incision made in the skin of the patient, and a closable wall port placed in another portion of the wall, adapted to form a portion of the chamber placed outside the incision, and wherein the closable wall port is adapted to enable transfer from the outside of the medical device to the inside of the medical device chamber through the wall of the medical device in which the wall port is mounted.

According to one embodiment, the medical device further comprises a multiport, and wherein the medical device comprises a portion of the chamber formed outside the incision adapted to change from a first to a second shape during the surgical procedure, and wherein: the medical device in the first shape allows the multiport to be positioned in close relation to the incision in the skin of the patient, to allow key hole surgery through the multiport placed in close relation to the incision in the skin, the medical device in the second shape allows the multiport to be positioned further from the incision in the skin of the patient, such that the portion of the chamber placed outside the skin is adapted to be formed with enough space to receive at least one of; a hand placed inside an inset comprising a glove, an apparatus, a non-body part, a body part, an instrument and a part used for the surgical procedure.

According to one embodiment, the medical device is adapted to create a sealed tunnel between; the tissue in proximity to the incision in the skin of the patient and the human organ or human tissue related to and in contact with said human organ sealed towards the medical device by the internal sealing device, thereby adapted to contain operational matter inside the sealed space.

According to one embodiment, the medical device is adapted to create a sealed tunnel between; a body port connected to the wall of the medical device, the body port adapted to be placed in close proximity to the incision in the skin of the patient, and the human organ or human tissue related to and in contact with said human organ sealed towards the medical device by the internal sealing device, thereby allowing the chamber to contain operational matter inside the sealed space.

According to one embodiment, the multiport comprises a two part port adapted to be disconnectably connected allowing the medical device to take the first and second shape, wherein the two parts is connected to the wall to create a portion of the sealed chamber placed outside the body.

According to one embodiment, the medical device further comprises at least one of; at least two closable body ports, and at least two closable multiports comprising two, three or more ports, adapted to enable transfer from the outside of the patient's body to the inside of the patient's body, through the incision made in the skin of the patient.

According to one embodiment, the medical device wall is adapted to enclose a chamber in which a portion of the surgical procedure can be performed, and wherein the chamber is adapted to be in fluid connection with a cavity having both a first external portion adapted to be placed on the outside of the body and a second internal portion being adapted to be placed inside the body of the patient, wherein the medical device is adapted to have the external and internal portions of the chamber adapted to be at least one of; forming together one chamber, and allowing a closable body port adapted to be placed in close proximity to the incision of the body between the external and internal portion of the chamber, to have the chamber to be divided in two separate closed parts of the chamber.

According to one embodiment, the medical device comprises a tube or channel adapted to be in fluid connection with and reaching from the outside to the inside of the body for allowing injection of an disinfection agent or injecting fluid to clean or disinfect remaining body parts inside the sealed space, when the surgical procedure is performed.

According to one embodiment, the medical device is adapted to at least one of; loosen the at least one internal sealing device from the human organ or human tissue related to and in contact with said human organ, and using the at least one internal sealing device to keep the sealed space closed after the internal sealing device has been loosened.

According to one embodiment, the medical device further comprises a closing sealing device, adapted to keep the sealed space closed when finishing the surgical procedure to avoid leakage of bodily matter from the sealed space.

According to one embodiment, the medical device chamber has a volume larger than 500 000 mm3.

According to one embodiment, the wall comprises a closable wall port adapted to enable transfer from the ambient environment to the inside of the chamber, the wall may be flexible and/or elastic.

According to one embodiment, the medical device further comprises at least one integrated glove or inset comprising an integrated glove enabling manual manipulation within the chamber of the medical device and/or inside the cavity of the patient.

According to one embodiment, the medical device further comprises a coupling adapted to connect an inset or port to the medical device. The coupling may be placed in at least one of: the wall of the medical device, and the incision made in the patient's skin.

According to one embodiment, the medical device further comprises an inset selected from: a glove inset, a hand access inset, a port inset, a multiport inset, and a gel port inset.

According to one embodiment, the medical device further comprises at least one of; a closable wall port or wall multiport, placed in the wall, and a closable body port or body multiport adapted to be placed in or in close relation to the incision of the patient, wherein the closable wall port or wall multiport and the closable body port or body multiport comprises connections adapted to be connecting the closable wall port or wall multiport to the closable body port or body multiport, and wherein the multiport comprise two, three, four or more or more ports placed in one multiport casing.

According to one embodiment, the closable body port comprises a first penetrable self sealing gel and the closable wall port comprises a second penetrable self sealing gel, and wherein the first and second penetrable self sealing gels are adapted to be placed tightly together by the wall and body ports being connected, such that they act as a single penetrable self sealing gel adapted to, in a non-compressed state, provide sealing, and in a compressed state, enable a hand or an object to be inserted through the self sealing gel while maintaining the seal.

According to one embodiment, the wall forming the chamber is adapted to hold a pressure within the chamber exceeding atmospheric pressure.

According to one embodiment, the first and second internal sealing device comprises loop shaped vacuum sealing members adapted to at least one of; encircle the incision made, encircle two or more positions on or around the at least one organ of the patient, and encircling two or more human organs to enclose a larger surgical area in the sealed space,

According to one embodiment, at least one vacuum sealing member is adapted to be placed to seal by means of vacuum towards the at least one human organ or related tissue, when an incision has been made in the organ of the patient.

According to one embodiment, the closing sealing device is adapted to seal with vacuum to keep the sealed space closed when finishing the surgical procedure to avoid leakage of bodily matter from the sealed space.

According to one embodiment, the medical device further comprises at least one fluid conduit leading from outside the body to inside the body, inside the chamber, adapted to allow sucking and injecting fluid into the chamber from outside the body, wherein the at least one fluid conduit is adapted to be at least one of; integrated in at least one of the medical device and the wall or body port placed in close proximity to the incision, and placed as a separate tube having the two ends of the fluid conduit displaced as a separate tube and there in between adapted to be integrated in at least one of the medical device and the wall or body port placed in close proximity to the incision.

According to one embodiment, the medical device chamber is adapted to encompass part of the surgical procedure to be performed, wherein the medical device further comprises; a closeable wall multiport placed in a portion of the wall, a closeable body multiport placed in a second portion of the wall and adapted to be placed in or closely related to an incision in the patient's body, and a sealing device is adapted to be at least one of; sealingly connected to at least one of; the wall and the closable body port, and an integrated part of at least one of; the wall and the closable body port, wherein the sealing device further being adapted to seal against at least one of the skin and tissue of the patient, wherein the wall, wall multiport and body multiport are adapted to together form an airlock sluice between a cavity in the body of the patient and the ambient environment, when the surgical procedure is performed.

According to one embodiment, the wall port of the medical device according to any one of the embodiments, may be displaceable between a first position and a second position, wherein the second position is situated more remote from the body port.

According to one embodiment, the wall multiport of the medical device may be adapted to be at least one of: connected to the body multiport, abutting the body multiport, locked to the body multiport, formed into an integrated port together with the body multiport.

According to one embodiment, at least one of; the closable wall multiport and closable body multiport comprises a penetratable self sealing gel, allowing an object or hand to be inserted through the self sealing gel.

According to one embodiment, the medical device further comprises at least one of; an additional body port, an additional body multiport, an additional body inset, an additional wall port, an additional wall multiport and an additional wall inset, wherein at least one of a wall port, a wall multiport, a body port, a body multiport, a wall inset and a body inset is exchangeable by at least one of; the additional body port, the additional body multiport, the additional body inset, the additional wall port, the additional wall multiport and the additional wall inset.

According to one embodiment, the medical device further comprises a sealing member adapted to seal the wall towards human tissue, comprising at least one of; a vacuum sealing member adapted to hold a pressure less than atmospheric pressure, and a pressure sealing member adapted to hold a pressure above atmospheric pressure.

According to one embodiment, the medical device further comprises a closable body port and closable multiport according, and at least one of; the closable body port comprises a closable body multi-port adapted to be placed from inside the chamber in an incision to be performed in the skin of the patient, and wherein the body multiport is adapted to be connected to at least one of; the wall port, and a wall multiport, when the medical device is applied on the patient's body for performing a surgical procedure, and the closable wall port comprises a closable wall multiport, wherein the wall multiport is adapted to be connected to at least one of; the body port, and a body multiport placed in an incision in the patient's body, when the medical device is applied on the patient's body for performing a surgical procedure.

According to one embodiment, the wall is a flexible wall and the medical device comprises a first inset, or an interconnectable port placed in the wall, the interconnectable port comprising; a first port or multiport, a second coupling adapted be interconnected with at least one of; the first port to form the interconnectable port, and the first inset, wherein the second coupling is disconnectable from the first port or first inset, and wherein at least one of: the first port or multiport is connected to a first portion of the flexible wall, and the first inset is connected to a third portion of the flexible wall, wherein the second coupling is connected to a second portion of the flexible wall, the flexible wall is adapted to form a chamber together with the second coupling and at least one of; the first port, and first inset, the second coupling adapted to allow a closable opening, the chamber is adapted to allow communication through the coupling into a cavity inside the patient's body, after an incision has been made in the skin of the patient, when a surgical procedure is performed, at least one of; the first port or multiport and first inset, in a first position, is adapted to connect to the second coupling in close relation to the patients skin, wherein the chamber having a first smaller volume, and at least one of; the first port or multiport and first inset in a second position is adapted to be disconnected from the second coupling, being separated from the coupling in a more remote position in relation to the patients skin, wherein the chamber having a second larger volume.

According to one embodiment, the first port forms a closable wall port or closable multiport when disconnected from the second coupling.

According to one embodiment, the second coupling forms a closable body port by itself when the first port or multiport has been disconnected from the second coupling. The multiport may comprise two, three, four or more individual ports.

According to one embodiment, the first inset comprises at least one of: a surgical glove and a device or instrument mount, for assisting in the surgical procedure.

According to one embodiment, the medical device further comprises at least one first coupling, and wherein at least one of; the first port or multiport and first inset is connected to the first coupling, and at least one of; the first port or multiport and first inset is indirectly connected to the second coupling, being adapted to be directly connected to the first coupling, wherein the first coupling is adapted to be reversibly directly connected to the second coupling.

According to one embodiment, at least one of the first and second coupling comprises a protruding member, and at least one of the first and second coupling comprises a recess, and wherein, the protruding member is adapted to engage the recess for locking the first coupling to the second coupling.

According to one embodiment, the interconnectable port is adapted to: in a first state, when the first port or the first coupling is adapted to be interconnected with the second coupling, enable a surgeon to operate using endoscopic surgery through the interconnected interconnectable port, and in a second state, when the second coupling is disconnected from the first port or first coupling enable the surgeon to perform steps of the procedure within the chamber formed by the flexible wall between the first port and second coupling of the interconnectable port.

According to one embodiment, the interconnectable port is adapted to, in the second state, enable the surgeon to remove a specimen from the body of the patient through the second coupling and into the chamber, and to perform one of: placing the specimen within the chamber, and removing the specimen from the chamber through the first port.

According to one embodiment, the medical device further comprises a second body port adapted to also be placed in the second coupling, wherein at least one of the first port, the second body port and the first coupling are adapted to be reconnected to the second coupling after having been disconnected from the second coupling, such that the surgeon can continue operating through the interconnected port after the specimen has been removed.

According to one embodiment, the medical device is adapted to have the chamber connected to a pressurized fluid supply such that the medical device can receive pressurized fluid and at least one of; hold the same pressure as a pressurized fluid filling a body cavity outside the chamber, hold a lower pressure than a pressurized fluid filling a body cavity outside the chamber, and hold a higher pressure than a pressurized fluid filling a body cavity outside the chamber.

According to one embodiment, the medical device further comprises at least one of; a nipple for fluid supply placed outside the body and a nipple for fluid supply placed outside the body in combination with a second nipple adapted to receive flow of fluid leaving the chamber to allow circulation of fluid.

According to one embodiment, the medical device further comprises at least one of a filtering unit for filtering fluid supplied to the chamber, a sterilization unit for directly or indirectly sterilizing fluid supplied to the chamber, a fluid tempering unit adapted change the temperature of fluid supplied to the chamber, a filtering unit for filtering fluid and a sterilization unit for directly or indirectly sterilizing fluid supplied to the chamber, a filtering unit for filtering fluid supplied to the chamber and a fluid tempering unit adapted change the temperature of fluid supplied to the chamber, a sterilization unit for directly or indirectly sterilizing fluid supplied to the chamber and a fluid tempering unit adapted change the temperature of fluid supplied to the chamber, and a filtering unit for filtering fluid supplied to the chamber, a sterilization unit for directly or indirectly sterilizing fluid supplied to the chamber and a fluid tempering unit adapted change the temperature of fluid supplied to the chamber.

According to one embodiment, the medical device is applied on the patient's body for performing a surgical procedure, the medical device further comprises at least one of: at least one inlet provided in at least one of: the wall of the medical device, and the skin of the patient for supplying a fluid to at least one of the chamber, and the cavity in the patient's body, and at least one outlet provided in at least one of: the wall of the medical device, and the skin of the patient for discharging the fluid from at least one of: the chamber, and the body cavity.

According to one embodiment, the medical device further comprises a pump adapted to circulate the fluid from the outlet to the inlet.

According to one embodiment, the medical device further comprises a wall comprises a pleated portion such that the flexible wall can be expanded.

According to one embodiment, the medical device further comprises a sensor. The sensor could have been a sensor adapted to sense at least one of: the blood flow of the patient, the saturation of the blood of the patient, an ischemia marker of the patient, the temperature at the skin of the patient, and the patient's skin tone, a pressure, a force, a time, a movement, a stretching, a distance, and a volume.

According to one embodiment, the sensor may be a sensor adapted to sense at least one of: the pressure in the sealing device, the pressure in the chamber, and the direct or indirect leakage of fluid from the chamber, a force, a time, a movement, a stretching, a distance, a volume, an electrical parameter, energy, energy balance, voltage, current, and temperature.

A method of creating a sealed chamber at least partially inside of the body of a patient is provided. The comprising making an incision in the skin of the body of the patient, inserting, through the incision, a first internal sealing device comprising a first internal sealing member, the first internal sealing device being connected to a tubular wall, positioning the first internal sealing member in connection with at least a part of at least one human organ or human tissue, such that a first seal is created between the medical device and at least the part of the at least one human organ or human tissue, inserting, through the incision, a second internal sealing device comprising a second internal sealing member, the second internal sealing device being connected to a tubular wall, positioning the second internal sealing member in connection with at least a part of at least one human organ or human tissue, such that a second seal is created between the medical device and at least the part of the at least one human organ or human tissue, such that a medical device chamber is created sealingly between the first and second internal sealing devices and the tubular wall.

According to one embodiment, the method further comprises at least one of the steps of: inserting at least one trocar through the incision in the skin of the patient, inserting at least one dissecting tool into the trocar, and dissecting an area of the organ or tissue.

According to one embodiment, the method further comprises the step of placing a body port in the incision in the skin of the patient, and wherein the tubular wall is connected to the body port, such that the sealed chamber is created between the tubular wall, the first internal sealing device, the second internal sealing device and the body port.

According to one embodiment, the method further comprises the step of positioning the first and second internal sealing devices comprises positioning the first and second sealing device such that they each encircle a tubular organ.

According to one embodiment, the method further comprises the step of positioning the first and second internal sealing devices comprises the steps of positioning the first sealing device encircling an intestine of the patient and positioning the second internal sealing device encircling the esophagus of the patient, such that the wall encapsulates the stomach of the patient, such that the stomach of the patient is placed in a sealed chamber between the first sealing member, the second sealing member, and the wall.

According to one embodiment, the wall comprises a closing portion adapted to be opened and closed for enclosing a human organ or human tissue in the medical device chamber, and wherein the method comprises the steps of: opening the wall the encircle an organ of the patient, and closing the wall to encapsulate the organ.

According to one embodiment, the internal sealing member is an internal vacuum sealing member, and wherein the method further comprises the step of applying a vacuum to the vacuum sealing member such that the vacuum sealing member seals by the applied vacuum.

According to one embodiment, the internal sealing member is an internal pressure sealing member, and wherein the method further comprises the step of applying a pressure to the pressure sealing member such that the pressure sealing member seals by means of the applied pressure.

According to one embodiment, the closing portion comprises a zipper, and wherein the step of closing the wall to encapsulate the organ comprises closing the zipper.

According to one embodiment, the method further comprises the step of filling the chamber with a pressurized fluid.

According to one embodiment, the method further comprises at least one of the following surgical procedures performed within the sealed chamber; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hernia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open joint surgery, combined open joint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

According to one embodiment, the method further comprises placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant.

According to one embodiment, the method further comprises placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

A medical device adapted to be positioned inside a patient's body via an incision made in the skin of the patient, the medical device comprises an internal sealing device, comprising an internal sealing member adapted to be positioned in connection to and seal against at least a part of at least one human organ or human tissue related to and in contact with said human organ, for creating a seal between the medical device and at least the part of the at least one human organ or human tissue related to and in contact with said human organ, and a tubular wall adapted to create a medical device chamber sealingly connected to the internal sealing device, such that a portion of at least one human organ or tissue is placed inside of the medical device chamber, wherein the internal sealing member has an adjustable circumference, for enclosing or encircling a portion of the at least one human organ or human tissue.

According to one embodiment, the medical device further comprises a body port placed in an incision in the skin of the patient, and wherein the wall is connected to the body port, such that a portion of at least one human organ is placed inside of the medical device chamber created between the first internal sealing member, the second internal sealing member and the body port.

According to one embodiment, the medical device further comprises a second internal sealing device comprising a second internal sealing member adapted to be positioned in connection to and seal against at least a part of at least one human organ or human tissue, for creating a seal between the medical device and at least the part of the at least one human organ or human tissue, wherein the wall is further connected to the second internal sealing member, such that a portion of at least one human organ is placed inside of the medical device chamber between the first and second internal sealing members.

According to one embodiment, the second sealing member has an adjustable circumference for enclosing or encircling a portion of the at least one human organ or tissue.

According to one embodiment, at least one of the first and second internal sealing members is circular and has an adjustable diameter, such that the circumference is adjusted by adjustment of the diameter.

According to one embodiment, the internal sealing device comprises at least one of; an internal vacuum sealing device, sealing by using a vacuum towards the human organ or human tissue related to and in contact with said human organ, an internal pressure sealing device, sealing using a pressure towards the human organ or human tissue related to and in contact with said human organ, and an internal adhesive sealing device, sealing by adhesively contacting the human organ or human tissue related to and in contact with said human organ.

According to one embodiment, the internal sealing member comprises at least one sealing surface adapted to connect to and seal against a tissue portion of the patient, wherein the sealing surface is placed in the front of the internal sealing device, such that the internal sealing device can seal axially against a surface of a tissue.

According to one embodiment, the internal sealing member comprises at least one sealing surface adapted to connect to and seal against a tissue portion of the patient, wherein the sealing surface is placed on the inner circumference of the internal sealing device, such that the internal sealing device can seal radially and thus against the outer circumference of a tubular organ or tissue.

According to one embodiment, at least one of the internal sealing members comprises a pleated portion, such that the circumference is adjustable by the adjustment of the pleated portion.

According to one embodiment, the internal sealing device is an internal vacuum sealing device comprising a pleated vacuum conduit comprising perforations adapted to generate suction against the tissue of the patient, such that a sealing is created between the pleated vacuum conduit and the organ or tissue of the patient.

According to one embodiment, the medical device further comprising a vacuum conduit adapted to connect the pleated vacuum conduit to a vacuum source outside the body of the patient. The perforations may be placed in the front end of the internal sealing device, such that the internal sealing device can seal against a planar surface of a tissue. According to one embodiment, the perforations are placed on the inner circumference of the pleated vacuum conduit, such that the internal sealing device can seal radially and thus against the outer circumference of a tubular organ or tissue.

According to one embodiment, the internal sealing device is adapted to be positioned, at least one of; on the human organ, on human tissue related to and in contact with said human organ, in an incision made in the at least one human organ of the patient, on the inside of the at least one human organ or human tissue related to and in contact with the inside of said human organ, and on the outside of the at least one human organ or human tissue related to and in contact with the outside of said human organ.

According to one embodiment, the medical device chamber is adapted to communicate with at least one of; the cavity inside the organ in the patient's body, a portion of the medical chamber placed outside the body, and a portion of the medical chamber placed outside the body, wherein a port placed in the incision of the body and connected to the wall, is adapted to be opened or closed to allow communication to the portion of the chamber placed outside the body when said port being open.

According to one embodiment, the internal sealing device is adapted to be positioned inside the body to seal the total chamber formed by the alternatives above, towards the outside thereof.

According to one embodiment, the medical device is adapted to seal fluid or other bodily matter from the inside of the at least one human organ, when an incision is made in the human organ, to avoid the spreading of such fluid or other bodily matter outside the sealed space.

According to one embodiment, the internal vacuum sealing member comprises a vacuum groove adapted to create a vacuum chamber together with the human organ or human tissue.

According to one embodiment, the internal vacuum sealing device comprises a second or more vacuum sealing members comprising at least a second vacuum groove creating a second vacuum chamber together with another part of the human body.

According to one embodiment, the medical device further comprising an additional vacuum sealing member adapted to seal against at least one of; the inside of the incision, and tissue connected to the cut part of the incision.

According to one embodiment, the medical device further comprises an additional vacuum sealing member adapted to seal the incision in the skin of the patient, on the outside thereof.

According to one embodiment, the additional vacuum sealing member is a loop shaped vacuum sealing member adapted to encircle the incision made in the skin of the patient, and wherein the additional vacuum sealing member is further adapted to connect to at least one of; the skin of the patient on the outside thereof, and human tissue of the patient inside the body, sealing towards at least one of; a muscle fascia, the outside of a muscle fascia, the inside of a muscle fascia, the muscle fascia of the rectus abdominis in the abdominal wall, the peritoneum, fat tissue, fibrotic tissue, muscle tissue, and a body organ, all positioned inside the body, wherein the at least one additional vacuum sealing member is adapted to be placed to seal with vacuum towards the human tissue in close relation to the incision, when an incision has been made in the skin of the patient.

According to one embodiment, the medical device comprises at least one of; a closable body port adapted to enable transfer from the outside of the patient's body to the inside of the patient's body through the incision made in the skin of the patient, and a closable wall port placed in another portion of the wall, adapted to form a portion of the chamber placed outside the incision, and wherein the closable wall port is adapted to enable transfer from the outside of the medical device to the inside of the medical device chamber through the wall of the medical device in which the wall port is mounted.

According to one embodiment, the closeable wall or body port comprises a two part port adapted to be disconnectably connected allowing the medical device to take the first and second shape, wherein the two parts are connected to the wall for creating a portion of the sealed chamber outside of the body.

According to one embodiment, the wall is adapted to create a medical device chamber having a volume larger than 500 000 mm3.

According to one embodiment, the wall is flexible and/or elastic.

According to one embodiment, the wall comprises at least one integrated glove or inset comprising an integrated glove enabling manual manipulation within the chamber of the medical device and/or inside the cavity of the patient.

According to one embodiment, the medical device further comprises a coupling adapted to connect an inset or port to the medical device.

According to one embodiment, the coupling is adapted to be placed in at least one of: the wall of the medical device, and the incision made in the patient's skin.

According to one embodiment, the medical device further comprising an inset selected from: a glove inset, a hand access inset, a port inset, a multiport inset, and a gel port inset.

According to one embodiment, the medical device further comprises a closable body port comprising a first penetrable self sealing gel and the closable wall port comprising a second penetrable self sealing gel, and wherein the first and second penetrable self sealing gels are adapted to be placed tightly together by the wall and body ports being connected, such that they act as a single penetrable self sealing gel adapted to, in a non-compressed state, provide sealing, and in a compressed state enable a hand or an object to be inserted through the self sealing gel while maintaining the seal.

According to one embodiment, the wall forming the chamber is adapted to hold a pressure within the chamber exceeding atmospheric pressure.

According to one embodiment, the wall port is displaceable between a first position and a second position, wherein the second position is situated more remote from the body port.

According to one embodiment, at least one of; the closable wall port and closable body port comprises at least one of an elastic membrane and a flexible membrane, and wherein the membrane is adapted to, in a non-compressed state, seal between the chamber and the environment outside the chamber, and in a compressed state enable a hand or an object to be inserted through the membrane.

According to one embodiment, the body port comprises a first sealing member adapted to be positioned at the inside of the patient's skin around an incision to be performed in the skin and a second sealing member adapted to be positioned at outside of the patient's skin around the incision, and a spring-loaded or elastic connecting member sealingly interconnecting the first and second sealing members, whereby the spring-loaded or elastic connecting member is adapted to seal between at least one of: (a) the second sealing member and the skin of the patient, (b) the first sealing member and tissue of the patient, and c) the connecting member and tissue in relation to the incision.

The medical device according to any one of the preceding embodiments, wherein the medical device further comprises at least one of: a filtering unit for filtering fluid supplied to the chamber, a sterilization unit for directly or indirectly sterilizing fluid supplied to the chamber, a fluid tempering unit adapted change the temperature of fluid supplied to the chamber, a filtering unit for filtering fluid and a sterilization unit for directly or indirectly sterilizing fluid supplied to the chamber, a filtering unit for filtering fluid supplied to the chamber and a fluid tempering unit adapted change the temperature of fluid supplied to the chamber, a sterilization unit for directly or indirectly sterilizing fluid supplied to the chamber and a fluid tempering unit adapted change the temperature of fluid supplied to the chamber, and a filtering unit for filtering fluid supplied to the chamber, a sterilization unit for directly or indirectly sterilizing fluid supplied to the chamber and a fluid tempering unit adapted change the temperature of fluid supplied to the chamber.

According to one embodiment, the medical device is applied on the patient's body for performing a surgical procedure, the medical device further comprises at least one of: at least one inlet provided in at least one of: the wall of the medical device, and the skin of the patient for supplying a fluid to at least one of the chamber, and the cavity in the patient's body, and at least one outlet provided in at least one of: the wall of the medical device, and the skin of the patient for discharging the fluid from at least one of: the chamber, and the body cavity.

According to one embodiment, the medical device further comprises a pump adapted to circulate the fluid from the outlet to the inlet.

According to one embodiment, at least one of the wall and body port comprises a protruding member, and at least one of the wall and body port comprises a recess, and wherein, the protruding member is adapted to engage the recess for locking the wall port to the body port.

According to one embodiment, the recess is a groove in at least one of the wall port and body port.

According to one embodiment, the wall port is adapted to be fixated to a first coupling and the body port is adapted to be fixated to a second coupling, and wherein the first and second couplings are adapted to be connected for connecting the wall port and body port.

According to one embodiment, the flexible wall comprises a pleated portion such that the flexible wall can be expanded.

According to one embodiment, the medical device further comprises at least one sensor adapted to sense at least one of; a pressure, flow and a physiological or physical parameter of the patient. According to one embodiment, the physiological or physical parameter is a parameter selected from: the blood flow of the patient, the saturation of the blood of the patient, an ischemia marker of the patient, the temperature at the skin of the patient, and the patient's skin tone, a pressure, a force, a time, a movement, a stretching, a distance, and a volume.

A method of creating a sealed chamber at least partially inside of the body of a patient is provided. The method comprises making an incision in the skin of the body of the patient, inserting, through the incision, an internal sealing device comprising an internal sealing member, the internal sealing device being connected to a tubular wall, positioning the internal sealing member in connection with at least a part of at least one human organ or human tissue, such that a seal is created between the medical device and at least the part of the at least one human organ or human tissue, and adjusting the circumference of the sealing member for enclosing or encircling a portion of the at least one human organ or human tissue, such that a sealed chamber is created by the tubular wall of the medical device, the internal sealing device, and the organ or tissue.

According to one embodiment, the method further comprises at least one of the steps of: inserting at least one trocar through the incision in the skin of the patient, inserting at least one dissecting tool into the trocar, and dissecting an area of the organ or tissue.

According to one embodiment, the method further comprising the step of placing a body port in the incision in the skin of the patient, and wherein the tubular wall is connected to the body port, such that the sealed chamber is created between the tubular wall, the internal sealing device and the body port.

According to one embodiment, the method further comprises the steps of: inserting, through the incision, a second internal sealing device comprising a second internal sealing member, the second internal sealing device also being connected to the tubular wall, and positioning the second internal sealing member in connection with at least a second part of the at least one human organ or human tissue, such that a seal is created between the second medical device and at least the part of the at least one human organ or human tissue, such that a sealed chamber is created by the tubular wall of the medical device, the first and second internal sealing devices, and the organ or tissue.

According to one embodiment, the method further comprises the step of adjusting the circumference of the second sealing member for enclosing or encircling a portion of the at least one human organ.

According to one embodiment, at least one of the first and second internal sealing members is circular and has an adjustable diameter, such that the circumference is adjusted by adjustment of the diameter.

According to one embodiment, the internal sealing member is an internal vacuum sealing member, and wherein the method further comprises the step of applying a vacuum to the vacuum sealing member such that the vacuum sealing member seals by the applied vacuum.

According to one embodiment, the internal sealing member is an internal pressure sealing member, and wherein the method further comprises the step of applying a pressure to the pressure sealing member such that the pressure sealing member seals by means of the applied pressure.

According to one embodiment, the internal sealing member comprises at least one sealing surface adapted to connect to and seal against a tissue portion of the patient, wherein the sealing surface is placed in the front of the internal sealing device, and wherein the step of positioning the internal sealing member in connection with at least a part of at least one human organ or human tissue further comprises applying the internal sealing member axially against a surface of an organ or tissue.

According to one embodiment, the internal sealing member comprises at least one sealing surface adapted to connect to and seal against a tissue portion of the patient, wherein the sealing surface is placed on the inner circumference of the internal sealing device, and wherein the step of positioning the internal sealing member in connection with at least a part of at least one human organ or human tissue further comprises applying the internal sealing member radially axially against the outer circumference of a tubular organ or tissue.

According to one embodiment, the method further comprises the step of filling the chamber with a pressurized fluid.

According to one embodiment, the method further comprises at least one of the following surgical procedures performed within the sealed chamber; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hernia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open joint surgery, combined open joint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

According to one embodiment, the method comprises placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,

According to one embodiment, the method includes placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

The medical device according to any of the embodiments herein may comprise an interconnectable port adapted to be at least partially placed in an incision in a patient's body is provided. The interconnectable port comprises a first and second port adapted to be interconnected to form the interconnectable port, wherein the second port is disconnectable from the first port. The first port is connected to a first portion of a flexible wall, and the second port is connected to a second portion of the flexible wall, and wherein the flexible wall is adapted to form a chamber in which a step of a surgical procedure me be conducted.

According to one embodiment, the interconnectable port enables choosing between performing an endoscopic procedure through the port, in a cavity in the body of the patient, and performing at least one step of the surgical procedure inside the chamber of the medical device using endoscopic instruments or a hand inserted into the chamber. As an example, a laparoscopic procedure may be performed until an object should be inserted into the body of the patient, or specimen should be removed from the body of the patient, at what time the interconnected port is disconnected such that the object or specimen can be transferred from the chamber of the medical device to a cavity in the patient, or in the opposite direction.

According to one embodiment of the interconnectable port, the first port forms a closable wall port when disconnected from the second port, such that the chamber can remain sealed.

According to one embodiment of the interconnectable port the second port forms a closable body port by itself when the first port has been disconnected from the second port, such that the chamber and cavity of the patient remains separated even as the interconnectable port is separated. The chamber may have a first volume when the first and second ports are interconnected and a second volume when the second port is disconnected from the first port, and the second could be larger than the first volume. According to one embodiment, the first volume is smaller than 100 000 mm3. According to one embodiment, the second volume is larger than 500 000 mm3.

According to one embodiment, the medical device comprises a first and second coupling, and the first port is connected to the first coupling, and the second port is connected to the second coupling. At least one of the first and second coupling may comprise a protruding member, and at least one of the first and second coupling may comprise a recess, which may be a groove, and the protruding member may be adapted to engage the recess for locking the first coupling to the second coupling.

According to one embodiment, the interconnectable port is adapted to, in a first state, when the first and second ports are interconnected, enable a surgeon to operate through the interconnected interconnectable port, and in a second state, when the second port is disconnected from the first part enable the surgeon to perform steps of the procedure within the chamber formed by the flexible wall between the first and second ports of the interconnectable port. The interconnectable port may be adapted to, in the second state, enable the surgeon to remove a specimen from the body of the patient through the second port and into the chamber, and to perform one of: placing the specimen within the chamber, and removing the specimen from the chamber through the second port.

According to one embodiment, the first and second ports are adapted to be reconnected after having been disconnected, such that the surgeon can continue operating through the interconnected port after the specimen has been removed.

The interconnectable port may further comprise an inset detachably fixated to at least one of the first and second coupling, such that the inset could be detached and replaced by a different inset. The inset may comprise at least one of: a surgical glove and a device or instrument mount.

According to one embodiment, at least one of the first and second ports comprises an elastic or flexible membrane adapted to, in a non-compressed state, provide sealing, and in a compressed state enable a hand or an object to be inserted through the membrane.

According to one embodiment of the interconnectable port the first port may comprise a first elastic or flexible membrane and the second port may comprise a second elastic or flexible membrane, and the first and second membranes may be adapted to be placed tightly together such that they act as a single elastic or flexible membrane adapted to, in a non-compressed state, provide sealing, and in a compressed state enable a hand or an object to be inserted through the membrane. The first and second membranes may comprise a plurality of self sealing holes enabling insertion of instruments through the membranes.

According to one embodiment, the interconnectable port further comprises at least one of: a vacuum seal adapted to hold a pressure less than atmospheric pressure between the interconnectable port and the patient's skin to seal between the interconnectable port and the patient's skin, and a pressure seal adapted to hold a pressure above atmospheric pressure between the interconnectable port and the patient's skin to seal between the interconnectable port and the patient's skin.

The interconnectable port according to any one of the preceding embodiments, may further comprises at least one of: a filtering unit for filtering the fluid, a sterilization unit for directly or indirectly sterilizing the fluid and a fluid tempering unit adapted change the temperature of the fluid,

A surgical method to be performed on the body of a patient using an interconnectable port is further provided. The interconnectable port comprises a first and second port, the method comprising: creating an incision in the skin of the patient, placing the interconnectable port in the incision, disconnecting the first port from the second port, such that a chamber is formed by a wall connected to the first and second port, and performing a step of the surgical procedure within the chamber. The step of performing a step of the surgical procedure within the chamber may comprises removing a specimen from the cavity in the body of the patient, transferring the specimen through the second port, and placing the specimen within the chamber of the medical device. The surgical method may further comprise: reconnecting the first and second ports such that the interconnectable port is formed, and performing a surgical step in a cavity of the patient, through the interconnectable port.

A medical device for performing a surgical procedure on a patient is provided. The medical device comprises a wall adapted to be at least partially applied on the patient's body to form a chamber in which a sealed environment can be maintained, the chamber being adapted to encompass part of the surgical procedure to be performed, wherein a portion of the wall directly or indirectly forms an elongated tubular enclosure having a single open end, the elongated tubular enclosure extending in the chamber and being adapted to fit a portion of an extremity of the patient inserted into the elongated tubular enclosure through the open end thereof, such that the surgical procedure can be performed on the portion of the patient's extremity, and a fluid evacuation system adapted to evacuate fluid from the interior of the elongated tubular enclosure, when the portion of the patient's extremity is placed in the enclosure.

By evacuating the fluid from the elongated tubular enclosure, the wall of the inside of the elongated tubular enclosure can fit snuggly against the extremity of the patient for example reducing the risk that bacteria or viruses caught in the skin of the patient, is transported to the incision site in the chamber of the medical device.

According to one embodiment, the elongated tubular enclosure comprises an adhesive surface adapted to contact the skin of the patient when the fluid has been evacuated from the interior of the elongated tubular enclosure.

According to one embodiment, a first portion of the elongated tubular enclosure may comprise an adhesive surface, and the medical device may further comprise a separating layer adapted to cover the first portion, and the separating layer may be adapted to be removed from the first portion, when the adhesive surface is placed onto the skin of the patient.

According to one embodiment, the medical device comprises at least one sealing device adapted to seal between the skin of the extremity of the patient and at least one of: the wall of the medical device and the elongated tubular enclosure. The sealing device may be adapted to exert an adjustable sealing force.

According to one embodiment, the sealing device comprises a hydraulically, pneumatically, or electrically powered pressure sealing member.

According to one embodiment, the sealing device comprises a vacuum sealing member attached to at least one of; the wall and enclosure and adapted to provide a pressure less than atmospheric pressure to create a vacuum seal between the skin of the patient's extremity and at least one of the wall and elongated tubular enclosure.

According to one embodiment, the medical device further comprises at least one of: a pressure creating member connected to the at least one of; the hydraulically, pneumatically and electrically powered sealing member, and a vacuum creating member connected to the vacuum sealing member.

According to one embodiment, the body multi port is fixated in the elongated tubular enclosure.

The medical device in any of the embodiments herein may comprise a wall adapted to be at least partially applied on the patient's body to form a first chamber adapted to hold a first pressure exceeding atmospheric pressure, in which a sealed environment can be maintained for encompassing part of the surgical procedure to be performed. A portion of the wall is adapted to contact the skin of the patient to create a substantially airtight seal between the wall portion and the skin of the patient, to enable an incision to be performed through the skin of the patient in sealed environment such that a fluid connection between a cavity within the patient and the first chamber can be created. The wall is adapted to maintain the seal between the wall portion and the patient's skin substantially airtight after such an incision has been performed. The medical device is adapted to hold a pressure in the first chamber, allowing fluid connection with the cavity, exceeding atmospheric pressure, when such an incision has been performed during the surgical procedure, wherein the wall comprises: a first wall part, designed to allow the first pressure to be maintained in the first chamber, and a second wall part, designed to allow a second pressure to be maintained in a second chamber adapted to create a sealing between the medical device and the patient's body, and the first and second pressures are different. By the provided medical device, a first positive or negative pressure could be provided for sealing between the skin and/or tissue of the patient, and a second positive pressure could be provided in the chamber of the medical device and/or in a cavity in the patient's body.

According to one embodiment of the medical device, the wall comprises a top portion and a bottom portion, which is adapted to contact and press against the patient's skin, surface to surface, to seal between the bottom wall portion and the skin of the patient. The first wall part is adapted to allow a pressure in the first chamber exceeding atmospheric pressure. The bottom wall portion may be adapted to be cut through when the incision in the skin of the patient is made in the sealed environment, such that the incision is performed inside of the sealed environment, reducing the risk that bacteria can enter the incision site.

According to one embodiment, the bottom wall portion comprises an adhesive surface for at least one of: fixating the wall to the patient's skin, and sealing between the bottom wall portion and the patient.

According to one embodiment, the bottom wall portion comprises a vacuum sealing member, at least one of; attached to and integrated in the wall and adapted to provide a pressure less than atmospheric pressure between the vacuum sealing member and the patient's skin to create a vacuum sealing between the skin and the wall.

The medical device of any if the embodiments may further comprise at least one of; a pressure adjustment device adapted to adjust the pressure exerted by the sealing device, and a monitoring unit for monitoring at least one of: the pressure exerted by the sealing device, the blood flow passing the sealing device, and the direct or indirect leakage of fluid from the chamber. By monitoring the pressure exerted by the sealing device, or the effects on the body of the patient, the exerted force can be adapted such that sufficient sealing is provided while doing minimal damage to the body of the patient.

According to one embodiment, the sealing device has a closed geometrical configuration adapted to be applied on a surface of the skin without encircling any limb of the patient and/or a closed geometrical configuration adapted to encircle a limb of the patient.

The medical device may further comprise at least one of; at least one glove integrated in the wall such that a surgeon is able to use the glove from outside the chamber to make manual manipulations inside the chamber while maintaining sealed environment in the chamber, at least one inset, and at least one inset comprising an integrated glove such that a surgeon is able to use the glove from outside the chamber to make manual manipulations inside the chamber while maintaining sealed environment in the chamber.

According to one embodiment, the wall comprises a bottom portion adapted to contact and press against the patient's skin, to seal between the bottom wall portion and the skin of the patient. The wall of the medical device is adapted to allow a pressure in the chamber exceeding atmospheric pressure. The bottom wall portion may be adapted to be cut through when an incision in the skin of the patient is to be performed in sealed environment.

In any of the embodiments herein, the medical device may comprise a wall adapted to be at least partially applied on the patient's body and further adapted to form a chamber alone or together with the patient's body, in which a sealed environment can be maintained for encompassing part of the surgical procedure to be performed, wherein a portion of the wall is adapted to be in contact with the patient's skin, and at least one wall inset or wall port attached to the wall and adapted to enable manipulation within the chamber, wherein the wall inset or wall port is displaceable between: a first position, in which the inset or wall port is fixated relative to the skin or tissue of the patient, and a second position, more remote from the patient's skin than the first position, in which the inset or wall port is moveable relative to the skin or tissue of the patient. Having the port or inset more remote from the patient's skin enables manipulation using hands or instruments within the chamber of the medical device, whereas the location close to the skin facilitates manipulation in a cavity in the body of the patient using endoscopic instruments or a hand.

According to one embodiment, the wall inset or wall port comprises a first coupling adapted to fixate the inset or wall port directly or indirectly to the skin or tissue of the patient.

According to one embodiment, the wall comprises a first and second coupling, the first coupling is adapted to be positioned at a first position, and wherein the second coupling is adapted to connect to the first coupling for locking the wall inset or wall port in the first position.

The medical device may further comprise a body inset or body port adapted to be mounted in another wall portion or the skin or tissue of the patient. The first coupling may be adapted to be positioned in connection with the body port or body inset, and the wall port and the body port may be adapted to form an integrated port when the first and second coupling are connected in the first position. The body port may comprise a second coupling adapted to connect to the first coupling.

According to one embodiment, the chamber may assume a first volume when the wall inset or wall port is in the first position, and assumes a second volume larger than the first volume, when the inset or wall port is in the second position.

The portion of the wall adapted to be applied on the patient's body when the surgical procedure is to be performed further comprises a vacuum sealing member adapted to hold a pressure less than atmospheric pressure between the wall portion and the patient's skin to seal between the wall portion and the patient's skin. The vacuum sealing member may be adapted to create a substantially airtight seal between the wall portion and the skin of the patient, to enable an incision to be performed through the skin of the patient in sealed environment such that a fluid connection between a cavity within the patient and the chamber can be created.

A medical device for performing an endoscopic surgical procedure on a patient is provided. The medical device comprising a wall adapted to at least partially be applied on the patient's body to form a chamber, by itself or together with the patient's body, in which a sealed environment can be maintained for encompassing part of the surgical procedure to be performed. A wall portion of the medical device is adapted to contact the patient's skin when the surgical procedure is performed, and a vacuum sealing member is adapted to hold a pressure in a second chamber between the skin and the wall, or in a second chamber and between the skin and the wall, contacting the skin of the patient, the pressure being: different than the pressure in the chamber, and less than atmospheric pressure, for providing sealing between the wall portion and the patient's skin. The vacuum sealing member thus enables the maintaining of a pressure exceeding atmospheric pressure in the chamber and being below atmospheric pressure in the vacuum sealing member.

The vacuum sealing members in any of the embodiments herein may comprise multiple vacuum sealing grooves adapted to be placed consecutively.

The medical device in any of the embodiments herein may further comprise at least one of an additional body port, additional body inset, additional wall port and additional wall inset, and at least one of the wall port and body port is exchangeable by the additional port or additional inset.

According to one embodiment, the wall portion adapted to be applied on the patient's body, when the surgical procedure is to be performed, further comprises at least one of; a vacuum sealing member adapted to hold a pressure less than atmospheric pressure between the wall portion and the patient's skin to seal between the wall portion and the patient's skin, and a pressure sealing member adapted to hold a pressure above atmospheric pressure between the wall and the patient's skin to seal between the wall and the patient's skin.

According to one embodiment, the vacuum sealing member or pressure sealing member is adapted to create a substantially airtight seal between the wall portion and the skin of the patient, to enable an incision to be performed through the skin of the patient in sealed environment such that a fluid connection between a cavity within the patient and the chamber can be created.

According to one embodiment, at least one of a closeable body multi-port may be adapted to be placed from inside the chamber in an incision to be performed in the skin of the patient, and the body multiport is adapted to be connected to at least one of; the wall port, and a wall multiport, when the medical device is applied on the patient's body for performing a surgical procedure, and the closable wall port comprises a closeable wall multiport, wherein the wall multiport is adapted to be connected to at least one of; the body port, and a body multiport placed in an incision in the patient's body, when the medical device is applied on the patient's body for performing a surgical procedure.

According to one embodiment, the medical device according to any one of the preceding claims comprises a first and second chamber, wherein the second chamber is adapted to hold a negative pressure for exerting a suction force on the skin and/or tissue of the patient for creating the sealing between the medical device and the patient. The first chamber may be adapted to be in fluid connection with a cavity of the patient via an incision in the skin of the patient, the chamber and the cavity in the patient forms a joint volume having the same pressure and partially being enclosed by the medical device. The first chamber may be adapted to hold a positive pressure between 5 mmHg and 120 mmHg, and wherein the second chamber is adapted to hold a negative pressure between 5 mmHg and 120 mmHg.

A system is further provided, the system comprised the medical device according to any one of embodiments above, a pressure creating member adapted to create a pressure exceeding 5mmHg in the first chamber of the medical device, and a vacuum creating device adapted to create a negative pressure of 5 mmHg in the second chamber of the medical device for creating a sealing between the medical device and the skin and/or tissue of the patient.

A second system is further provided comprising: the medical device according to any one of embodiments, a first pressure creating member adapted to create a pressure exceeding 5 mmHg in the first chamber of the medical device, and a second pressure creating member adapted to create a second pressure exceeding 5 mmHg in the second chamber of the medical device for creating a sealing between the medical device and the skin of the patient.

The second pressure may be higher than the first pressure, such that the first pressure of the sealing member can withstand the force exerted by the pressure in the chamber on the sealing device.

A medical device for performing hip joint surgery on a patient is provided. The medical device comprising: a wall adapted to least partly be placed on the patient's body for forming a chamber alone or together with the patient's body, such that a sealed surgical environment is created in the chamber. The chamber is adapted to encompass an incision of the hip joint surgery in the skin of the patient and at least one step of the hip joint surgery to be performed, while maintaining the sealed surgical environment. The medical device further comprises a sealing device connected to the wall and adapted to seal between the wall and the skin or tissue of the patient, wherein the sealing device forms a special loop at least partly encircling at least a portion of one or more of the following; the leg, the pelvic region, the abdominal region, the inguinal region, the gluteal region, the thoraxial region and the region of the lower back, such that the sealing device seals between the incision site of the hip joint surgery and at least one of: the anal orifice, the urethra orifice, and the perineum region. By creating a sealed environment, the risk of infections can be substantially reduced. The risk of infections is particularly large when performing prosthesis surgery inside the joints of patients, which is why the maintaining of a controlled environment is important. The sealing device is positioned such that both of the urethra and the anus are excluded from the enclosed chamber forming the sealed surgical environment.

The medical device according to any of the embodiments herein may further comprise a first sealing device connected to the wall and adapted to seal between the wall and the skin or tissue of the patient to seal the hip surgery incision and at least a part of the hip joint surgery operational field from at least one of: the anal orifice, the urethra orifice and at least part of the perineum region, while still maintaining the chamber sealed, the sealing device comprising at least one of: a first part of the sealing device, and a first holding member adapted to hold the medical device in place by encircling at least one leg of the patient or the prolongation thereof to be involved in the sealing of the chamber. The medical device further comprises at least one of a second part of the sealing device, and a second holding member adapted to hold the medical device in place by encircling the whole body left to right on the level above the legs selected from at least one of; the level of the pelvic, abdominal or thorax region of the patient to further be involved in the sealing of the chamber. The medical device further comprises at least one of; a third part of the sealing device adapted to seal from the medial cranial part of the leg where it is meeting the inguinal region or its prolongation, further in a cranial direction lateral of the patient's ventrodorsal midsagittal plane, on the side where the hip surgery is performed, and wherein the third part of the sealing device at least partly extends in cranial direction until meeting the second sealing device, and wherein the wall is interconnecting to the first, second and third part of the sealing device to seal the chamber and/or a third holding member adapted to seal the chamber and exclude from the chamber at least one of; the anal orifice, the urethra orifice and at least part of the perineum region.

The medical device according to any one of the embodiments herein may comprise a slit for placing the chamber around at least a portion of the extremity of the patient radially in relation to the length axis of the extremity. The slit may be adapted to be at least partially closed by closing elements mounted on the wall of the medical device.

The medical device may comprise a first and a second portion located on a first and second side of the slit when the chamber is placed around at least a portion of the extremity. The first and second portions are adapted to be interconnected by means of at least one of: an adhesive surface and a mechanical connecting member.

According to one embodiment, at least one inset or port attached to the wall is adapted to be displaceable between a first position, in which the inset or port is situated relatively close to the patient's skin or tissue of the patient and directly or indirectly is connecting to the skin or tissue of the patient, and a second position, in which the inset or wall port is situated more remote from the patient's skin than in the first position, when the wall at least partly is applied on the patient's body.

The medical device in any of the embodiments herein may be adapted to form a chamber at least partially encapsulating the knee joint of the patient, the chamber being adapted to encompass at least a part of the knee joint surgery to be performed while maintaining a sealed environment for the surgical procedure, and a closeable wall port or inset placed in a portion of the wall. The closeable wall port or inset is adapted to be at least one of: abutting, attaching, and placed in a fixed position in relation to a body port placed in an incision in the knee region of the patient's body, such that an arthroscopic step of the knee joint surgery can be performed.

A method of performing a portion of knee joint surgery on a patient using a medical device is provided. The method comprises applying a wall on the skin of the patient to form a chamber in which a sealed environment can be maintained for encompassing part of the knee joint surgery to be performed. The wall comprises a closeable wall port connecting the closeable wall port to a body port, such that the closeable wall port is at least one of: abutting the body port and attached to the body port, such that an arthroscopic step of the knee joint surgery can be performed.

A medical device for performing a surgical procedure on a patient is provided. The medical device comprises a wall adapted to be at least partially applied on the patient's body to form a chamber together with the patient's body, in which a sealed environment can be maintained for encompassing part of the surgical procedure to be performed, at least one of; a closeable wall port and inset placed in a portion of the wall and being adapted to be detachably connected to a closeable body port placed in an incision in the patient's body connecting to a body cavity, when the medical device is applied on the patient's body for performing the surgical procedure, at least one of; a closeable body port and an inset adapted to be placed in an incision in the patient's body and further adapted to be detachably connected to the closeable wall port, when the medical device is applied on the patient's body for performing the surgical procedure, a sealing device connected to the wall and adapted to seal against the skin of the patient, when the medical device is applied on the patient's body. At least one of; the closable wall port, the closable body port and the closable wall port and the closable body port together, are adapted to form an airlock sluice between a cavity in the body of the patient and the ambient environment, when the medical device is applied on the patient's body for performing the surgical procedure.

The medical device according to anyone of the embodiments may further comprise a coupling adapted to be at least one of; an integrated part of at least one of the body port or the body inset, an integrated part of at least one of the wall port or the wall inset, a body coupling adapted to be placed in close relation to the skin of the patient, and further adapted to be connected to the wall port or inset, and a wall coupling adapted to be placed in close relation to the wall of the medical device. The coupling may further be adapted to be connected to the body port or inset.

The wall in any of the embodiments herein may be adapted to hold a liquid at least partially filling the chamber; the liquid may be at least one of; an isotonic solution and an antibiotic liquid.

The medical device of any of the embodiments herein may be adapted for use in knee joint procedures, and thus comprise at least one of; a first opening arranged such that the leg of a patient can enter the medical device and a second opening arranged such that the leg of a patient can exit the medical device.

According to one embodiment, the medical device comprises a fluid system for withdrawing fluid from the fluid outlet and supplying fluid to the fluid inlet. The fluid system may further comprise a fluid pump for circling fluid from the fluid outlet to the fluid inlet. The fluid system may comprise at least one of: a filtering unit for filtering the fluid, a sterilization unit for directly or indirectly sterilizing the fluid, a fluid tempering unit adapted change the temperature of the fluid.

The medical device in any of the embodiments may further comprise an energy transferring coupling adapted to transfer energy from outside the chamber into the sealed environment, such that a tool can be energized. The medical device may be adapted to hold a pre-placed energy consuming tool within the sealed environment before the medical device is placed in connection with the patient.

The medical device according to any embodiment may comprise a first fluid inlet placed in the wall and adapted for supplying fluid to the chamber, a first fluid outlet placed in the wall and adapted for discharging fluid from the chamber, a second fluid inlet adapted to be placed in an incision in the skin of the patient, in the area of the joint, for supplying fluid to the joint, and a second fluid outlet adapted to be placed in an incision in the skin of the patient, in the area of the joint, for discharging fluid from the joint. The fluid may be a liquid fluid, such as an isotonic solution and an antibiotic liquid. The fluid system may comprise a fluid pump for circulating the fluid from the first or second fluid outlet to the first or second fluid inlet.

The medical device may comprise a pressure adjustment device adapted to adjust the pressure in the sealing member such that the pressure in the sealing member is between one of: 10mmHg - 160mmHg, 20mmHg - 140mmHg, 30 mmHg - 120mmHg, 30 mmHg - 100 mmHg, 40 mmHg - 100 mmHg, 40 mmHg - 80 mmHg, and 50 mmHg - 70 mmHg.The pressure adjustment device may comprise at least one of: a fluid pump for pumping a gaseous fluid to the sealing member, a fluid pump for pumping a liquid fluid to the sealing, a spring loaded device involved in causing the pressure, and a prefilled reservoir involved in causing the pressure.

A monitoring unit may in any of the embodiments be adapted to encircle an extremity of the patient and measure the blood pressure of the patient periodically or monitor the blood pressure of the patient by exerting a pressure on the encircled extremity exceeding the systolic arterial pressure, such that the blood flow is momentarily stopped. The monitoring unit may be adapted to measure the blood pressure of the patient with an interval of one of: 1 - 5 minutes, 5 - 10 minutes, 10 - 20 minutes, and 20 - 30 minutes.

The medical device according to any of the embodiments herein may further comprise an energy transferring member for transferring energy from outside of the chamber to inside of the chamber, and an information transferring member for transferring information from outside of the chamber to inside of the chamber, whilst maintaining the sealed environment. The energy transferring member may be adapted to transfer at least one of; pneumatic, hydraulic and kinetic energy. According to one embodiment, the energy transferring member comprises a valve such that the energy transferring member can be opened for enabling transfer of at least one of; kinetic, pneumatic and hydraulic energy through the wall of the medical device.

According to one embodiment, the energy transferring member may be adapted to transfer electric energy via at least one of; a wireless link, and a cable connection. In an alternative embodiment, the energy transferring member may comprise an inductive coupling for transferring electric energy by a magnetic field between a first conductor positioned on the outside of the chamber, and a second conductor positioned on the inside of the chamber.

The medical device according to any of the embodiments herein may further comprise an information transferring member is adapted to transfer data from the inside of the chamber to the outside of the chamber, through the wall of the medical device. According to one embodiment, the information transferring member comprises at least one of: an optical information transferring member, a capacitive information transferring member, an inductive information transferring member, and a radio based information transferring member. The optical information transferring member may be a infra red information transferring member. The step of transferring energy through the wall of the medical device may comprise at least one of: pneumatic, hydraulic and kinetic energy through the wall of the medical device.

A part of the wall of the medical device may form an elongated tubular enclosure extending within the chamber of the medical device, contacting the skin of the patient and being adapted to separate the skin of the patient from the sealed environment of the chamber, such that the chamber is formed between the wall of the medical device and the elongated tubular enclosure.

The medical device may further comprise an evacuation valve for evacuating a fluid from the chamber. The evacuation valve may be connected to a filtering unit for filtering the evacuated fluid. The medical device may further comprise a control system for controlling the opening of the evacuation valve. The control system may be adapted to control the evacuation valve on the basis of at least one of: the pressure in the chamber, manual detection of the visibility in the chamber, the visibility in the chamber, detection of smoke in the chamber, the temperature in the chamber, and a time related variable.

A medical device for use in an endoscopic surgical procedure is further provided. The medical device comprises a flexible wall enclosing a chamber. The medical device further comprises a device connecting portion positioned in the flexible wall and adapted to connect to a trocar connecting portion of a trocar, such that a fluid connection is created between the chamber enclosed by the wall, and a cavity within the patient.

The medical device may further comprise a surgical instrument enclosed within the chamber and adapted to operate within the cavity of the patient through the trocar.

The surgical instrument may comprise a handle portion. The handle portion may be operably encapsulated by the wall such that the handle portion can be manipulated from outside the chamber.

According to one embodiment, the medical device comprises a penetrable second self sealing membrane adapted to seal between the chamber and the ambient environment.

The second self sealing membrane may be positioned at the device connecting portion of the medical device, such that the self sealing membrane is abutting a first self sealing membrane of the trocar when the medical device and the trocar are connected, such that an integrated self sealing membrane is formed enabling insertion of the endoscopic instrument, contained in the medical device, into the trocar through the integrated self sealing membrane.

The second connecting portion may comprise a sleeve adapted to connect to the first connecting portion of the trocar by one of: engaging the trocar on the outside thereof, and engaging the trocar on the inside thereof. The sleeve may comprise a sealing member for sealing between the sleeve and the trocar.

The device connecting portion of the medical device may comprise a recess or protruding member adapted to engage a corresponding recess or protruding member of the trocar connecting portion.

A medical device system for performing endoscopic surgery on a patient is further provided. The medical device system comprises a medical device comprising a flexible wall enclosing a chamber. The medical device further comprises a device connecting portion positioned in the flexible wall, and a trocar adapted to be positioned though the skin of the patient for establishing a fluid connection with a cavity within the patient, wherein the trocar comprises a trocar connecting portion . The device connecting portion of the medical device may be adapted to be connected to the trocar connecting portion of the trocar for establishing a fluid connection between the chamber of the medical device and the cavity in the patient.

According to one embodiment, the wall port is displaceable between a first position, in which the wall port is situated relatively close to the tissue or skin of the patient, and a second position, in which the wall port is situated more remote from the skin of the patient than in the first position.

According to one embodiment, the wall port is adapted to be locked to the body port.

According to one embodiment, at least one of the wall port and body port comprises a penetrable self sealing gel, such that an object or hand can be inserted through the wall port while maintaining the sterile environment.

According to one embodiment, the chamber is adapted to hold a fluid having a pressure exceeding atmospheric pressure.

Any of the body ports described in relation to the medical device may be body multi ports comprising at least two ports adapted to enable passage of at least one of; one or more tools, one or more devices and one or more parts of the human body, from outside of the patient's body to inside of the patient's body or the opposite direction. A body port may further comprise one port adapted to enable passage of at least one of; one or more tools, one or more devices and one or more parts of the human body, from outside of the patient's body to inside of the patient's body or the opposite direction.

The endoscopic instruments described herein may for example be endoscopic graspers, endoscopic claw graspers, an endoscopic stone graspers, an endoscopic needle holders, an endoscopic hooks, a cameras, scissors, knives, an ultrasound dissectors, suction instruments, endoscopic dissectors or endoscopic diathermy instruments.

The medical device may in any of the embodiments have a wall comprising a transparent layer enabling viewing into the chamber of the medical device.

The medical device in any of the embodiments may comprise a wall adapted to be at least partially applied on the patient's body to form a chamber adapted to hold a pressure exceeding atmospheric pressure for maintaining a sterile environment in which part of the surgical procedure can be performed. A portion of the wall is adapted to contact the skin of the patient to create a substantially airtight seal between the wall portion and the skin of the patient, to enable an incision to be performed through the skin of the patient in sterile environment such that a fluid connection between a cavity within the patient and the chamber can be created. The wall is adapted to maintain the seal between the wall portion and the patient's skin substantially airtight after such an incision has been performed, and the medical device is adapted to hold a pressure in the chamber, which is in fluid connection with the cavity, exceeding atmospheric pressure, when such an incision has been performed during the surgical procedure. By creating the sterile chamber prior to making the incision in the skin of the patient, the incision can be made in a sterile environment and the risk that bacteria enters the body of the patient in connection with the creation of the incision is thereby dramatically reduced.

The medical device in any of the embodiments may comprise a top portion and a bottom portion, which is adapted to contact and press against the patient's skin, surface to surface, to seal between the bottom wall portion and the skin of the patient, and wherein the wall is adapted to allow a pressure in the chamber exceeding atmospheric pressure.

The medical device in any of the embodiments may comprise a wall sealing device adapted to seal between a portion of the wall and the patient's body, when the wall is applied on the patient's body. The sealing device comprises a pressure sealing member adapted to pneumatically, hydraulically or mechanically press the wall portion against the body of the patient such that the chamber is at least substantially sealed from the ambient atmosphere allowing a pressure inside the chamber higher than ambient atmospheric pressure during the surgical procedure, thus the wall sealing device being adapted to seal the chamber, after the medical device has been applied at least partially on the patient's body, when at least one of; an incision has been performed in the wall of the chamber contacting the patient's body and an incision has been performed in the skin of the patient forming part of the chamber.

Another feature that could be included in the various embodiments disclosed herein is that a portion of the wall directly or indirectly forms an elongated tubular enclosure having an open end. The enclosure extends in the chamber and is adapted to fit a portion of an extremity of the patient inserted into the enclosure through the open end thereof. In other embodiments the medical device could comprise a sealing member encircling the extremity of the patient and adapted to seal between the skin of the patient and the wall.

Another feature that could be included in the various embodiments disclosed herein is that the enclosure could be collapsible or deformable, for facilitating the insertion of the extremity into the enclosure, whereas the wall enclosing the chamber could be rigid. However, in other embodiments it is equally conceivable that the wall enclosing the chamber is partially or entirely collapsible.

Another feature that could be included in the various embodiments disclosed herein is that the medical device could further comprise two gloves integrated in the wall enabling manual manipulation within the sterile environment. The gloves could be connected to the wall by means of pleated sections connecting the gloves to the wall and enabling free movement of the gloves within the sterile environment.

Another feature that could be included in the various embodiments disclosed herein is that where the wall is at least partially collapsible, the wall could be inflatable by a gaseous fluid for defining the chamber.

Another feature that could be included in the various embodiments disclosed herein is that the sealing member could comprise a vacuum sealing member adapted to seal between an extremity of the patient and the partially collapsible wall, by the vacuum sealing member being adapted to hold a pressure less than 1 bar for creating a vacuum sealing between the skin of the patient and the wall. The vacuum is created by a vacuum pump connected to the vacuum sealing member by means of a vacuum conduit. The vacuum sealing member could for example be made from a semi-rigid polymer material.

Another feature that could be included in the various embodiments disclosed herein is an air evacuation system, for evacuating air from the enclosure after the portion of an extremity has been placed in the enclosure, thereby tightening the wall around the extremity of the patient. The air evacuation system could comprise a conduit in fluid connection with the enclosure thereby enabling the evacuation of the air from therein.

Another feature that could be included in the various embodiments disclosed herein is a pneumatic pressure sealing member attached to at least one of the wall and enclosure and adapted to press against the patient's skin to create a pressure sealing between the skin of the patient's extremity and at least one of the wall and enclosure, using a pneumatic pressure pressing against the skin of the extremity. The pneumatic pressure could be created by a pneumatic pump being connected to the pneumatic pressure sealing member by means of a fluid conduit adapted to transport a pressurized gas from the pneumatic pump to a cavity within the pressure sealing member. The pressure sealing member could be inflatable such that the size and/or shape of the cavity can be changed for allowing the pneumatic pressure sealing member to press against the skin of the extremity and thereby seal between the extremity and the pneumatic pressure sealing member. The pneumatic pressure sealing member can for example be made from an elastic material such as an elastic polymer.

Another feature that could be included in the various embodiments disclosed herein is that an adhesive could be adapted to provide at least one of: fixation of at least one of the wall and enclosure to the skin of the patient and sealing between the skin of the patient and at least one of the wall and enclosure. The adhesive could for example encircle an extremity of a patient and thereby create a seal. The adhesive could for example be a pressure sensitive adhesive such as 3M's pressure sensitive adhesive Scotch-Grip 4268-NF.

Another feature that could be included in the various embodiments disclosed herein is that the wall has a cylindrical or tube shaped structure. Where the wall is at least partially collapsible, it could be inflatable by means of a pneumatic conduit, which could be connected to a tank containing pressurized gas.

Another feature that could be included in the various embodiments disclosed herein is a pneumatic system for transferring pneumatic force into the chamber. The pneumatic system could be connected to a pneumatic tool, which in the example shown is an orthopedic saw, adapted to be operated within the sterile environment. The pressure provided to the sterile environment and the pneumatic system could be controlled and monitored by a control/monitoring system connected to the pneumatic conduits, respectively. The wall could be sealed to the extremity of the patient by means of a pneumatic pressure sealing member which could encircle the extremity.

Another feature that could be included in the various embodiments disclosed herein is the medical device could incorporate a pneumatic pressure sealing member.

Another feature that could be included in the various embodiments disclosed herein is an air-lock system comprising two zipper-closed consecutive openings, where the outer zipper could be opened for placing and/or removing objects in the airlock between the zippers from the outside of the chamber, and the inner zipper could be opened from the inside of the chamber for placing and/or removing objects from the airlock. For example the airlock system could be used to remove a specimen from the sterile environment while keeping the environment sterile, or the airlock could be used to insert a medical instrument or an implant into the sterile environment.

Another feature that could be included in the various embodiments disclosed herein is a multi-port comprising laparoscopic ports, through which laparoscopic trocars or endoscopic instruments could be inserted.

Another feature that could be included in the various embodiments disclosed herein is an instrument mount for retaining instruments within the sterile environment.

Another feature that could be included in the various embodiments disclosed herein is a non-collapsible transparent window for enabling viewing into the chamber and the sterile environment. Where the wall is at least partially collapsible, the non-collapsible transparent window could ensure that the surgeon has adequate visual control over the procedure since the partially collapsible wall, even if preferably made from a transparent material, could provide limited visibility, e.g. if the medical device is not fully inflated, or in seams or bends of the material.

Another feature that could be included in the various embodiments disclosed herein is a self sealing access port, which for example could be a port comprising a material of gel type. A self sealing access port could enable the insertion of trocars and/or instruments as well as a person's hand, enabling manual manipulation within the chamber.

Another feature that could be included in the various embodiments disclosed herein is an endoscopic body port in direct connection with the extremity of the patient, within the chamber. The endoscopic port could for example enable an arthroscopic procedure with trocars to be performed within the sterile environment and either by the instruments being manipulated from the inside of the sterile environment or by the instruments reaching from wall ports in the wall, or the self sealing port in the wall and to the port within the sterile environment.

Another feature that could be included in the various embodiments disclosed herein is a system for tempering the fluid entering the chamber. The tempering part of the system could be provided to heat or cool the gaseous fluid inflating the collapsible wall. In cases when the surrounding environment is cold, the tempering part of the system could be used to raise the temperature of the sterile environment to provide beneficial operating circumstances both for the patient and for the surgeon. In cases when the surrounding environment is hot, the tempering part of the system could be used to lower the temperature of the sterile environment both to provide beneficial operating circumstances, for the patient and for the surgeon, and for providing a temperature inhibiting bacterial and viral infections. The tempering unit could comprise a temperature regulating device for setting the required temperature.

Another feature that could be included in the various embodiments disclosed herein is a sterilizing unit adapted to sterilize the gaseous fluid entering the chamber. The gaseous fluid entering the chamber could originate from a pressurized tank and could be pre-sterilized. In other embodiments, it is conceivable that the gaseous fluid is the surrounding air which is pressurized. In the embodiments where the surrounding air is used to inflate the collapsible wall and constitute the operating environment this air needs to be properly sterilized.

Another feature that could be included in the various embodiments disclosed herein is a system for circulating a fluid through the chamber of the medical device. The system could comprise a fluid conduit connected at an inlet placed in the wall for supplying fluid to the chamber, and an outlet for draining the fluid from the chamber. The fluid is circled by means of a pumping unit and is circled via a sterilizing/filtering unit adapted to remove impurities and sterilize the fluid.

Another feature that could be included in the various embodiments disclosed herein is a filtering unit adapted to remove particles that could be suspended in the gas. The filtering unit could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit could for example comprise a filter containing activated carbon.

Another feature that could be included in the various embodiments disclosed herein is that the medical device could be adapted to be placed in relation to an extremity of the patient e.g. a leg or an arm. The medical device could for example be used for performing an amputation both in a hospital environment and in environments where the risk of infections are considerably higher, such as in third world countries or in zones of catastrophe. The medical device could enable surgical procedures to be performed in a fully enclosed environment without any risk of infection disease even in very remote locations and without the access to water or electricity.

Another feature that could be included in the various embodiments disclosed herein is a self sealing port. The self sealing port could comprise a self sealing membrane fixated to a rigid part of the self sealing port. The self sealing membrane could have a small self sealing hole centrally in the membrane. The self sealing membrane could for example be made from a gel-type material being elastic enough to enable a deformation large enough for a person's hand to pass through the small self sealing hole, while still contracting enough to create an airtight seal between the sterile environment and the outside environment. The self sealing port could be fixable to a retractor comprising one intra body ring adapted to be positioned on the inside of the patients skin, and one ring adapted to be placed on the outside of the patients skin. Between the intra body ring and the ring adapted to be placed on the outside of the patients skin, a retractor membrane is positioned which is adapted to exert a pressure of the cut surfaces of the incision for retracting the skin and thereby keeping the wound open. The retractor membrane is preferably made from a resilient of elastic polymer material. The ring adapted to be placed on the outside of the patients skin could comprise an integrated roll up system which could be a spring loaded roll up system adapted to create a suitable pressure on retractor membrane for keeping the wound open.

Another feature that could be included in the various embodiments disclosed herein is a self sealing port connected to a retractor membrane made from a material elastic enough such that one end of the retractor membrane could be fixedly fixated to the rigid part of the port.

Another feature that could be included in the various embodiments disclosed herein is the wall separating the chamber from the outside environment is adapted to be fixated to the skin of the patient by means of an adhesive or by means of a difference in pressure between the outside of the chamber and the inside thereof.

Another feature that could be included in the various embodiments disclosed herein is that the medical device could further comprise an upper and a lower coupling for fixating ring shaped objects, which for example could be insets or ports, where insets for example could be surgical gloves, or holding devices and ports could be endoscopic ports or hand access ports. The coupling could comprise a releasing lever for releasing the object from the coupling. The lever could be connected to latching members which are spring loaded from the rear in order to secure the groove of the object. The inset fixated to the lower coupling could for example be a surgical glove, enabling manual manipulation within the body of the patient.

Another feature that could be included in the various embodiments disclosed herein is a valve member adapted to close the opening in the upper coupling when the upper coupling is not in use.

Another feature that could be included in the various embodiments disclosed herein is that the coupling could be adapted to enable the changing of objects in the coupling from for example a surgical gloves, to a multi-port comprising three endoscopic ports. To be able to quickly switch from an endoscopic system to a hand access or manual manipulation system could be of major importance if complications arise during the procedure and could remove the need for a traditional conversion from laparoscopic to open surgery, a conversion which inevitably causes problems increasing the risk of complications and/or postoperative care.

Another feature that could be included in the various embodiments disclosed herein is that the medical device could be adapted to be applied on the patient prior to the start of the surgical procedure. The medical device could for example be adapted to be fixated to the abdominal area of a patient by means of an adhesive. By fixating the medical device to the patient prior to the start of the surgical procedure a incision in the patient could be performed inside the chamber of the medical device. The medical device could for example be connected to a source of pressurized fluid, which could be lead into the chamber through a fluid conduit.

Another feature that could be included in the various embodiments disclosed herein is that the chamber of the medical device could be adapted to hold a gaseous fluid having a pressure exceeding 1 bar such that a laparoscopic procedure could be performed partially within the chamber and partially within the patient.

Another feature that could be included in the various embodiments disclosed herein is that the medical device could comprise one laparoscopic port detachably fixated to the wall. The laparoscopic port could be adapted to be attached by means of a coupling and thereby detachable. The medical device could further comprise a valve member for closing the hole in the wall while exchanging the laparoscopic port to another inset or port, such that the inset or port can be exchanged while maintaining the sterile environment within the chamber. The detachable insets or ports could be adapted to be fixated to the wall by means of couplings and thereby possible to quickly exchange.

Another feature that could be included in the various embodiments disclosed herein is surgical gloves, which could be integrated in the wall. The surgical gloves could enable manual manipulation within the chamber of the medical device while keeping the chamber hermetically closed.

Another feature that could be included in the various embodiments disclosed herein is that the medical device is an inflatable medical device which could be positioned prior to the start of a normal laparoscopic procedure but remains deflated until the very end of the procedure when the medical device is inflated, for example when a specimen is to be removed.

Another feature that could be included in the various embodiments disclosed herein is a cleaning device for cleaning a transparent window from the inside of the chamber. The cleaning device could comprise an arrangement for supplying a fluid adapted to assist in the cleaning of the transparent window via a fluid conduit to a nozzle placed in proximity to the transparent window. The fluid could for example be an isotonic solution, such as saline solution, or an antiseptic solution such as Chlorhexidine.

Another feature that could be included in the various embodiments disclosed herein is that the medical device could comprise a window contacting member for wiping the window clean after the fluid has been sprayed thereon for mechanically removing objects from the window.

Another feature that could be included in the various embodiments disclosed herein is that the medical device could be adapted to be applied on the abdominal region of the patient. The medical device could be adapted to be fixated to the patient by means of a vacuum affecting the patient along a vacuum groove, which could be connected to a vacuum pump by means of a vacuum conduit.

Another feature that could be included in the various embodiments disclosed herein is a detachable port, which could be positioned in the wall according to any of the embodiments disclosed herein. The port could be attached using a coupling enabling a quick exchange of the port. The port could for example be a self sealing port which could comprise a self sealing membrane fixated to a rigid part of the self sealing port. The self sealing membrane could have a small self sealing hole centrally placed in the membrane. The self sealing membrane could for example be made from a gel-type material being elastic enough to enable a deformation large enough for a person's hand to pass through the small self sealing hole, while still contracting enough to create an airtight seal between the sterile environment and the outside environment.

Another feature that could be included in the various embodiments disclosed herein is that the medical device could include a filtering unit adapted to remove impurities and optionally sterilize the fluid. The filtering unit could be adapted to remove particles that could be suspended in the gas. The filtering unit could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit could for example comprise a filter containing activated carbon.

Another feature that could be included in the various embodiments disclosed herein is a coupling enabling the changing of objects in the coupling for example from a surgical glove to an endoscopic port unit comprising endoscopic ports. To be able to quickly switch from an endoscopic system to a hand access or manual manipulation system could be of major importance if complications arise during the procedure and could remove the need for a traditional conversion from laparoscopic to open surgery, a conversion which inevitably causes problems increasing the risk of complications and/or postoperative care. In other embodiments the laparoscopic ports and surgical gloves could additionally be exchanged for holding devices, for example holding instruments or implants.

Another feature that could be included in the various embodiments disclosed herein is that the medical device could be adapted to be applied on the patient prior to the start of the surgical procedure, such that an incision in the patient could be performed in the sterile environment.

Another feature that could be included in the various embodiments disclosed herein is that where the wall of the medical device is at least partially collapsible, it could be adapted to be inflated by a pressurized fluid entering the medical device through a fluid conduit

Another feature that could be included in the various embodiments disclosed herein is that the medical device is adapted to placed in contact with the skin of the patient prior to the beginning of the operation and the entire portion of the wall contacting the skin of the patient comprises an adhesive, such that an incision in the patient runs through the wall and further through the skin of the patient. This reduces the risk that infectious objects can be transferred from the skin of the patient to the incision in the patient if the skin of the patient is not adequately disinfected.

Another feature that could be included in the various embodiments disclosed herein is that the coupling could be integrated in the wall. The coupling could enable the changing of objects from for examples ports to insets in form of surgical gloves or holding members. The coupling could additionally comprise a valve member for closing the hole in the coupling when objects should be exchanged such that the sterile environment in the chamber remains sterile.

Another feature that could be included in the various embodiments disclosed herein is that the medical device could comprise a holding device, which could for example be adapted to hold surgical instruments and/or implants or parts of implants.

Another feature that could be included in the various embodiments disclosed herein is that the medical device comprises a coupling integrated in the wall. For example could an airlock for transferring objects between the chamber and the outside thereof be fixated to the coupling. The airlock could comprise a first valve member separating the airlock from the sterile environment, and e second valve separating the airlock from the outside environment. The airlock space could be provided between the first and second valve and could for example be used for passing a medical instrument or device into the chamber, or for passing a specimen or sample out of the chamber.

Another feature that could be included in the various embodiments disclosed herein is that the upper coupling could be used for manual manipulation and preparation within the medical device, such that could be needed for preparing or changing an instrument or preparing an implant for implantation. The manual manipulation could be performed by means of a glove fixated to the wall by the coupling.

Another feature that could be included in the various embodiments disclosed herein is that a surgical glove could be fixated to the upper coupling. In other embodiments, the surgical glove could be exchanged by for example a surgical port or a holding device, as further disclosed with reference to other embodiments herein.

Another feature that could be included in the various embodiments disclosed herein is that a coupling of the medical device could comprise a releasing lever for releasing the object. The lever could be connected to latching members which could be spring loaded from the rear in order to secure the groove of the object.

Another feature that could be included in the various embodiments disclosed herein is that the medical device could be adapted to enable the changing of objects in the coupling from a surgical glove to a multi-port comprising endoscopic ports. To be able to quickly switch from an endoscopic system to a hand access or manual manipulation system could be of major importance if complications arise during the procedure and could remove the need for a traditional conversion from laparoscopic to open surgery, a conversion which inevitably causes problems increasing the risk of complications and/or postoperative care.

Another feature that could be included in the various embodiments disclosed herein is that the chamber could be adapted to, after an incision has been created in the skin of the patient, be in fluid connection with a cavity within the patient's body via the incision in the skin of the patient.

Another feature that could be included in the various embodiments disclosed herein is that the medical device could be adapted to hold a pressure within the chamber exceeding 1 bar. The medical device could be enable to hold a pressure in the chamber exceeding 1 bar by means of an adhesive fixating the medical device to the skin of the patient. The medical device could be adapted to be positioned in connection with the skin of the patient, for creating a substantially airtight seal between the wall of the medical device and the skin of the patient prior to the incision in the skin of the patient which creates the fluid connection between the cavity within the patient and the chamber.

Another feature that could be included in the various embodiments disclosed herein is that the medical device is adapted to enable manual manipulation within the chamber by the wall forming the chamber to have a volume larger than 500 000 mm3.

Another feature that could be included in the various embodiments disclosed herein is that the medical device could comprise a vacuum sealing member adapted to hold a pressure less than atmospheric pressure between the wall and the patient's skin to seal between the wall and the patient's skin. The vacuum sealing member could be fixated to the patient by means of a vacuum affecting the patient along a vacuum groove. The vacuum groove could in turn be connected to a vacuum pump by means of a vacuum conduit. This construction enables maintaining of the pressure within the chamber and in the sterile environment above 1 bar by means of for example a pressurized fluid in a pressurized fluid tank delivered to the chamber through the fluid conduit.

Another feature that could be included in the various embodiments disclosed herein is that the medical device could further comprise an opening between the chamber and the outer environment for allowing passage of objects between the chamber and the outer environment.

Another feature that could be included in the various embodiments disclosed herein is that the medical device could additionally comprises a pressure sealing adapted to be inflated for pressing against the skin of the patient and thereby sealing between the sterile environment within the medical device and the outside environment. The pressure sealing could for example be inflated though a fluid conduit connected to a pressure source, in this embodiment a pressurized tank. The wall of medical device could for example be fixated to the patient by means of an adhesive adapted to withstand the force created by the inflated pressure sealing.

Another feature that could be included in the various embodiments disclosed herein is that the fixation of the wall could be assisted or replaced by a band pressing the wall of the medical device against the skin of the patient. The band could be required if the pressure needed to seal between the skin if the patient and the wall needs to be particularly large, which for example could depend on the positioning of the medical device.

Another feature that could be included in the various embodiments disclosed herein is that the medical device could comprise a detachable port arrangement, which could be positioned in the chamber of the medical device. The port arrangement includes an intra body ring adapted to be placed at the inside of the patients skin around an incision, and an outer ring adapted to be placed on the outside of the patients skin around the incision. Between the intra body ring and outer ring an annular retractor membrane is positioned adapted to exert a pressure on the skin or human tissue at the incision to seal between the rings and the skin or human tissue and additionally for retracting the skin and thereby keep the incision open. The retractor membrane may be made from a resilient or elastic polymer material. The outer ring may comprise an integrated roll up system, which may be spring loaded and adapted to create a suitable pressure on the retractor membrane to keep the incision open.

The port arrangement may also include a coupling having a rigid annular seating attached to the outer ring, spring loaded latching members arranged in radial bores in the seating and a hand lever connected to the latching members to enable retraction thereof against the action of springs. The port arrangement may further include a self sealing body port held by the coupling. The body port may comprise a disc-shaped self sealing membrane fixated to a rigid ring having an outer circumferential groove that receives the latching members of the coupling. The self sealing membrane has a small self sealing hole centrally in the membrane. For example, the self sealing membrane may be made from a gel-type material being elastic enough to enable a deformation large enough for a person's hand to pass through the small self sealing hole while still contracting enough to create an airtight seal between the chamber and the outside environment.

The coupling enables quick exchange of the body port. Thus, the surgeon may release the body port from the port arrangement by simply pulling the hand lever so that the latching members disengage from the groove of the body port, whereby the body port can be removed.

Another feature that could be included in the various embodiments disclosed herein is that the medical device could comprise an upper and a lower coupling for fixating ring shaped objects which for example could be insets or ports, where insets for example could be surgical gloves or holding devices and ports could be endoscopic ports or hand access ports, such as self sealing ports. The coupling could comprise a releasing lever for releasing the object.

Another feature that could be included in the various embodiments disclosed herein is that the medical device could comprise a valve member adapted to close the opening in the upper coupling when the upper coupling is not in use. The upper coupling is for example used for manual manipulation and preparation within the medical device, such that could be needed for preparing or changing an instrument or preparing an implant for implantation. The lower coupling could for example be used for laparoscopic manipulation within the body of the patient, or manual manipulation for e.g. removing a specimen or positioning an implant.

Another feature that could be included in the various embodiments disclosed herein is that an inset, for example a surgical glove, could be fixated to the lower coupling.

Another feature that could be included in the various embodiments disclosed herein is that the medical device could have a coupling integrated in the wall. The coupling enables the changing of objects from for examples ports to insets in form of surgical gloves or holding members. The coupling could comprise a valve member for closing the hole in the coupling when objects should be exchanged such that the sterile environment remains sterile. The holding device could for example be adapted to hold surgical instruments and/or implants or parts of implants. The wall of the medical device is according to this embodiment adapted to be fixated to the skin of the patent by the vacuum sealing.

Another feature that could be included in the various embodiments disclosed herein is that at least a portion of the wall of the medical device could be partially collapsible, and the collapsible wall portion could be adapted to be inflated by a gaseous fluid for defining the chamber. The collapsible wall portion could be inflated by a pressurized fluid entering the chamber through a fluid conduit. The inflation of the wall, in which a window is integrated, positions the window in a position suitable for viewing the procedure being performed on the patient. The inflation of the collapsible wall portion could continue until the pressure in the chamber exceeds 1 bar, which is required in some applications.

Another feature that could be included in the various embodiments disclosed herein is that the medical device could be used in surgical procedures where transitions between open and laparoscopic surgery is needed. A portion of the wall of the medical device could be displaceable between a first position and a second position, where the medical device has an upper coupling and a lower coupling separated from each other, when the wall is in the second position for enabling a larger freedom of motion for the surgeon within the chamber of the medical device, whereas the upper coupling is locked to the lower coupling, when the wall is in the first position, which could enable the use of a laparoscopic trocar or hand access for further reach within the body of the patient. The medical device could further be used in embodiments where manual manipulation in a sterile environment is required, e.g. for preparing a medical device for use in the surgical procedure. Other applications could for example be that the surgeon wishes to remove a specimen from the body of the patient after a laparoscopic procedure has been concluded. The laparoscopic procedure could in these instances be performed with the wall of the medical device being in the first position, being close to the skin of the patient, whereafter the wall could be displaced into the second position for enabling the specimen to be removed from the body of the patient and placed in the chamber of the medical device without contacting the outside environment, and without the release of the pressure that usually fills the abdomen in laparoscopic procedures. The lower coupling could comprise a lever for releasing and/or locking the upper coupling to the lower coupling.

Another feature that could be included in the various embodiments disclosed herein is that an object comprising an airlock could be coupled to the wall of the medical device, for example by means of a coupling. The airlock can be adapted for transferring objects between the outer environment and the chamber of the medical device. The airlock could comprise an inner wall and an outer wall on each side of the airlock enabling the enclosing of an object within the airlock. Another example of an object to which the surgical glove can be exchanged is a port such as a laparoscopic port for performing a laparoscopic procedure.

Another feature that could be included in the various embodiments disclosed herein is that the upper and/or lower coupling could comprises a valve member for closing the opening in the coupling when the objects should be exchanged. The valve member could for example be a flap valve.

Another feature that could be included in the various embodiments disclosed herein is that the wall of the medical device could be placed in contact with the skin of the patient prior to the beginning of the operation and the entire portion of the wall contacting the skin of the patient could comprise an adhesive, such that an incision in the patient runs through the wall and further through the skin of the patient. This reduces the risk that infectious objects can be transferred from the skin of the patient to the incision in the patient if the skin of the patient is not adequately disinfected. The body of the patient could be inflated to allow better visibility within the body from for example a fluid conduit connected to a pressure tank. However, it is equally conceivable that the body of the patient is inflated from within trough another incision in the body of the patient.

Another feature that could be included in the various embodiments disclosed herein is an object coupled to the medical device could be exchanged by a plurality of different objects while the wall of the medical device is locked in the first position, i.e. with the upper coupling locked to the lower coupling. For example, an integrated surgical glove could be exchanged by an airlock comprising a first valve member separating the airlock from the sterile environment, and a second valve separating the airlock from the environment outside the chamber. The airlock space provided between the first and second valves could for example be used for passing a medical instrument or device into the sterile environment, or for passing a specimen or sample out of the sterile environment, or exchanged by a holding member which for example could be adapted to hold surgical instruments and/or parts of an implant, or exchanged for an endoscopic port unit comprising endoscopic ports. The medical device could according to this embodiment be adapted to be fixated to the skin of the patient by a large portion of the wall of the medical device being adhesive, and by means of the vacuum sealing.

Another feature that could be included in the various embodiments disclosed herein is that the medical device could comprise a retainer placed in the skin of the patient for retaining an opening enabling the passing from the chamber of the medical device to a cavity in the body of the patient. The opening enables a pressure being larger than the pressure of the external environment to be present in the in both the cavity of the patient and in the chamber of the medical device, which enables a semi-laparoscopic procedure to be performed both using traditional trocars and by means of hand access through the use of the medical device. The retainer could be a retainer disclosed herein, or could be a different retainer including traditional retainers using hooks. For manual manipulation within the medical device and/or the body of the patient a surgical glove is attached to the wall of the medical device, for example by means of an upper coupling comprising a releasing lever, both further described throughout the application.

Another feature that could be included in the various embodiments disclosed herein is that the chamber of the medical device could have a volume being larger than 500cm3 for enabling the manipulation of objects within the chamber and/or placing objects of considerable size within the chamber.

The different aspects or any part of an aspect or different embodiments or any part of an embodiment may all be combined in any possible way. Any method or any step of method may be seen also as an apparatus description, as well as, any apparatus embodiment, aspect or part of aspect or part of embodiment may be seen as a method description and all may be combined in any possible way down to the smallest detail. Any detailed description should be interpreted in its broadest outline as a general summary description, and please note that any embodiment or part of embodiment as well as any method or part of method could be combined in any way.

### Brief description of drawings

The invention is now described, by way of example, with reference to the accompanying drawing, in which:
Fig.1a - 1c shows the medical device when applied to a leg of a patient,
Fig.1d shows the medical device when applied to an arm of a patient,
Fig. 2a shows a vacuum seal in detail,
Fig. 2b shows an air evacuation pump, in detail,
Fig. 2c shows a pressure sealing in detail,
Fig. 2d shows an adhesive sealing,
Fig. 2e shows an embodiment of the medical device comprising an elongated tubular enclosure,
Fig. 2f shows an embodiment of the medical device comprising an elongated tubular enclosure, in cross-sectional view.
Fig. 2g shows an embodiment of the medical device comprising an elongated tubular enclosure, when inflated,
Fig. 2h shows an embodiment of the medical device comprising an elongated tubular enclosure, when a leg of the patient has been inserted into the elongated tubular enclosure.
Fig. 2i shows an embodiment of the medical device when the elongated tubular enclosure fits snuggly around the leg of the patient.
Fig. 3a shows an embodiment of a medical device, in perspective,
Fig. 3b - 3e shows embodiments of a medical device, in perspective,
Fig. 4a - 4c shows embodiments of a self sealing port,
Fig. 5a shows an embodiment of the medical device, in section,
Fig. 5b shows a coupling, in section,
Fig. 5c shows the exchange of objects in a coupling,
Fig. 5d - 5e shows a coupling in the wall of the medical device,
Fig. 5f - 5g shows the medical device when used,
Fig. 5h - 5i shows embodiments of the medical device, in section,
Fig. 5j - So shows the medical device when used,
Fig. 5p shows an embodiment of the medical device, in section,
Fig. 5q - 5r shows embodiments of a medical device, in perspective,
Fig. 5s - 5u shows embodiments of the medical device, in section,
Fig. 6a - 6g shows an embodiment of the medical device being fixated to the patient and used,
Fig. 6h - 6i shows embodiments of the medical device, in section,
Fig. 7 shows another embodiment of the medical device, in section,
Fig. 8, 9a, 9b, 10 and 11 shows an embodiment of the medical device comprising a vacuum sealing and a coupling to which objects could be connected,
Fig. 12 shows an embodiment of the medical device, in section,
Fig. 13 shows an embodiment of the medical device in section,
Fig. 14 - 16 shows another embodiment of the medical device, in section,
Figs. 17a - 17b shows an embodiment of the medical device in section, being fixated to the patient and used,
Fig. 18a - 18k shows alternative embodiments of the medical device, when fixated to the abdomen of the patient.
Fig. 19 shows an embodiments of the medical device, in section,
Fig. 20 shows the medical device when applied to the abdomen of a patient,
Fig. 21a - 29b shows different embodiments of the medical device in perspective, when applied to the body of the patient,
Fig. 30 - 32 shows different embodiments of the medical device in perspective, when applied to the body of the patient.
Fig. 33 shows an embodiment of a medical device, in perspective,
Figs. 34a - 34j shows an embodiment of the medical device, comprising pressure or vacuum sealing members being connected to monitoring units and/or adjustment devices,
Fig. 35 shows an embodiment of the medical device applied on an extremity of the patient,
Fig. 36 shows an embodiment of the medical device applied on an extremity of the patient, comprising a fluid circulating system,
Figs. 37a - 39 shows embodiments of the medical device comprising information and/or energy transferring members,
Fig. 40a - 40c shows an embodiment of the medical device comprising an integrated trocar,
Fig. 40d shows an embodiment of the medical device when being used in a surgical procedure,
Figs. 40e - 40g" shows different embodiments of trocars adapted to be used in combination with a medical device,
Fig. 40h, 40h' shows an embodiment of the trocar when being used with a medical device,
Fig. 40i - 40l shows an embodiment of an encapsulated trocar, when used,
Fig. 40m - 40q‴ shows embodiments of trocars and medical devices when used in combination,
Fig. 40q"" shows an alternative embodiment of a combination of a trocar and a medical device,
Figs. 41a - 43b are flow charts describing methods adapted to be performed using the medical devices according to the different embodiments presented herein.
Figs. 44a - 49c shows embodiments of a medical device adapted to be sealed and fixated to the tissue or an organ on the inside of the body of the patient.
Figs. 50a - 53 shows embodiments of a medical device adapted to be sealed and fixated to the tissue or an organ of the body of the patient, such that a chamber is created on the inside of the body of the patient.

### Detailed description

A medical device is provided which is adapted to be fixated and/or sealed against the body of a patient by means of a vacuum sealing device. The vacuum sealing device is adapted to apply a pressure below atmospheric pressure (a negative pressure) between a portion of the inside of the body of the patient, i.e. inside the skin of the patient, and the medical device. The negative pressure creates a suction which presses the portion of the inside of the patient's body against the vacuum sealing device, thereby creating a sealing connection between the portion of the inside of the body of the patient and the medical device. The vacuum sealing device could for example be adapted to connect to and seal against muscle tissue, muscle fascia, fat tissue, the inside of the patient's skin, subcutaneous tissue, the surface of a section made in the body of the patient, a bodily organ, or fibrotic tissue. Muscular tissue suitable for connection and sealing by the vacuum sealing device for example includes the muscles of the abdomen, such as the inside or outside of the rectus abdominis, the inside or outside of the transverse abdominal muscles, the inside or outside of the internal oblique muscle, the inside or outside of the external oblique muscle, or a surface of a section of any of the muscles above. Muscular fascia suitable for connection and sealing by the vacuum sealing device for example includes the transversalis fascia, the internal oblique fascia, the external oblique fascia, the rectus abdominis fascia and the parietal fascia. Fat tissue suitable for connection and sealing by the vacuum sealing device for example includes extraperitoneal fat tissue and subcutaneous fat tissue. A bodily organ could for example be an organ of the gastrointestinal system; such as the esophagus, the stomach, the small or large intestine, or the anus, an organ may additionally be the gall bladder or bile duct, or an organ of the urinary system; such as the urethra, the urinary bladder, the ureter or the kidneys of the patient.

As the vacuum sealing member connects to and seals against a portion of the patient's body inside the patient's body, the surface of the skin can be kept unaffected, which means that no hematomas or discoloring of the skin will occur. In addition, the superficial blood flow of the skin of the patient is maintained substantially unaffected, reducing the risk of ischemia in portions of the skin.

The medical device could be fixated to the body of the patient by means of the vacuum sealing device, or by means of an additional holding member, which for example could be adapted to fixate the medical device by squeezing or clamping a portion of the skin and/or muscular and/or fascia layer, for example the abdominal wall.

The medical device provided herein could in its different embodiments be used in endoscopic or open surgery, or a combination of endoscopic and open surgery to allow hand access or insertion or removal of objects or instruments from the surgical area. In some embodiments the medical device could be adapted to be used in arthroscopic surgery, in which case the medical device could comprise one of more chambers adapted to be filled with a liquid. The sealing properties of the medical device could for example be used as a means for creating a completely sterile and closed surgical environment within the body of the patient, outside the body of the patient, or reaching between the inside and outside of the body of the patient. The sterile and closed surgical environment may be used in open surgery, endoscopic surgery, arthroscopic surgery or in a mix thereof. The medical device could thus be used as a miniature operating theater in conditions where a proper operating thereafter is not available. The medical device could in instances in which it is used as a miniature operating theater be adapted for a specific surgical procedure, in which case the objects and/or instruments needed in that particular surgery could be pre-placed within the medical facilitating shipping and handling and keeping the objects and/or instruments sterile at all time. Additionally, the medical device provided herein could provide a possibility to combine any of traditional laparoscopic surgery and/or single incision laparoscopic surgery and/or natural orifice transluminal endoscopic surgery and/or hand-assisted laparoscopic surgery and/or open surgery and/or combined multiport laparoscopic surgery as well as arthroscopic surgery could be combined with open joint surgery with single or multiports.

The medical device provided herein could be adapted for and used with benefit in for example appendectomy, cholecystectomy, nephrectomy, hysterectomy, oophorectomy, adrenalectomy, gastric bypass, Nissen fundoplication, intestinal shunts, hernia repair, splenectomy, colon or small intestine resection, liver resection, for creating a cecostomy, colostomy, duodenostomy, Ileostomy, appendicostomy, esophagostomy, gastrostomy, urostomy, nephrostomy, ureterostomy, vesicostomy. Endoscopic and open surgery could be combined as well as arthroscopic and open joint surgery could be combined and for example also include cryoablation or ultrasound knife technology. In arthroscopy, the medical device could be used for the removal of separated bone or cartilage tissue or the insertion of ligaments or fixation elements such as screws, nails, plates, prosthetic joints or elements for lubrication. Furthermore, any type of cancer surgery could when applicable use the medical device. The medical device also provides the opportunity to insert any type of medical device or other foreign parts into the body still combined with laparoscopic or arthroscopic surgery.
The medical device provided herein could in its different embodiments be used as a complement to endoscopic surgery, such as laparoscopic, gastroscopic and arthroscopic surgery, to allow hand access or insertion or removal of objects or instruments from the surgical area inflated. The medical device could further be used in preparation for a possible conversion from endoscopic to open surgery, for reducing complications associated with such conversion. In some embodiments the medical device could be used with a circulating liquid in for example arthroscopic surgery. In some embodiments the medical device could be used as a means for creating a completely sterile and closed surgical environment to be used in open surgery, endoscopic surgery or a mix thereof. The medical device could thus be used as a miniature operating theater in conditions where a proper operating thereafter is not available. The medical device could in instances in which it is used as a miniature operating theater be adapted for a specific surgical procedure, in which case the objects and/or instruments needed in that particular surgery could be pre-placed within the medical facilitating shipping and handling and keeping the objects and/or instruments sterile at all time. Embodiments disclosed herein enable surgical procedures to be performed in a more sealed environment which reduces the risk of bacterial and viral infections. Creating the proper environment for surgical procedures is expensive, time consuming and an environmental burden as most of the sterile products etc. used during a surgical procedures are deposable. The operating theaters are generally equipped with special air filtering ventilation systems reducing the number of particles in the operating environment. Even if the operating theaters are properly sterilized, the air is properly filtered and the medical staff uses proper protective clothing the size of the area which needs to be sterilized in combination with the passing of medical staff in and out of the operating theater risks of errors significant. A normal surgical procedure involves as much as ten different staff members, performing the surgery, assisting in the surgery, being tutored or being involved in the anesthesia. Several of these members of the medical staff needs to move from the controlled environment of the operating theater to less controlled environments outside of the theater as they may be involved in parallel surgical procedures or need to prepare or coordinate subsequent procedures. By sealing the operating field closer to the body of the patient, the area which needs to have the highest level of sterility can be made smaller, which also reduces the number staff that needs to be in direct contact with the most sensitive areas of the surgery and eliminates the need for any medical staff to pass from the most sensitive areas of the surgery to the outside of the operating theater.

In surgical procedures requiring the removal of a material from within the human body, this procedure is preferably done with minimally invasive surgery. Minimally invasive surgery is advantageous since the risk of infections is less with smaller incisions, the time for recovery is shorter and the scars remaining after the procedure are smaller. When for example removing a section of the intestinal system it is sometimes unclear how much of the intestine that needs to be removed and where the intestine in need of removal is located. This could in most cases be determined through optical inspection using a fiber optic camera, well known in the art of laparoscopy and arthroscopy, placed in a trocar inserted through a small incision in the body of the patient. For enabling optical inspection the cavity within the body needs to be created, which is typically done by means of pressurized CO2 gas being introduced through the trocar. Returning to the removal a section of the intestinal system this removal is confined by the size of the laparoscopic trocars, typically not being larger than φ18mm. The medical device provided herein could provide a possibility to combine any of traditional laparoscopic surgery and/or single incision laparoscopic surgery and/or natural orifice transluminal endoscopic surgery and/or hand-assisted laparoscopic surgery and/or open surgery. In surgical procedures where a portion of the intestinal system temporarily is placed outside of the body of the patient, these parts of the intestinal system needs to be kept humid at all time to reduce the forming of scar tissue after the surgical procedure is concluded. By keeping the intestinal portion in the chamber of the medical device disclosed herein, the intestines can be kept in a controlled, sealed, tempered and humid environment which reduces the risk that complicating scar tissue is formed or the risk that the patient gets post operative problems with infections etc..

In hand assisted surgery the surgeon can insert a hand through a small incision in the abdomen. Hand assisted surgery has the benefit of providing sensory perception and the possibility to guide the surgical instruments whilst maintaining the possibility of visually observing the entire procedure on a TV screen overhead. Furthermore, it enables the removal of large organs intact, for example making it possible to evaluate cancer. Hand assisted surgery could also be beneficial to surgeons who are still learning laparoscopic techniques. The medical device may preserve the main idea of Minimal Access Surgery (MAS) and enhance the safety and efficiency of MAS by allowing the completion of the operation with a hand inside the body of the patient. The medical device makes it possible to maintain the intra-abdominal pressure to facilitate the better view and magnification of an endoscopic camera.

In the following a detailed description of embodiments of the invention will be given with reference to the accompanying drawings. It will be appreciated that the drawings are for illustration only and are not in any way restricting the scope of the invention. Thus, any references to directions, such as "up" or "down", are only referring to the directions shown in the figures. It should be noted that the features having the same reference numerals have the same function, it should further be noted that features in the different embodiments having the same last two digits are similar i.e. 74, 474 and 774. A feature in one embodiment could thus be exchanged for a feature from another embodiment having the same last two digits unless clearly contradictory. The descriptions of the similar features having the same last two digits should thus be seen as complementing each other in describing the fundamental idea of the feature and thereby showing the features versatility.

Endoscopy is to be understood as any form of key-hole surgery or minimally invasive surgery using endoscopic instruments. Endoscopy for example includes laparoscopy including Single Incision Laparoscopic Surgery (SILS), arthroscopy, Natural Orifices Translumenal Endoscopic Surgery (NOTES) for example including gastroscopy and proctoscopy and hand-assisted endoscopic surgery. Endoscopic surgery could be used in many different cavities in the human body such as but not limited to abdomen, thorax, joints, and any by the surgeon created cavity for example such as during hernia repair or pelvic surgery.

Inset is to be understood as an indentation in the wall, or a part which could be inserted into the wall of the medical device for making up part of the wall. An insert may for example be a circular object which may be locked to a coupling or opening in the wall of the medical device, or integrated in the wall of the medical device. An inset could for example comprise an integrated glove, or a device or instrument mount for holding for example an implant. The inset, when positioned in the wall of the medical device, keeps the environment in the chamber, enclosed by the wall, sealed, and thus is not possible to pass i.e. objects cannot be transferred through the inset.

Vacuum, when used for providing sealing properties is to be understood as negative pressure i.e. pressure below atmospheric pressure and thus providing a suction to the area on which it is applied, it is not be understood as an absolute vacuum.

A medical implant may for example be an arthroplastic prosthesis, a heart assisting device, and energized implant, control logic, a filling esthetical implant, implantable medicament dispenser, a powering unit, a vascular implant, an urological implant, an abdominal implant, a drug-releasing implant, a gynecological implant or any other type of active or passive implants.

An instrument, surgical instrument or endoscopic instrument may for example be a scalpel, at trocar, tweezers, a suturing instrument, scissors, a camera, a clamping instrument, a dissecting instrument, a gripping instrument, a bonding instrument, such as a suturing or stapling instrument, or a severing instrument, such as a scalpel or an electromagnetic or ultrasonic diathermy, it may be a laparoscopic grasper, a laparoscopic claw grasper, a laparoscopic stone grasper, a laparoscopic needle holder, a laparoscopic hook, a laparoscopic dissector, or a laparoscopic diathermy instrument. Many different kind of instruments used for arthroscopy or joint replacements as well as any orthopedic type of instrument or machine are other examples.

Physiological or physical parameter could for example but not limited to be at least one of any kind of; pressure, force, time, movement, stretching, distance, volume, the blood pressure of the patient, the blood flow of the patient, the saturation of the blood of the patient, or an ischemia marker such as lactate, the temperature at the skin of the patient, the skin tone etc.

Functional parameter of the device could for example but not limited to be at least one of any kind of; pressure, force, time, movement, stretching, distance, volume, electrical parameter, energy, energy balance, voltage, current, any digital or analog sensor input and temperature.

The reference numerals in the appended figures refer to the following elements, in the listing below examples are recited, however, they are only to be seen as examples of how the elements may be implemented and not to be understood as limiting the scope of the term:
A = Ambient environment, the environment outside o the surgical environment enclosed or partially enclosed by the chamber of the medical device or within the body of the patient.
C = Chamber
F = Fat
I = Incision
L = Leg
M = Muscle
O = Object
P = Peritoneum
R1, R2 = Position 1, Position 2
S = Skin
ST = Subcutaneous tissue
T = Tumor
VP = Vacuum Perforations
0 = Medical device
1 = Wall, e.g. a layer of the medical device enclosing a chamber. The wall can be a rigid wall or an flexible, elastic and/or inflatable wall. The wall could be transparent, translucent and/or gas tight and may be made from a transparent polymer material, such as polyvinyl chloride (PVC) with a plasticizer additive. The wall may be a rigid transparent material for example glass or a transparent polymer material such as an acrylic glass, a polycarbonate, polyethylene terephthalate, an acrylic fiber material or a copolymer containing polyacrylonitrile.
2 = Glove, e.g. denotes an indentation in the wall of the medical device enabling manual manipulation by medical staff from the outside of the ambient environment or from a second chamber.
3 = Pleated section, e.g. denotes a flexible wall portion comprising a plurality of creases enabling the extension of the material of the wall. The pleated portion may additionally be elastic.
4 = Enclosure
5 = Sealing device, e.g. denotes any sealing or fixating element or combination of elements together performing a sealing or fixating function.
5'= Vacuum sealing member, e.g. denotes an element involved in providing sealing by means of maintaining a pressure below atmospheric pressure.
5"= Pressure sealing member, e.g. denotes an element involved in providing sealing by means of pressing against the an element, which could involve maintaining a fluid pressure above atmospheric pressure.
5"'=Adhesive sealing member, e.g. denotes an element involved in providing sealing by means adhesion.
6 = Vacuum conduit, e.g. a flexible polymer conduit designed such that it does not collapse when transferring a pressure being lower than the pressure outside the conduit. The conduit may be re-enforced by for example by annular metallic ring-shaped elements, such that the conduit maintains its flexibility.
7 = Separating layer, e.g. a layer having a non-stick surface such that it can be removed from an adhesive surface. The non-stick surface could for example comprise a PTFE coated surface.
8 = Vacuum pump, e.g. a pump adapted to create a pressure below atmospheric pressure such that air from a chamber can be evacuated, could for example be in the form of a piston or membrane pump.
9 = Fluid pump, e.g. any pump capable of moving a liquid or gaseous fluid, could for example be in the form of a piston or membrane pump.
10 = Fluid conduit, e.g. any conduit capable of transferring a liquid or gaseous fluid, could for example be a flexible polymer tubing.
11 = Chamber, e.g. any enclosed space.
12 = Tank.
13 = Pneumatic system, e.g. a system for handling pneumatic fluid which could have a pressure different from the atmospheric pressure. The pneumatic system may be a system for powering pneumatic tools or inflating a chamber. The pneumatic system may for example comprise pneumatic/fluid conduits, valves, gauges, pressure sensors, sterilizing/filtering/tempering/humidifying units, pumps and/or tools consuming pneumatic energy.
15 = Control/monitoring system, e.g. a gauge o valve for monitoring or controlling a fluid flow or pressure.
16 = Tool e.g. a powered tool for surgical use, such as an orthopedic drill, saw, reamer, stapler etc.
17 = Wall port, e.g. any closable/openable port placed in a wall such that an object may be transferred from one side of the wall to the other side of the wall through the port. The wall port may for example comprise a self sealing silicon or gel membrane or an iris or flap valve.
18 = Airlock sluice, e.g. any element enabling transfer of an object between a first and second area via a sluice chamber such that some form of closing is provided between the first and second area at all times while the object is transferred.
19 = Zipper e.g. a classic zipper, a sealed classic zipper or a Ziploc^{®} type zipper.
20 = Instrument and/or device mount, e.g. denotes any mount or holding device capable of holding objects which could be necessary in or around a surgical procedure.
21 = Window, e.g. any transparent sheet material.
22 = Body port, e.g. any closable/openable port placed in the body such that an object may be transferred from outside the body to a cavity within the body through the port. The body port may for example comprise a self sealing silicon or gel membrane or an iris or flap valve.
23 = Elastic element, e.g. a flat elastic sheet material for example made from a polymer material or glass or carbon fiber.
24 = Guide slot, e.g. a slot in the wall of the medical device made from the same sheet material as the wall and adapted to guide the elastic element.
25 = Lifting member, e.g. a polymer wire adapted to connect the bottom portion of the wall of the medical device and the top portion of the wall of the medical device for lifting the bottom portion.
26 = Sterilizing/filtering/tempering/humidifying unit, e.g. a combined unit adapted to enable sterilization, filtering, tempering and humidification of a passing gaseous or liquid fluid passing the unit.
26a = Sterilizing unit, e.g. using a sterilizing method which may comprises the use of heat, such as electrical or chemical heat and/or irradiation, such as UV-light and/or though the addition of a chemical sterilizing agent.
26b = Filtering unit, e.g. unit comprising a mechanical filter, such as a filter textile, and/or a chemical filter such as activated carbon, or by a combination of a mechanical and chemical filters.
26c = Tempering unit, e.g. a unit comprising a heating element, such as an electrical or chemical heating element, or a cooling element, such as a refrigerator element or a chemical cooling elements such as liquid hydrogen or dry ice.
26d = Humidification unit, e.g. a unit adapted to increase the humidity of a passing gaseous fluid by for example introducing water particles or mixing the fluid with vaporized water.
27 = Circulating pump, e.g. any pump capable of moving a liquid or gaseous fluid, could for example be in the form of a piston or membrane pump.
28 = System for fluid circulation, e.g. a system for handling a circulating fluid. The system for fluid circulation may for example comprise fluid conduits, valves, gauges, pressure sensors, sterilizing/filtering/tempering/humidifying units and fluid pumps.
29 = Fluid conduit, e.g. any conduit capable of transferring a liquid or gaseous fluid, could for example be a flexible polymer tubing.
30 = Inlet, 30' = Inlet in body
31 = Outlet, 31' = Outlet in body
32 = Spring, e.g. an elastic member adapted to provide an elastic action when force is applied, such as a metal or polymer helical spring.
33 = Protruding member 33' Magnet
34 = Recess / Groove
35 = Intra body ring, or inner retractor member, e.g. a ring adapted to be positioned inside the body of a patient, for example subcutaneously.
36 = Connecting member, e.g. a flexible and/or elastic sheet material adapted for sealing and/or connecting and/or retracting.
37 = Outer ring, or outer retractor member e.g. a ring adapted to be positioned outside the body of a patient, for example in connection with the skin.
38 = Valve member, e.g. adapted to close an opening or hole, may for example comprise a flap valve.
39 = Lever
42 = Inset, e.g. denotes any exchangeable portion or any indentation or projection.
42' = Wall inset, e.g. an inset provided in a wall portion.
42" = Body inset, e.g. an inset adapted to be provided in the body of a patient.
43 = Self sealing membrane, e.g. denotes a membrane adapted to have flexible or elastic properties such that the membrane can allow transfer of an object therethrough and afterwhich the membrane closes by itself by means of its flexible and/or elastic properties.
44 = Self sealing hole, e.g. a hole in a membrane adapted to seal by itself when no external force affects the membrane.
45 = Seating
46 = Roll up system, e.g. a manually operated, sprig loaded or powered system for rolling a sheet material.
47 = Interconnected port, e.g. denotes a closeable/openable port created by the connection of at least two ports, such as for example a body port and a wall port. first and
48 = Multiport, e.g. a closeable port or port unit comprising a plurality of closeable ports or port units
48' = Wall multiport, e.g. a multiport adapted for positioning in a wall portion.
48" = Body multiport, e.g. a multiport adapted for positioning in the body of a patient.
49 = Support, e.g. denoted any structural element adapted to absorb or transfer force.
50 = Adhesive surface, e.g. a surface of a sheet material comprising an adhering substance or composition such that the surface adheres to other objects. The substance or composition may for example be a pressure sensitive adhesive such as 3M's pressure sensitive adhesive Scotch-Grip 4268-NF.
51 = Motor, e.g. an electric, pneumatic, hydraulic or combustion motor adapted to create or transfer mechanical work.
52 = Kinetic energy transferring member, e.g. any element adapted to transfer kinetic energy of some form.
53 = Kinetic energy coupling, e.g. any coupling capable of transferring kinetic energy from a first kinetic energy transferring member to a second kinetic energy transferring member.
54 = O-ring
55 = Information transferring member, e.g. any element adapted to transfer information of some form, for example data.
56 = Fluid coupling, any coupling capable of handling the transfer of a gaseous or liquid fluid.
57 = Magnet
58 = Electric energy coupling, e.g. any element adapted to transfer electric energy, the electric energy coupling may be a standard electric coupling or a coupling for transferring wireless energy.
59 = Sleeve
60 = Tool holder, e.g. a chuck for gripping a drilling or milling tool.
61 = Window contacting member, e.g. a member comprising a soft polymer element for wiping a window.
62 = Fluid conduit (for window cleaning)
63 = Nozzle (for window cleaning)
64 = Fluid arrangement (for window cleaning)
65 = Camera, e.g. an endoscopic camera, such as a camera used in laparoscopic or arthroscopic procedures. The camera may be a camera based on fiber optic technology.
66 = Coil
67 = Pressure creating member
68 = Bellows, e.g. a chamber having a wall with a pleated structure.
69 = Objects, e.g. medical device such as implants or medical instruments, such as instruments used in a surgical procedure.
70 = Incision retractor
71 = Inner wall
72 = Connection
73 = Sensing probe
74 = Diode
75 = Detector
76 = Cuff
77 = Covering sheet, e.g. a removable sheet material with the purpose of covering a portion of a medical device.
78 = Lead
79 = Retractor member, e.g. an element adapted to retract the skin around an incision made in the skin of a patient.
80 = Coupling, e.g. a mechanical element adapted to fixate an object, such as an inset or port, or to couple to an additional coupling.
81 = Specimen, e.g. bodily matter which is to be removed from the body of the patient, a specimen could for example be a tumor, a portion of the intestinal system, or an organ such as the appendix or gallbladder of the patient.
82 = Pouch
83 = Key-hole recess, e.g. a recess capable of holding a protrusion when the protrusion is in a first portion and releasing the protrusion when the protrusion is in a second position.
84 = Connecting portion
85 = Bushing
86 = Iris valve
87 = Holding member, e.g. a mechanical element for holding and fixating a medical device to the body of the patient, e.g. by engaging the skin of the patient.
88 = Closing portion, e.g. a magnetic or vacuum operated closing portion adapted to seal a first edge against a second edge.
89 = Instrument, e.g. a surgical and/or endoscopic instrument which may be a gripping instrument, a bonding instrument, such as a suturing or stapling instrument, or a severing instrument, such as a scalpel or an electromagnetic or ultrasonic diathermy, it may be a laparoscopic grasper, a laparoscopic claw grasper, a laparoscopic stone grasper, a laparoscopic needle holder, a laparoscopic hook, a laparoscopic dissector, or a laparoscopic diathermy instrument.
89s = Stapling instrument, e.g. a bonding instrument adapted to bond tissue or skin of the patient by means of staplers.
92 = Trocar, e.g. a hollow element adapted to be inserted into the body of a patient for enabling insertion of an instrument into a cavity in the patient.
93 = Adjustment device
94 = Sealing sub device
95 = Organ, e.g. the esophagus, the stomach, the small intestine, the large intestine, the anus, the gallbladder, the bile duct, the kidney, the renal pelvis, the ureter, the urine bladder, the urethra, the heart, the lung, or the bronchus,
96 = Holding member, e.g. a structural element capable of holding.
97 = Hole
98 = Monitoring unit, e.g. a unit adapted to receive input parameters to be monitored and provide some form of output based on the received input.
99 = Open end, e.g. a hole or opening of an indentation capable of receiving something inserted therethrough.

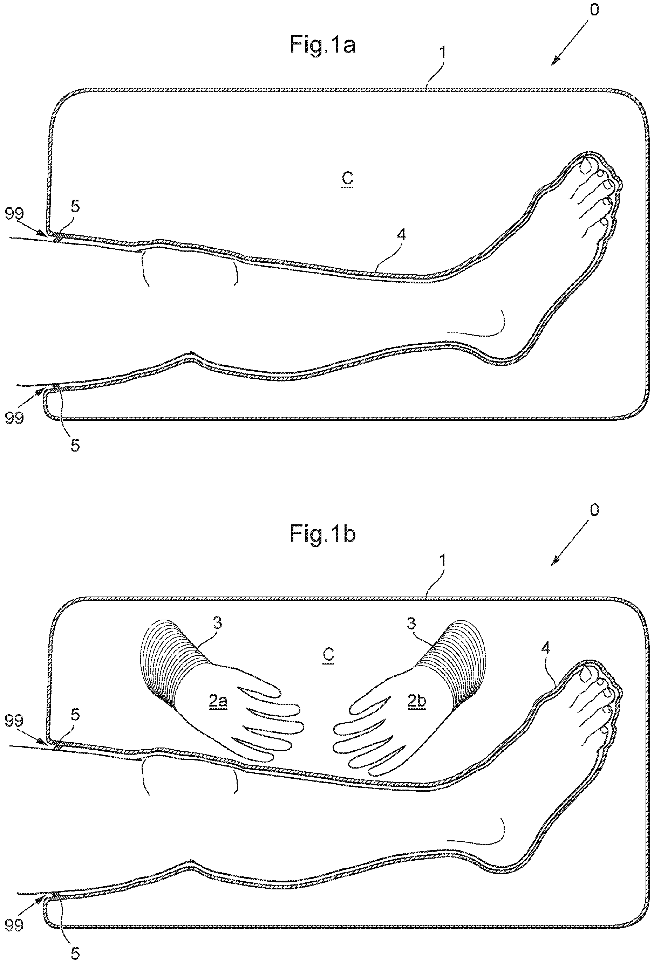

Figs. 1a - 5u shows an embodiment of the medical device being adapted for surgical procedures on an extremity of a patient, such as a leg or an arm of the patient. The medical device could for example be used for performing foot surgery, ankle surgery, knee surgery or fracture surgery involving femur, tibia or fibula. Surgery on the patients arm for example includes hand surgery, elbow surgery shoulder surgery and fraction surgery on for example the scapula, clavicula, humerus, radius or ulna.

Fig. 1a shows an embodiment of a medical device 0 for performing surgical procedures. The medical device 0 comprises an at least partially collapsible wall 1 enclosing a chamber **C** in which a sealed environment can be maintained for performing the surgical procedure. The at least partially collapsible wall 1 comprises a wall portion forming at least one elongated tubular enclosure 4 having an open end 99. The enclosure 4 extends in the chamber **C** and is adapted to fit a portion of an extremity of a patient inserted into the enclosure through the open end thereof. In the embodiment shown in fig. 1a the extremity is a leg of the patient.

The medical device 0 comprises a sealing device 5 encircling the leg of the patient and being adapted to seal between the skin of the patient and the at least partially collapsible wall 1. The sealing device shown in fig. 1a comprises an elastic sealing member, for example comprising an elastic polymer material, exerting a pressure on the skin of the patient and thereby providing the seal. According to the embodiment shown in fig. 1a only the portion of the wall 1 comprising the elongated enclosure 4 is collapsible or deformable, for allowing the insertion of the extremity into the elongated enclosure 4, whereas the rest of the wall 1 enclosing the chamber **C** is rigid, however, in other embodiments it is equally conceivable that the entire wall 1 enclosing the sealed environment is collapsible.

Fig. 1b shows the medical device 0 in an embodiment similar to that of fig. 1a with the difference that the embodiment of fig. 1b further comprises two gloves 2a, 2b integrated in the wall 1 enabling manual manipulation within the sealed environment. The gloves 2a, 2b are connected to the wall 1 by means of pleated sections 3 connecting the gloves 2a, 2b to the wall 1 and enabling free movement of the gloves 2a, 2b within the sealed environment.

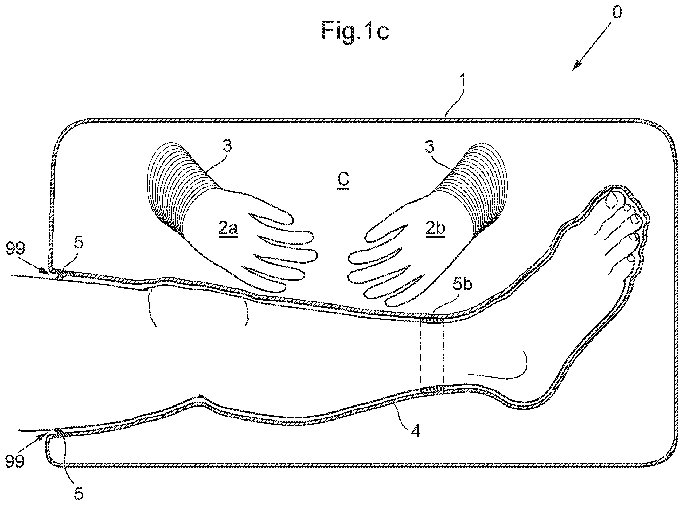

Fig. 1c shows the medical device 0 according to an embodiment similar to the embodiment shown in fig. 1a, with the difference that the medical device 0 comprises a second sealing device 5b is fixated to the wall 1 making up the elongated tubular enclosure 4 encircling the extremity in a more distal location such that the foot of the patient can be isolated, which is advantageous as the foot in very difficult to disinfect. The second sealing device 5b comprises an elastic sealing member exerting pressure of the distal portion of the leg of the patient.

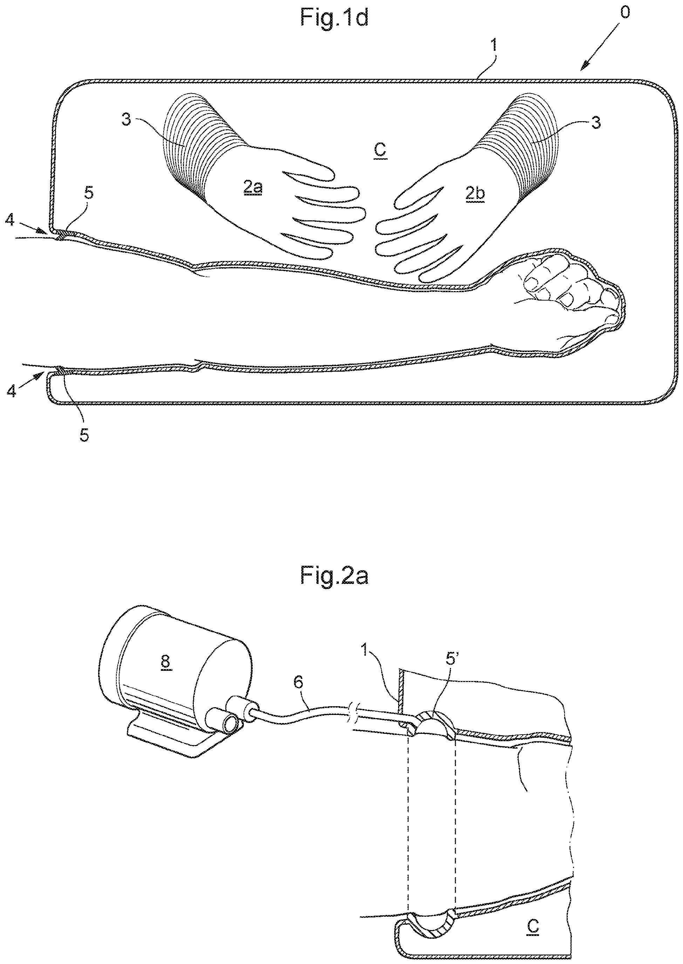

Fig. 1d shows an embodiment of the medical device 0 similar to that of fig. 1b, the difference being that the embodiment in fig. 1d is adapted to receive an extremity in the form of an arm.

Fig. 2a shows a sealing device in detail, in an embodiment in which the sealing device comprises a vacuum sealing member 5' adapted to seal between an extremity of the patient and the partially collapsible wall 1 by the vacuum sealing member 5' being adapted to hold a pressure less than 1 bar (an underpressure) for creating a vacuum seal between the skin of the patient and the medical device. The vacuum is created by a vacuum pump 8 connected to the vacuum sealing member 5' by means of a vacuum conduit 6. The vacuum sealing member 5' could for example be made from a semi-rigid polymer material.

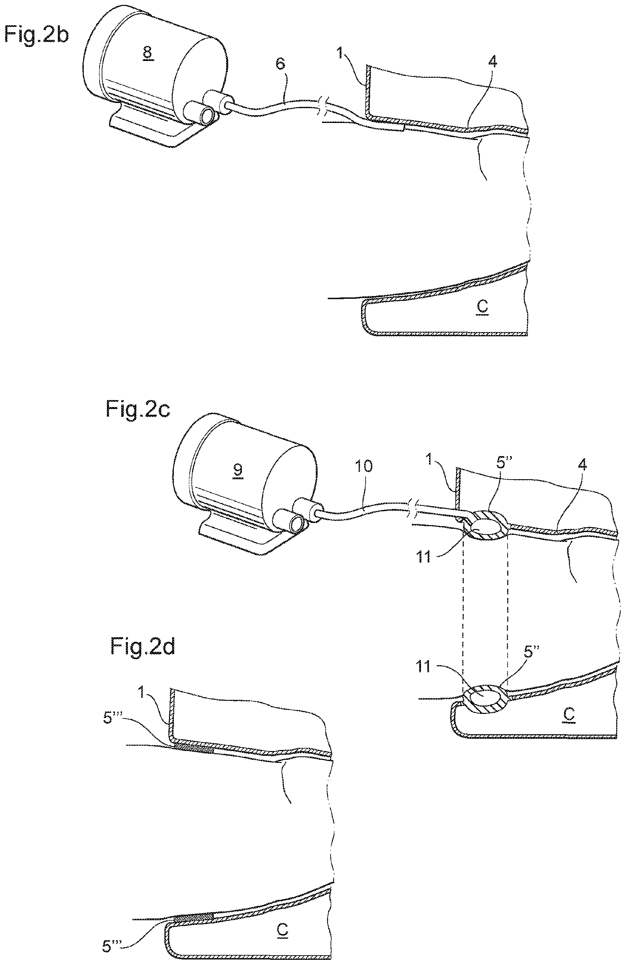

Fig. 2b shows an embodiment of the medical device in which the medical device comprises an air evacuation system for evacuating air in the elongated enclosure 4 after the portion of an extremity has been placed in the elongated enclosure 4, thereby tightening the wall 1 of the enclosure 4 around the extremity of the patient. The air evacuation system comprises a vacuum conduit 6 in fluid connection with the elongated enclosure 4 and thereby enabling the evacuation of the air or fluid from the enclosure 4.

Fig. 2c shows a sealing device in detail in an embodiment in which the sealing device comprises a pneumatic pressure sealing member 5" adapted to seal between an extremity of the patient and the partially collapsible wall 1 using a pneumatic pressure pressing against the skin of the extremity. The pneumatic pressure is created by a fluid pump 9 being connected to the pneumatic pressure sealing member 5" by means of a fluid conduit 10 adapted to transport a pressurized gas from the fluid pump 9 to a chamber 11 within the pressure sealing member 5", the pressure sealing member 5" being inflatable such that the size and/or shape of the cavity 11 can be changed for allowing the pneumatic pressure sealing member 5" to press against the skin of the extremity and thereby sealing between the extremity and the pneumatic pressure sealing member 5". The pneumatic pressure sealing member 5" may for example be made from an elastic material such as an elastic polymer.

Fig. 2d shows a sealing device in detail in an embodiment in which the sealing device comprises an adhesive sealing member 5‴ adapted to seal between an extremity of the patient and the partially collapsible wall 1 by means of an adhesive contacting the skin of the patient and the elongated enclosure 4 of the medical device. In the embodiment shown in fig. 2d the adhesive sealing member 5‴ encircles the extremity (in this case the leg) of the patient thereby creating a seal. The adhesive may for example be a pressure sensitive adhesive such as 3M's pressure sensitive adhesive Scotch-Grip 4268-NF.

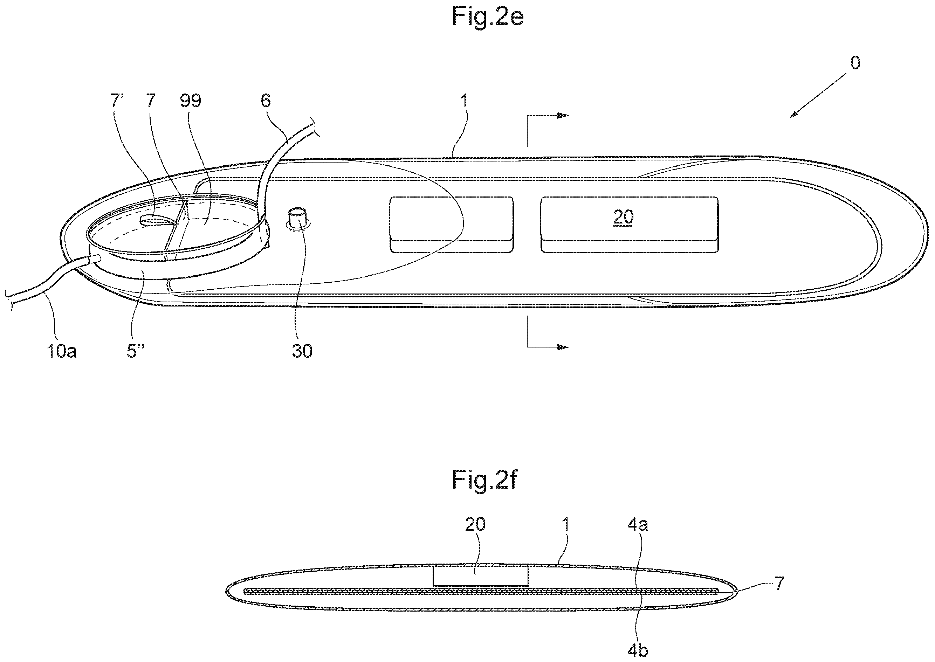

The medical device in figs. 2e - 2i comprises an elongated tubular enclosure 4 extending in the chamber **C** of the medical device. The elongated tubular enclosure 4 has an adhesive surface facing the skin of the patient and being adapted to snuggly adhere to the skin of the patient for reducing the risk that bacteria from the skin of the patient contaminates the environment of the chamber **C.**

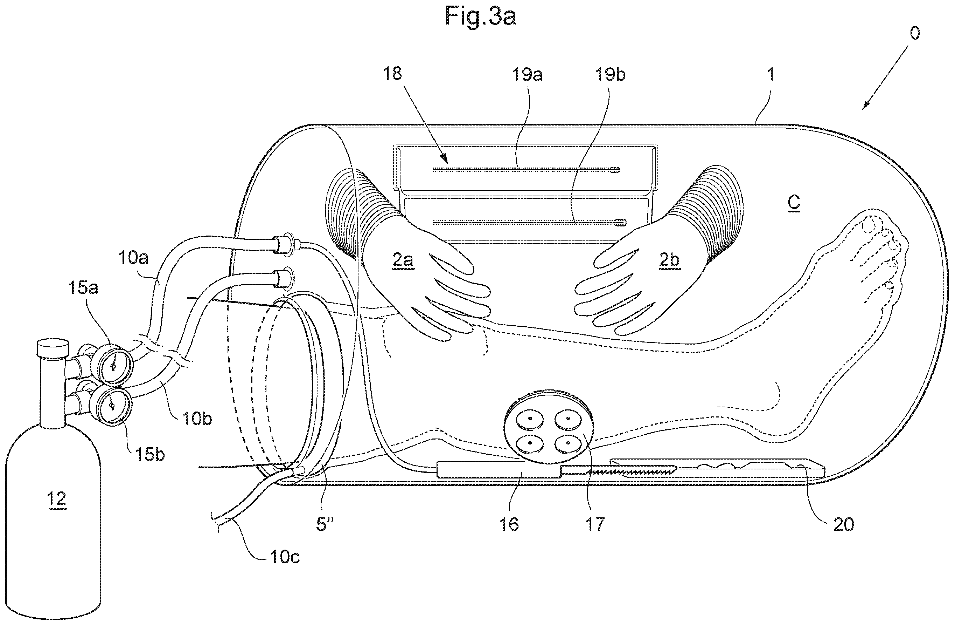

Fig. 2e shows an embodiment of the medical device 0 when deflated for packaging and shipping. The medical device 0 comprises a flexible wall 1 made from a transparent polymer material, such as polyvinyl chloride (PVC) with a plasticizer additive. The wall 1 is adapted to form a chamber **C** for containing a sealed environment in which steps of a surgical procedure can be performed. The medical device 0 comprises a fluid inlet 30 positioned in the wall 1 for supplying a fluid to the chamber and thus inflating the chamber. The fluid inlet 30 is adapted to be connected to a fluid providing member, such as a fluid pump (such as the fluid pump 9 in fig. 2c, or a pressurized tank, such as the pressurized tank 12 in fig. 3a). The fluid supplied may be a gaseous fluid, such as CO2 gas, or a liquid fluid, such as saline solution or an antibiotic liquid. The wall 1 of the medical device 0 forms an elongated tubular enclosure 4 having a single open end 99 adapted for insertion of an extremity of the patient. The enclosure 4 extends in the chamber and is adapted to fit a portion the extremity inserted into the elongated tubular enclosure 4 through the open end 99, such that the surgical procedure can be performed on the portion of the patient's extremity partly within the chamber. The medical device 0 further comprises a fluid evacuation system adapted to evacuate fluid from the interior of the elongated tubular enclosure 4, when the portion of the patient's extremity is placed in the elongated tubular enclosure 4. The fluid evacuation system comprises a vacuum conduit 6 adapted to be connected to a vacuum creating member, such as a vacuum pump (for example disclosed as 8 in fig. 2b).

The medical device 0 according to the embodiment shown in fig. 2e further comprises a sealing device comprising a pressurized sealing member 5" adapted to provide an adaptive sealing force. A fluid conduit 10a is connected to the pneumatically powered sealing member 5" for providing the pneumatic pressure. The other end of the fluid conduit 10a is connected to a pressure creating member, such as a pneumatic pump or a pressurized tank. In alternative embodiments, the sealing device may be hydraulically or electrically powered.

The inside of the elongated tubular enclosure 4 is an adhesive surface adapted to adhere to the skin of the patient, such that the incision in the skin of the patient is performed through the wall 1 of the elongated tubular enclosure 4. The adhesive layer of the elongated tubular enclosure 4 adhering snuggly against the skin of the patient, the risk that bacteria from the skin of the patient will enter the area of the incision is greatly reduced. The adhesive surface may for example comprises a pressure sensitive adhesive such as 3M's pressure sensitive adhesive Scotch-Grip 4268-NF. When the medical device 0 is packaged, the medical device 0 further comprises a separating layer 7, adapted to separate an adhesive surface of a first portion of the elongated tubular enclosure (shown as 4a in fig. 2f), from a second portion of the elongated tubular enclosure (shown as 4b in fig. 2f), such that the elongated tubular enclosure can easily be expanded or inflated after having been packaged as a flat package (which is shown in fig. 2e). The separating layer 7 comprises a non-stick surface which is easy to remove from the adhesive surface. The non-stick surface could for example be a PTFE covered surface. The separating layer 7 further comprises a gripping portion 7' such that the separating portion 7 can easily be removed after the elongated tubular enclosure 4 has been expanded or inflated.

The medical device 0 further comprises an instrument mount 20 placed within the chamber adapted for retaining instruments within the chamber.

Fig. 2f shows the medical device according to the embodiment shown in fig. 2e in a sectional view from the left, in which the separating layer 7, the first 4a and second 4b portion of the wall of the elongated tubular enclosure 4, and the wall 1 of the medical device creating the chamber can be seen in section.

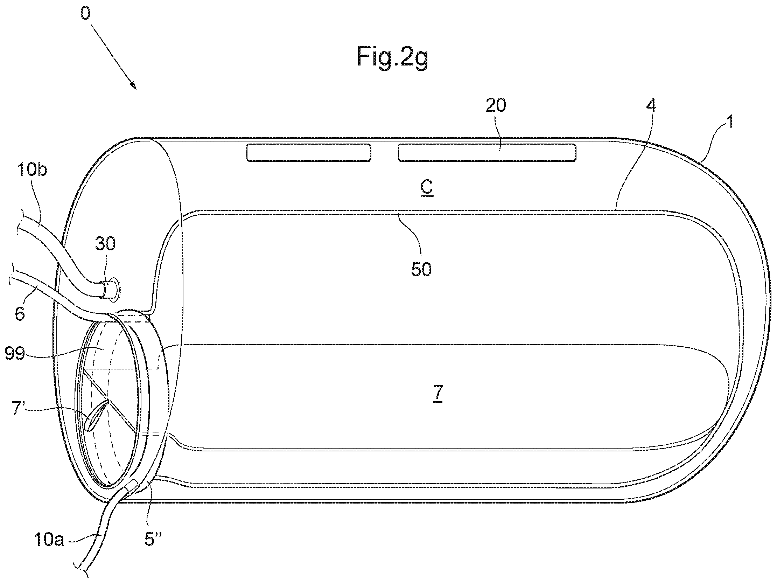

Fig. 2g shows the medical device 0 when the wall 1 of the medical device 0 has been inflated by the injection of a fluid through the fluid inlet 30 positioned in the wall 1 and connected to a fluid conduit 10b, such that a chamber **C** is formed. The fluid entering the chamber **C** could require filtering, and thus the fluid conduit 10b may pass a filtering unit. The filtering unit being adapted to remove particles by a mechanical filter, such as a filter textile, or by means of a chemical filter such as activated carbon, or by a combination of a mechanical and chemical filter. Furthermore, a sterilization device may be provided on the fluid conduit 10b for sterilizing the fluid entering the chamber **C.** The sterilization device could for example be a sterilization device sterilizing the fluid by means of heat, a chemical sterilization device, or a sterilization device sterilizing by means of radiation, such as UV-radiation. A fluid tempering device may also be provided on the fluid conduit 10b for changing the temperature of the fluid passing fluid tempering device. The fluid sterilization device, tempering device and tempering unit is further shown in e.g. fig. 210.

In fig. 2g, the elongated tubular enclosure 4 has been inflated through the vacuum conduit 6, which now has provided a pressurized fluid into the elongated tubular enclosure 4. The inflation of the elongated tubular enclosure 4 facilitates the insertion of the extremity of the patient into the elongated tubular enclosure 4 even when the inner surface of the elongated tubular enclosure 4 is an adhesive surface 50. During the inflation, the open end 99 may be covered by a covering member (not shown) such that a pressure buildup is possible within the elongated tubular enclosure. The elongated tubular enclosure 4 is made from a transparent polymer material rigid enough to retain its shape even when the covering member is removed and the pressure within the elongated tubular enclosure 4 is released. After the inflation and thus separation of the first and second portions of the wall 1 of the elongated tubular enclosure 4, which were connected when the medical device 0 was packaged, the separating layer 7 is removed by pulling the separating layer 7 out of the elongated tubular enclosure 4 by means of the gripping portion 7'. The instrument mount 20 is further shown in fig. 2g as being placed in the top portion of the chamber **C,** however, the position of the mount 20 may be adapted for the particular instruments to be retained by the mount 20.

The medical device 0 according to the embodiment shown in fig. 2g comprises a pressure seal 5" supplied with a pressurized fluid via a fluid conduit 10a. However, in alternative embodiments it is equally conceivable that the pressure sealing member 5" is replaced by a vacuum sealing member, a mechanically pressing sealing member, such as an elastic sealing member, or an adhesive sealing member (examples of which are given in relation to other embodiments herein).

The fluid conduits 10a, 10b are for example connected to a pressure creating member such as a pneumatic pump or a pressurized tank (disclosed throughout the disclosure).

The medical device 0 may additionally comprise a monitoring unit adapted to monitor a physiological parameter of the patient and adapted to control a pressure creating member inflating the sealing member 5" or the inflatable wall 1 such that the inflation of the sealing member, or the inflation of the chamber **C** can be adjusted such that the blood flow of the extremity of the patient is not hindered by the pressure in the sealing member 5" or by the pressure in the chamber **C.** The monitoring unit is further described under reference to figs. 209a - 209g.

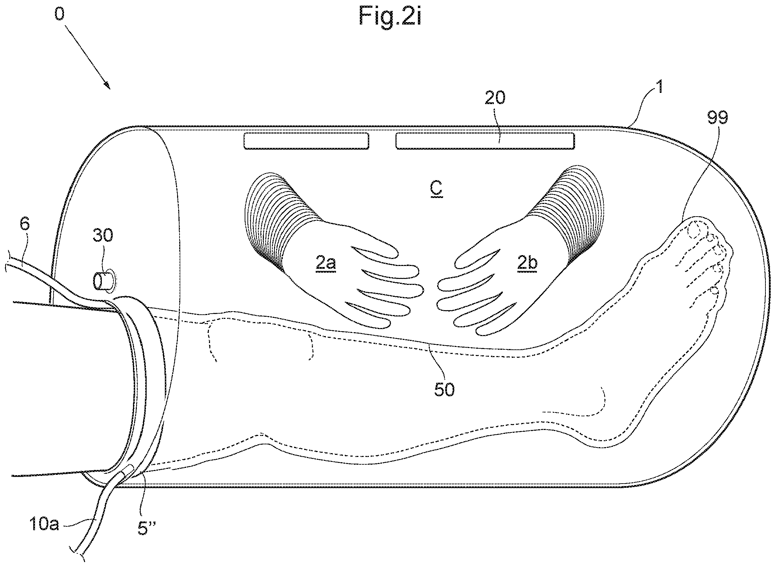

The medical device 0 may further comprise a glove integrated in the wall 1 such that a surgeon is able to use the glove from outside the chamber **C** to make manual manipulations inside the chamber **C** while maintaining the sealed environment in the chamber **C.** An example of an integrated glove is shown in fig. 2b and 2i.

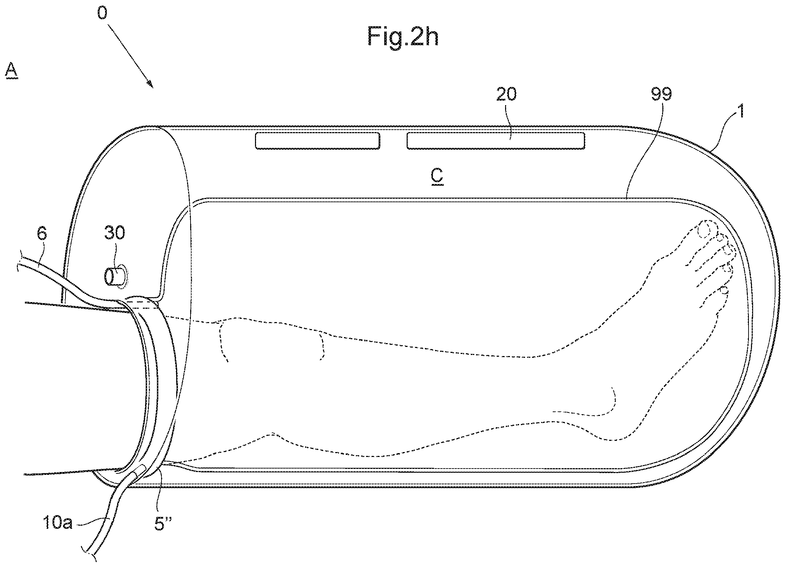

Fig. 2h shows the medical device 0 when the extremity of the patient has been inserted into the elongated tubular enclosure 4 and the sealing member 5" has been inflated, such that a substantially airtight seal is created between the ambient environment **A** and the elongated tubular enclosure 4. The vacuum conduit 6 is integrated in the sealing member 5" such that the vacuum conduit 6 is in fluid connection with the elongated tubular enclosure 4 whilst the sealing member 5" provides sealing between the elongated tubular enclosure 4 and the ambient environment **A.**

Fig. 2i shows the medical device 0 when the inflated fluid in the elongated tubular enclosure 4 has been evacuated by the vacuum conduit 6, such that the wall 1 of the elongated tubular enclosure 4 snuggly encloses the extremity and the adhesive surface 50 of the inside of the elongated tubular enclosure 4 adheres to the skin of the extremity of the patient, such that an adhering layer 50 on the patients skin is formed. The adhering layer is cut through in the beginning of a surgical procedure inside the chamber **C** of the medical device 0. By the adhering layer 50 covering all of the skin which otherwise would be exposed to the environment of the chamber **C,** the risk that bacteria from the skin of the patient should contaminate the environment of the chamber **C** is substantially reduced. Fig. 2i further shows gloves 2a, 2b integrated in the wall 1 of the medical device 0 and enabling manual manipulation within the chamber **C** of the medical device 0.

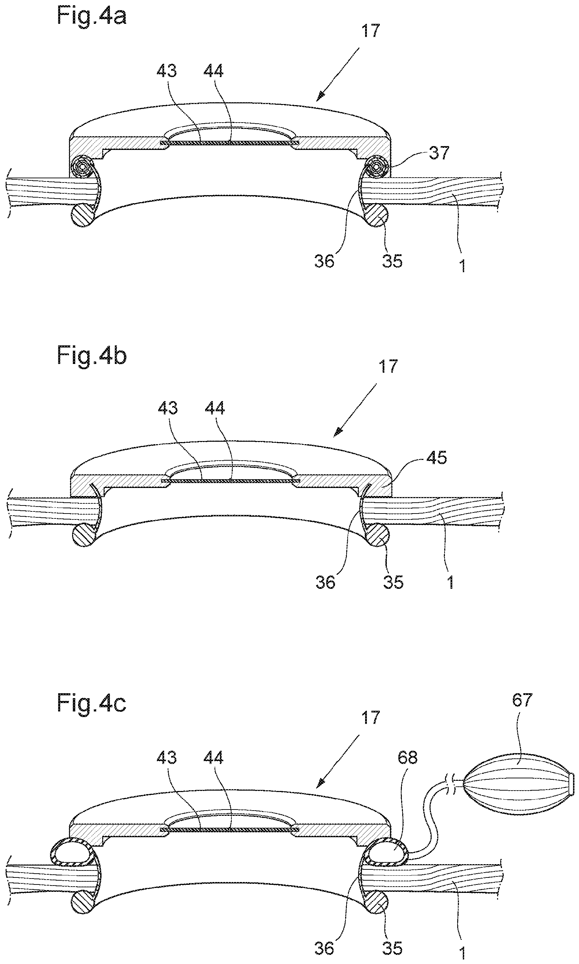

After an incision has been performed in the wall 1 of the elongated tubular enclosure 4 and the skin of the patient, a port may be placed in the wall 1 of the elongated tubular enclosure 4 and in the skin of the patient for creating a fluid connection between the chamber **C** and a cavity in the patient. The port may be fixated to the wall 1 of the elongated tubular enclosure 4 by being integrated in the wall, or the port may be fixated in the skin of the patient, such as for example disclosed under reference to figs. 4a - 4c. The port disclosed in figs. 4a - 4c comprises a sealing device having a first sealing member adapted to be positioned at the inside of the patient's skin around an incision cut in wall of the elongated tubular enclosure 4 and the patient's skin, made from within the chamber **C,** a second sealing member adapted to be positioned at the outside of the patient's skin inside the chamber, around the incision in the skin of the patient and the elongated tubular enclosure 4. The sealing further comprising an elastic connecting member sealingly interconnecting the first and second sealing members, such that the elastic connecting member seals between the second sealing member and the elongated tubular enclosure 4 and/or the first sealing member and the tissue of the patient.

The medical device 0 may further comprise a wall port provided in the wall 1 of the medical device 0 for enabling transfer between the chamber **C** of the medical device 0 and the ambient environment **A** (such a wall port for example disclosed as 17 in figs. 3a - 3e). The wall port may comprises an elastic membrane adapted to, in a non-compressed state, seal between the chamber **C** and the ambient environment **A,** and in a compressed state enable a hand or object to be inserted through the membrane.

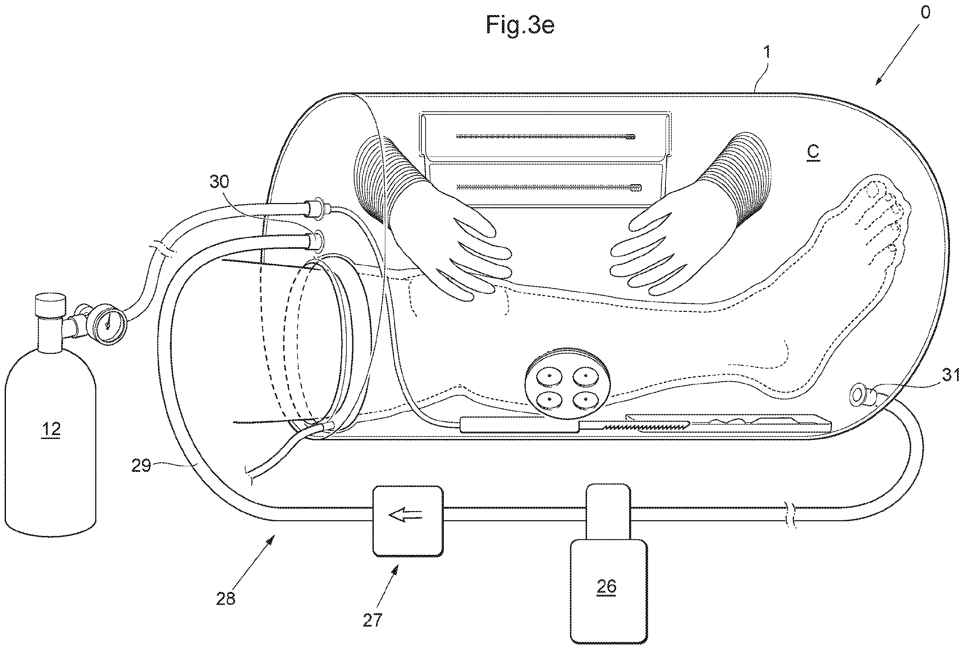

In alternative embodiments, the medical device 0 may further comprise an outlet member provided in the wall for discharging the fluid from the chamber **C,** and a pump adapted to circulate the fluid from the outlet member to the inlet member 30 (such as for example disclosed in fig. 3e).

Fig. 3a shows an embodiment of the medical device 0 in which the medical device 0 comprises a partially collapsible wall 1 having a cylindrical or tube shaped structure. The partially collapsible wall 1 is inflatable by means of a fluid conduit 10b, here connected to a tank 12 containing pressurized gas. The tank 12 is further connected to a fluid conduit 10a for transferring pneumatic force into the chamber **C** enclosed by the partially collapsible wall 1. Shown in the embodiment herein, the fluid conduit 10a is connected to a pneumatic tool 16, which in the example shown is an orthopedic saw, adapted to be operated within the chamber **C.** The pressure provided to the chamber **C** and the fluid conduit 10a is controlled and monitored by two control/monitoring systems 15a, 15b connected to the fluid conduits 10a, 10b, respectively. In the embodiment shown, the partially collapsible wall 1 is sealed from the extremity of the patient by means of a pneumatic pressure sealing member 5" (further disclosed with reference to fig. 2c) encircling the extremity. In fig. 3a the medical device 0 is shown incorporating the pneumatic pressure sealing member 5" receiving a fluid pressure by means of a fluid conduit 10c, however it is equally conceivable that the pneumatic pressure sealing member 5" is assisted or replaced by any of the other sealing members disclosed herein. The medical device 0 shown in fig. 3a further comprises an airlock sluice 18 comprising two zipper-closed consecutive openings, where the outer zipper 19a could be opened for placing and/or removing objects 69, such as implants, in the airlock sluice 18 between the zippers 19a, 19b from the outside of the chamber **C** and the inner zipper 19b could be opened from the inside of the chamber **C** for placing and/or removing objects 69 from the airlock sluice 18. For example the airlock sluice 18 could be used to remove a specimen from the chamber **C** while keeping the environment closed, or the airlock sluice 18 could be used to insert a medical device 0 or an implant into the chamber **C.** The medical device 0 disclosed with reference to fig. 3a further incorporates a wall port 17 (which are to be regarded as optional) through which laparoscopic trocars or endoscopic instruments could be inserted. The medical device 0 according to the embodiment disclosed with reference to fig. 3a further includes an instrument mount 20 for retaining instruments within the chamber **C.**

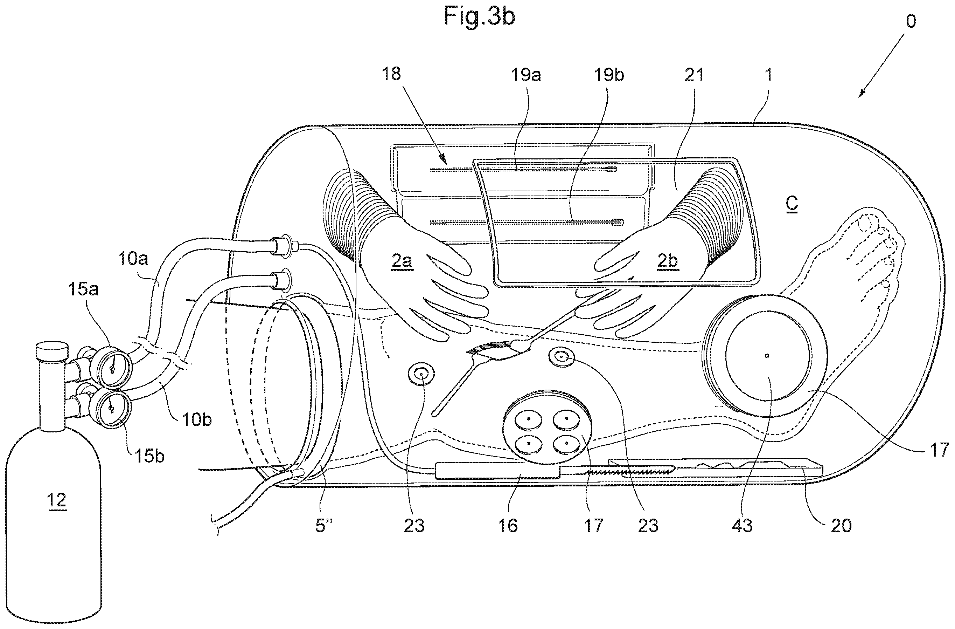

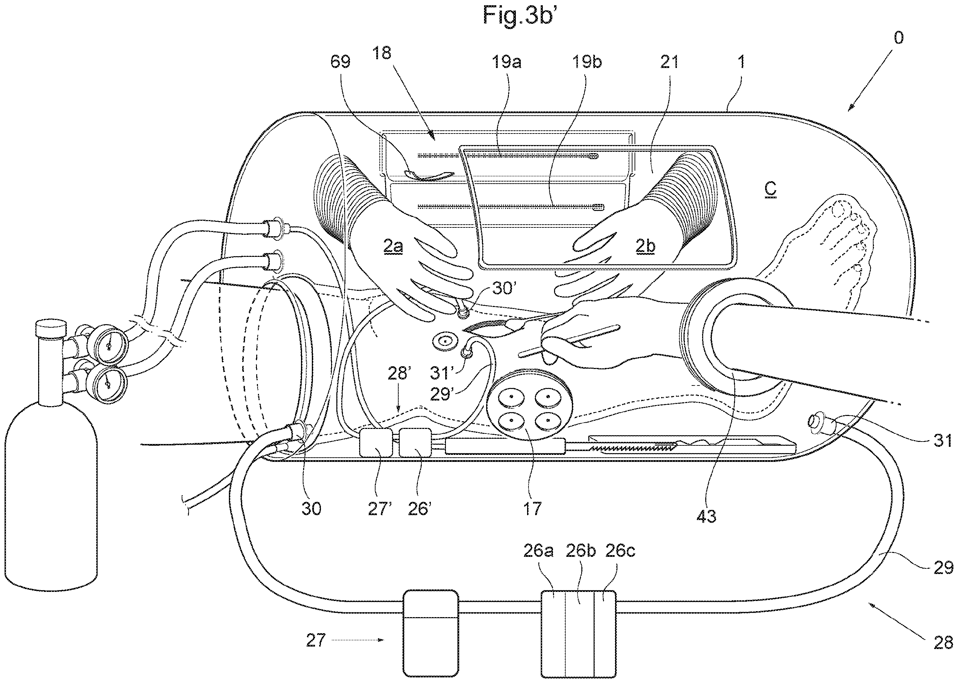

Fig. 3b shows the medical device 0 similar to the embodiment shown in fig. 3a, however the partially collapsible wall 1 of the medical device 0 according to fig. 3b comprises a non-collapsible transparent window 21 for enabling viewing into the chamber **C.** The non-collapsible transparent window 21 will ensure that the surgeon has adequate visual control over the procedure since the partially collapsible wall 1, even if preferably made from a transparent material could provide limited visibility, e.g. if the medical device 0 is not fully inflated, or in seams or bends of the material. The medical device 0 shown in fig. 3b further comprises a wall port 17 comprising a self sealing membrane 43, which for example could comprise a material of gel type, such as the GelPort available from Applied medical inc. Rancho Santa Margarita, CA. The wall port 18 with the self sealing membrane 17 enables the insertion of trocars and/or instruments as well as a persons (preferably the surgeons) hand, enabling manual manipulation within the chamber **C.** The medical device 0 as shown in fig. 3b further comprises two body ports 23 which incorporate endoscopic ports in direct connection with the extremity of the patient, within the chamber **C.** The body ports 23 could for example enable an arthroscopic procedure with trocars to be performed within the chamber **C** and either by the instruments being manipulated from the inside of the chamber **C** or by the instruments reaching from the ports 17 in the collapsible wall 1 and to the body port 23 within the chamber **C.**

Fig. 3b' shows the embodiment of the medical device 0 according to fig. 3b when the arm of the surgeon is inserted through the self sealing membrane 43 of the wall port 17 in the partially collapsible wall 1 for enabling manual manipulation therein. Manual manipulation through the wall port 17 could for example be used as a last resort should complications occur during the procedure, or if additional hands are required for a specific step of the operation. Fig 3b' further shows an internal system 28' for fluid circulation, and an external system 28 for fluid circulation. Both systems comprises fluid conduits 29; 29', in the external system 28 connected at an inlet 30 placed in the partially collapsible wall 1 for supplying fluid to the chamber **C,** and an outlet 31 for draining the fluid from the chamber **C,** and in the internal system 28' connected to an inlet 30' in the patient's body and an outlet 31' in the patient's body for circulating fluid in a body cavity. The fluid is circulated by means pumping units 27; 27' and is circulated via a sterilizing/filtering/tempering unit 26', in the external system disclosed as containing a sterilizing 26a, filtering 26b, and tempering 26c unit adapted to remove impurities, sterilize and temper the fluid. In embodiments where the fluid is a gaseous fluid, the filtering unit is adapted to remove particles that could be suspended in the gas. The filtering unit 26a could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit 26a could for example comprise a filter containing activated carbon.

Sterilizing methods could comprises the use of heat, such as electrical or chemical heat, it is furthermore conceivable that the sterilizing is performed using irradiation, such as UV-light and/or though the addition of a chemical sterilizing agent.

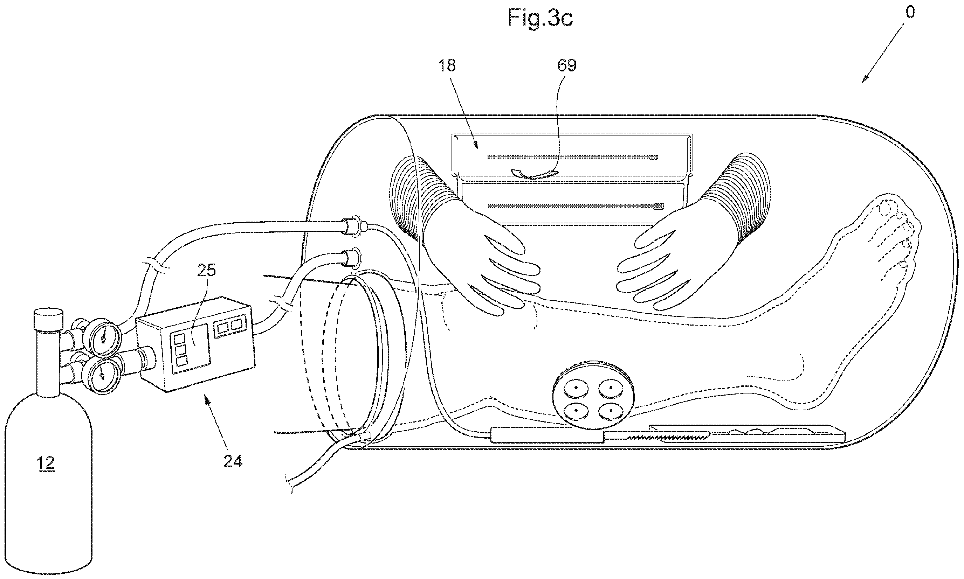

Fig 3c shows the embodiment of the medical device 0 also shown in fig. 3a with the addition of a unit 26c for tempering the fluid entering the chamber **C.** The tempering part of the system could be provided to heat or cool the gaseous fluid inflating the medical device 0. In cases when the surrounding environment is cold the tempering part of the system 24 could be used to raise the temperature of the chamber to provide beneficial operating circumstances both for the patient and for the surgeon. In cases when the surrounding environment is hot, the tempering part of the system 24 could be used to lower the temperature of the sealed environment both to provide beneficial operating circumstances, both for the patient and for the surgeon, and for providing a temperature inhibiting bacterial and viral infections. The tempering unit 24 may comprise a temperature regulator for setting the required temperature. Fig. 3c further shows an object in the airlock sluice 18 being a knee joint prosthesis or a portion of a knee joint prosthesis to be implanted. The prosthesis is being inserted into the chamber C through the air lock sluice 18 further disclosed under reference to fig. 3a.

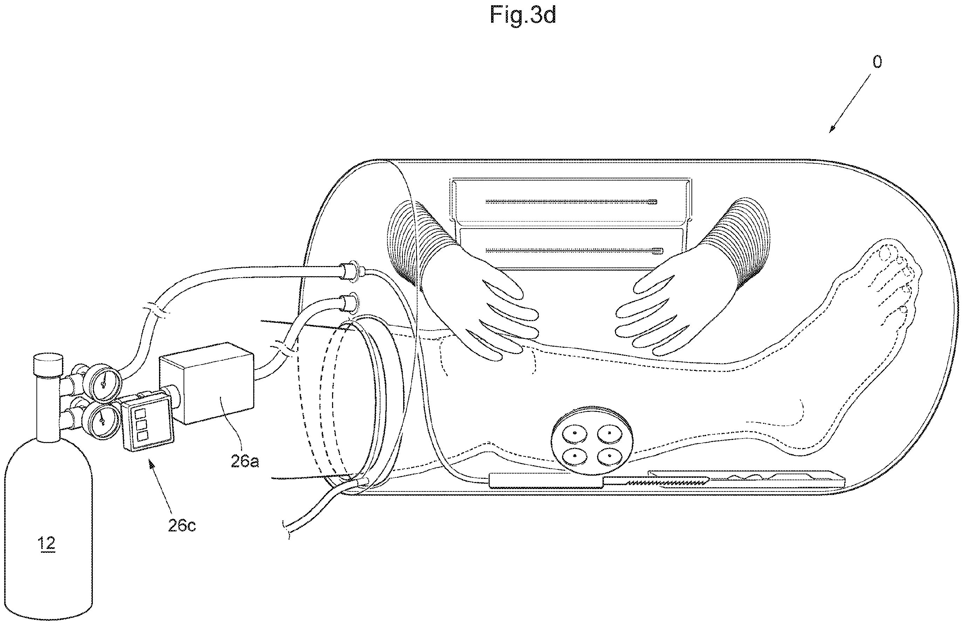

Fig. 3d shows the medical device 0 according to fig. 3c but further comprising a sterilizing unit 26a adapted to sterilize the gaseous fluid entering the medical device 0. In the embodiment disclosed herein, the gaseous fluid entering the medical device 0 originates from a pressurized tank 12 and could be pre-sterilized, however, in other embodiments, it is conceivable that the gaseous fluid is the surrounding air which is pressurized (such as further disclosed with reference to fig. 3e). In the embodiments where the surrounding air is used to inflate the medical device 0 and constitute the operating environment this air needs to be properly sterilized.

Fig. 3e shows an embodiment of the medical device 0 in which the medical device 0 comprises a system 28 for circulating a fluid through the chamber **C** of the medical device 0. The system comprises a fluid conduit 29 connected at an inlet 30 placed in the partially collapsible wall 1 for supplying fluid to the chamber **C,** and an outlet 31 for draining the fluid from the chamber **C.** The fluid is circulated by means of a pumping unit 27 and is circulated via a sterilizing/filtering/tempering unit 26 adapted to remove impurities, sterilize and temper the fluid. In embodiments where the fluid is a gaseous fluid, the filtering unit is adapted to remove particles that could be suspended in the gas. The filtering unit could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit could for example comprise a filter containing activated carbon.

Sterilizing methods could comprises the use of heat, such as electrical or chemical heat, it is furthermore conceivable that the sterilizing is performed using irradiation, such as UV-light and/or though the addition of a chemical sterilizing agent.

Figs. 1a - 3e show different embodiments of a medical device directed to an extremity of the patient e.g. a leg or an arm. The medical device could for example be used for performing an amputation both in a hospital environment and in environments where the risk of infections are considerably higher, such as in third world countries or in zones of catastrophe. The medical device disclosed enables surgical procedures to be performed in a fully enclosed environment without any risk of infection disease even in very remote locations and without the access to water or electricity.

Fig. 4a shows a wall port 17 in a close-up, in section, where the wall port 17 comprises a self sealing membrane 43 fixated to a rigid part 45 of the wall port. The self sealing membrane 43 having a small self sealing hole 44 centrally in the membrane 43. The self sealing membrane 43 is for example made from a gel-type material being elastic enough to enable a deformation large enough for a person's hand to pass through the small self sealing hole 44 while still contracting enough to create an airtight seal between the sealed environment and the outside environment. Wall port 17 is fixated to a hole in the wall 1 of the medical device by means of a first intra chamber ring 35 adapted to be positioned on the inside of the wall 1, and a second outer ring 37 adapted to be placed on the outside of the wall 1. Between the intra chamber ring 35 and the outer ring 37 adapted to be placed on the outside of the wall 1 an elastic connecting member 36 is positioned. The connecting member 36 is preferably made from a resilient or elastic polymer material. The outer ring 37 adapted to be placed on the outside of the wall 1 comprises an integrated roll up system which could be a spring loaded roll up system adapted to create a suitable pressure on connecting member 36.

Fig. 4b shows an alternative embodiment of the wall port 17, the difference being that the connecting member 36 is made from a material elastic enough such that one end of the retractor membrane 36 could be fixedly fixated to a rigid part 45 of the wall port 17.

Fig. 4c shows an alternative embodiment of the wall port 17, with the difference that the connecting member 36 is connected to an inflatable bellows 68 which in turn is connected to a pressure creating member 67 for inflating the bellows 68 and tighten the connecting member 36 in a direction substantially perpendicular to the wall 1 of the medical device.

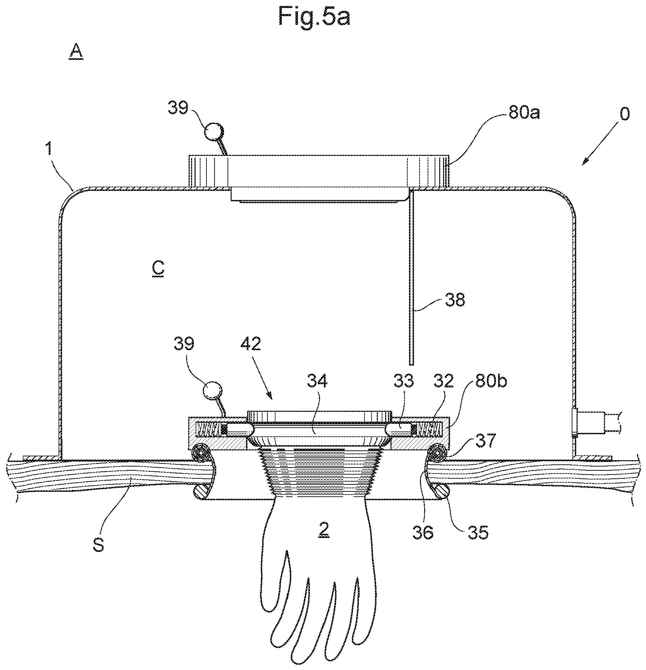

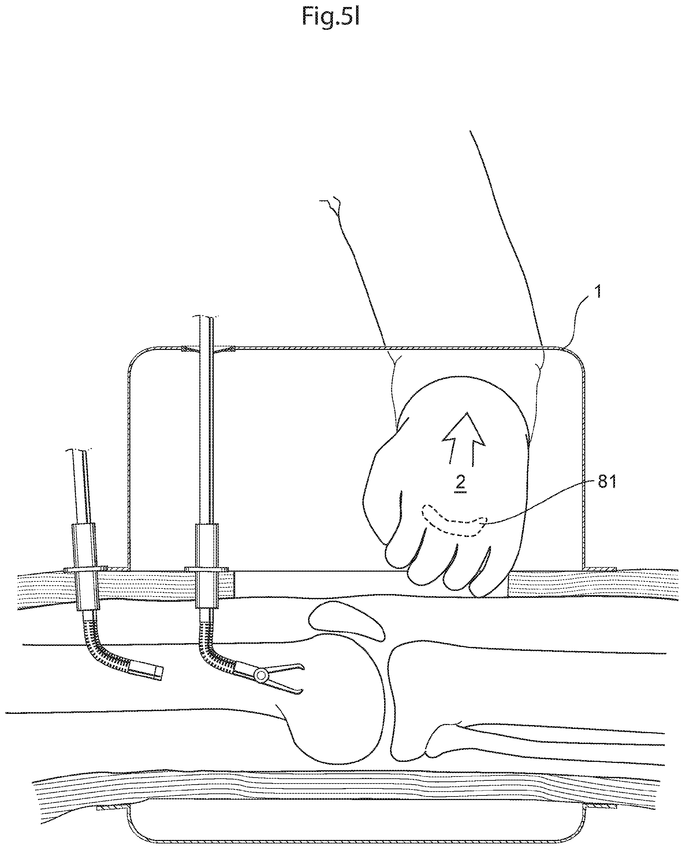

Fig. 5a shows an embodiment of the medical device 0 in which the medical device 0 comprises a wall 1 separating the environment of the chamber **C** in the medical device 0 from the ambient environment **A.** The wall 1 is adapted to be fixated to the skin **S** of the patient, by means of an adhesive or by means of a difference in pressure between the inside of the chamber **C** and the ambient environment **A.** The medical device 0 further comprises an upper 80a and a lower 80b coupling for fixating ring shaped objects which for example could be insets 42 or ports, such as the ports 17; 23 disclosed in figs. 1 - 3 and the insets for example could be a surgical glove 2 as disclosed in various embodiments herein, or holding devices. The ports may further be hand access ports, such as the port disclosed with reference to fig. 4a and 4b. The couplings 80a; 80b comprises a releasing lever 39 for releasing the inset 42 or port. The lever 39 is connected to protruding members 33 which are spring 32 loaded from the rear in order to secure the recess 34 of the object. In the embodiment disclosed in fig. 5a, the inset 42 fixated to the lower coupling 80b is a surgical glove 2 enabling manual manipulation within the body of the patient. The medical device 0 shown in fig. 5a further comprises a valve member 38 adapted to close the opening in the upper coupling 80a, when the upper coupling 80a is not in use.

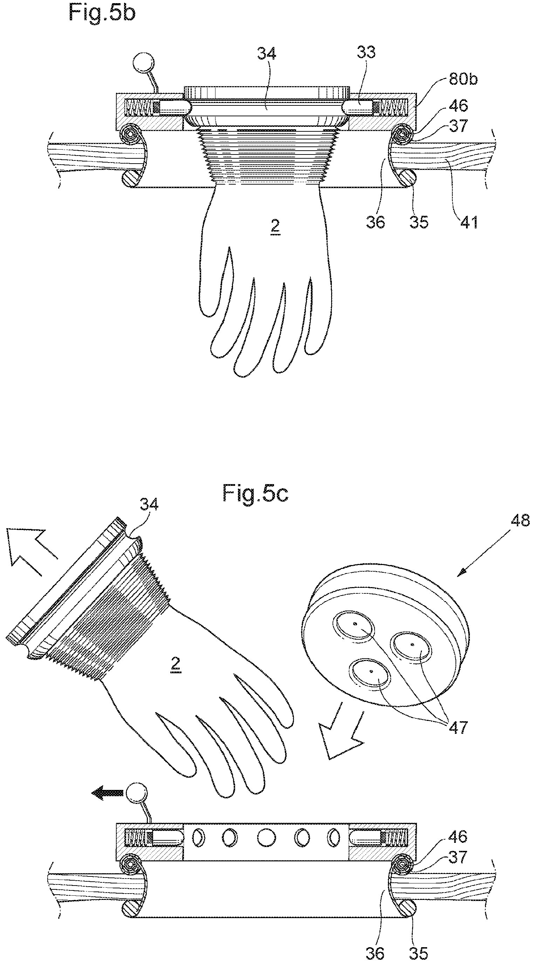

Fig. 5b shows the lower coupling 80b in further detail when an inset 42 in the form of a surgical glove 2 is fixated to the lower coupling 80b. A retractor system comprising an intra body ring 35 and an outer ring 37 adapted to be placed on the outside of the patients skin S further comprises a connecting member 36 in the form of a retractor membrane positioned such that it exerts a pressure on the cut surfaces of the incision for retracting the skin S and thereby keeping the incision open. The retractor membrane 36 could be rolled into a roll up system 46 inside of the outer ring 37 for tightening the retractor membrane 36 such that the incision is kept open.

Fig. 5c shows the changing of objects in the lower coupling 80b from an inset 42 comprising a surgical glove 2 to a multi-port 48 comprising three endoscopic ports. To be able to quickly switch from a purely endoscopic system to a hand access or manual manipulation system could be of major importance if complications arise during the procedure and could remove the need for a traditional conversion from endoscopic to open surgery, a conversion which inevitably causes problems increasing the risk of complications and/or postoperative care.

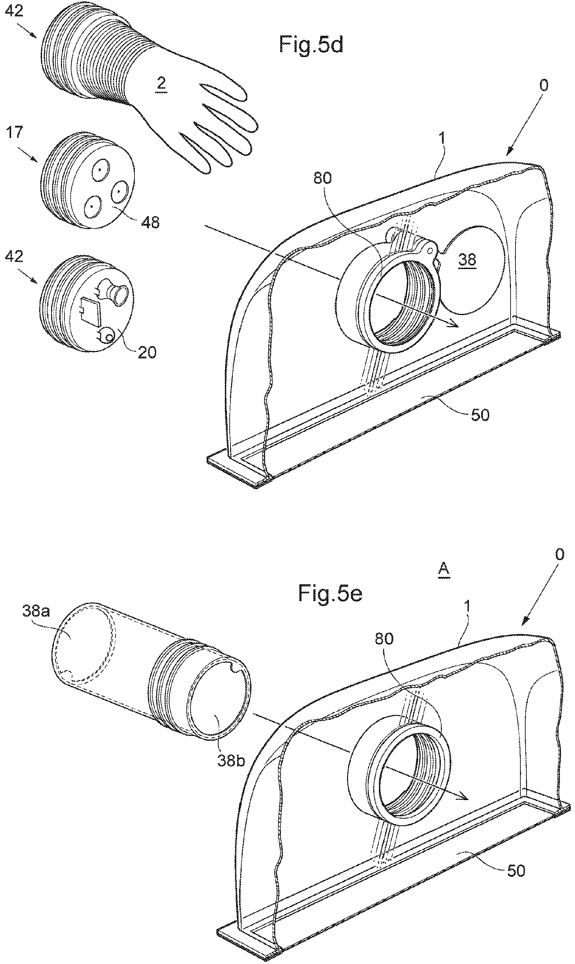

Fig. 5d shows a section of the medical device 0 showing a coupling 80 integrated in the wall 1. The coupling 80 enables the changing of objects from for examples ports, such as the wall port 17 disclosed in other embodiments herein, to insets 42 in form of a surgical glove 2 or an instrument / device mount 20. The coupling 80 comprises a valve member 38 for closing the hole in the coupling 80 when objects should be exchanged such that the environment in the medical device 0 can remain sterile. The instrument or device mount 20 could for example be adapted to hold surgical instruments and/or implants or parts of implants. The medical device 0 is according to this embodiment adapted to be fixated to the skin of the patent by a large portion of the wall of the medical device comprising an adhesive surface 50.

Fig. 5e shows the medical device 0 according to the same embodiment as in fig. 5d, but when the ports of insets in the coupling 80 have been replaced by an airlock sluice 18 comprising a first 38a and second 38b valve member enclosing a sluice chamber placed between the first 38a and second 38b valve members. The airlock sluice 18 can be used to enable transfer of objects from the ambient environment **A** to the chamber and vice versa.

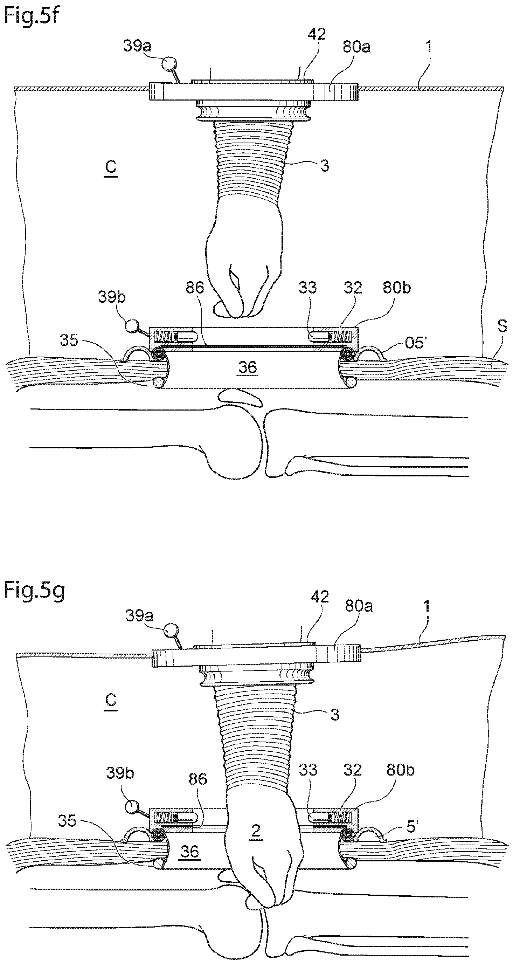

Fig. 5f illustrates how an incision in the patient's skin **S** has been created by the surgeon by means of an inset 42 comprising a glove 2 latched to a first coupling 80a. The second coupling 80b is fixated and sealed by means of a sealing device comprising a vacuum sealing member 5'. The medical device shown in fig. 5f further comprises a second sealing member that is provided partially within the patient's body 35; 36 which cannot be mounted until the incision in the patient's skin has been concluded. The two different sealing members 5'; 35; 36 disclosed could in some embodiments be replaced by only one of the sealing members, or by a different sealing member, such as the adhesive or pressure sealing members disclosed in other portions herein. The first coupling 80a comprises an iris valve 86 which may be opened to enable the surgeon to access the inside of the patient's body, or closed to separate the patient's body from the chamber **C** enclosed by the wall 1 of the medical device.

Fig. 5g shows the medical device when the second sealing device 35, 36, 37 have been placed in the incision cut in the patient's skin. The wall 1 enclosing the chamber **C** is elastic or flexible which enables the surgeon to move the first coupling 80a in relation to the second coupling 80b for getting better access to different angles within the patient's body, for example for removing a specimen or placing a prosthetic part in the knee joint. The embodiment of fig. 5f further comprises an iris valve 86 placed in the first coupling 80a for closing the coupling 80a such that the chamber is sealed from at least one of the ambient environment and a cavity in the patient. The closing of the iris valve 86 enables activity within the chamber **C** without maintaining a pressure in the chamber **C,** at the same time as a pressure and/or sealed environment can be maintained within the body of the patient.

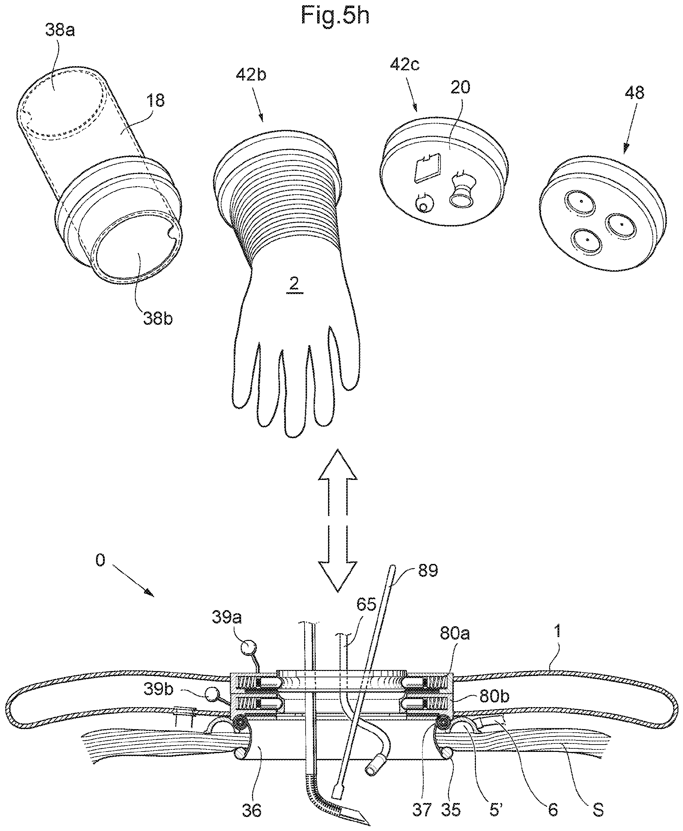

Fig. 5h shows the medical device 0 adapted to be at least partially placed in an incision cut in a patient's body. The medical device comprises a first and second coupling 80a; 80b adapted be interconnected. The first coupling 80a is adapted to be disconnected from the second coupling 80b, and the first coupling 80a is connected to a first portion of a flexible wall 1, and the second coupling 80b is connected to a second portion of the flexible wall 1. The flexible wall 1 forms a chamber in which a sealed environment can be maintained, the chamber **C** is heavily enlarged when the first coupling 80a is disengaged from the second coupling 80b, thus creating operating space within the chamber enclosed by the wall 1. The first 80a and second 80b coupling comprise a latching system for locking an inset 42b; 42c, airlock sluice 18 or a port (such as the multiport 48 disclosed). The inset 42 may for example be an inset 42b comprising a glove 2 for manual manipulation within the body of the patient, or within the enclosed chamber, or an inset comprising an instrument or device mount 20 for holding instruments or medical devices within the chamber. Alternatively the insets 42b; 42c can be replaced by an airlock sluice 18 with a first 38a and second 38b valve member or a port, such as the multiport 48, comprising a plurality of ports enabling the insertion of a surgical instrument 89, or a camera 88 into the chamber or a cavity in the body of the patient.

In the embodiment shown in fig. 5h the medical device is fixated to the body of the patient by means of a vacuum fixating member 5' connected to a vacuum conduit 6, which in turn is connected to a vacuum source. Furthermore, the medical device in fig. 5h comprises a second sealing device having a first sealing member in the form of an intra body ring 35 adapted to be positioned at the inside of the patient's skin **S** around an incision to be performed in the skin **S** and a second sealing member in the form of an outer ring 37 adapted to be positioned at the outside of the patient's skin **S** around the incision, and a spring-loaded or elastic connecting member 36 sealingly interconnecting the intra body ring 35 and outer ring 37. The spring-loaded or elastic connecting member 36 seals between at least one of the second coupling 80b and the skin of the patient, and the intra body ring 35 and tissue of the patient. As shown in fig. 5h the surgeon can use the multiport 48 placed in the first coupling 80a to insert laparoscopic instruments into the body of the patient.

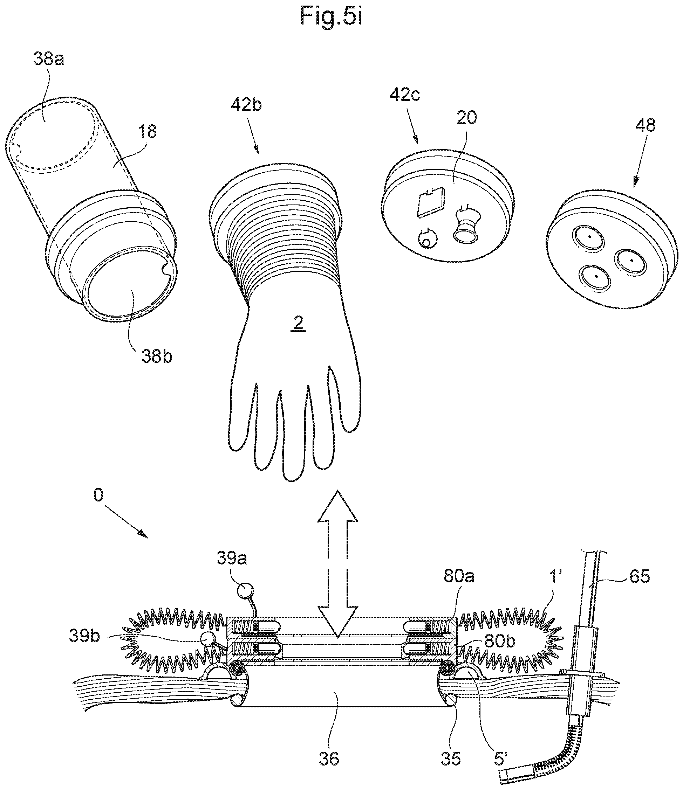

Fig. 5i shows the port in an embodiment very similar to the embodiment shown in fig. 5h but with the difference that the wall 1' has a bellows structure and thus taking up less space when in its deflated state. The bellows structure enables the wall 1' to be packaged in a small package and thus not obstructing the procedure. In some applications the wall can be used only on very special occasions or emergencies, such as when some part of the procedure goes wrong and conversion from laparoscopic surgery to more open surgery is required. The use of the inflated chamber then enables the pressure in the cavity within the patient to be maintained since the chamber can hold a pressure. Fig. 5i also shows a camera 65 placed in the skin of the patient to enable visual inspection of a cavity within the patient's body, the camera being placed outside of the ports and sealed environment.

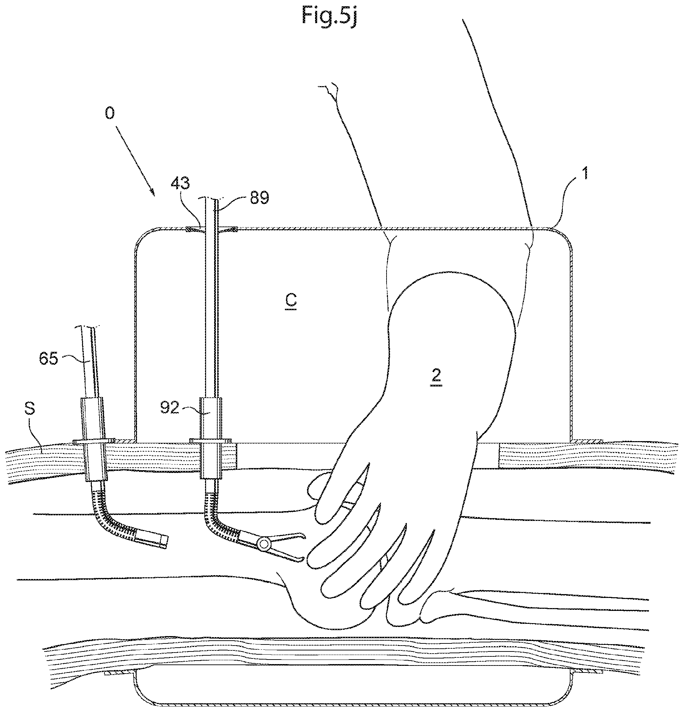

Fig. 5j shows an embodiment of the medical device 0, in which the medical device 0 comprises a glove 2 integrated in the wall 1 and a camera 65 placed in the skin **S** outside of the medical device 0. The medical device 0 further comprises a trocar 92 placed trough an incision in the skin **S** of the patient and into an area of the knee of the patient. A surgical instrument 89 travels through a self sealing membrane 43 in the wall 1 of the medical device and further through the trocar 92 placed in the incision in the skin **S** of the patient and into a cavity in the patient's body. Fig 5j furthermore schematically illustrates how the surgeon manipulates the knee in the chamber **C** using the glove 2.

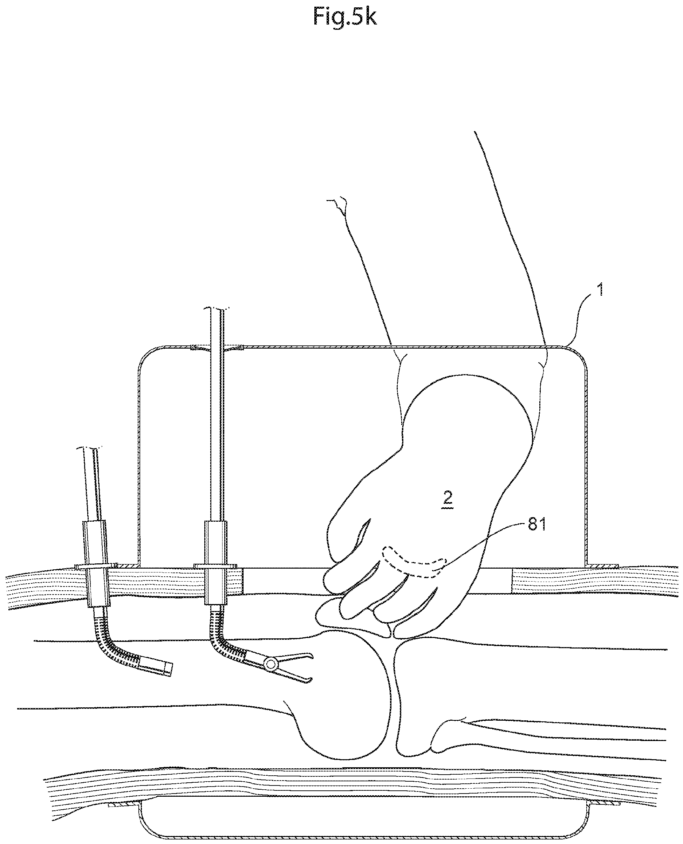

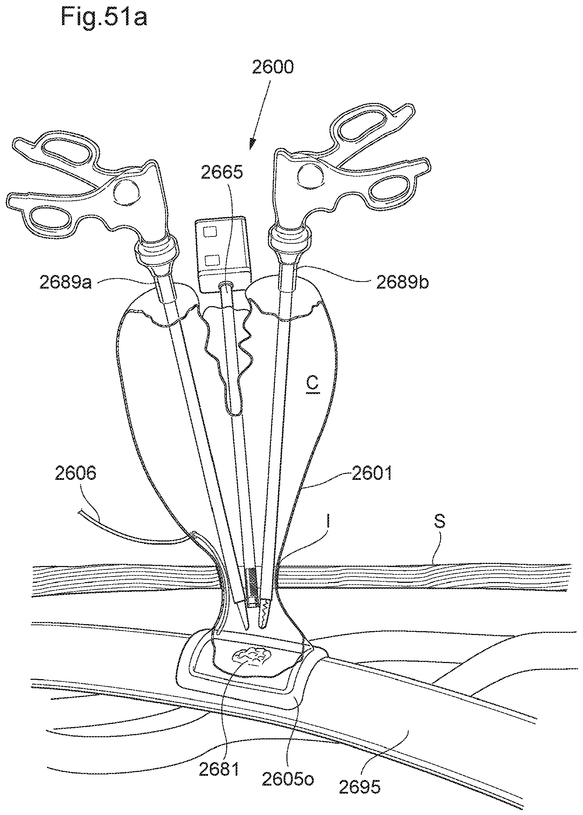

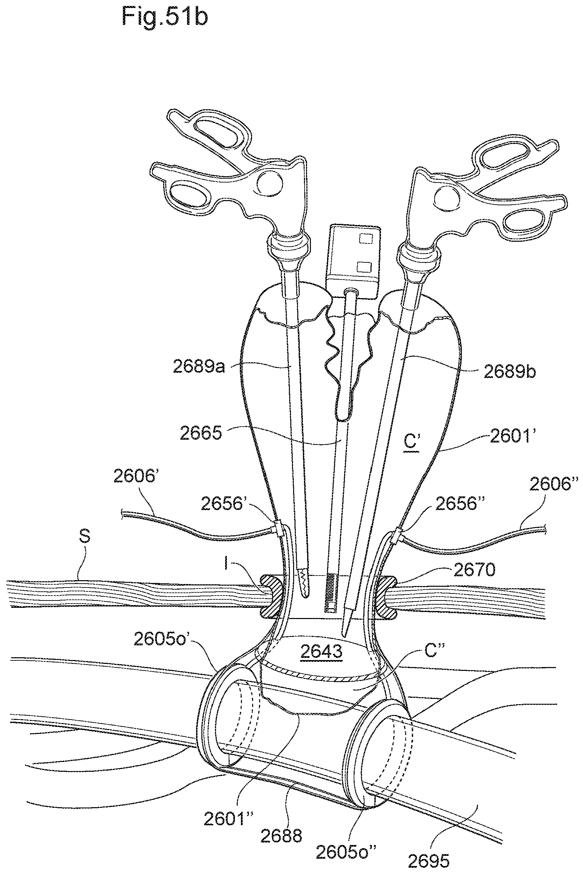

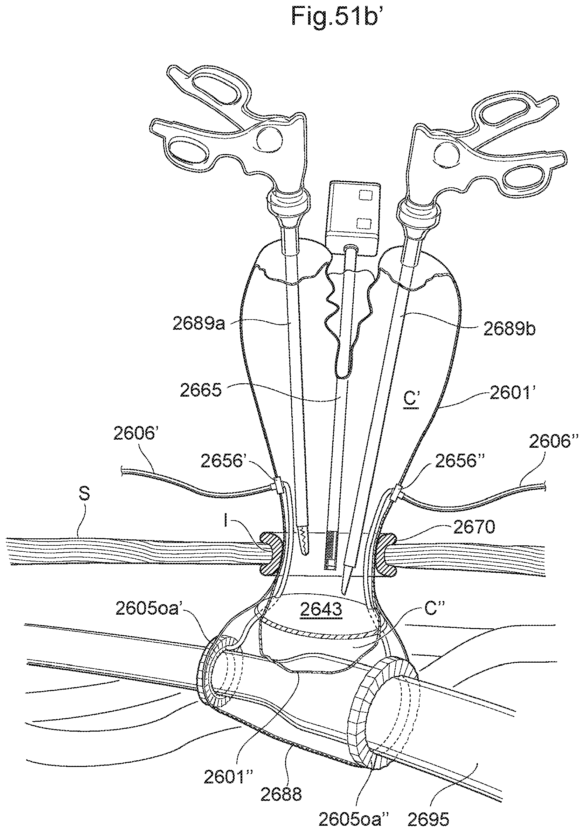

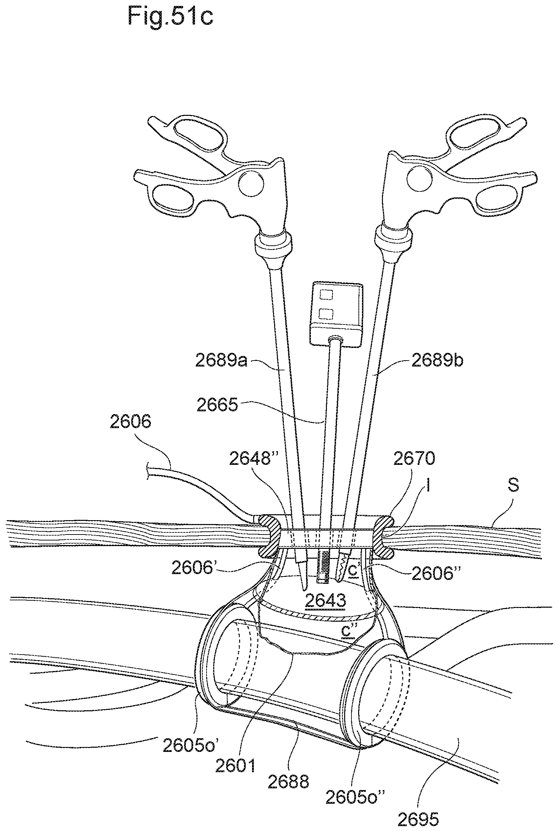

Fig. 5k, 51 shows the medical device when the surgeon removes a specimen 81 from the knee of the patient using the glove 2 integrated in the wall 1 of the medical device.

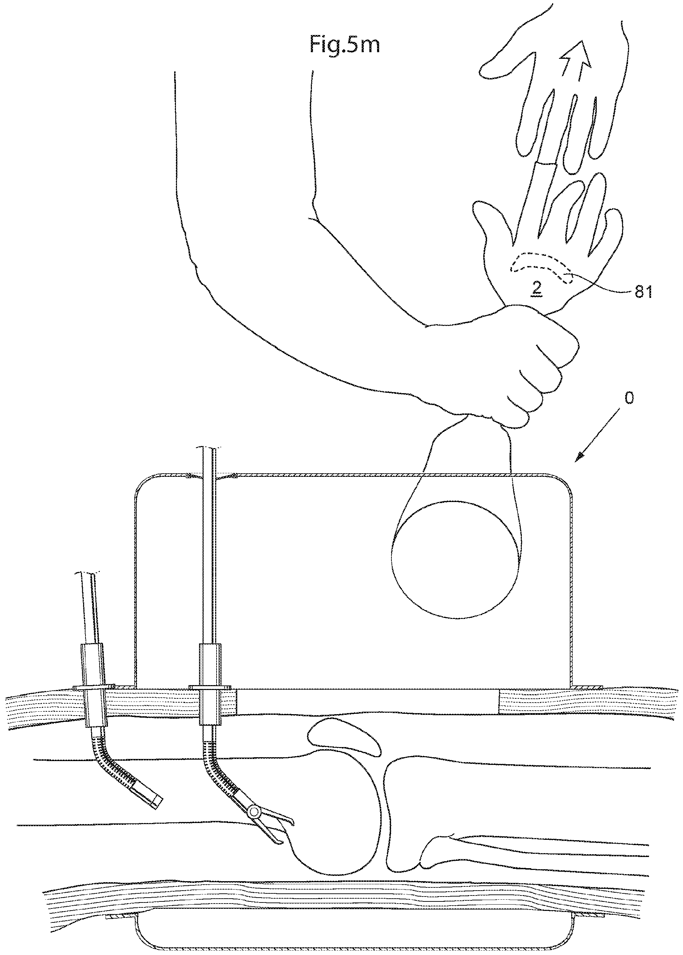

Fig. 5m shows the medical device 0, when the surgeon has turned the integrated glove 2 inside-out such that the specimen 81 can be contained within the integrated glove. By isolating the specimen 81 inside of the glove 2, the specimen 81 is removed both from the rest of the body of the patient and from the ambient environment, and thereby does not risk to infect any other portion of the patient's body or medical staff with any infectious disease.

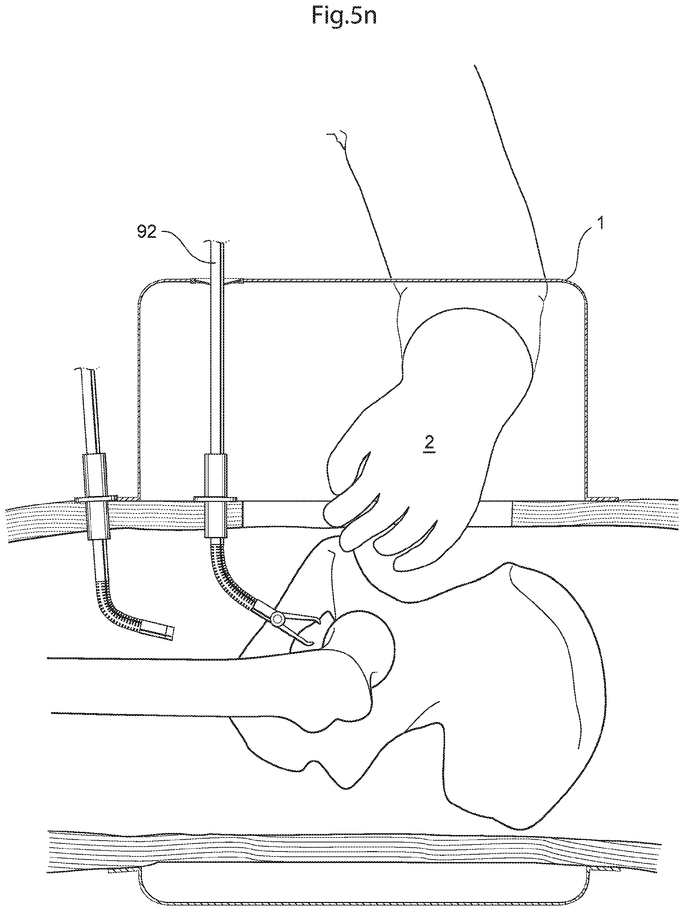

Fig. 5n shows and embodiment similar to the embodiment disclosed with reference to fig. 5j, the difference being that the medical device is adapted for performing procedures on the hip joint of the patient.

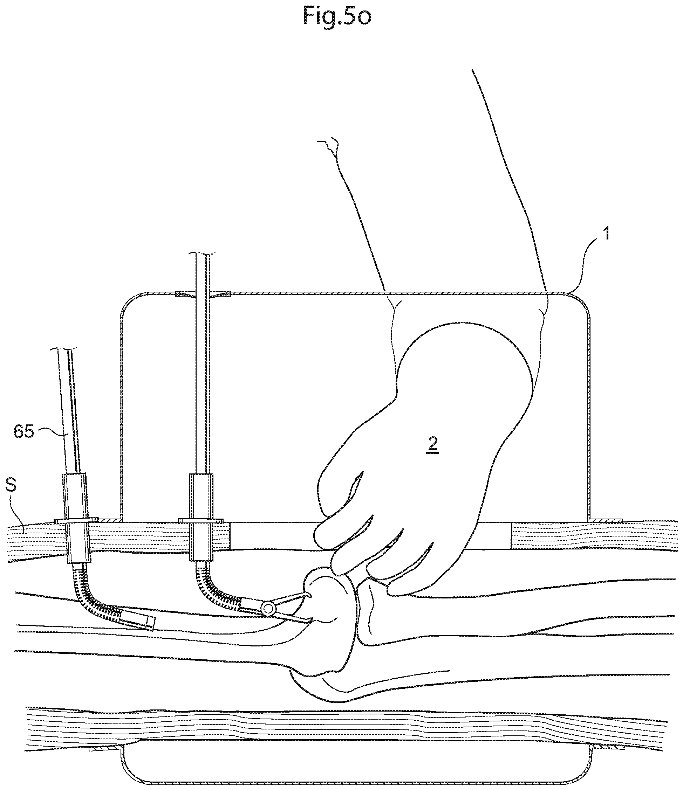

Fig. 5o shows an embodiment of the medical device, in which the medical device comprises a glove 2 integrated in the wall 1 and a camera 65 placed in the skin **S** outside of the medical device. The medical device further comprises a trocar placed through the skin **S** of the patient inside the chamber **C** of the medical device and into an area of the knee of the patient. Fig 5o schematically illustrates how the surgeon manipulates the knee in the sealed environment using the glove.

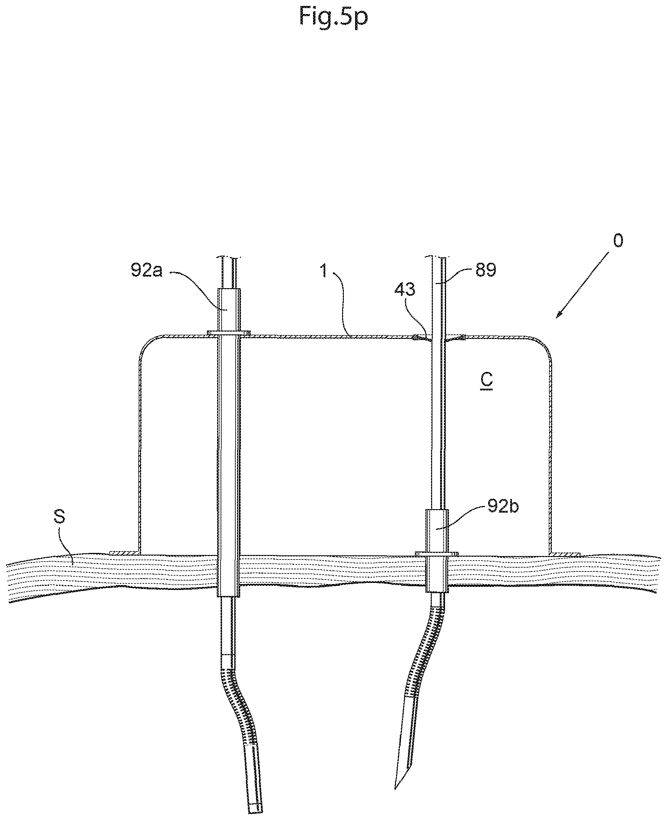

Fig. 5p shows the medical device 0 according to an embodiment in which the medical device 0 comprises a wall 1 placed on the patient's skin **S** and thereby enclosing a chamber **C** for creating a sealed environment. The medical device 0 further comprises two trocars 92a; 92b, one 92a traveling through both the wall 1 of the medical device 0 and the skin **S** of the patient and thus enabling laparoscopic tools to be inserted into the patient through both the wall 1 of the medical device 0 and the skin of the patient through one single trocar. The other trocar 92b being placed inside the chamber **C** and enabling a laparoscopic tool to be inserted through the skin **S** of the patient. The endoscopic instrument is in this embodiment inserted into the chamber **C** enclosed by the wall 1 through a membrane 43 in the wall sealing against the tool 92b.

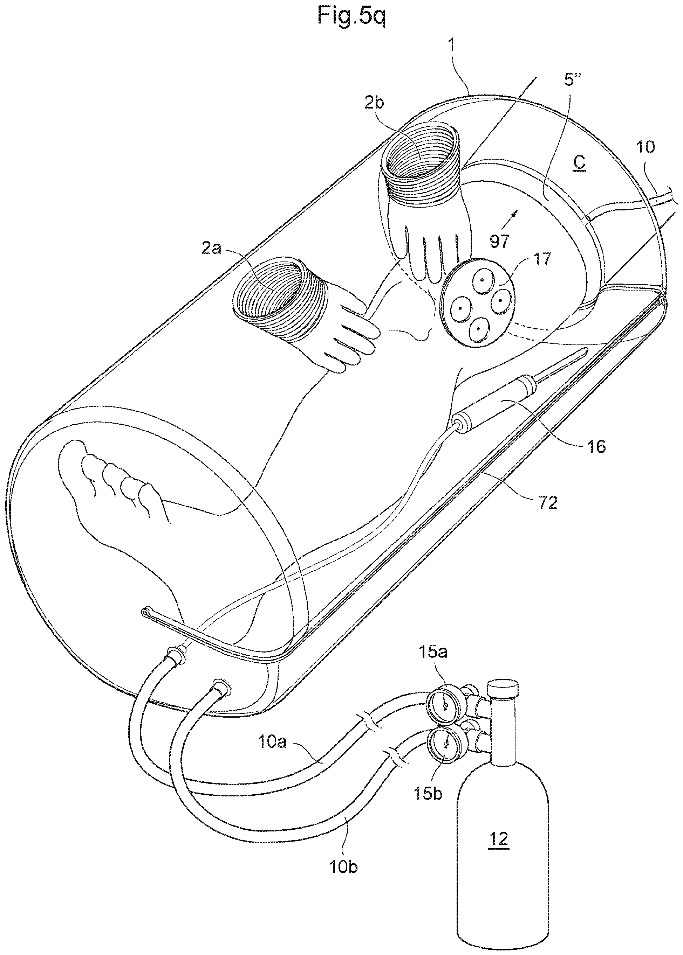

Fig. 5q shows the medical device in an embodiment similar to, an possible to combine with the features of, the embodiments shown in figs. 3a - 3e. The difference being that the wall 1 of the medical device 0 does not form an elongated tubular enclosure extending in the chamber **C,** but instead the wall 1 comprises a hole 97 through which an extremity of the patient is inserted. The hole 97 is surrounded by a sealing device adapted to seal against the skin of the patient by a pressurized sealing member 5" pressing against the skin by means of a hydraulic or pneumatic force being distributed by means of a conduit 10. The wall 1 is placed around the patient's extremity (in this case leg) by being possible to open by means of a connection 72 in the wall comprising a sealing and a closing device, for example as shown in fig. 5q designed as a zipper.

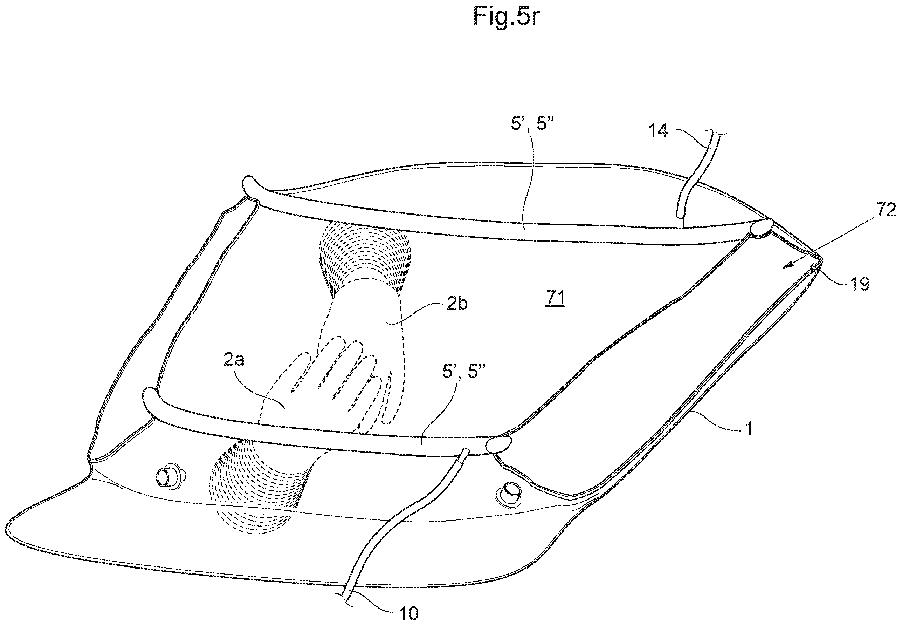

Fig. 5r shows the medical device according to an embodiment similar to the embodiment shown in fig. 5q when in its open state. The difference between the embodiment shown in fig 5q and the embodiment shown in fig. 5r is that the medical device shown in fig. 5r comprises an inner wall, closing or layer 71 which is part of the wall 1 and adapted to separate the chamber of the medical device from the skin of the patient when the medical device is assembled. The medical device comprises a vacuum 5' or pressure 5" sealing member adapted to encircle the extremity of the patient when the medical device is assembled. The connection 72 between different portions of the wall 1 is in 5r, as well as in 5q designed as a zipper 19 adapted for connecting the portions of the wall 1 to each other for creating the chamber.

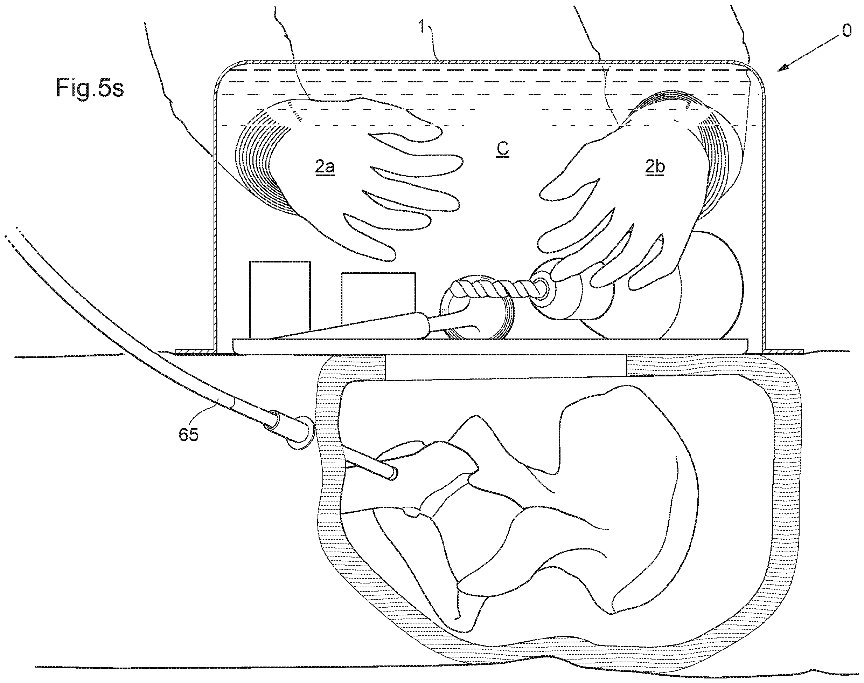

Fig. 5s shows the medical device 0 in an embodiment similar to other embodiments shown herein, the difference being the medical device 0 is adapted to contain a liquid such that a liquid operating environment is created in which an arthroscopic procedure with liquid can be performed. The embodiment shown in fig. 5s is shown with in an embodiment where the medical device 0 is adapted for an arthroscopic hip joint procedure and prosthetic parts and tools are pre-placed inside of the chamber **C** within the wall 1 and thus within the liquid inside of the chamber **C.**

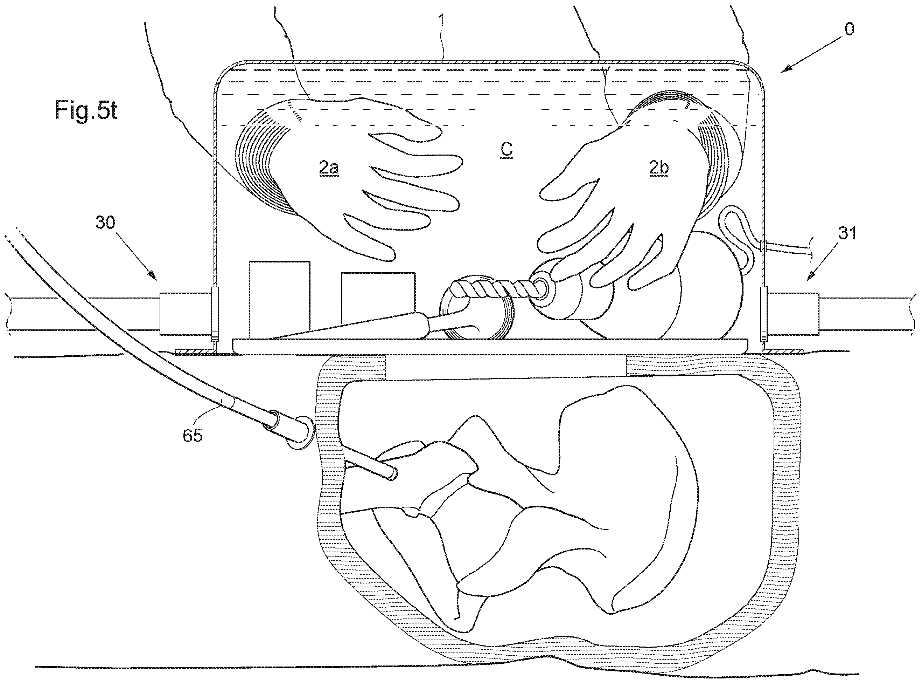

Fig. 5t shows the medical device 0 according to an embodiment similar to the embodiment shown in fig. 5s, the difference being that a system is provided for circulating the fluid inside of the chamber **C.** The liquid system comprises an inlet 30 for injecting the liquid into the chamber **C,** and an outlet 31 for enabling liquid within the chamber **C** to circle.

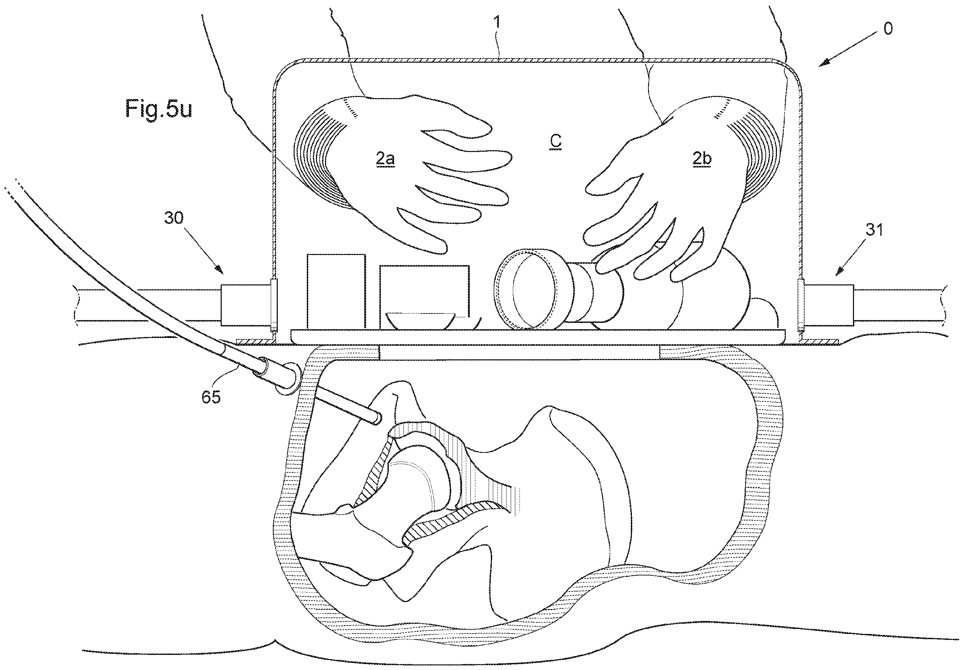

Fig. 5u shows the medical device 0 according to an embodiment similar to the shown in fig. 5t, the difference being that the chamber **C** is not adapted to hold a liquid, but rather a gaseous fluid such as CO₂ gas.

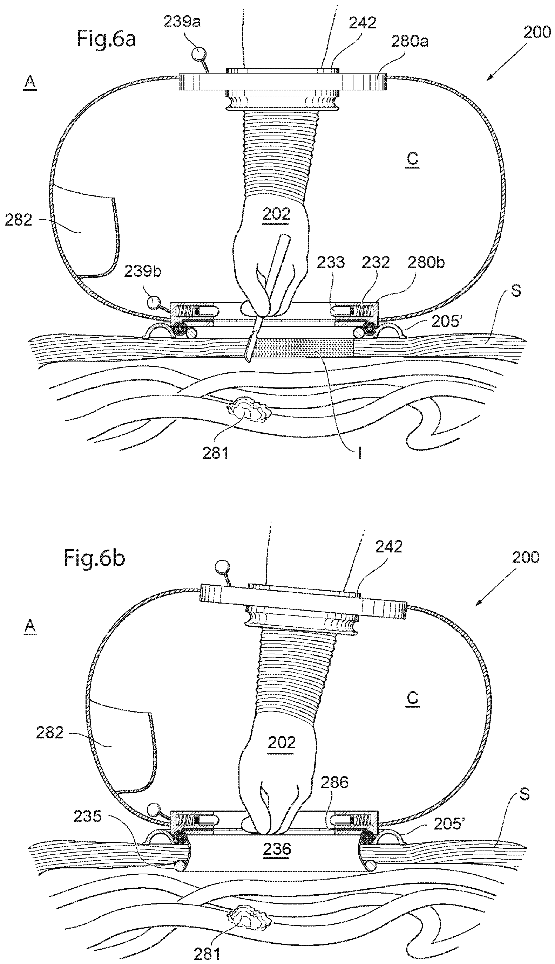

Fig. 6a shows the medical device 200 in an embodiment when the wall 201 of the medical device 200 is inflatable, and the medical device 200 is in its inflated state. When the chamber **C** is deflated it has a first volume i.e. when the first 280a and second 280b couplings are interconnected, and when the chamber **C** is inflated and the first coupling 280a is disconnected from the second coupling 280b it has a second volume, and the second volume is larger than the first volume. According to the embodiment shown in fig. 6a, the second volume is larger than 500 000 mm3. According to the embodiment shown in fig. 6a, the chamber **C** is formed by the inflatable wall 201 and is adapted to hold a pressure exceeding atmospheric pressure.

According to the embodiment shown in fig 6a - 6g the chamber **C** further comprises a pouch 282 for holding a specimen 281 removed from within the body of the patient not to contaminate any other part of the body and create the possibility of delaying the removal of the specimen until the procedure is concluded. The pouch 282 may be a pouch 282 which could be closed for creating a pouch-chamber within the chamber **C** for isolating the removed specimen 281 within the chamber **C.**

In fig. 6a a state is illustrated in which the incision **I** in the patient's skin **S** is being created by the surgeon by means of an inset 242 latched to the first coupling 280a and comprises a glove 202 enabling manual manipulation within the chamber **C.** The second coupling 280b is fixated and sealed by means of the vacuum sealing member 205' since the sealing device that is provided partially within the patient's body cannot be mounted until the incision **I** in the patient's skin is concluded. The two different sealing devices disclosed could in some embodiments be replaced by only one of the sealing device, or by a different seal, such as the adhesive of pressure sealing device disclosed in other portions herein.

Fig. 6b shows the medical device, when the second sealing 235, 236, 237 have been placed in the incision **I** cut in the patient's skin **S.** The wall 201 enclosing the chamber **C is** elastic or flexible which enables the surgeon move the second part 231a in relation to the first part 231b for getting better access to different angles within the patient's body, for example for removing a specimen 281. The embodiment of fig. 6b further comprises an iris valve 286 placed in the second coupling 280b for closing the hole in the second lower coupling 280b such that the chamber **C** is sealed from at least one of the ambient environment **A** and a cavity in the patient. The closing of the iris valve 286 enables the maintaining of a cavity within the chamber **C** without maintaining a pressure in the chamber **C,** at the same time as a pressure and/or sealed environment can be maintained within the body of the patient.

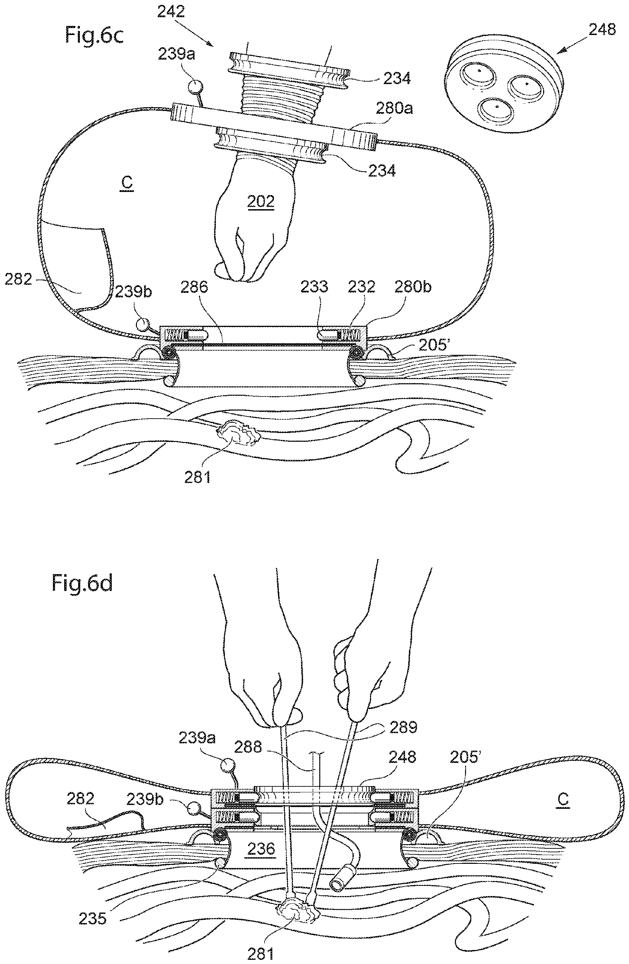

Fig. 6c shows the medical device according to figs. 6a and 6b when the inset 242 comprising the glove 202 is removed and replaced by a body multi-port 248 comprising a plurality of ports. The multi-port may be used for manipulating objects within the chamber **C** of the inflated medical device using surgical instruments 289, or within the body of the human patient, when the first and second couplings are connected (as shown in fig. 6d).

Fig. 6d shows the medical device when the first and second couplings 280a; 280b are connected such that surgical instruments 289 can be used for manipulating within the body of the patient. An endoscopic camera 288 may be provided in the multi port 248 for enabling visual inspection of the cavity within the patient's body. The process of cutting away a specimen 281 from the intestines of a patient in a laparoscopic way is shown in fig. 6d. The first coupling 280a is connected to the second coupling 280b by the protruding members (233 in fig. 6c) of the second coupling 280b engaging the recess (234 in fig. 6c) of the first coupling 280a.

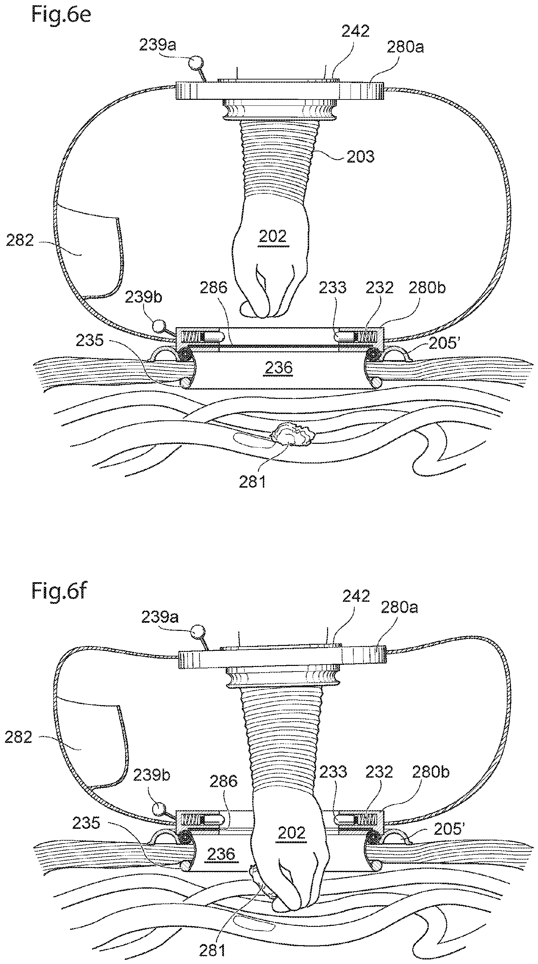

In Fig. 6e, the inset 242 comprising the glove 202 has been fixated to upper coupling 280a to enable the surgeon to operate within the cavity of the patient's body and within the chamber **C** enclosed by the wall 201 of the medical device. By the inset 242 comprising the glove 202 comprising a pleated portion 203, the surgeon is able to reach into the cavity of the patient for removing the specimen 281 dissected from the intestines in prior steps.

Fig. 6f shows that the wall 201 of the medical device is collapsible such that the inset comprising the glove 202 can be moved in relation to the lower coupling 280, which gives the surgeon further freedom when removing the loose specimen 281 from the intestines of the patient.

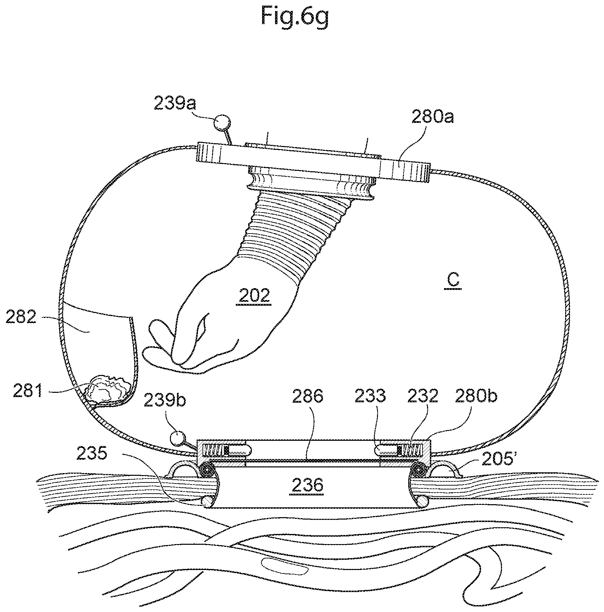

Fig. 6g shows the step of placing the removed specimen 281 in the pouch 282 such that the specimen 281 does not contaminate any other part of the body. The pouch 282 may be a pouch 282 which could be closed for creating a pouch-chamber within the chamber **C** for isolating the removed specimen 281 within the chamber **C.**

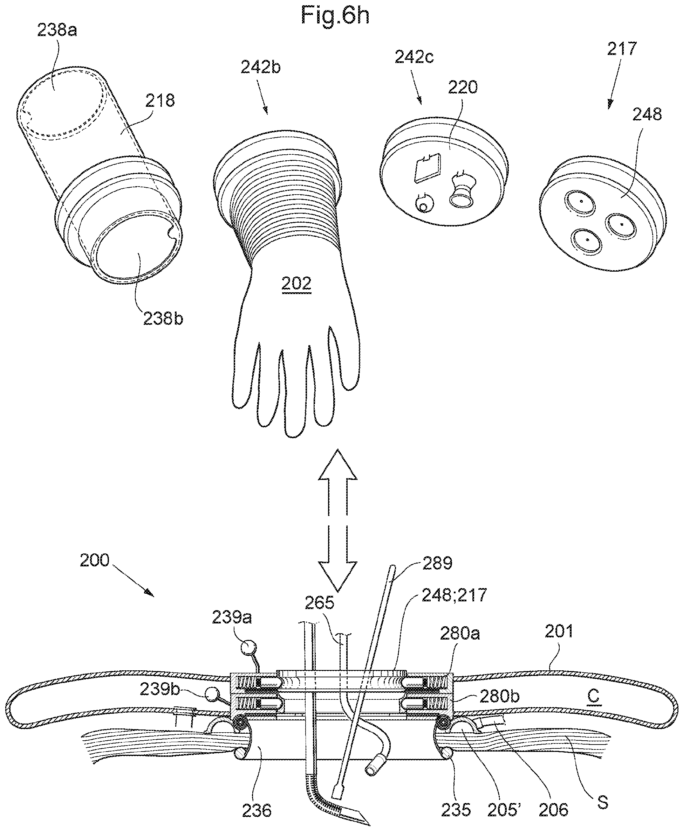

Fig. 6h shows the medical device 200 adapted to be at least partially placed in an incision cut in a patient's body. The medical device 200 comprises a first 280a and second 280b coupling adapted be interconnected. The first upper coupling 280a is adapted to be disconnected from the second lower coupling 280b, and the first upper coupling 280a is connected to a first portion of the flexible wall 201, and the second lower coupling 280b is connected to a second portion of the flexible wall 201. The flexible wall 201 forms a chamber **C** in which a sealed environment can be maintained, the chamber **C** is heavily enlarged when the first coupling 280a is disengaged from the second coupling 280b, thus creating operating space within the chamber **C** enclosed by the wall 201. The first and second couplings comprise latching systems for locking an inset 242 or wall port 217 in the upper coupling 280a. The inset 242b; 242c may for example be a glove 202 for manual manipulation within the body of the patient, or within the enclosed chamber **C,** a device o instrument mount 220 for holding instruments or medical devices within the chamber **C.** Alternatively the insets 242b; 242c can be replaced by an airlock sluice 218 with a first 238a and second 238b valve member or a wall port 217, such as the wall multiport 248 provided in fig. 6h, enabling the insertion of a surgical instrument 289 and a camera 265 into the cavity in the body of the patient.

In the embodiment shown in fig. 6h the medical device is fixated to the body of the patient by means of a vacuum fixating member 205' connected to a vacuum conduit 206, which in turn is connected to a vacuum source. Furthermore, the medical device in fig. 6h comprises a second sealing function having a first sealing member 235 in the form of an intra body ring adapted to be positioned at the inside of the patient's skin around an incision to be performed in the skin and a second sealing member 237 in the form of an outer ring adapted to be positioned at the outside of the patient's skin around the incision, and a spring-loaded or elastic connecting member 236 sealingly interconnecting the first 235 and second 237 sealing members. The spring-loaded or elastic connecting member 236 seals between at least one of the lower coupling 280b and the skin **S** of the patient, and the first sealing member 235 and tissue of the patient.

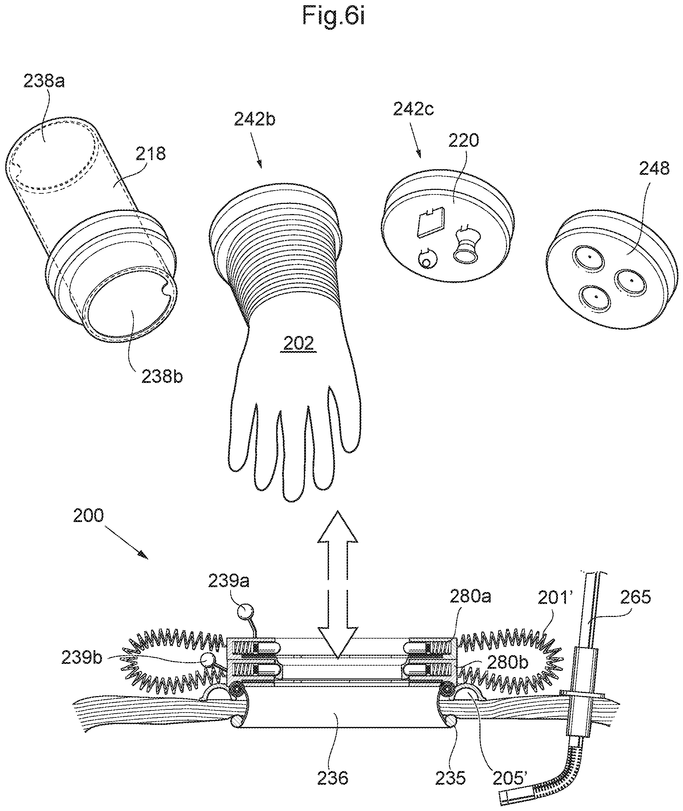

Fig. 6i shows the port in an embodiment very similar to the embodiment shown in fig. 6h but with the difference that the wall 201' has a bellows structure and thus taking up less space when in its deflated state. The bellows structure enables the wall 201' to be packaged in a small package and thus not obstructing the procedure. In some applications the wall can be used only on very special occasions or emergencies, such as when some part of the procedure goes wrong and conversion from laparoscopic surgery to more open surgery is required. The use of the inflated chamber then enables the pressure in the cavity within the patient to be maintained since the chamber can hold a pressure. A camera 265 is also shown in fig. 6i, placed outside of the medical device and enabling visual inspection of the cavity within the medical patient.

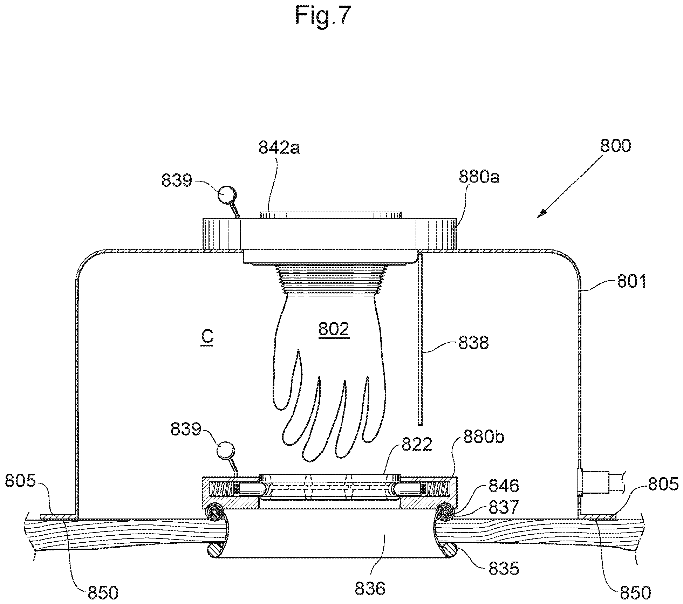

Fig. 7 shows a medical device 800 comprising a wall 801 adapted to be at least partially applied on the patient's body to form a chamber C together with the patient's body, in which a sealed environment can be maintained for encompassing part of the surgical procedure to be performed. The medical device further comprising an upper coupling 880a placed in a portion of the wall 801, an a closeable body port 822 placed in a lower coupling 880b placed in an incision in the patient's body, the medical device 800 further comprises a sealing device 805 connected to the wall 801 and adapted to seal against the skin of the patient. The sealing device disclosed in fig. 7 is comprises an adhesive surface 850 adapted to adhere to the skin of the patient.

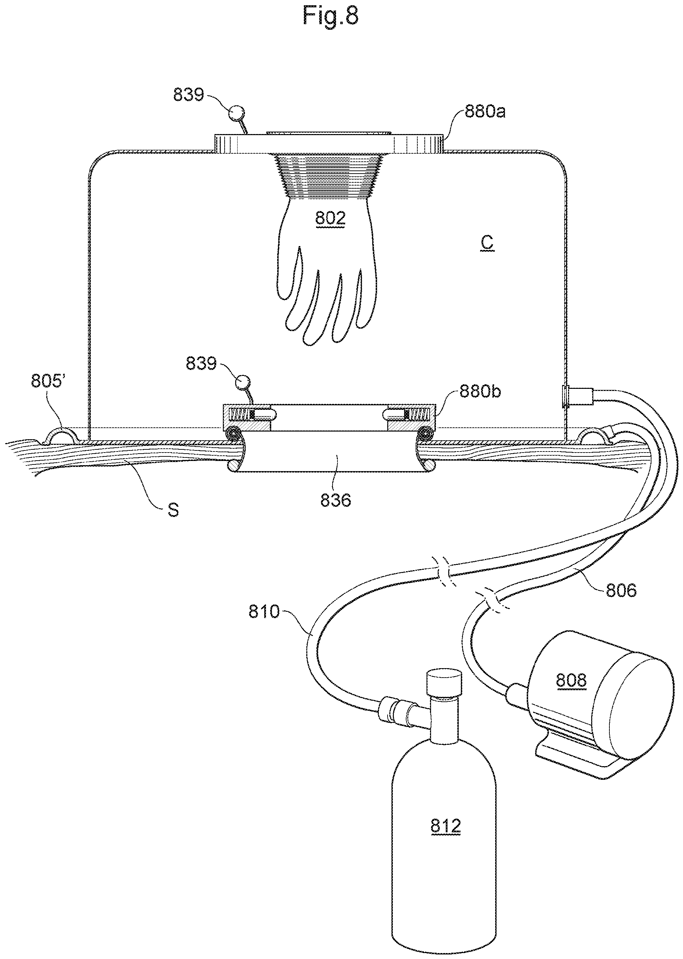

The upper coupling 880a, comprises a valve 838 for closing the coupling 880a when changing from a first to a second object (such as a wall port or wall inset) in the coupling 880a. According to the embodiment shown in fig. 8, the medical device comprises a retractor comprising one intra body ring 835 adapted to be positioned on the inside of the patients skin, and one ring 837 adapted to be placed on the outside of the patients skin. Between the intra body ring 835 and the ring 837 adapted to be placed on the outside of the patients skin a retractor membrane 836 is positioned which is adapted to exert a pressure of the cut surfaces of the incision for retracting the skin and thereby keeping the wound open. The retractor membrane 836 is preferably made from a resilient of elastic polymer material. The ring 837 adapted to be placed on the outside of the patients skin comprises an integrated roll up system 846 which could be a spring loaded roll up system adapted to create a suitable pressure on retractor membrane 836 for keeping the wound open. The lower coupling 880b is fixated to the rigid part, which in some embodiments is an integral part of the wall 801 separating the sealed environment from the ambient environment. The coupling 880a comprises a releasing lever 839 for releasing the object centered therein. The lever 839 is connected to protruding latching members 833 which are spring loaded 832 from the rear in order to secure the recess or groove 834 of the port of the object. In other embodiments, the medical device 800 could be provided with a retractor 836 of a different design, or none at all.

Fig. 8 shows the medical device 800 as shown in fig. 7, when fixated to the skin of the patient by means of a vacuum sealing member 805' connected to a vacuum pump 808 via a vacuum conduit 806. The vacuum sealing member 805' is adapted to seal between the medical device and the skin S of the patient by means of the vacuum sealing member 805' holding a pressure below atmospheric pressure. The sealed environment is here adapted to hold a pressure exceeding atmospheric pressure and is adapted to be inflated through a fluid conduit 810 connected to a pressure source, here being a pressure tank 812. The medical device is adapted to hold a pressure in the sealed environment exceeding atmospheric pressure such that the abdomen of the patient can be inflated for allowing increased visibility in the cavity thereby created. In other embodiments, the vacuum sealing member 805' could be assisted or replaced be an adhesive portion adapted to be connected to the skin S of the patient.

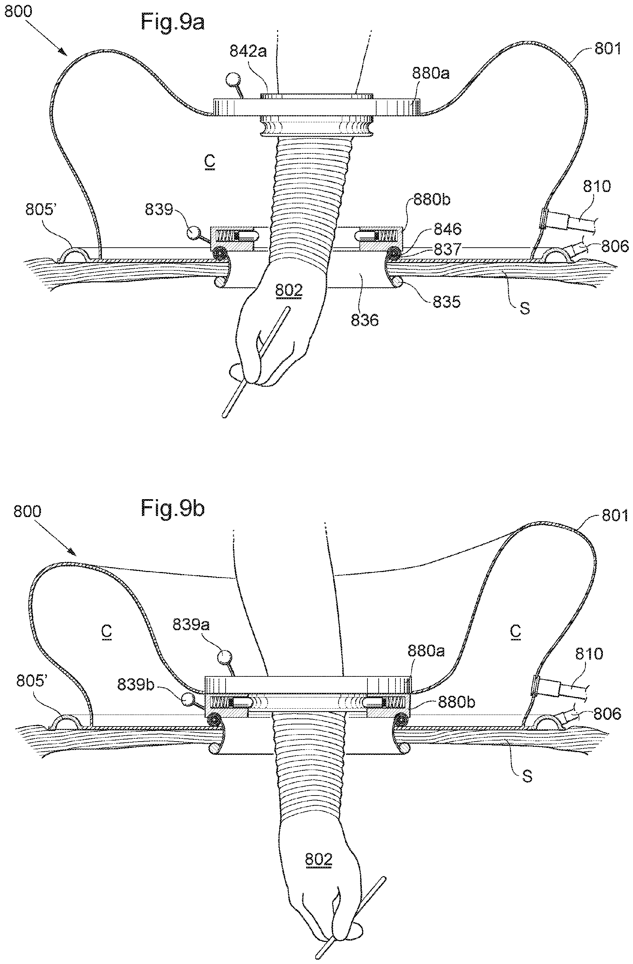

Fig. 9a shows the medical device 800 in an embodiment where the upper coupling 880a in the medical device 800 can be in a first and second position, the second position is disclosed in fig. 9a, while the first position is located closer to the skin of the patient and disclosed in fig. 9b. The medical device could for example be used in surgical procedures where transitions between open and laparoscopic surgery is needed. The medical device 800 could be placed in the second position such that the wall port 831a is locked to the body port 831b for enabling a larger freedom of motion for the surgeon within the chamber C of the medical device 800, while the first position could enable the use of a laparoscopic trocar or hand access for further reach within the body of the patient. The medical device could further be used in embodiments where manual manipulation in a sealed environment is required, e.g. for preparing a medical device 800 for use in the surgical procedure. Other applications could for example be that the surgeon wishes to remove a specimen form the body of the patient after a laparoscopic procedure has been concluded. The laparoscopic procedure could in these instances be performed with the medical device 800 being in the first position (as shown in fig. 9b), being close to the skin S of the patient, whereafter the medical device 800 could be placed in the second position for enabling the specimen to be removed from the body of the patient and placed in the chamber C of the medical device 800 without contacting the ambient environment, and without the release of the pressure that usually fills the abdomen in laparoscopic procedures. According to the embodiment shown in figs. 9a - 11, the wall 801 is collapsible such as to enable the transferring between the first and second position. The lower coupling 880b comprises a lever 839 for releasing and/or locking upper coupling 880a to the lower coupling 880b. The medical device 800 is according to the embodiment shown in fig. 9a fixated to the patient by means of a vacuum sealing member 805.

Fig. 9b shows the medical device 800 when locked in the first position, i.e. the upper coupling 880a is locked to the lower coupling 880b. The integrated surgical glove 802 now enables manual manipulation with great freedom within the body of the patient. The medical device comprises two levers 839a and 839b, wherein the first lever 839a is connected to the upper coupling 880a and adapted to lock an inset 842 or port (here being a wall inset 842a comprising a surgical glove 802) to the upper coupling 880a, while the second lever 839b is connected to the lower coupling 880b for locking the upper coupling 880a to the lower coupling 880b. The medical device 800 is according to the embodiments shown in fig. 9a - 11 is circular when seen from above, such that the medical device 800 takes the shape of a doughnut when placed in the first position.

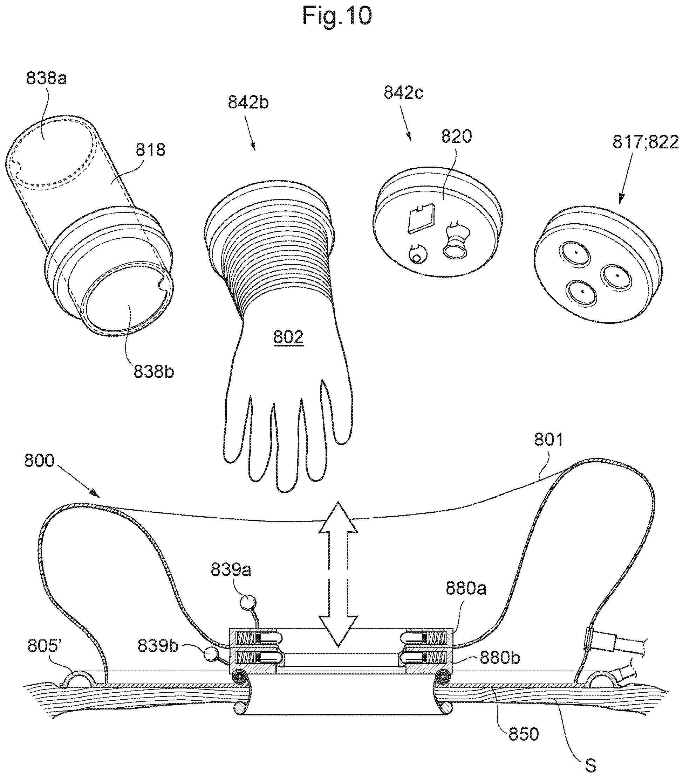

Fig. 10 shows the medical device 800 when locked in the first position, i.e. the upper coupling 880a is locked to the lower coupling 880b. The integrated surgical glove 802 disclosed with reference to the embodiments shown in figs. 7 - 9b could be exchanged by a plurality of different objects of which examples are shown in fig. 10. Fig. 10 shows an airlock 818 comprising a first valve member 838a separating the airlock 818 from the ambient environment, and a second valve 838b separating the airlock 818 from the sealed environment of the chamber C. The airlock 818 is provided between the first 838a and second 838b valve and could for example be used for passing a medical device 800 into the sealed environment, or for passing a specimen or sample out of the sealed environment. Fig. 10 further shows an inset 842c comprising a device or instrument mount 820 which for example could be adapted to hold surgical instruments and/or parts of an implant. The insets could alternatively be exchanged for an endoscopic wall or body port (which could depend on the positioning of the upper coupling 880a), the port may be a multi-port 848 comprising a plurality of endoscopic ports. The medical device 800 is according to this embodiment adapted to be fixated to the skin S of the patient by a large portion of the medical device 800 comprising an adhesive surface 850, and by means of the vacuum sealing member 805'.

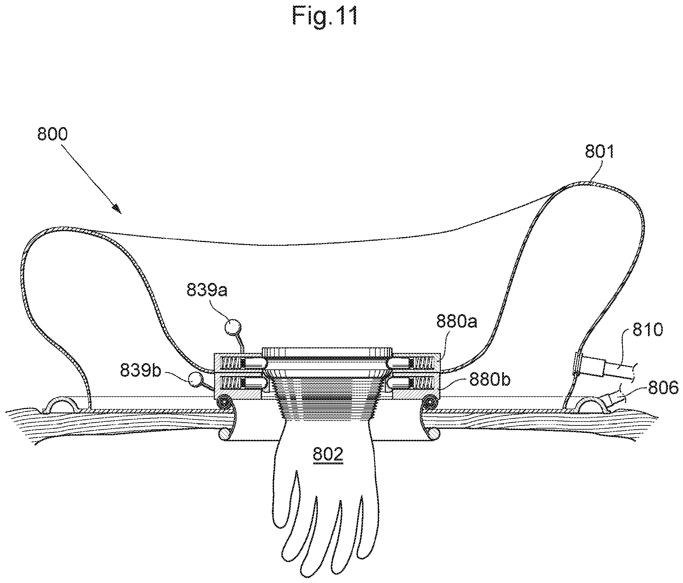

Fig. 11 shows the medical device 800 further disclosed with reference to figs. 7 - 10 when in its first position with the upper coupling of the wall port 831a locked to the lower coupling of the body port 831b. The medical device 800 comprises two levers 839a and 839b, where the first lever 839a in this case locks the inset 842a comprising the surgical glove 802 to the upper coupling 880a, and the upper coupling 880a to the lower coupling 880b.

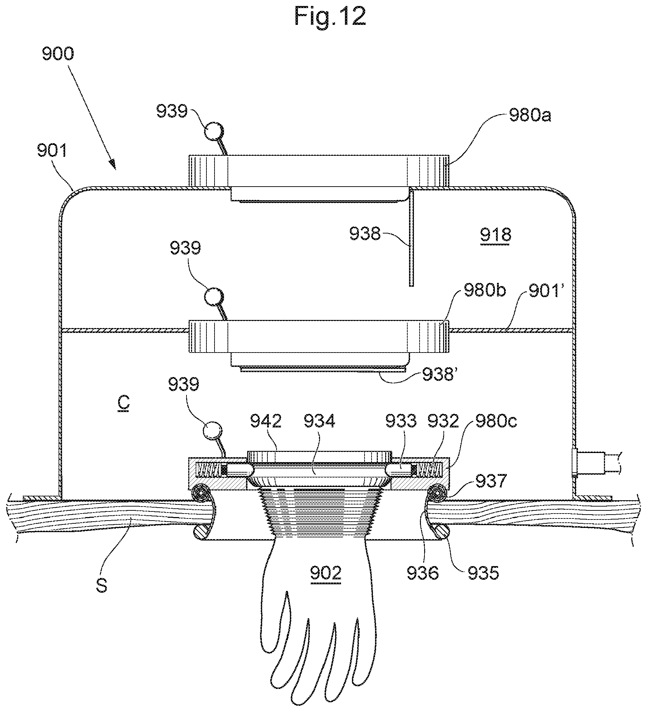

Fig. 12 shows a medical device 900 comprising an upper 980a, middle 980b and a lower 980c coupling for fixating ring shaped objects which for example could be insets or ports, where insets for example could be an inset 942 comprising surgical gloves 902, or devices/instrument mounts, ports could be endoscopic ports or hand access ports, such as the self sealing port disclosed with reference to fig. 4a and 4b. The couplings 980a;b;c comprise releasing levers 939 for releasing the object. The levers 939 are connected to protruding latching members 933 which are spring loaded 932 from the rear in order to secure the recess or groove 934 of the object. In the embodiment disclosed in fig. 12, the inset 942 fixated to the lower coupling 980c is a surgical glove 902 enabling manual manipulation within the body of the patient. The medical device shown in fig. 12 further comprises a valve member 938 adapted to close the opening in the upper coupling 980a, when the upper coupling 980a is not in use. The upper coupling 980a could for example be used for manual manipulation and preparation within the medical device 900, such that could be needed for preparing or changing an instrument or preparing an implant for implantation. The lower coupling 980c is for example used for laparoscopic manipulation within the body of the patient, or manual manipulation for e.g. removing a specimen or positioning an implant. The upper coupling 980a comprises a valve 938 for closing the hole in the coupling 980a such that the chamber C is sealed. The medical device 900 further comprises an intermediary coupling 980b placed in a wall portion 901' and comprising a valve 938'. The valve 938 in the upper coupling 980a and the intermediary coupling 980b together forms an airlock sluice 918 forming part of the chamber C. The airlock sluice 918 is adapted to allow reversible passage of objects from the chamber C through the first valve 938' into the sluice 918 and further through the second valve 938 out to the ambient environment outside of the sealed environment, and vice versa, whereby the risk of contamination is reduced. The first valve 938' is adapted to be opened for an object to pass through whilst the second valve 938 remains closed by itself, thus without any object passing through the second valve 938, which enables the medical device to be hermetically sealed whilst an object passes from the chamber C of the airlock sluice 918 to the chamber C being a part of the operational area.

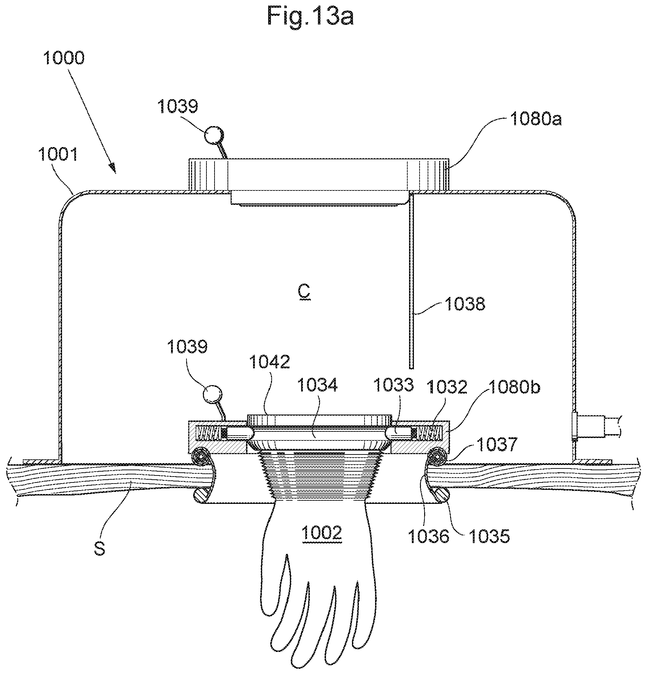

Fig. 13 shows a medical device comprising an upper 1080a and a lower 1080b coupling for fixating ring shaped objects which for example could be insets or ports, where insets for example could be an inset 1042 comprising a surgical glove 1002, or an inset comprising a device/instrument mount. Ports could be endoscopic ports or hand access ports, such as the self sealing port disclosed with reference to fig. 4a and 4b. The coupling comprises a releasing lever 1039 for releasing the object. The lever 1039 is connected to latching protruding members 1033 which are spring loaded 1032 from the rear in order to secure the recess or groove 1034 of the object. In the embodiment disclosed in fig. 13, the inset 1042 fixated to the lower coupling 1080b comprises a surgical glove 1002 enabling manual manipulation within the body of the patient. The medical device shown in fig. 13 further comprises a valve member 1038 adapted to close the opening in the upper coupling 1080a, when the upper coupling 1080a is not in use. The upper coupling 1080a is for example used for manual manipulation and preparation within the medical device 1000, such that could be needed for preparing or changing an instrument or preparing an implant for implantation. The lower coupling 1080b is for example used for endoscopic manipulation within the body of the patient, or manual manipulation for e.g. removing a specimen or positioning an implant.

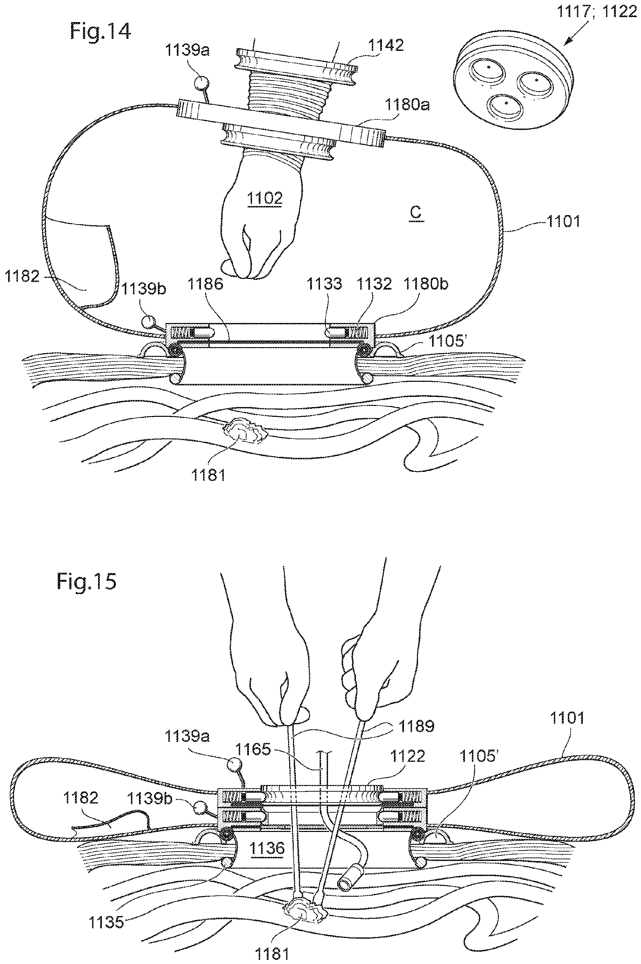

Fig. 14 shows a surgical port when an inset 1142 comprising the glove 1102 is placed in the upper coupling 1180a is removed and replaced by a wall/body port 1117; 1122 here, in the form of a multi port comprising a plurality of ports. The upper coupling 1180a thus forms a wall port. The wall port 1117 may be used for manipulating objects within the chamber C of the inflated medical device using surgical instruments 1189, or within the body of the human patient, when the first upper coupling 1180a and the lower couplings 1180b are connected (as shown in fig. 15).

Fig. 15 shows the medical device when the upper and lower coupling s 1180a;b are connected forming an interconnected port such that surgical instruments 1189 can be used for manipulating within the body of the patient. An endoscopic camera 1165 may be provided in the body port 1122 for enabling visual inspection of the cavity within the patient's body. The process of cutting away a specimen 1181 from the intestines of a patient in a laparoscopic way is shown in fig. 15.

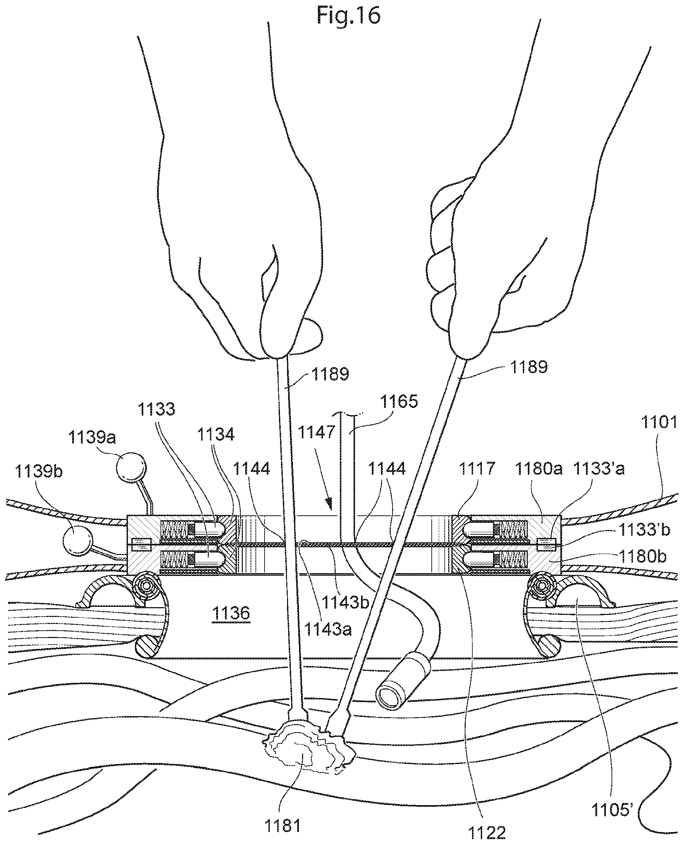

Fig. 16 shows an alternative embodiment of the medical device in which a wall port 1117 is connected to an upper coupling 1180a and a body port 1122 is connected to the lower coupling 1180b. The upper and lower couplings 1180a; 1180b are connected by means of magnetic connecting members 1133'a, 1133'b such that the wall port 1117 and body port 1122 is connected, forming an interconnected port 1147. The first and second part each comprises a latching system comprising protruding latching members 1133 adapted to engage a recess in the form of a groove in 1134 in multiport insets placed in the first 1131b and second 1131a parts. The multiport insets comprise elastic membranes 1143a, 1143b adapted to, in a non-compressed state, provide sealing, and in a compressed state enable a surgical instrument to be inserted through the membrane. The elastic membrane 1143a of the second part 1131a and the elastic membrane 1143b of the first part 1131b are adapted to be placed tightly together such that they act as a single elastic membrane adapted to, in a non-compressed state, provide sealing, and in a compressed state enable the instruments 1189 to be inserted through the membranes 1143a, 1143b. The elastic membranes comprises a plurality of self sealing holes 1144 through which the instruments 1189 travel. The membranes 1143a, 1143b snuggly encircles the instruments and thus maintains the seal between the cavity in the patient and the ambient environment. In alternative embodiments similar to the embodiment shown in fig. 16 at least one of said first and second part comprises a penetrable self sealing gel, such that an object or hand can be inserted through the surgical port while maintaining a seal. Much as in the embodiment of fig. 16 the first part 1131b may comprises a first penetrable self sealing gel and the second part 1131a may comprise a second penetrable self sealing gel. The first and second penetrable self sealing gels may be adapted to be placed tightly together such that they act as a single penetrable self sealing gel adapted to, in a non-compressed state, provide sealing, and in a compressed state enable a hand or an object to be inserted through the self sealing gel while maintaining the seal.

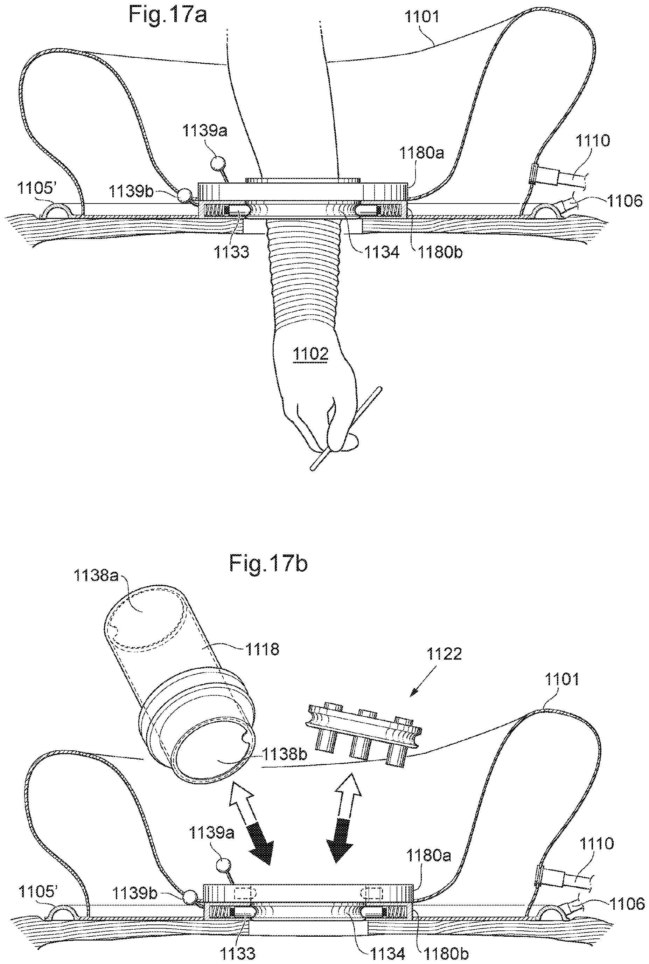

Fig. 17a shows the medical device 1100 when locked in the first position, i.e. the upper coupling 1131a is locked to the lower coupling 1131b. The integrated surgical glove 1102 now enables manual manipulation with great freedom within the body of the patient. The medical device comprises two levers 1139a and 1139b, wherein the first lever 1139a is connected to the upper coupling 1180a and adapted to lock the object (here being a surgical glove 1102) to the coupling 1180a, while the second lever 1139b is connected to the lower coupling 1180b for locking the upper coupling 1180a to the lower coupling 1180b. The medical device 1100 is according to the embodiments shown in fig. 17a - 17b circular when seen from above, such that the medical device 1100 takes the shape of a doughnut when placed in the first position.

Fig. 17b shows the exchanging of objects when the upper coupling 1180a has been locked to the lower coupling 1180b. The object comprises the surgical glove 1102 has been removed and can for example be replaced by an airlock 1118 for transferring objects between the outer environment and the chamber of the medical device. The airlock 1118 comprises an inner wall 1138b and an outer wall 1138a on each side of the airlock 1118 enabling the enclosing of an object within the airlock 1118. Another example of an object to which the surgical glove 1102 can be exchanged is a port device 1122 here shown as a body multi-port comprising laparoscopic ports for performing a laparoscopic procedure. In some embodiments (not shown) the upper and/or lower coupling comprises a valve member for closing the opening in the coupling when the objects should be exchanged, the valve member could for example be a flap valve.

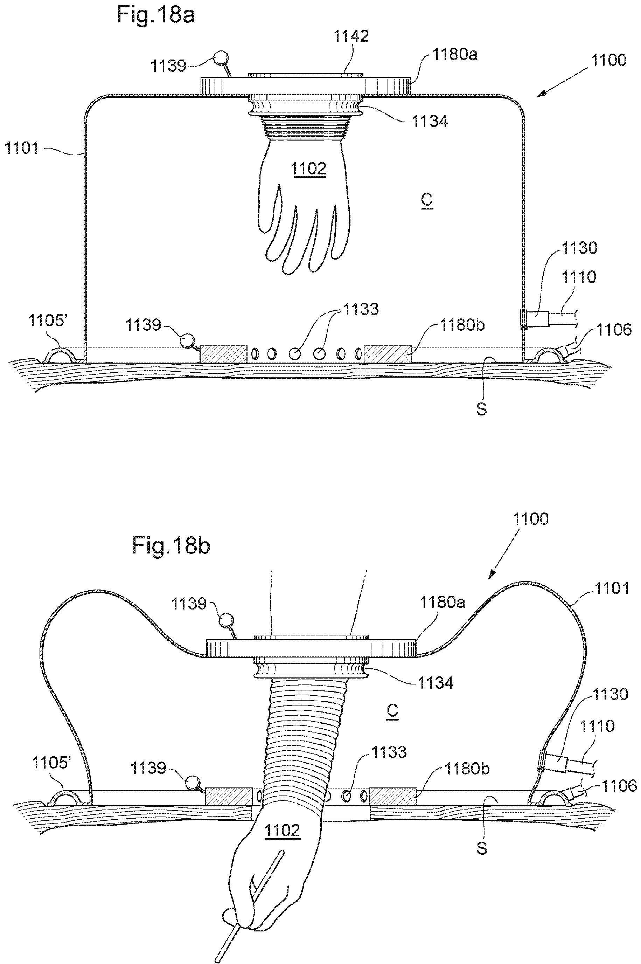

Fig. 18a - 18e shows an embodiment of the medical device where the medical device does not comprise a bottom portion of the wall 1101, i.e. a wall portion contacting the skin of the patient and making the wall of the medical device fully encapsulating the chamber C. In the embodiment shown in figs. 18a - 18e, the chamber C has a circular cross-section perpendicular to the skin of the patient. However, in other embodiments, it is equally conceivable that the medical device has a cross section having a different shape.

Fig. 18a shows a medical device for performing a surgical procedure comprising a wall 1101 adapted to enclose a chamber C together with the skin S of the patient for encompassing part of the surgical procedure. A wall port 1131a is placed in the wall 1101 in which an inset can be positioned (such as the glove inset 1102 shown in fig. 18a). The medical device 1100 further comprises a seal 1105' connected to the wall 1101 and adapted to seal against the patient's body, such that the chamber C is formed by the wall 1101 and the patient's body. In the embodiment shown in fig. 18a the medical device comprises a vacuum seal 1105' adapted to hold a pressure less than atmospheric pressure between the patient's skin S and the vacuum seal 1105' to seal the chamber C by sealing towards the patient's skin S. In alternative embodiments, the vacuum seal 1105' may be replaced or assisted by a pressure seal adapted to hold a pressure above atmospheric pressure between the pressure seal and the patient's skin S to seal the chamber C by sealing towards the patient's skin S.

Fig. 18b shows the medical device 1100 of the embodiment described with reference to fig. 18b when the wall 1101, preferably made from a transparent flexible material is compressed by the wall port is being moved closer to a body port placed in an incision in the skin S of the patient. The wall 1101 comprises an inlet 1130 for supplying a fluid to the chamber C, the wall 1101 being adapted to hold a pressure in the chamber C exceeding atmospheric pressure such that the chamber C is inflated by the fluid. In laparoscopic surgery a cavity within the patient is inflated by means of a pressurized fluid for creating an area in which the laparoscopic surgery can be performed under visual inspection. As the chamber C is adapted to hold a pressure exceeding atmospheric pressure the pressure in the cavity in the patient can be maintained even when the incision in the skin of the patient has been performed. In alternative embodiments, the medical device further comprises a fluid outlet provided in the wall 1101 for discharging the fluid from the chamber C, in which case the medical device further may comprise a pump adapted to circulate the fluid from the outlet to the inlet. The inlet 1101 may further be connected to at least one of: a filtering unit for filtering fluid supplied to the chamber C, a sterilization member for directly or indirectly sterilizing fluid supplied to the chamber C, a fluid tempering device adapted change the temperature of fluid supplied to the chamber C.

In the embodiment shown in fig. 18b the body port comprises protruding members 1133, and the wall port comprises a recess 1134 in the form of a groove adapted to receive the protruding members 1133. The protruding members 1133 are adapted to engage the groove 1134 for locking the wall port to the body port. The protruding members 1133 are connected to a lever 1139 for operating the protruding members 1133 such that the wall port can be quickly released from the body port after having been connected.

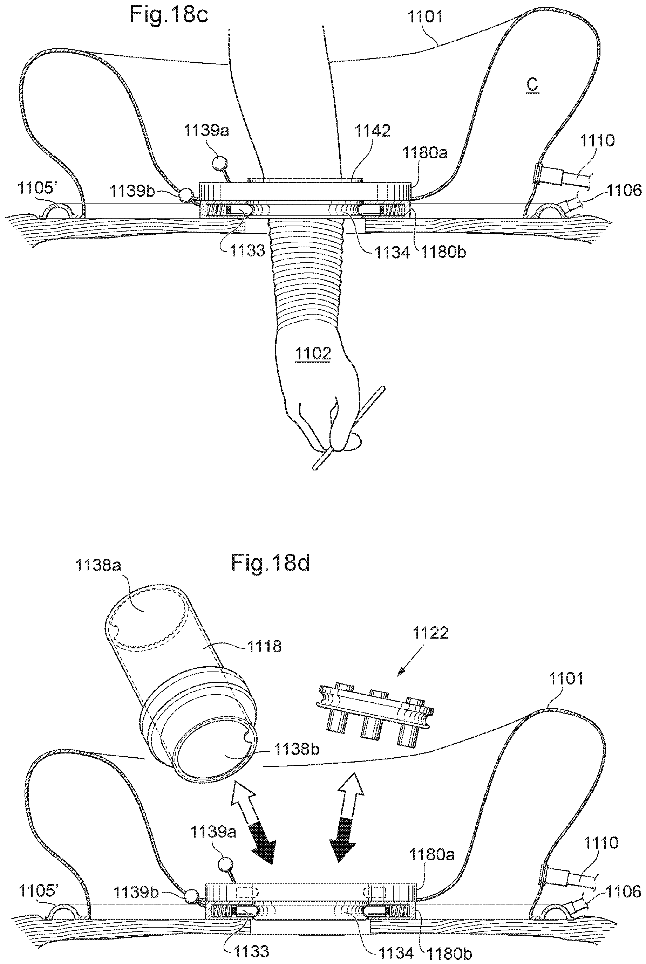

Fig. 18c shows the medical device when the wall port is detachably connected to the body port placed in an incision cut in a patient's body, which enables manual manipulation within the cavity of the patient by means of the glove 1102 positioned in a glove inset fixated to the wall port. The wall port is locked to the body port by the protruding members 1133 of the body port engaging the groove 1134 of the wall port. The chamber C has a smaller volume when the wall port is connected to the body port. According to the embodiment shown in fig. 18c the smaller volume is smaller than 100 000 mm3, however in other embodiments, the smaller volume may be smaller than one of: 50 000 mm3, smaller than 150 000mm3, smaller than 250 000mm3 or smaller than 350 000 mm3, while the larger volume, when the wall port is disconnected from the body port (such as in fig 18a) is larger than 500 000 mm3 for enabling manual manipulation to be made within the chamber C.

Fig. 18d shows the medical device when the wall port has been connected to the body port and the glove 1102 shown in fig. 18c is replaced by either a multi port inset 1122 adapted to enable passage of two parallel objects to the inside of the patient's body, or an airlock sluice 1118 comprising a first valve 1138b sealing between the cavity of the patient and the chamber of the airlock sluice, and a second valve 1138a sealing between the chamber of the airlock sluice 1118 and the ambient environment, such that the cavity of the patient can be sealed at all times even when an object is transferred from the ambient environment to the cavity.

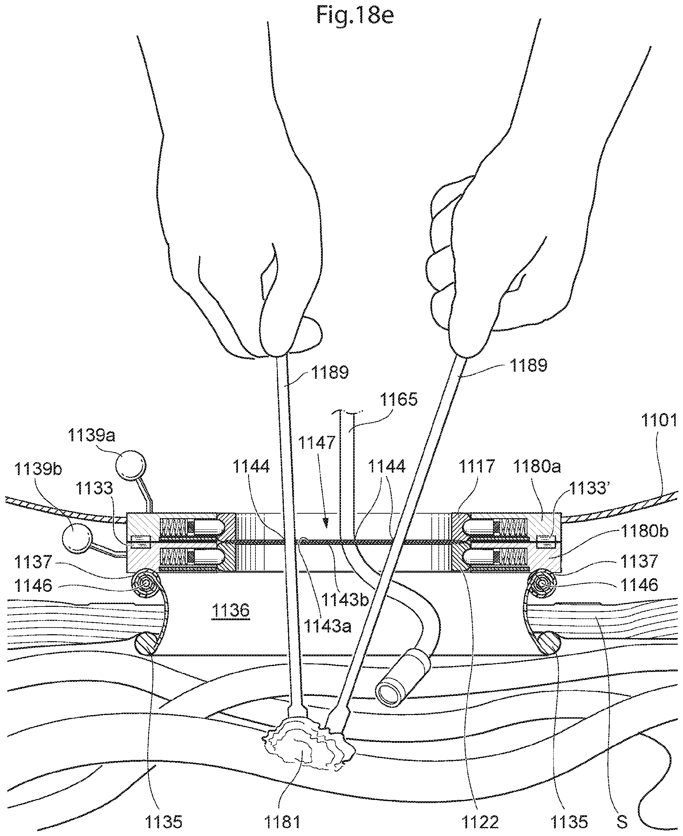

Fig. 18e shows an embodiment when both the wall port and body port comprises disc-shaped self sealing membranes 1143a, 1143b. In the body port, the self sealing membrane is positioned in the top portion of the port, whereas in the wall port, the membrane 1143a is positioned in the bottom part of the wall port. The self sealing membranes 1143a, 1143b have small self sealing holes 1144 such that the instruments 1188 can be inserted through the self sealing membranes 1143a, 1143b. For example, the self sealing membranes 1143a, 1143b may be made from a gel-type material. The wall port, and body port may form an integrated port when the wall port and body port is connected, with the membranes 1143a, 1143b contacting each other such that the instruments 1189 engage an integrated sealing membrane (made up of the first and second membranes 1143a, 1143b) in the integrated port. If the ports had two spaced-apart membranes, the two membranes would reduce the ability to move the instruments 1189 far more than the two integrate membranes acting as a single integrated membrane. The sealing membranes 1143a, 1143b are adapted to, in a non-expanded state, provide a seal, and in an expanded state, enable an instrument 1189 to be inserted through the membranes 1143a, 1143b.

Fig. 18e further shows a fixating/sealing device for the body port. The fixating / sealing device comprises a first sealing member 1135 being an intra body ring 1135 adapted to be positioned at the inside of the patient's skin around an incision performed in the skin, and a second sealing member 1137 in form of an outer ring 1137 adapted to be positioned at the outside of the patient's skin around the incision, and a spring-loaded or elastic connecting member 1136 in form of a retractor membrane 1136 sealingly interconnecting the first 1135 and second 1137 sealing members. The spring-loaded or elastic connecting member 1136 seals between the body port and the tissue of the patient. The fixating / sealing device additionally retracts the skin and thereby keeps the incision open. The retractor membrane 1136 is preferably made from a resilient or elastic polymer material. The outer ring 1137 comprises an integrated roll up system 1146, which may be spring loaded and adapted to create a suitable pressure on retractor membrane 1136 to keep the incision open.

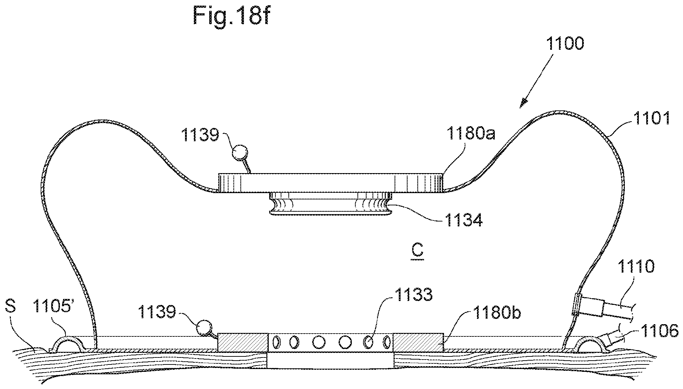

Fig. 18f shows the medical device 1100 when the wall port 1131a is disconnected from the body port 1131b by the protruding members 1133 connected to the lever 1139 being disengaged from the recess 1134 (groove) of the wall part 1131a, such that a additional steps of the surgical procedure can be performed within the chamber C of the medical device 1100. For example, the disconnection could be required when a specimen has been removed from the cavity of the patient and should be removed from the cavity. The specimen may in the embodiment shown in fig. 18f be positioned in the chamber C while the surgical procedure is concluded, such that the environment of the chamber C can remain sealed from the ambient environment when a specimen needs to be removed from the cavity within the patient. In alternative embodiments the wall 1101 may further comprise a pouch for holding the specimen within the chamber C. The pouch can be a closeable pouch, such that a pouch-chamber can be created for isolating the removed specimen within the chamber C.

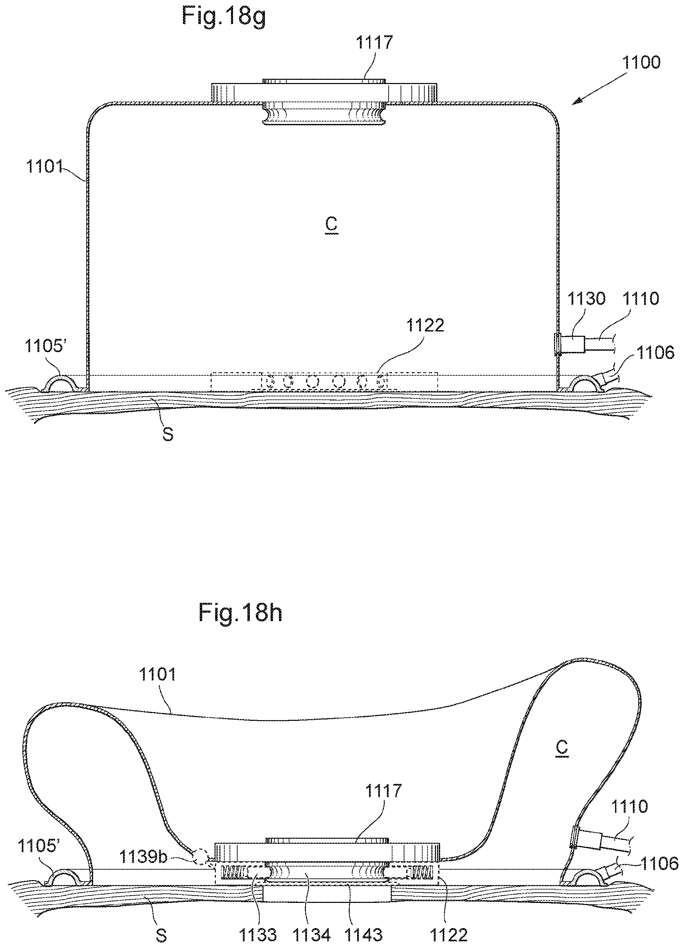

Fig. 18g shows an embodiment of the medical device 1100 in which the medical device comprises a wall 1101 partially applied on the patient's skin S body forming a chamber C together with the patient's skin S, in which a sealed environment can be maintained for encompassing part of the surgical procedure to be performed. The medical device further comprises a closeable wall port 1117 placed in a portion of the wall 1101 and being adapted to be connected to a closeable body port 1122, closeable by means of a self sealing membrane 1143 in the body port 1122. The body port 1122 being placed in an incision in the patient's skin S connecting the chamber C of the medical device to a cavity of the patient's body, when the medical device is applied on the patient's skin S for performing the surgical procedure. The medical device further comprises a sealing device 1105', in the embodiment disclosed in fig. 123'g being a vacuum seal 1105', connected to the wall 1101 and adapted to seal against the skin S of the patient. The wall port 1147a thus forming an airlock sluice together with the body port 1147b, between the cavity in the patient's body and the ambient environment, when the medical device is applied on the patient's body for performing the surgical procedure.

The closeable wall port 1147a comprises an interconnecting member comprising a recess 1134 adapted to connect to an interconnecting member of the body port 1147b in the form of protruding members such that the closeable wall port 1147a is locked to the closeable body port 1147a.

The vacuum seal 1105' shown in fig. 18g is connected to a vacuum conduit 1106 for supplying negative pressure to the vacuum seal 1105'. The vacuum conduit is in turn connected to a vacuum creating member, such as a vacuum pump (disclosed for example under reference to fig. 2a).

A fluid inlet 1130 connected to a fluid conduit 1110 is furthermore shown in fig. 18g. The fluid inlet is adapted for supplying fluid to the chamber C for inflating the wall 1101 of the medical device 1100.

Fig. 18h shows the medical device when an incision has been made in the skin S of the patient and the wall port 1147a is connected to the body port 1147b by means of the protruding members 1133 of the body port engaging the recess or groove 1134 of the wall port 1117. The protruding members 1133 are connected to a releasing lever 1139b for retracting the protruding members and thereby release the wall port 1117 from the body port 1122.

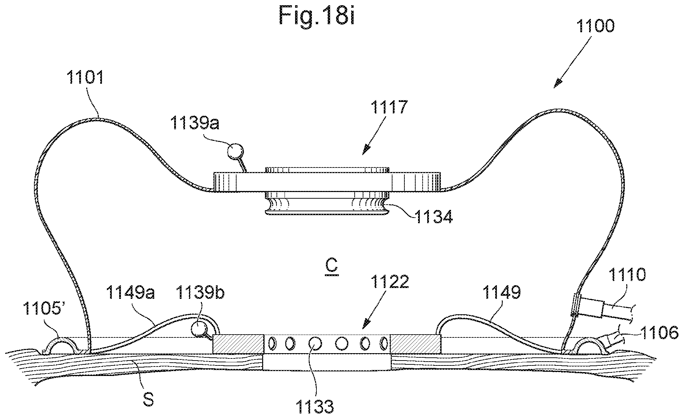

Fig. 18i shows an alternative embodiment of the medical device disclosed with reference to figs. 18a - 18h in which the body port 1122 is fixated by means of supports 1149a, 1149 attached to the sealing device 1105' and/or wall 1101 of the medical device 1100. Fig. 18i shows the wall port 1117 when it is being displaced between a first position, in which the wall port 1117 is fixated to the body port 1122 (such as for example shown in fig. 18h), and a second position in which the wall port 1117 is detached from the body port 1122 and positioned more remote from the body port 1122. The wall port 1117 and body port 1122 are closable by means of self sealing membranes positioned in the wall port 1117 and body port 1122.

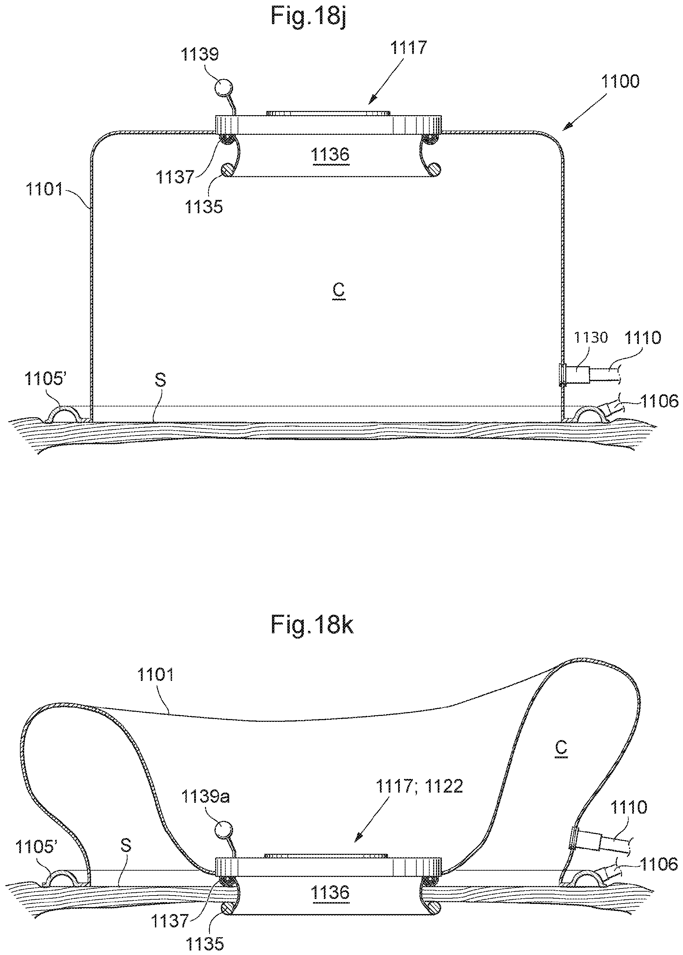

Fig. 18j shows an embodiment of the medical device similar to the embodiment disclosed with reference to figs. 18g - 18i, the difference being that the wall port 1117 comprises a portion adapted to be placed in an incision in the patient's skin for fixating and sealing the wall port in the patient's skin. The portion comprising a sealing adapted to be placed in the incision comprising a first sealing member in the form of an intra body ring 1135 adapted to be positioned at the inside of the patient's skin around the incision. The sealing further comprises a second sealing member in the form of an outer ring 1137 adapted to be positioned at the outside of the patient's skin around the incision, and a spring-loaded or elastic connecting member 1136, sealingly interconnecting the first 1135 and second 1137 sealing members, such that the spring-loaded or elastic connecting member 1136 seals between the second sealing member and the skin S of the patient and/or the first sealing member and the tissue of the patient inside of the patients skin S.

Fig. 18k shows the medical device shown in fig. 18j, when a portion of the wall port 1117 is placed in the incision in the skin S of the patient, such that the intra body ring 1135 fixates the wall port 1117 to the skin S of the patient. The port 1117/1122 now becomes a body port separating the cavity in the patient from the ambient environment and enabling laparoscopic steps to be performed through the port 1117/1122 inside the cavity in the patient. If a specimen needs to be removed from the cavity in the patient, the portion of the wall port placed in the incision can be retracted from the incision, or the portion could be disconnected from the rest of the wall port, thus keeping the wound open whilst allowing a step of the operation to be performed in the full-size chamber of the medical device through the wall port.

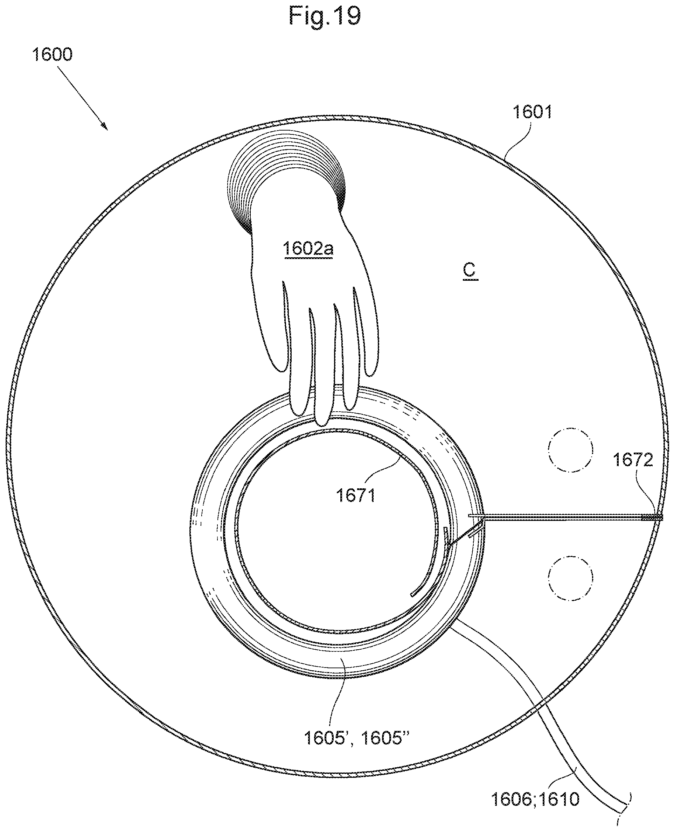

In the embodiment shown in fig. 19, an elongated tubular enclosure is formed by placing a wall 1601 forming the chamber around a portion of the extremity of the patient and closed by means of a connection 1672 which is positioned between the inner wall 1671, in connection with the skin of the patient, and the outer wall 1601. The connection connecting one portion of the medical device with a second portion of the medical device could for example be made by means of an adhesive, a mechanical connection system, or a vacuum system. The chamber C is placed encircling the extremity of the patient, in a side view. The inner layer 1671 is placed with an overlap and the connection 1672 is closed by means of the zipper. The medical device is herein provided with a pressure or vacuum sealing member 1605'; 1605".

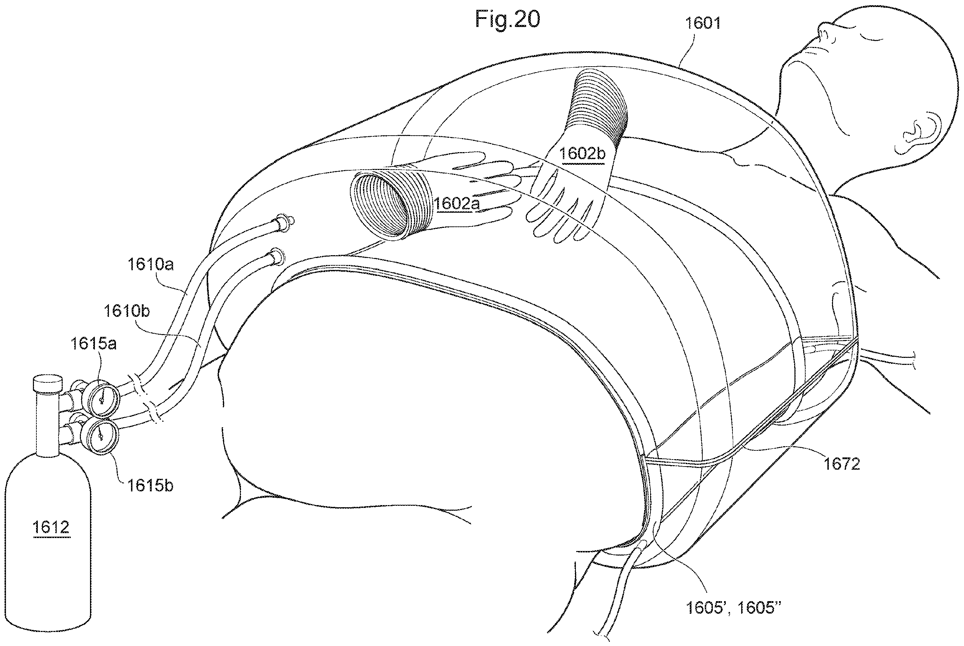

Fig. 20 shows the medical device when applied on a portion of the upper body of a patient, thus enabling surgical procedures in the abdominal or thoracic regions. The chamber is wrapped around the patient by means of the medical device comprising a slit and connected by means of the connection 1672 and sealed against the skin of the patient by means of pressure 1605' or vacuum 1605" seals. The slit and the connecting members enables the placement of the chamber around at least a portion of the extremity of the patient radially in relation to the length axis of the extremity.

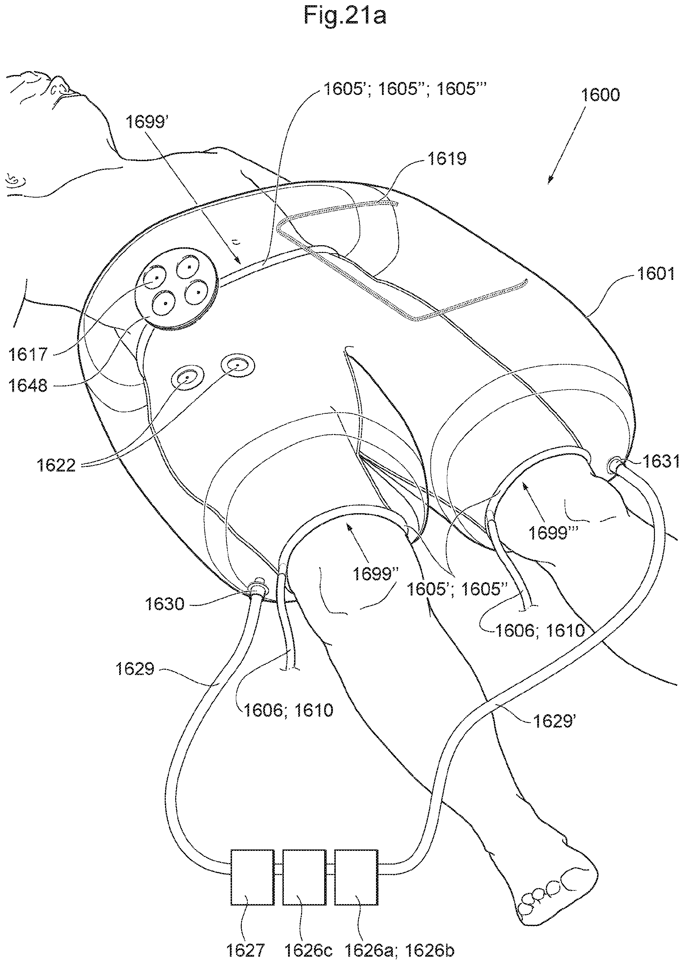

Fig. 21a shows an embodiment of the medical device in which the medical device 1600 comprises three open ends adapted to receive the two legs of the patient and the upper body, accordingly. The medical device 1600 is thus designed as a pair of pants adapted to be fitted onto the patient to provide a pants-like chamber surrounding the patient and being adapted to be inflated to provide an isolated environment in which a surgical procedure can be performed. The open ends encircling the patient's legs above the patient's knees are equipped with seals, here shown as vacuum 1605' or pressure 1605" seals adapted to be connected to vacuum 1606 or pressure 1614 conduits. The open end encircling the upper body of the patient is equipped with an adhesive seal 1605, however it is equally conceivable that the adhesive seal is supported or replaced by a vacuum or pressure seal, such as the seals disclosed encircling the legs of the patient. The medical device comprises a system for circulating a fluid inside of the camber of the medical device. The system comprises a first conduit 1629' connected to an outlet 1631 from the chamber and to a unit for heating 1626a and/or sterilizing 1626b the fluid. The system further comprises a filtering unit 1626c and a pump 1627 for circulating the fluid to a second conduit 1629 and to an inlet 1620 placed in the wall of the medical device. The medical device is also shown with a multi port 1648 placed in the wall and comprising four ports 1647 for enabling transfer between the ambient environment and the chamber. The medical device also comprises endoscopic ports 1622 placed in the skin of the patient for enabling an operation to be performed within a joint or a cavity in the patient's body. A zipper-closed opening 1619 is further provided in the wall of the medical device and adapted to enable transfer from the ambient environment into the inside of the chamber, of for example medical devices and/or implants. The medical device 1600 shown in fig. 157a is for example ideal for use in a hip joint surgery where the entire surgical area can be isolated from the ambient environment and the operation can be performed in a sterile liquid or gas circled in the chamber.

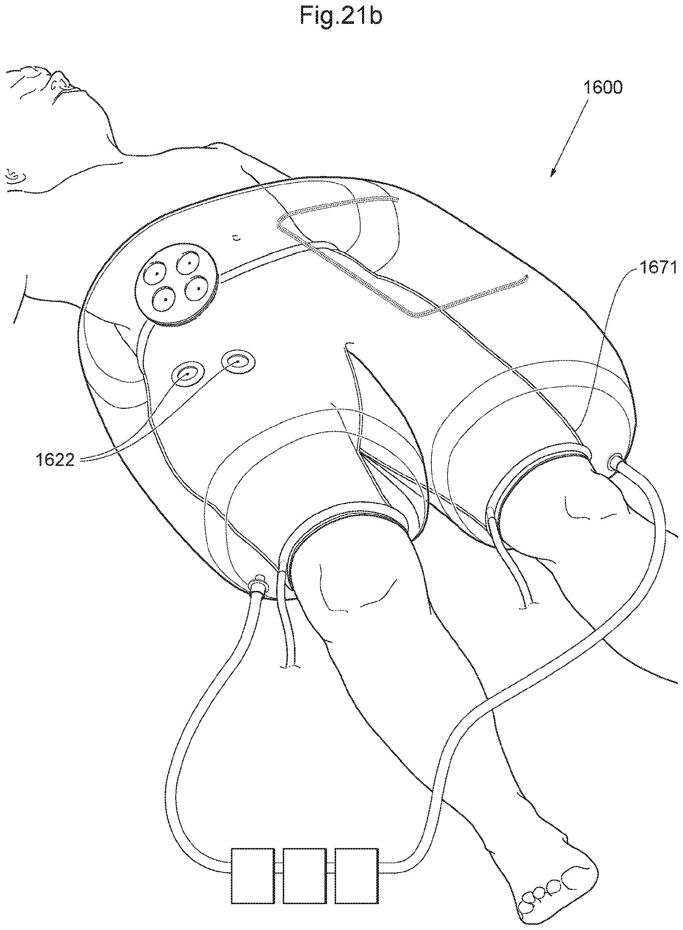

Fig. 21b shows the medical device 1600 in an embodiment comprising the features described with reference to fig. 21a with the difference that the medical device of fig. 21b further comprises an inner layer 1671 such that the chamber is formed by the wall 1601 without involving the patient's body. The inner layer 1671 forms a tubular enclosure formed by the wall 1601. The inner layer thus separates the chamber from the skin of the patient such that the environment in the chamber is not contaminated by for example bacteria which could reside on the patient's skin. The inner layer can be cut through when the incision is to be made in the patient's skin such that only a very small portion of the patient's skin needs to be exposed to the environment in the chamber. The inner layer 1671 could for example be an adhesive polymer layer. In the embodiments shown in fig. 21b two body ports 1622 are placed in the wall of the inner layer. The body ports may be pre-placed in the wall, or may be placed in the wall after the medical device has been positioned with the body of the patient. The advantage with placing the ports 1622 in the inner wall 1671 and in the body of the patient after the medical device 1600 has been applied on the patient's body is that the ports 1622 can be contained within the chamber of the medical device before being placed in the skin of the patient, such that the chamber serves as a sterile package for the body ports 1622.

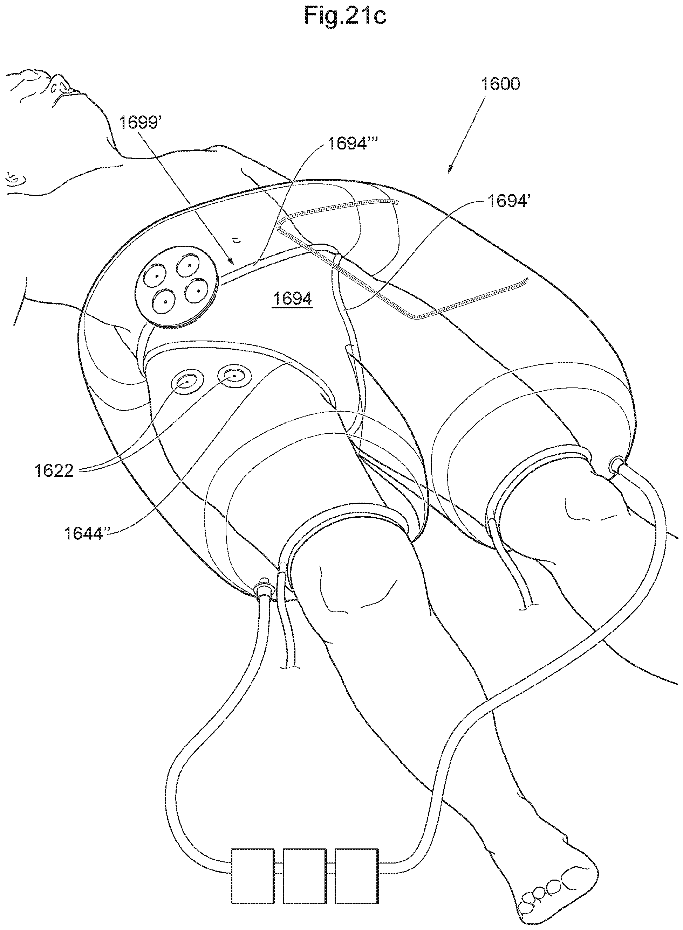

Fig. 21c shows the medical device in an embodiment including the features of the embodiment disclosed with reference to fig. 21a. The difference being that the embodiment of fig. 21c comprises an additional sealing subdevice 1694 comprising a wall and a sealing device adapted to be placed between the chamber and the skin of the patient. The sealing subdevice is formed to separate the anus or opening of urethra from the chamber to avoid connection between the anus or opening of the urethra and the chamber. The sealing subdevice 1694 comprises seals in the inguinal area 1694'; 1694" which could be adhesive, pressure or vacuum seals. The sealing subdevice is further connected at a connecting joint 1694‴ to the open end of the medical device 1600 encircling the upper body of the patient, however it is furthermore conceivable that the sealing subdevice comprises a separate seal adapted to seal between the medical device 1600 and the skin of the patient.

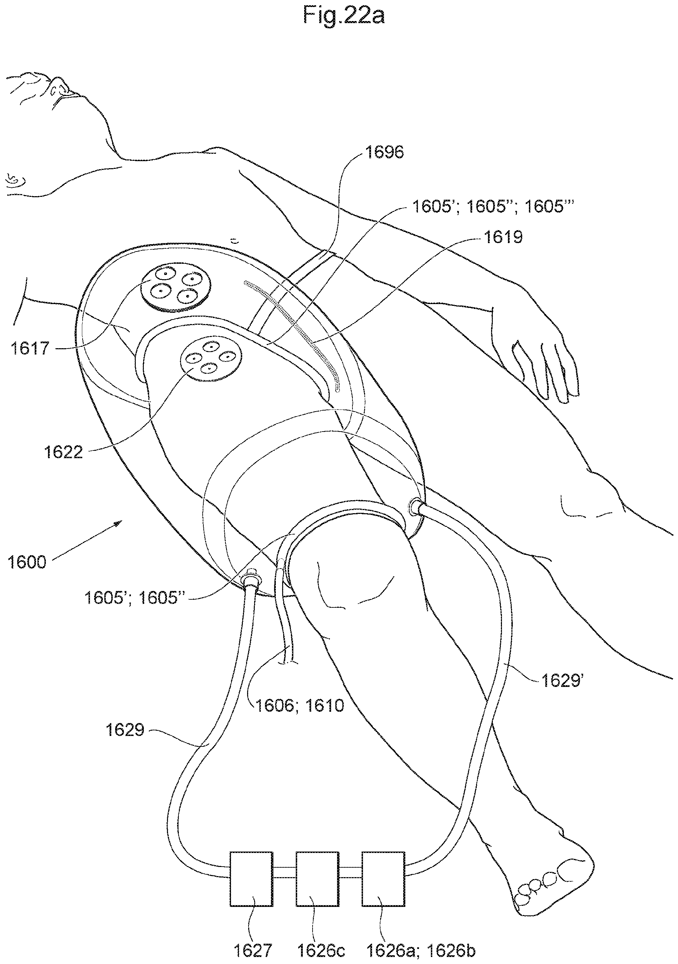

Fig. 22a shows a medical device similar to the medical device shown in fig. 21a with the difference that the medical device of fig. 22a is only adapted to be placed on one side of the patient and provide sealing 1605; 1605'; 1605" in the inguinal area. One advantage with the construction of fig. 22a is that the anus and urethra openings are separated from the chamber, and the fluid volume in the device is smaller in comparison to that of the embodiment disclosed in fig. 21a. The features included in fig. 22a are equivalent to the features included in the embodiment disclosed in fig. 21a.

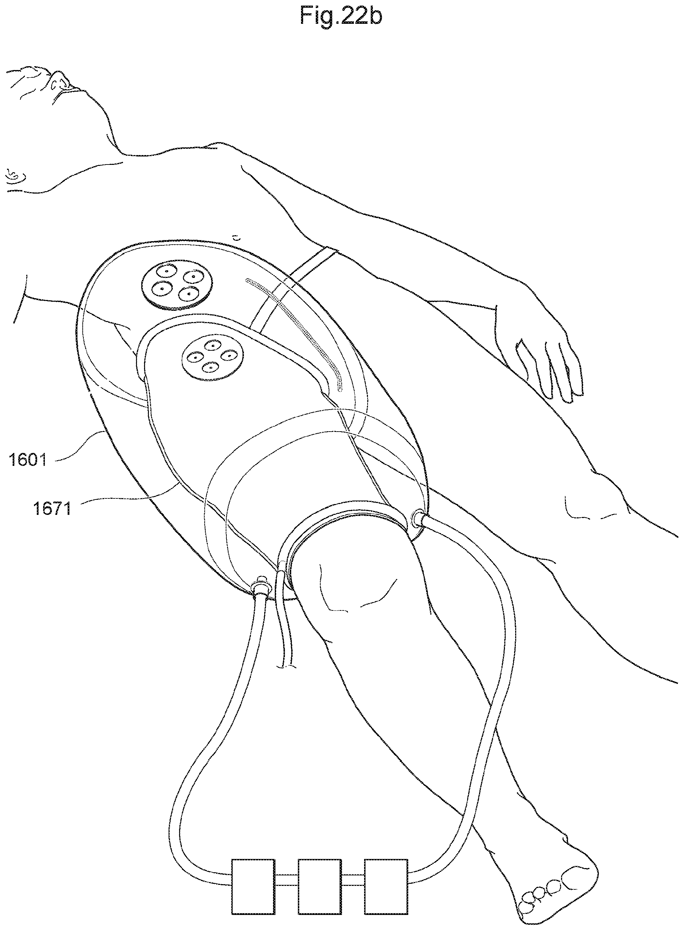

Fig. 22b shows the medical device in an embodiment similar to the embodiment shown in fig 22a, the difference being that in the medical device in the embodiment of fig. 22b comprises an inner wall 1671 in the same way as 21b. The inner wall 1671 forms an elongated tubular enclosure through which the leg of the patient is placed. The inner wall 1671 forming the elongated tubular enclosure thus separates the skin of the patient from the chamber of the medical device such that the chamber is formed by the wall 1601 and the inner wall 1671 without involving the skin of the patient.

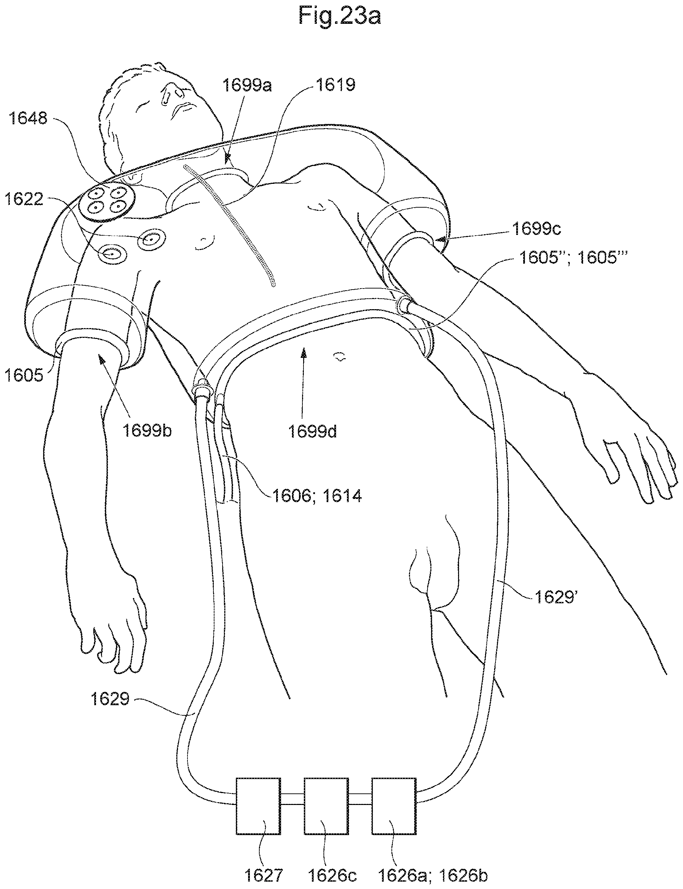

Fig. 23a shows an embodiment of the medical device 1600 similar to the embodiment disclosed with reference to fig. 21a, the difference being that the embodiment of fig. 23a comprises 4 open ends and is adapted to accommodate the upper body of the patient an seal at the arms, neck and chest of the patient. The seals could be any one of the seals 1605; 1605'; 1605" disclosed herein, i.e. adhesive, pressure or vacuum. The medical device further comprises a system for circulating a fluid, wall 1648 and body 1622 ports and zipper-closed opening 1619, all further described with reference to fig. 21a. The embodiment of fig. 23a is for example suitable for performing an arthroscopic shoulder joint procedure.

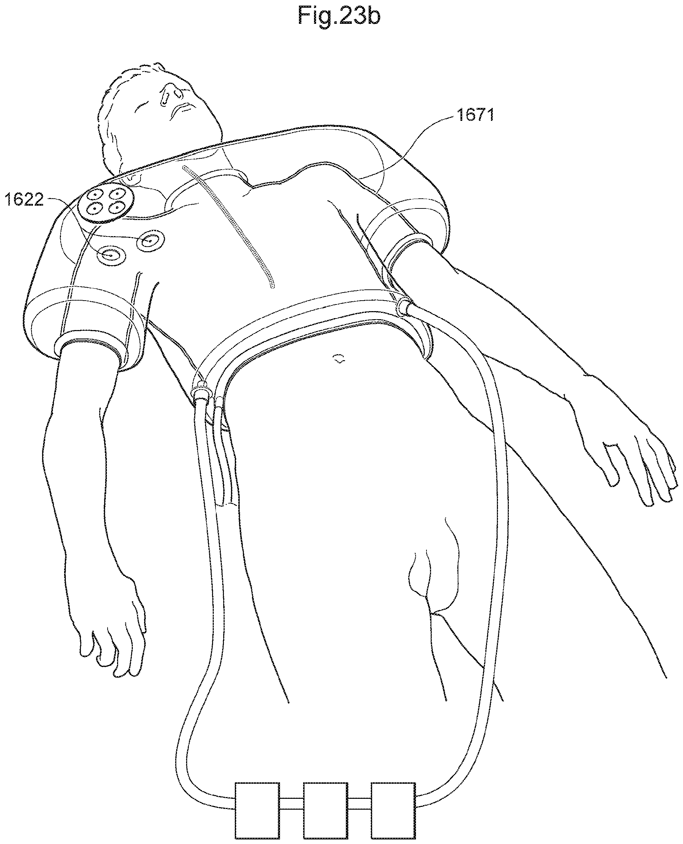

Fig. 23b shows an embodiment of the medical device comprising the features of the embodiment disclosed in fig. 23a, with the difference that the medical device in the embodiment of fig. 23b comprises an inner wall 1671, much like in the embodiment described with reference to fig. 21b. The inner wall 1671 is adapted to separate the skin of the patient from the chamber of the medical device and can be cut through when the incision in the patient for performing the surgical procedure should be performed.

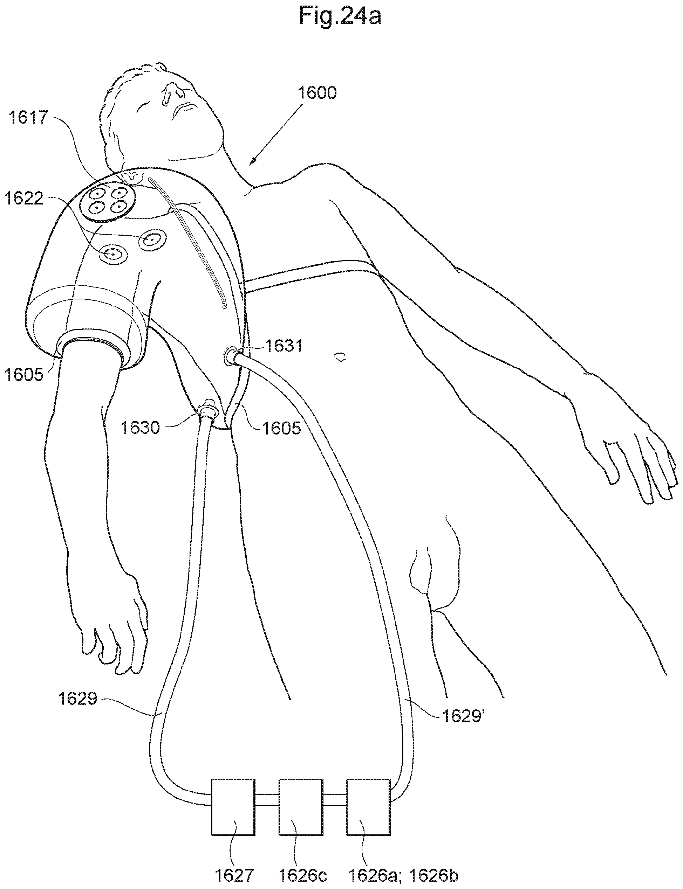

Fig. 24a shows a medical device 1600 similar to the medical device 1600 shown in fig. 23a with the difference that the medical device of fig. 24a is only adapted to be placed on one side of the patient and provide sealing 1605 along the side of the patient's chest. One advantage with the construction of fig. 24a is that the fluid volume in the device is smaller in comparison to that of the embodiment disclosed in fig. 23a. The features included in fig. 24a are equivalent to the features included in the embodiment disclosed in fig. 23a.

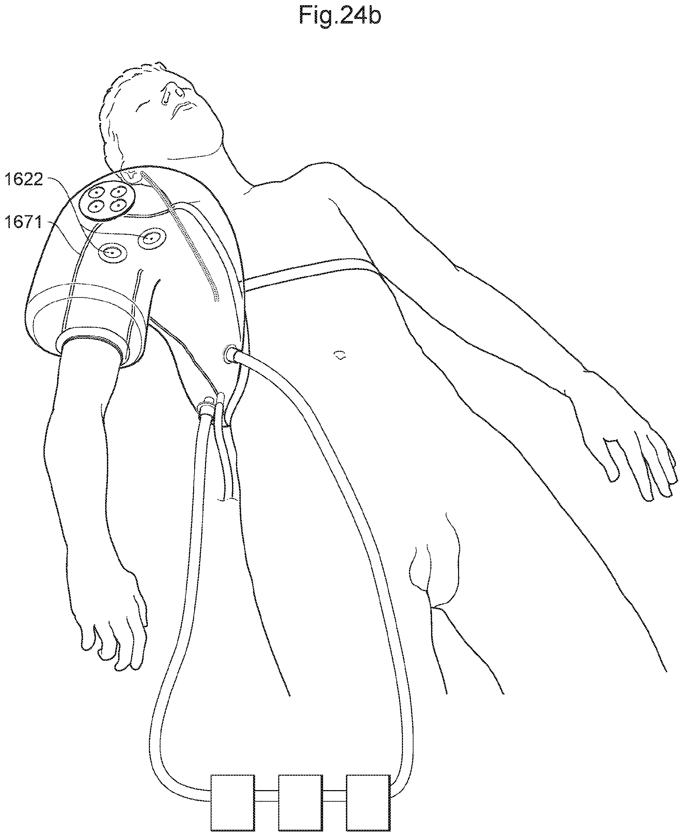

Fig. 24b shows an embodiment of the medical device similar to the medical device described in fig. 24a with the difference that the medical device of the embodiment in fig. 24b comprises an inner wall 1671, much like in the embodiment described with reference to fig. 23b. The inner wall 1671 is adapted to separate the skin of the patient from the chamber of the medical device and can be cut through when the incision in the patient for performing the surgical procedure should be performed.

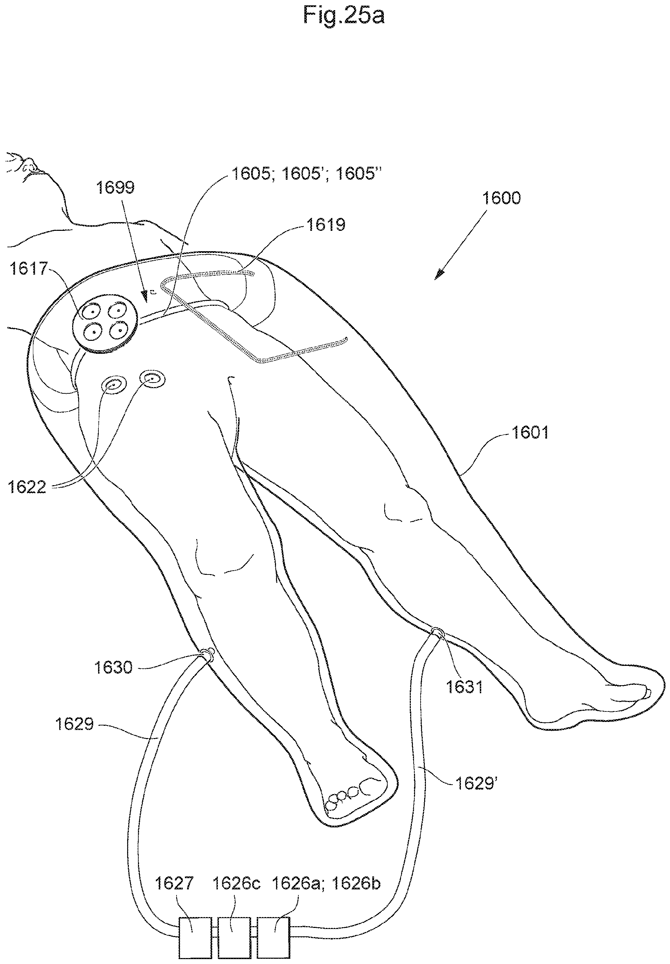

Fig. 25a shows an embodiment of the medical device in which the medical device only comprises one open end adapted to encircle the upper body of the patient and thereby provide sealing against the skin of the patient. The sealing could comprise any one of the seals disclosed herein, i.e. adhesive, pressure or vacuum seal. The medical device further comprises a system for circulating a fluid, wall 1648 and body 1622 ports and zipper-closed opening 1619, all further described with reference to fig. 21a. The embodiment of fig. 25a is for example suitable for performing an arthroscopic procedure involving the pelvis.

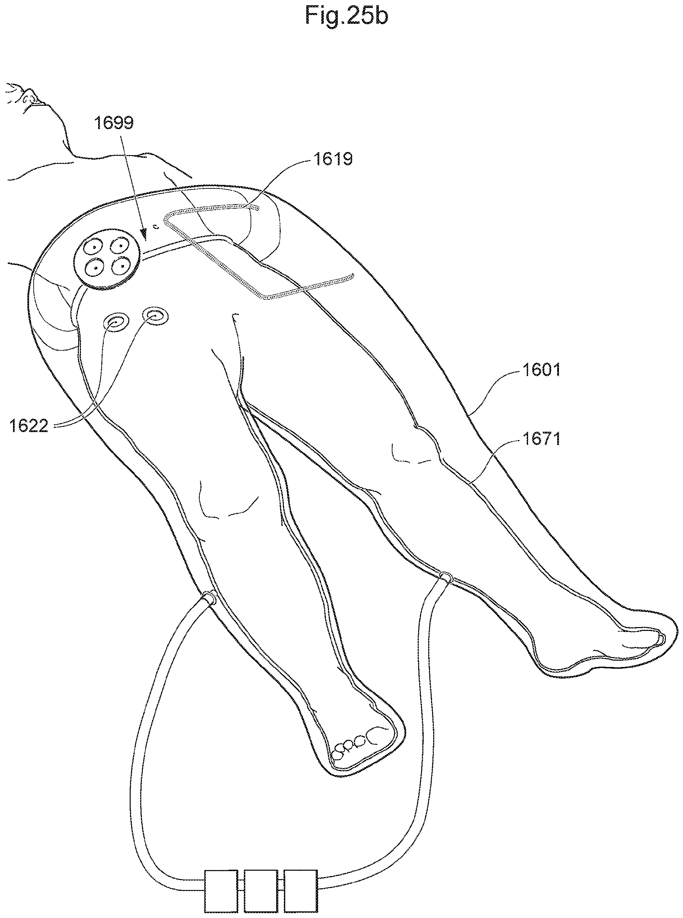

Fig. 25b shows an embodiment of the medical device similar to the medical device described in fig. 25a with the difference that the medical device of the embodiment in fig. 25b comprises an inner wall 1671, much like in the embodiment described with reference to fig. 21b. The inner wall 1671 is adapted to separate the skin of the patient from the chamber of the medical device and can be cut through when the incision in the patient for performing the surgical procedure should be performed. The inner wall 1671 has the advantage of separating the feet of the patient from the chamber which is beneficial since the feet of the patient is difficult to properly sterilize.

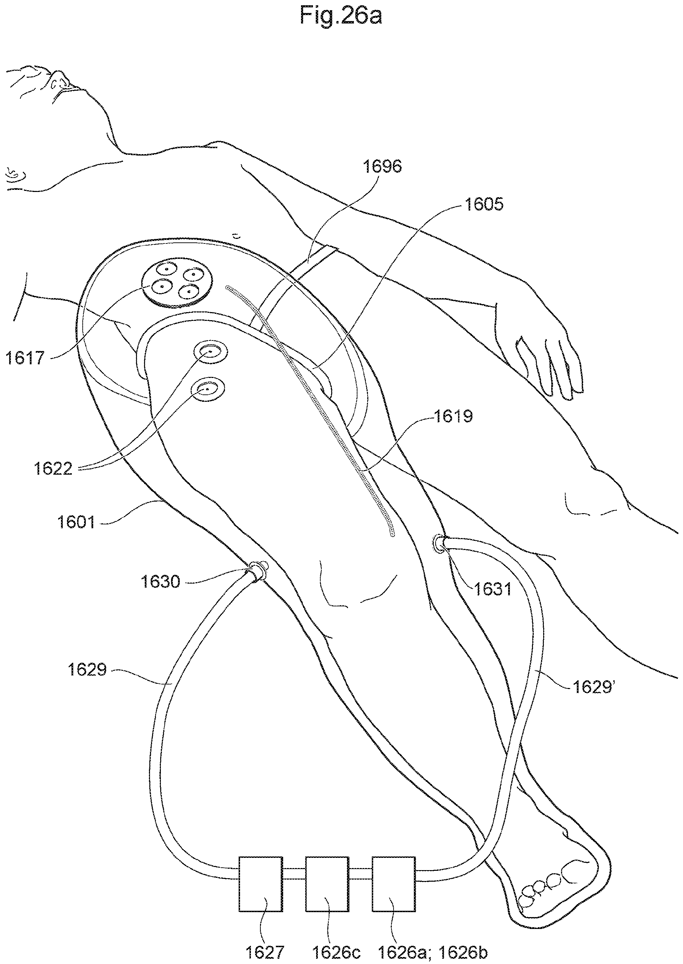

Fig. 26a shows a medical device similar to the medical device shown in fig. 25a with the difference that the medical device of fig. 25a is only adapted to be placed on one side of the patient and provide sealing 1605; 1605'; 1605" in the inguinal area. One advantage with the construction of fig. 26a is that the anus and urethra openings are separated from the chamber, and the fluid volume in the device is smaller in comparison to that of the embodiment disclosed in fig. 25a. The features included in fig. 26a are equivalent to the features included in the embodiment disclosed in fig. 25a.

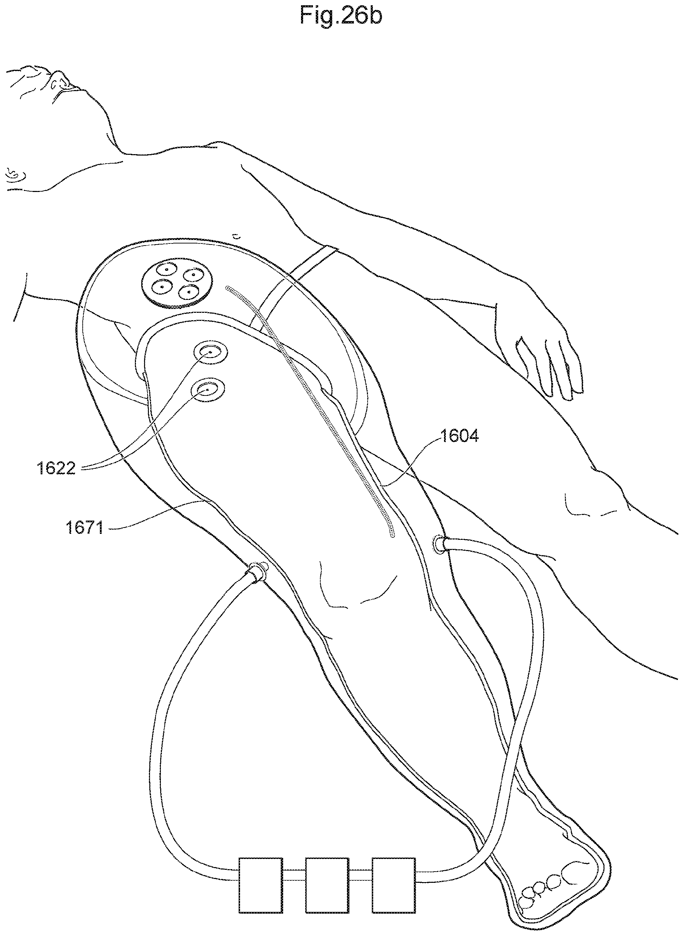

Fig. 26b shows the medical device in an embodiment similar to the embodiment shown in fig 26a, the difference being that in the medical device in the embodiment of fig. 26b comprises an inner wall 1671 in the same way as 25b and 21b. The inner wall 1671 forms an elongated tubular enclosure through which the leg of the patient is placed. The inner wall 1671 forming the elongated tubular enclosure thus separates the skin of the patient from the chamber of the medical device such that the chamber is formed by the wall 1601 and the inner wall 1671 without involving the skin of the patient. The inner wall 1671 has the advantage of separating the feet of the patient from the chamber which is beneficial since the feet of the patient is difficult to properly sterilize.

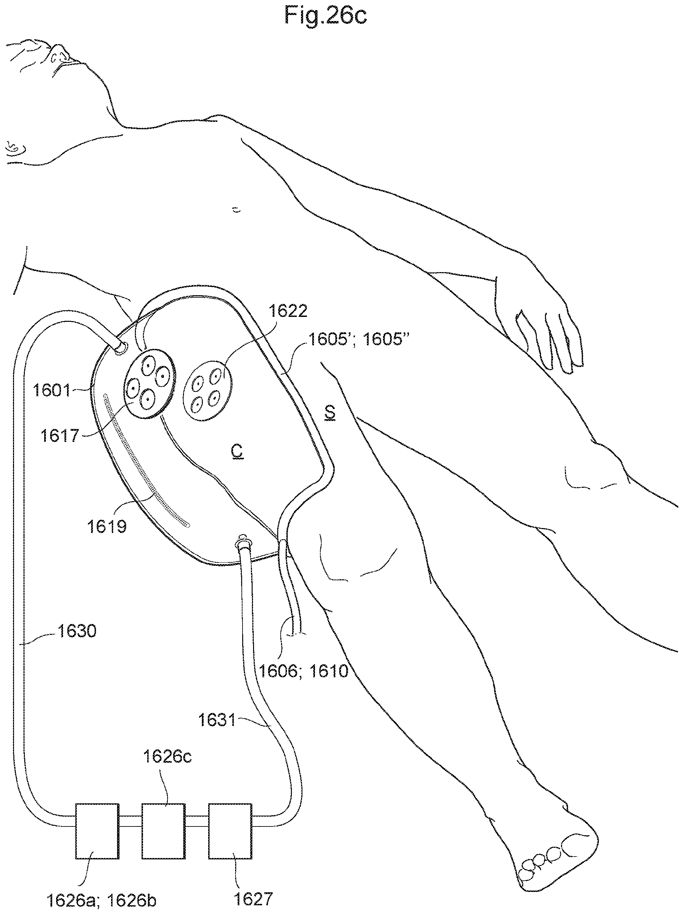

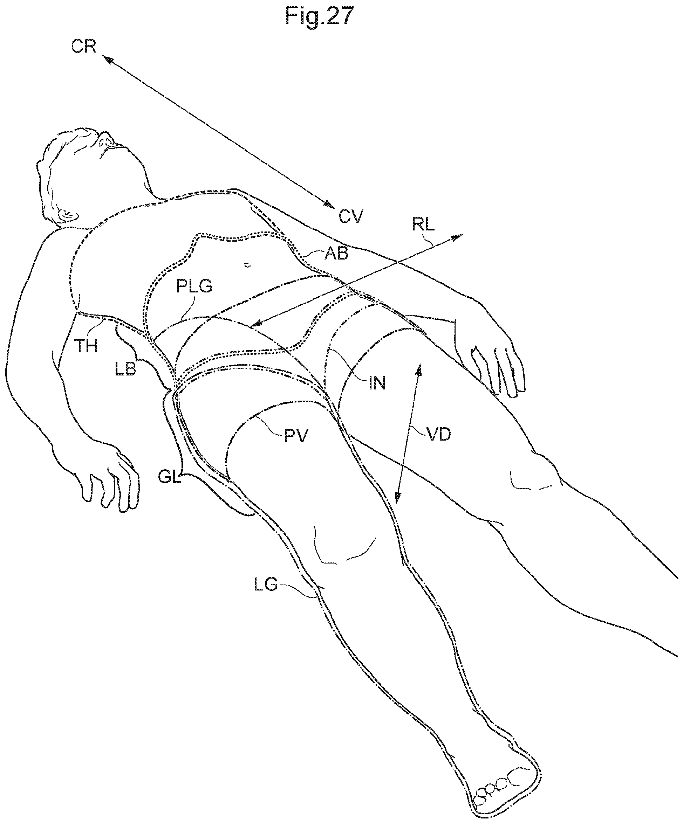

Fig. 26c and 26d shows medical devices similar to the medical device shown in figs. 22a, 22b, 26a and 26b. The medical devices shown in fig. 26c and 26d are adapted for performing hip joint surgery on a patient in a sealed and controlled environment, such that the risk of infections can be substantially reduced. The risk of infections is particularly large when performing prosthesis surgery inside the joints of patients, which is why the maintaining of a controlled environment is of utmost importance. The medical device of fig. 26c comprises a wall 1601 placed on the patient's body forming a chamber (C) together with the patient's body and is some instances together with an adhesive surface applied on against the skin S of the patient for reducing the risk that bacteria from the skin S enters the incision of the surgical procedure. The wall 1601 encloses the chamber C, such that a sealed surgical environment is created in the chamber C, in which an incision of the hip joint surgery in the skin S of the patient and at least one step of the hip joint surgery can be performed, while maintaining the sealed surgical environment. The medical device further comprises a sealing device 1605 connected to the wall 1601 and adapted to seal between the wall 1601 and the skin S of the patient. The sealing device 1605 forms a special loop encircling a portion of the leg (LG fig. 27), the pelvic region (PV fig. 27), the abdominal region (AB fig. 27), the inguinal region (IN fig. 27), the gluteal region (GL fig. 27), the thoraxial region (TH fig. 27), and the region of the lower back (LB fig. 27), all further defined under reference to fig. 27, such that the sealing device 1605 seals between the incision site of the hip joint surgery and the anal orifice and/or the urethra orifice and/or the perineum region. The sealing device 1605 of the embodiment disclosed under reference to fig. 26c seals laterally on the leg in a ventrodorsal direction to the frontal part of the leg above the knee, the sealing thereafter turns in a cranial direction towards the cranial or proximal part of the leg, crossing the inguinal region or its prolongation, further in a cranial direction lateral of the patient's ventrodorsal midsagittal plane, on the side where the hip surgery is performed. The sealing continues cranially across the abdominal region of the patient and then turns in a latero-dorsal direction crossing the lower lateral part of the thoraxial region and continuing in a medial-dorsal direction to the lower back region. Before reaching the midsagittal plane, the sealing device turns in a caudal direction across the gluteal region and continues until intersecting with the sealing device and thus forming the special loop encircling the area of the incision performed in the hip joint surgery. The placing of the sealing device 1605 forming the special loop, as shown in fig. 26c excludes the anus, urethra and perineum regions from the sealed chamber C of the medical device which is of utmost importance as these regions are very difficult to properly disinfect.

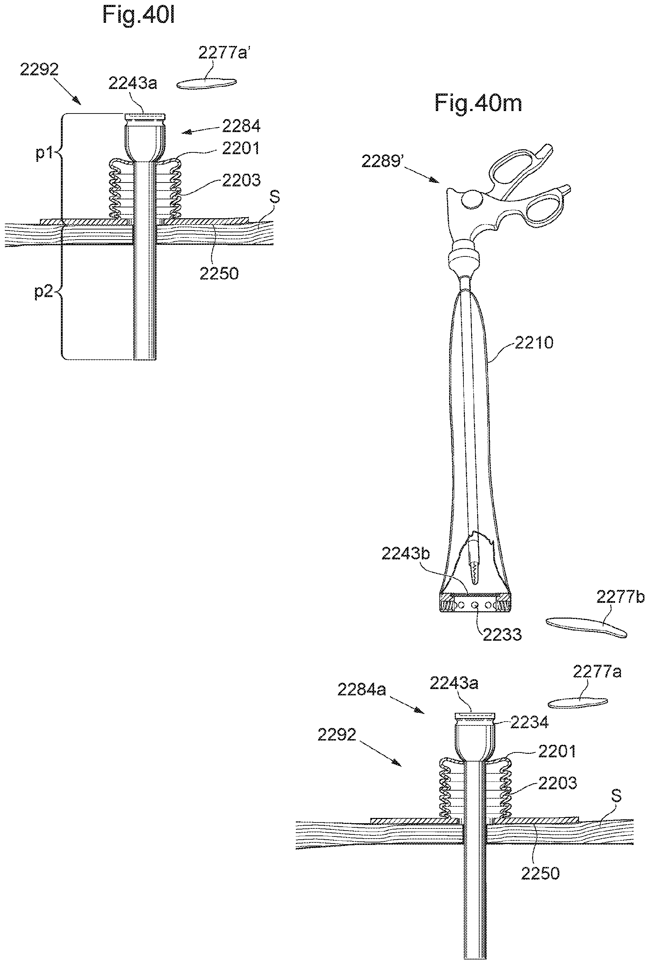

The medical device shown in fig. 26c further comprises a wall port 1617 (in the form of a multiport) adapted for insertion of instruments into the sealed environment of the medical device. The instruments may be equipped with couplings, such as the couplings disclosed under reference to figs. 40l - 40p, such that the instruments can be kept sealed when not in use in the sealed environment of the medical device. The medical device further comprises a body port 1622 (in the form of a multiport) placed in an incision performed in the skin of the patient, the body port enabling manipulation in a cavity in the patient using surgical instruments. The wall port 1617 and/or body port 1622 may comprise couplings 1680 such that the wall port 1617 and body port 1622 can be connected for forming an interconnected port, for example using any of the ports adapted to be connected disclosed in other embodiments herein. The wall port 1617 may thus be fixated to a flexible and/or elastic part of the wall 1601 such that the wall port 1617 can be moved in relation to the body port 1622. The wall port 1617 or body port 1622 may be attached to the wall 1601 of the medical device 1600 by means of a coupling 1680 fixated to the wall 1601, such that the wall port 1617 and/or body port 1622 is detachably fixated to the wall 1601 and thus replaceable by an additional port or an inset (such as any of the insets disclosed herein). Different embodiments of couplings are disclosed herein and may be used in the medical device according to the embodiment disclosed in fig. 26c.

The wall of the medical device further comprises a zipper 1619 for opening the wall 1601 of the medical device. The zipper 1619 may for example be a Ziploc-type zipper adapted to contain a fluid in the chamber C having a pressure exceeding atmospheric pressure. The wall 1601 of the medical device may be a flexible or elastic wall such that the leg of the patient can be moved during the surgical procedure, which me be required, for example in prosthesis surgery, to align and test the functionality of the implanted prosthesis.

The medical device of fig. 26c further comprises a fluid circulating system comprising a fluid inlet 1630 and a fluid outlet 1631 connected to a sterilizing/filtering unit 1626a, 1626b and a tempering unit 1626c (further described in other embodiments of the disclosure). The fluid is circulated by means of a circulating pump 1627. The fluid contained in the chamber C of the medical device could be a gaseous fluid, such as CO2 gas, or a liquid fluid, such as an isotonic or antibiotic fluid, which is used in an arthroscopic procedure in the hip joint of the patient.

The sealing member 1605 shown in the embodiment o fig. 1662c comprises an adhesive sealing member adapted to attach and/or seal the medical device to the skin S of the patient by adhering to the skin S of the patient. However, the adhesive sealing member may be assisted or replaced by a pressure o vacuum sealing member, such as the pressure and/or vacuum sealing members disclosed in other embodiments herein.

In alternative embodiments, the wall of the medical device may further comprise an inset positioned in the wall 1601 which for example may comprises a device and/or instrument mount of a glove for enabling manual manipulation within the chamber C.

When performing a hip joint surgical procedure using the medical device disclosed in figs. 1662c and 1662d, the incision in the incision site of the hip joint surgery is made inside of the chamber C of the medical device, after the medical device have been placed and sealed against the skin S of the patient.

According to one embodiment, at least one of; the body port, the wall port, the second wall port and the second body port, comprising a multi-port, comprising at least two ports integrated in the multi port, and adapted to enable passage of an object to the inside of the patient's body via the cut incision in the skin, when performing the surgical procedure.

The medical device of fig. 26c may further comprise an air or liquid lock sluice fixated to the wall of the medical device and adapted to enable insertion of objects into the chamber of the medical device while maintaining the sealed environment. The air or liquid lock sluice may be an air or liquid lock sluice according to any of the embodiments described herein.

Fig 27 is an anatomical figure showing the thoraxial region TH, reaching from under the ribs and in cranial direction CR to the clavicles. Below the thoraxial region, the abdominal region AB reaches from below the ribs in caudal direction CV to the pubic symphysis and following the edges of pelvis, below which the inguinal region IN reaches from the lower abdomen to the upper parts of the thighs. The pelvic region PV is defined to mean the entire region encircling the body of the patient along the pelvic bone in a cranial-caudal (CR - CV) direction. The leg LG of the patient is defined as the entire leg up to the iliac crest, above which a region defined as the prolongation of the leg PLG starts and reaches further in a cranial direction. On the rear side of the patient's body the region of the lower back LB is defined as the region above the gluteal region GL, the gluteal region reaching from the lower portion of the gluteal muscles to the upper portions of the gluteal muscles in line with the lower lumbar vertebras, the region of the lower back LB then continues to mid level thoracic vertebras. The direction indicator VD indicates ventrodorsal direction and RL indicates right-left direction.

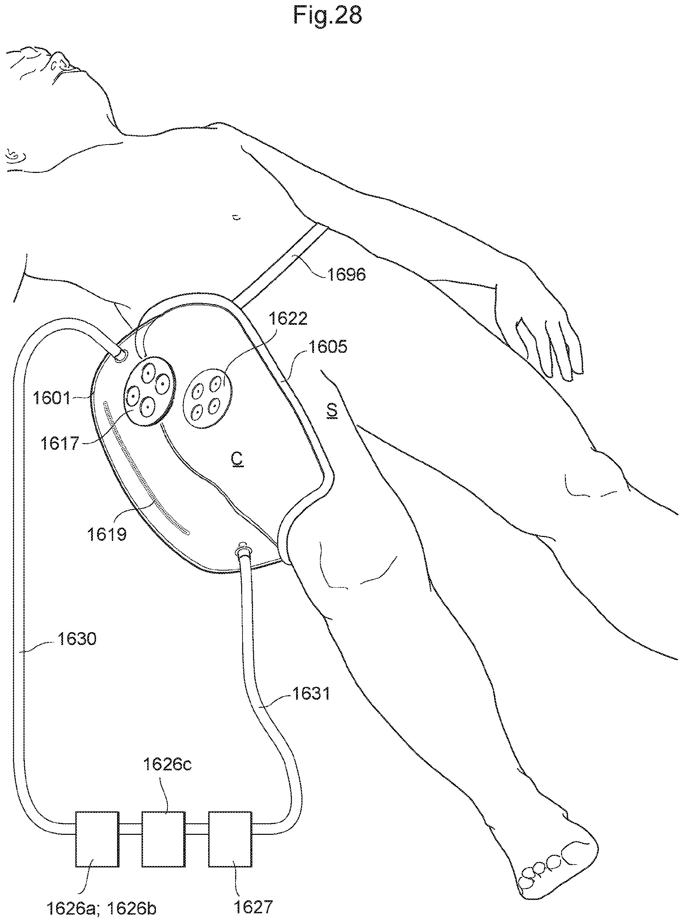

Fig. 28 shows an embodiment of the medical device identical to the embodiment shown in fig. 26c, with the difference that the medical device comprises a holding member 1696 fixating the medical device and enabling the use of a sealing device 1605 comprising a pressure sealing member adapted to seal against the skin S of the patient by pressing on the skin S of the patient. The holding member 1696 is adapted to be placed encircling the abdominal region and the lower back of the patient (see fig. 27). In alternative embodiments the holding member 1696 may be adapted to be placed encircling a single leg of the patient, or both legs of the patient, lower on the patient's body, such that the holding member encircles the gluteal region and the inguinal and pelvic regions.

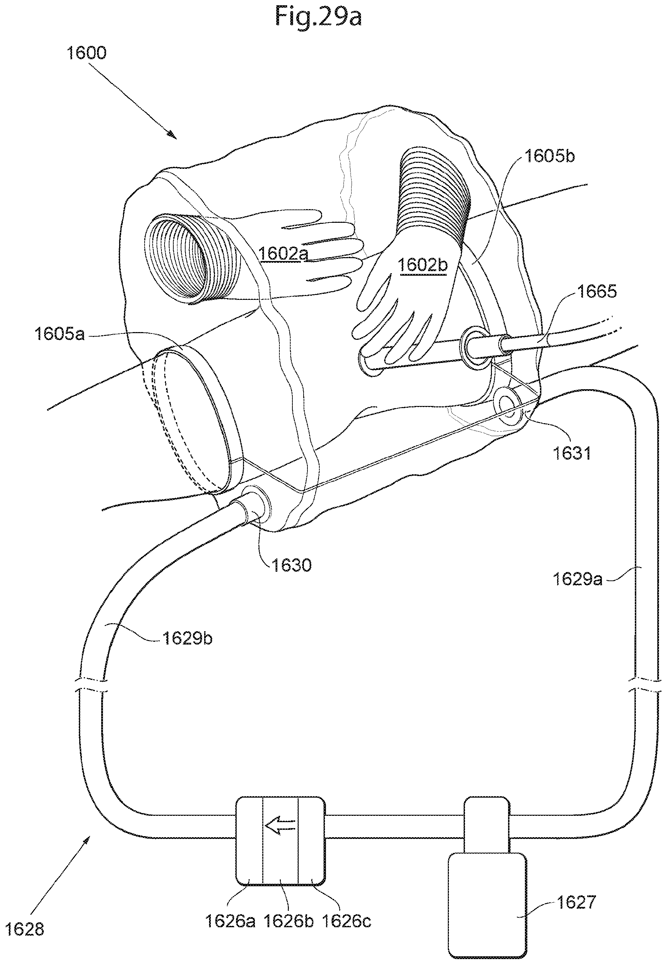

Fig. 29a shows an embodiment of the medical device where the layer 1601 is collapsible such that it can be collapsed for package and/or transportation and inflated when put to use in a surgical procedure. In the embodiment of fig. 29a, the layer 1601 is made from a flexible material, such as a flexible polymer material or a woven material. The material could be transparent or translucent for enabling viewing into the sealed environment of the medical device. The medical device is shown with a system 1628 for circulating a liquid or gaseous fluid. The fluid is circled form an outlet 1631 in the wall 1601 through a first conduit 1629a via a pump 1626 and via a system for sterilization 1627c, filtering 1627b and heating 1627a, through a second conduit 1629b to an inlet 1630.

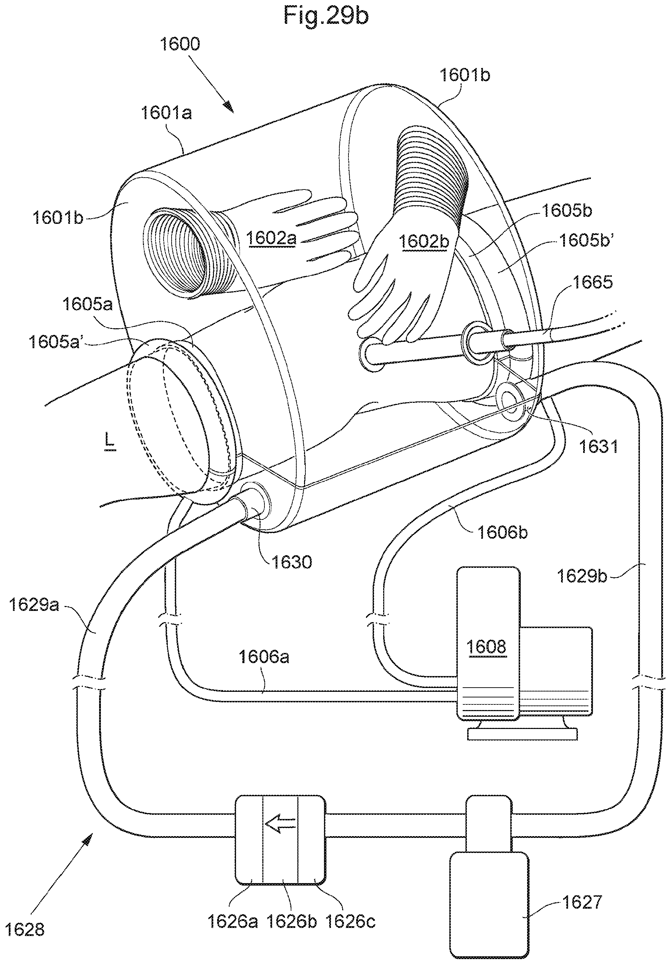

Fig. 29b shows the medical device according to an embodiment comprising the features of the embodiment disclosed with reference to fig. 29a and further comprising vacuum seals 1605a', 1605b' encircling the leg L of the patient for sealing between the medical device and the skin of the patient. The vacuum seals 1605a', 1605b' are connected to a vacuum pump 1608 through vacuum conduits 1606a, 1606b transferring the vacuum from the vacuum pump 1608 to the vacuum seals 1605a', 1605b'. The vacuum seals described with reference to fig. 29b could be used in combination with the seals 1605a, 1605b which for example could comprise two elastic ring shaped members 1605a, 1605b, sealing by pressing against the skin of the patient, or without the seals 1605a, 1605b, in which case the vacuum seals could be materially integrated in the second portions 1601b of the layer 1601.

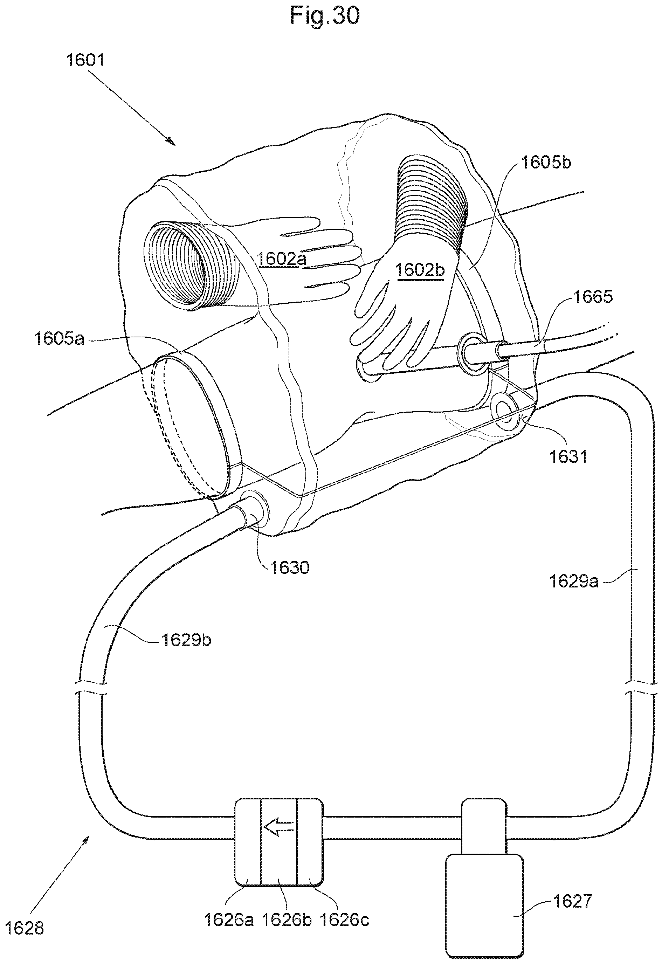

Fig. 30 shows an embodiment of the medical device where the layer 1601 is collapsible such that it can be collapsed for package and/or transportation and inflated when put to use in a surgical procedure. In the embodiment of fig. 30, the layer 1601 is made from a flexible material, such as a flexible polymer material or a woven material. The material could be transparent or translucent for enabling viewing into the sealed environment of the medical device. The medical device is shown with a system 1628 for circulating a liquid or gaseous fluid. The fluid is circled form an outlet 1631 in the wall 1601 through a first conduit 1629a via a pump 1626 and via a system for sterilization 1627c, filtering 1627b and heating 1627a, through a second conduit 1629b to an inlet 1630.

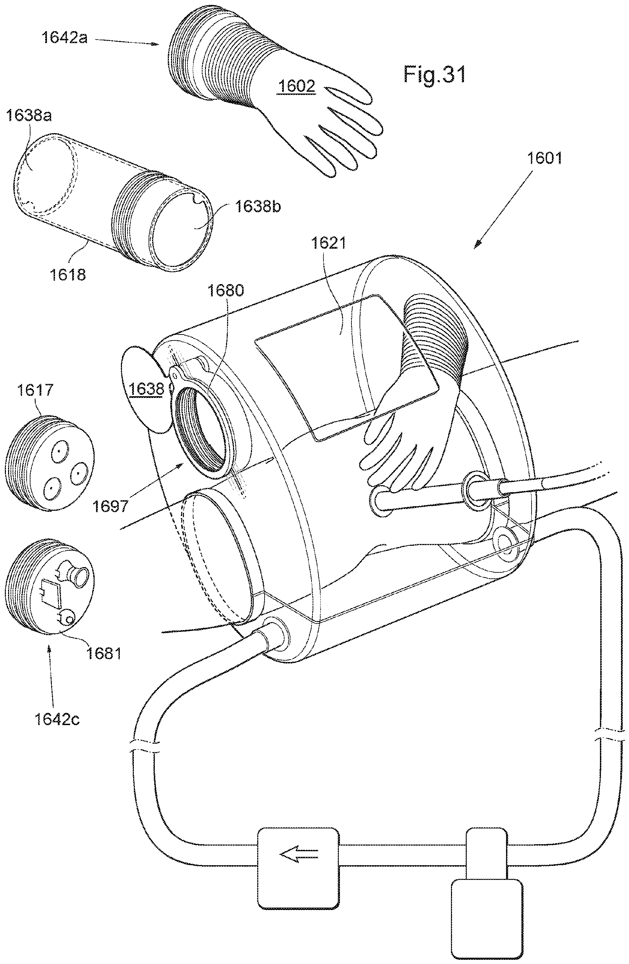

Fig. 31 shows the medical device according to an embodiment in which the medical device comprises a non-collapsible transparent window 1621 for enabling viewing into the sealed environment. The window is preferably used in embodiments where the layer is a collapsible layer, as further disclosed with reference to fig. 30. The non-collapsible transparent window 1621 will ensure that the surgeon has adequate visual control over the procedure since the partially collapsible layer, even if preferably made from a transparent material could provide limited visibility, e.g. if the medical device is not fully inflated, or in seams or bends of the material. The non-collapsible transparent window 1621 could for example be made from acrylic glass, a polycarbonate, polyethylene terephthalate, an acrylic fiber material or a copolymer containing polyacrylonitrilea, or made from tempered glass. In other embodiments (not shown) the medical device could further comprise a cleaning device for cleaning the transparent window 1621 from the inside of the sealed environment. The cleaning device could comprise an arrangement for supplying a fluid adapted to assist in the cleaning of the transparent window via a fluid conduit to a nozzle placed in proximity to the transparent window. The fluid could for example be an isotonic solution, such as saline solution, or an antiseptic solution such as Chlorhexidine. The medical device could further comprises a window contacting member for wiping the window clean after the fluid has been sprayed thereon for mechanically removing objects from the window. The embodiment shown in fig. 31 further comprises a coupling 1631 positioned in the layer 1601. In the embodiment shown in fig. 31 the coupling 1631 comprise screw-threads corresponding with screw-threads of the objects which could be coupled to the coupling 1631. The coupling 1631 as shown in fig. 31 further comprises a valve member 1638 which could be closed when no objects are placed in the coupling 1631 or during the process of changing objects, when otherwise the coupling 1631 would be left open. Examples of objects which could be placed in the coupling 1631 are shown in fig. 31, however, other suitable objects are also conceivable and the scope of the invention is not to be limited to the particular objects shown, rather, the concept of having changeable objects is to be regarded as part of the invention. The examples of objects shown in fig. 31 are now further described: An airlock 1618 is shown comprising an outer opening 1638 between the surrounding environment and the airlock 1618 and an inner opening 1682 between the airlock 1618 and the sealed environment of the medical device. The airlock 1618 could enable transfer between the surrounding environment and the airlock, and between the airlock and the sealed environment of the medical device, for enabling the transfer of for example prosthesis or prosthetic parts or surgical instruments. The airlock could further enable transfer of objects from the sealed environment, into the airlock and further to the surrounding environment, which for example could enable the transfer of specimens from the body of the patient. Both the transferring of objects into the sealed environment and out from the sealed environment could by means of the airlock be conducted whilst keeping the sealed environment hermetically closed. Fig. 31 further shows a holding member 1681 for prosthetic parts or surgical instruments. By means of placing the holding member 1681, or exchanging the holding member to a different holding member, prosthetic parts or surgical instruments could be introduced into the sealed environment, which for example could be used if different steps or unexpected events in the procedure require different instruments. A port unit 1617 is further shown, the port unit as shown in fig. 31 comprises three ports adapted for laparoscopic and/or arthroscopic instruments. In some embodiments the port unit could comprise a single larger port unit which could enable hand access into the sealed environment, the single larger port unit could for example be a gel-port type port unit.

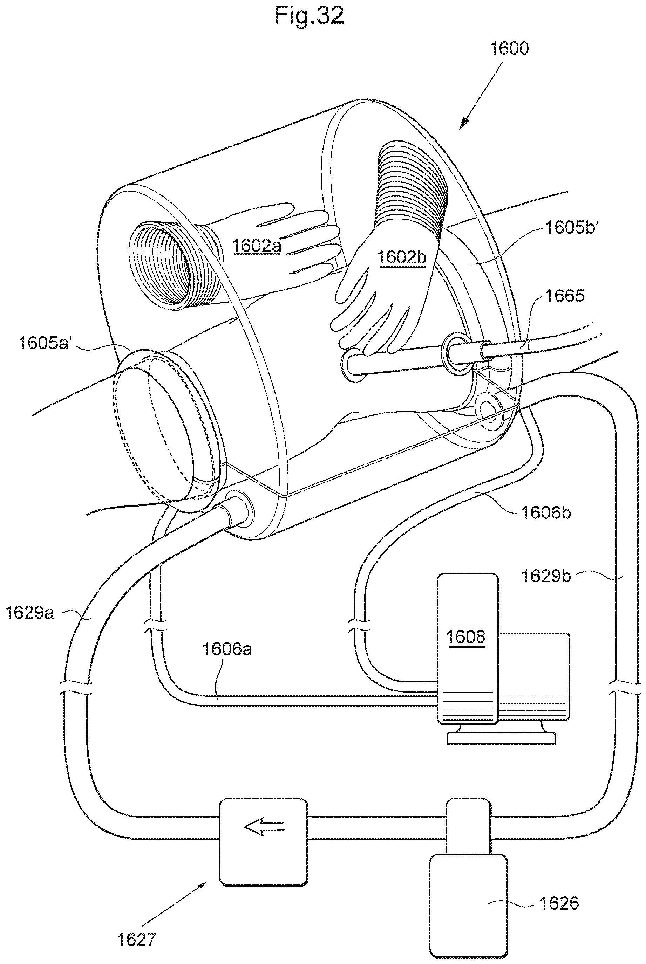

Fig. 32 shows an embodiment further comprises vacuum sealing members 1605a', 1605b' adapted to seal between the skin of the patient and the medical device by the vacuum sealing members 1605a', 1605b' being adapted to hold a pressure less than 1 bar, thus creating a vacuum seal between the skin of the patient and the medical device. The vacuum sealing members 1605a', 1605b' are connected to a vacuum pump 1608 through vacuum conduits 1606a, 1606b transferring the vacuum from the vacuum pump 1608 to the vacuum seals 1605a', 1605b'. The vacuum seals described with reference to fig. 32 could be used in combination with other types of seals.

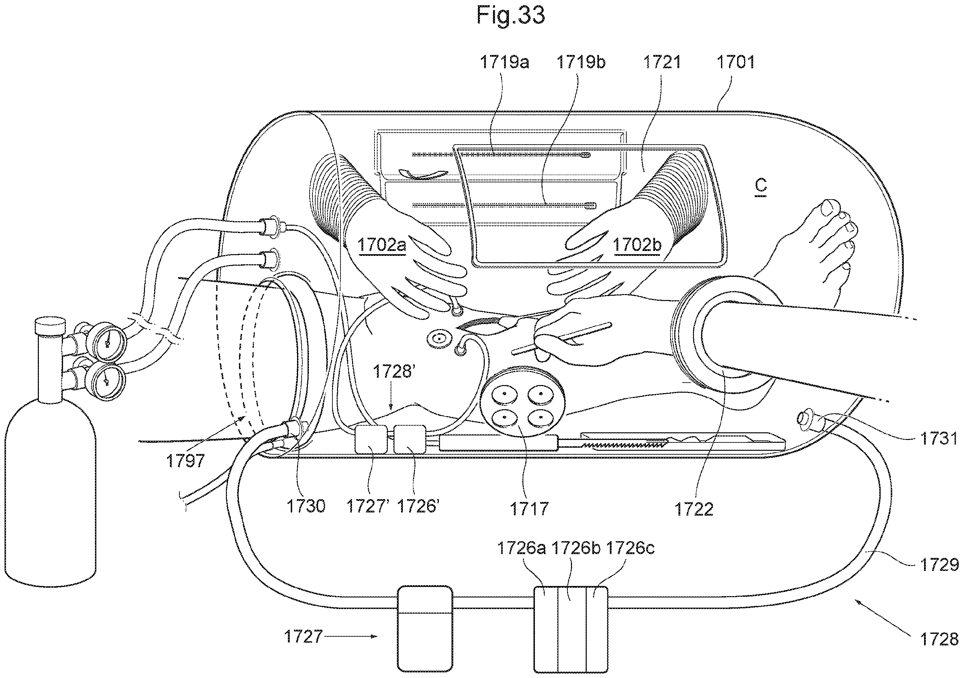

Fig. 33 shows an embodiment of the medical device when the arm of the surgeon is inserted through the self sealing wall port 1717b in the partially collapsible wall 1701 for enabling manual manipulation therein. Manual manipulation through the self sealing wall port 1717b could for example be used as a last resort should complications occur during the procedure, or if additional hands are required for a specific step of the operation. Fig. 33 further shows an internal system 1728' for fluid circulation, and an external system 1728 for fluid circulation. Both systems comprises fluid conduits 1729; 1729', in the external system 1728 connected at an inlet 1730 placed in the partially collapsible wall 1701 for supplying fluid to the chamber C, and an outlet 1731 for draining the fluid from the chamber C, and in the internal system 1728' connected to an inlet 1730' in the patient's body and an outlet 1731' in the patient's body for circulating fluid in a body cavity. The fluid is circulated by means pumping units 1727; 1727' and is circulated via a sterilizing/filtering/tempering unit 1726', in the external system disclosed as containing a sterilizing 1726a, filtering 1726b, and tempering 1726c unit adapted to remove impurities, sterilize and heat the fluid. In embodiments where the fluid is a gaseous fluid, the filtering unit is adapted to remove particles that could be suspended in the gas. The filtering unit 1726a could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit 1726a could for example comprise a filter containing activated carbon.

Sterilizing methods could comprises the use of heat, such as electrical or chemical heat, it is furthermore conceivable that the sterilizing is performed using irradiation, such as UV-light and/or though the addition of a chemical sterilizing agent.

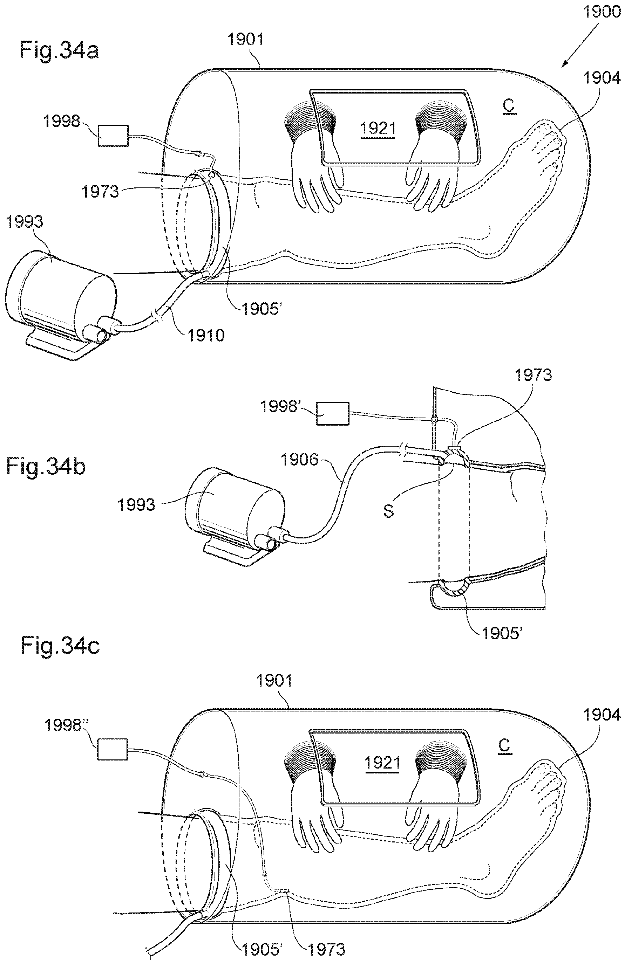

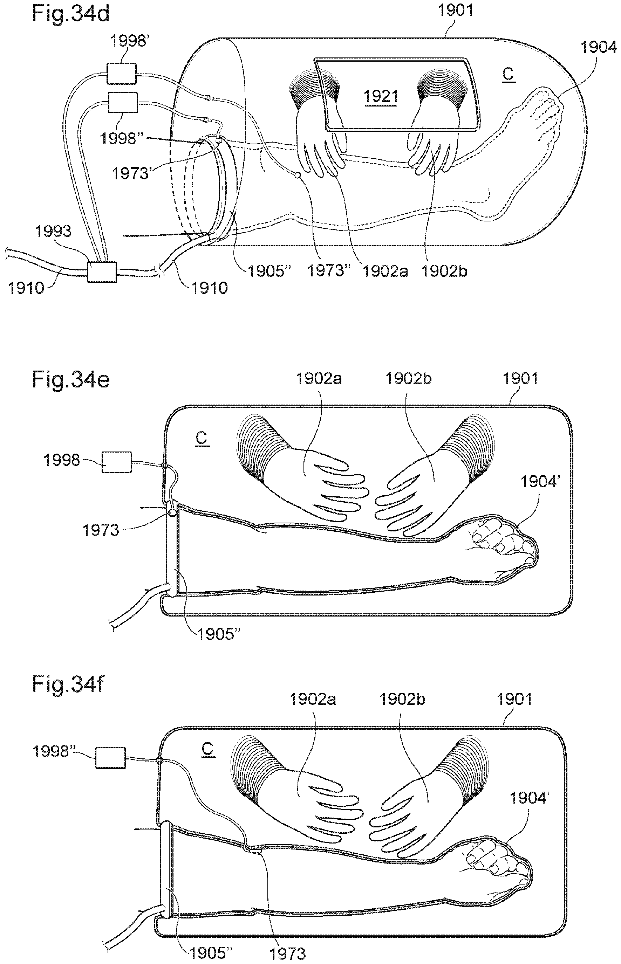

Fig. 34a shows an embodiment of the medical device 1900 for performing a surgical procedure on a patient. The medical device 1900comprises a wall 1901 adapted to at least partially be applied on an extremity of the patient's body being a leg o the patient to form a chamber C. The chamber C may be formed, as shown in fig. 34a, by itself, between the exterior wall 1901 and a portion of the wall 1901 forming an elongated tubular enclosure 1904 extending within the chamber C and thus forming a layer between the skin of the patient and the chamber C. The elongated tubular enclosure 1904 is fixated to the rest of the wall 1901 at the area of the wall 1901 at which a sealing member 1905" is positioned. When the surgical procedure is performed, the wall of the elongated tubular enclosure 1904 is cut through such that an incision in the skin of the patient can be made. By placing the layer in contact with the skin of the patient prior to making the incision in the skin of the patient, the risk that any bacteria present on the skin of the patient will reach the area of the incision if reduced. The layer of the wall forming the elongated tubular enclosure 1904 may comprise an adhesive surface such that the wall 1901 adheres to the skin S of the patient, which further reduces the connecting points between the chamber C and the skin S of the patient, which thus further reduces the risk that bacteria present on the skin of the patient will reach the area of the incision and cause infectious decease. In alternative embodiments, the chamber C may be formed together with the patient's body, in which case the elongated tubular enclosure 1904 is omitted.

The sealing shown in fig. 34a is a pressure sealing member 1905" adapted to pneumatically or hydraulically press the wall 1901 against the body of the patient such that the chamber C is at least substantially sealed from the ambient atmosphere allowing a pressure inside the chamber C to be higher than the ambient atmospheric pressure during the surgical procedure. In other embodiments, such as other embodiments shown herein, the sealing 1905" may be mechanically powered for example by means of an adjustable band which may be combined with an elastic band.

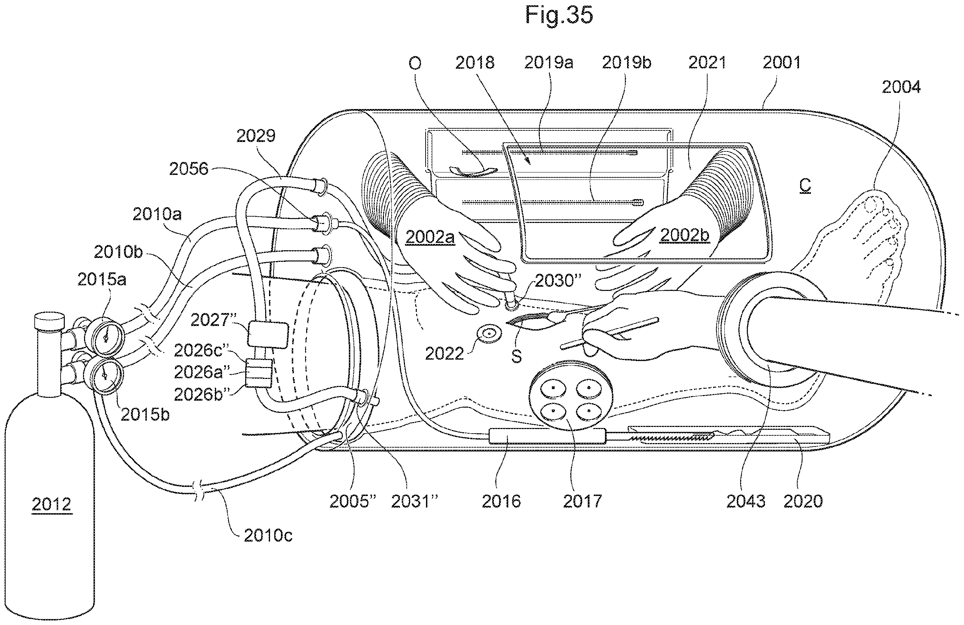

The pressure sealing 1905" is adapted to seal the chamber C after the medical device has been applied at least partially on the patient's body such that an incision can be performed inside the chamber C for creating a fluid connection between the chamber C and a cavity in the patient. The sealing member 1905" is adapted to remain unaffected by the incision and thus sealing with unaffected force. The pressure sealing member 1905" is in fluid connection with a fluid conduit 1910 for providing a pressurized fluid to the pressure sealing member 1905". The fluid conduit 1910 is in turn connected to a pressure adjustment device 1993 in the form of a pump adapted to fill the pressure sealing 1905" with pressurized air from the ambient environment for adjusting the pressure in the sealing member 1905". The pump 1993 may in other embodiments be replaced by a tank comprising a pressurized fluid (for example the tank 2012 disclosed in the embodiment of fig. 35).

The embodiment of fig. 34a further comprises a monitoring unit 1998 connected to a lead connected to a monitoring probe 1973 fixed to the sealing member 1905". The monitoring probe 1973 comprises a pressure sensor adapted to detect change in the pressure in the sealing 1905". In alternative embodiments, the monitoring unit 1998 may be adapted to measure the blood pressure of the patient by means of measuring the blood flow in the peripheral blood vessels, by means of for example an ultra sonic flow meter. An ultrasonic flow meter measures the difference of the transit time of ultrasonic pulses propagating in and against the flow direction. This time difference is a measure of the average velocity of the fluid along the path of the ultrasonic beam. By using the absolute transit times the averaged fluid velocity can be calculated. The measurement of the blood pressure enables the adjustment of the sealing member 1905" such that the sealing member 1905" does not hinder the blood flow passing the sealing member 1905", whilst allowing the sealing member 1905" to provide sufficient pressure for sealing the chamber C from the ambient environment. The pressure adjustment device 1993 may for example be adapted to adjust the pressure in the sealing member 1905" such that the pressure in the sealing member 1905" is below the systolic blood pressure and thus do not hinder the blood flow. The systolic blood pressure varies somewhat with the age of the patient but should be within a range of 90 mmHg - 160mmHg. The pressure in the sealing member 1905" should thus not exceed the values of that range for not hindering the blood flow. The pressure could for example be in a range of 10mmHg - 160mmHg, 20mmHg - 140mmHg, 30 mmHg - 120mmHg, 30 mmHg - 100 mmHg, 40 mmHg - 100 mmHg, 40 mmHg - 80 mmHg and 50 mmHg - 70 mmHg.

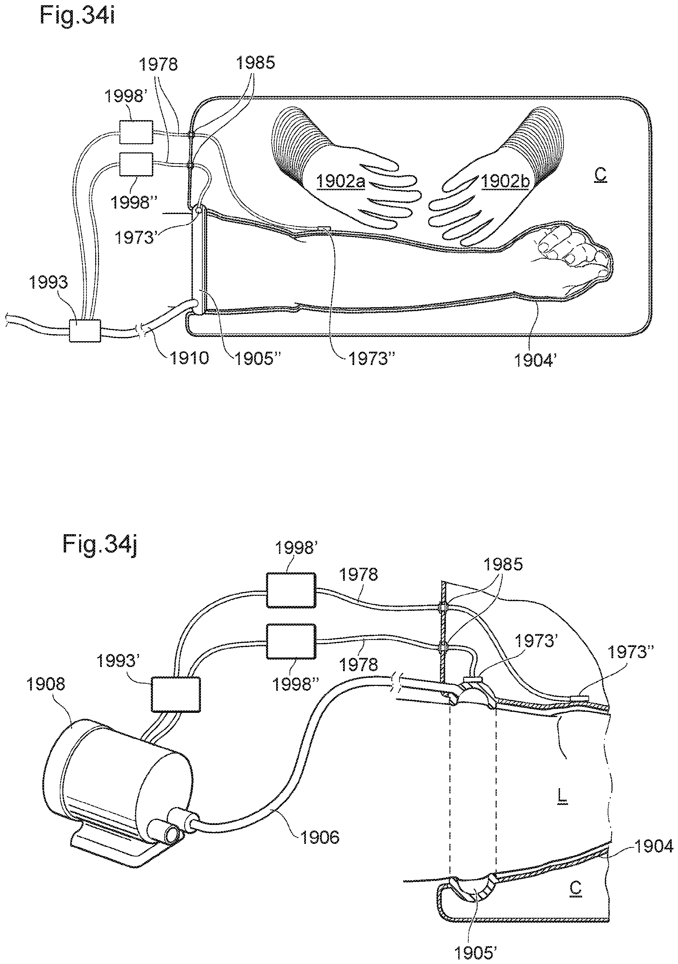

Fig. 34b shows an alternative to the pressure sealing member 1905" disclosed in fig. 34a in which the pressure sealing member is replaced by a vacuum sealing member 1905' providing sealing between the wall of the medical device 1901 and the skin S of the patient by means of a pressure in the vacuum sealing member 1905' being lower than the ambient atmospheric pressure. The negative pressure in the vacuum sealing member is e.g. provided by a vacuum pump connected by means of a vacuum conduit 1906. The negative pressure in the vacuum sealing member 1905' may be monitored by means of a vacuum monitoring unit 1998' connected to a monitoring probe 1973, connected to the vacuum sealing member 1905'. The operation and effect of the vacuum sealing member 1905' is described in further detail with reference to fig. 34i.

In alternative embodiments the pressure in the sealing member 1905" does not need to be monitored, only controlled such that the blood flow passed the sealing member 1905" remains substantially unaffected. The blood flow may, as previously mentioned, be measured by means of an ultra sonic flow meter. The vacuum adjustment device 1993 may in other embodiments be adapted to adjust the pressure in the vacuum sealing member 1905' as response to the blood pressure of the patient, the blood flow of the patient and/or the temperature at the skin of the patient.

The medical device may further comprise a pressure sensor for measuring the pressure inside the chamber C. By measuring the temperature inside the chamber C, fluid leakage at the sealing 1905" can be determined, which can be used as input in the control of the sealing member 1905".

Fig. 34c shows an alternative embodiment of the medical device in which a probe 1973 is positioned in the knee region of the patient on the dorsal side of the leg of the patient. The probe 1973 may for example be a temperature sensing probe 1973 adapted to sense the temperature of the body of the patient for the purpose of determining that the blood flow from at that area of the patient is sufficient. When the blood flow is hindered, the temperature of the skin of the patient raises which can be used as an input variable to the pressure adjustment device shown for example as 1993 of fig. 34c. In alternative embodiments the probe 1973 may be an ultra sonic flow meter for measuring the blood flow of the patient. The probe 1973 is preferably placed on the dorsal side at the knee region of the leg as the blood vessels are more superficial in this region. The probe 1973 may for example be adapted to measure the blood flow of the popliteal vein, the popliteal artery, the saphenous vein and/or the tibial artery.

Fig. 34c shows an embodiment of the medical device in which the medical device comprises two monitoring units 1998'; 1998", wherein the first monitoring unit 1998' is adapted to monitor the blood flow at an area of the patient by means of an ultra sonic flow meter probe 1973", and the second monitoring unit 1998" is adapted to monitor at least one of the pressure in the sealing device, the pressure in the chamber, and the direct or indirect leakage of fluid from the chamber by means of a pressure sensing probe 1973'.

In any of the embodiments disclosed it is equally conceivable that the medical device comprises two monitoring units 1998'; 1998", wherein the first monitoring unit is adapted to monitor the blood pressure o the patient, and the second monitoring unit 1998" is adapted to monitor at least one of: the pressure in the sealing device, the pressure in the chamber C, and the direct or indirect leakage of fluid from the chamber C.

In any of the embodiments disclosed it is equally conceivable that the medical device comprises two monitoring units 1998'; 1998", wherein the first monitoring unit 1998' is adapted to monitor a temperature of the body of the patient, and the second monitoring unit 1998" is adapted to monitor at least one of: the pressure in the sealing device, the pressure in the chamber C, and the direct or indirect leakage of fluid from the chamber C.

Fig. 34c further shows a pressure adjustment device 1993 adapted to adjust the pressure in the sealing device 1905" based on input from the first 1998' and second 1998" monitoring unit, such that the resulting pressure in the sealing member 1905" can be a mediation between providing sufficient sealing and maintaining the blood flow of the patient unaffected. The pressure adjustment device comprises an electrically controlled pressure regulating valve adapted to control pressure from a pressure source (e.g. a pump) distributed to the pressure adjustment device by means of a fluid conduit 1910.

The sealing member 1905" may be a sealing member 1905" adapted to be pressurized by means of a gaseous fluid, or by means of a liquid fluid. The liquid fluid may be pumped by means of a fluid pump. In embodiments where the sealing member 1905" is adapted to be pressurized by a gaseous fluid, the gaseous fluid may come from a pressure tank (for example shown as 2012 in fig. 35) or may be pressurized ambient air supplied by a pneumatic pump 1908.

The sealing member 1905" in the embodiment of fig. 34a - 34h may be substituted by a vacuum sealing member 1905' as for example shown in fig. 34b and 34i, and further disclosed throughout the description. The vacuum sealing member 1905' is adapted to hold a pressure less than atmospheric pressure between the wall 1901 and the patient's skin to seal between the wall 1901 and the patient's skin.

Fig. 209d shows an embodiment of the medical device similar to the embodiment shown in fig. 209a, the difference being that the medical device with the sealing 1905" and the elongated tubular enclosure 1904' is adapted to fit an extremity being an arm of the patient.

Fig. 209e shows an embodiment of the medical device similar to the embodiment shown in fig. 209b, the difference being that the embodiment shown in fig. 209d with the sealing 1905" and the elongated tubular enclosure 1904' is adapted to fit an extremity being an arm of the patient. The embodiment comprises an ultra sonic flow sensing probe 1973 adapted to sense the blood pressure of the patient and being adapted to be positioned on the inside of the patients arm where the blood vessels are superficial. The ultra sonic flow sensing probe 1973 may be adapted to sense the flow in one or more of the cephalic vein, the basilic vein, the median cubital vein, the brachial artery, the ulnar artery and the radial artery.

Fig. 209f shows an embodiment in which the medical device comprises a pressure sealing member 1905", equivalent to the sealing 1905" disclosed with reference to fig. 34a - 34f. The medical device further comprises a blood pressure monitoring unit 1998‴ operated as a sphygmomanometer. The blood pressure monitoring unit comprises an inflatable cuff 1976 which is snugly placed around the upper arm of the patient. The pressure monitoring unit 1998‴ is operated by the cuff 1976 being inflated to a pressure initially in excess of the systolic arterial pressure and then reduced to below diastolic pressure over a period of about 30 seconds. When no blood is flowing passed the cuff 1976, the cuff 1976 pressure will be essentially constant. When blood flow is present, but restricted, the cuff 1976 pressure, which is monitored by a pressure sensor of the blood pressure monitoring unit 1998‴, will vary periodically in synchrony with the cyclic expansion and contraction of the arteries. The values of the systolic and diastolic pressure can thus be computed. In alternative embodiments the measurement of the oscillating pressure may be assisted or replaced by an ultra sonic flow meter measuring the blood flow at a relevant place of the patients arm. The blood pressure monitoring unit 1998‴ may be adapted to measure the blood pressure of the patient periodically, such as for example with an interval of: 1 - 5 minutes, 5 - 10 minutes, 10-20 minutes, or 20 - 30 minutes. The periodical monitoring of the blood pressure enables the sealing member 1905" to be inflated/deflated such that the blood flow in the arm of the patient is substantially unaffected or periodically occluded whilst the sealing member 1905" provides sufficient sealing.

In instances when the chamber C is inflated with a pressure exceeding atmospheric pressure the sealing needs to provide a sealing force exceeding the force created by the pressure from the pressure inside the chamber C. The pressure in the sealing may then exceed the pressure in the chamber C by e.g. 10mmHg, 20mmHg, 30mmHg, 40mmHg, 50mmHg, 60mmHg or 100mmHg. As an example the pressure in the sealing 1905" can be 120 mmHg, not to exceed the systolic blood pressure, and the pressure in the chamber C can be 80 mmHg such that the resulting pressure exerted on the skin of the patient and providing the sealing force is 40 mmHg.

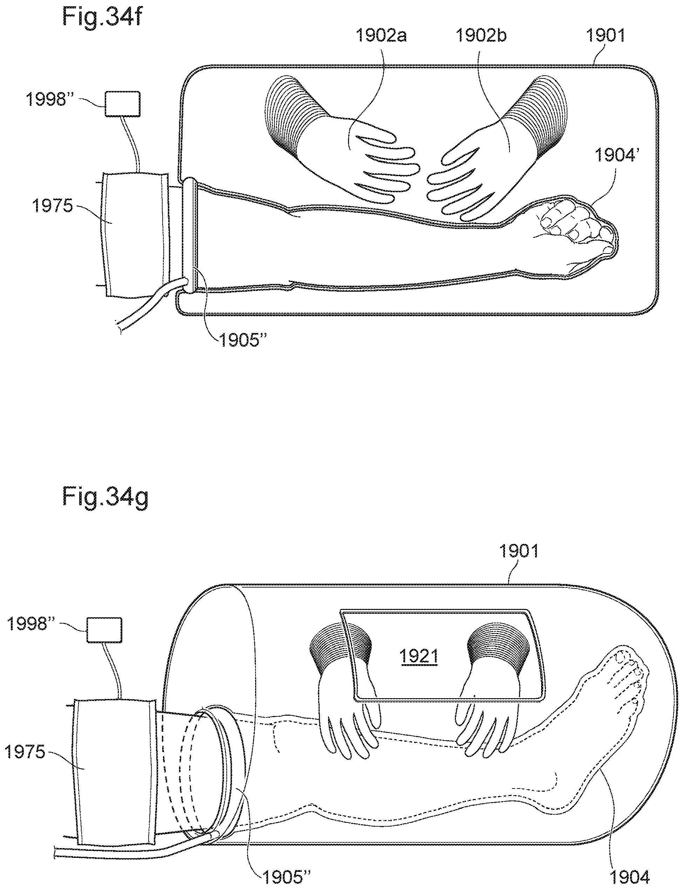

Fig. 34h shows an embodiment of the medical device similar to the embodiment described with reference to fig. 34g, the difference being that the medical device is adapted to be positioned on an extremity of the patient being a leg of the patient, and thus the sealing member 1905" and the elongated tubular enclosure is adapted for being positioned on a leg of the patient.

Fig. 34i and 34j (34j being a sectional enlargement of a portion of 34i) shows an embodiment of the medical device in which the medical device further comprises a vacuum monitoring unit 1998' connected to the adjustment device 1993'. The pressure monitoring unit 1998 is connected to a pressure sensing probe 1973 via a sensing lead 1978 penetrating the wall 1901 of the medical device by means of a bushing 1985 positioned in the wall 1901. The pressure sensing probe 1973 is adapted to sense the pressure in the sealing member 1905" and to provide the pressure adjustment device 1993' with a pressure signal to be used in the adjustment of the pressure in the vacuum sealing member 1905'. The pressure adjustment device 1993' is in turn connected to a vacuum pump 1908 in connection with the vacuum sealing member 1905' by means of a vacuum conduit 1906 for adjusting the pressure in the vacuum sealing member 1905'.

The embodiment shown in figs. 34i, 34j also shows an alternative or complement to the vacuum monitoring unit 1998', being a probe 1973 placed inside the chamber C at an area of the leg of the patient. The alternative or complementing probe 1973 may be adapted to measure the pressure in the chamber C, the blood pressure of the patient, and the blood flow of the patient (for example by means of ultrasound).

By measuring the blood flow or blood pressure of the patient, the adjustment device 1993' could be adapted to adjust the pressure in the sealing member 1905' such that the blood flow of the patient remains unaffected.

The embodiment shown in figs. 34i, 34j thus shows a medical device comprising a first monitoring unit 1998' adapted to sense the pressure in the vacuum sealing member 1905' and a second monitoring unit 1998" adapted to sense the pressure in the chamber C, or the blood flow, or the blood pressure of the patient. The medical device further comprises an adjustment device 1993' adapted to adjust the pressure in the vacuum sealing member 1905' in response to the input from both the first 1998' and second 1998" monitoring unit, for mediating between keeping the chamber C sealed and keeping the blood flow of the patient un affected. The adjustment device 1993' may be adapted to adjust the pressure in the vacuum sealing member 1905' for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.

According to one embodiment, the pressure in the chamber C is between 5 mmHg and 75 mmHg above atmospheric pressure, and the negative pressure in the vacuum sealing member is between -6 mmHg and -76 mmHg.

The vacuum sealing member 1905' is adapted to create a substantially airtight seal between the wall 1901 and the skin of the patient, to enable an incision to be performed through the skin of the patient in sealed environment such that a fluid connection between a cavity within the patient and the chamber C is formed.

Fig. 35 shows an embodiment of the medical device further comprising an airlock sluice 2018, comprising an outer and inner zipper 2019a, 2019b provided in the wall 2001 for allowing passage of an object O between the chamber C and the environment outside of the chamber C when the sealing member 2005" is sealing between the skin of the patient and the wall 2001.

The wall 2001 of any of the embodiments described under reference to figs. 34a - 36 may be at least partially collapsible and adapted to be inflated by a fluid. According to the embodiment shown in fig. 35, the fluid may be a compressed gaseous fluid contained in a pressure container 2012 and transported to the chamber C by means of a fluid conduit 2010b. The pressure of the pressure container and/or of the chamber C may be monitored by means of a control / monitoring device 2015b. The control / monitoring device may in other embodiments be implemented as a digital unit. In yet other embodiments, the control / monitoring unit could be adapted to measure the flow of compressed fluid in the fluid conduit 2010b for maintaining the inflation rate at a level such that the chamber or objects within the chamber is not damaged. The chamber C according to the embodiment shown in fig. 35 is adapted to hold a pressure exceeding atmospheric pressure, and wherein the sealing member 2005" is thus adapted to seal between the wall 2001 of the medical device and the skin of the patient such that the pressure in the chamber C, exceeding atmospheric pressure, can be maintained. Having the pressure exceeding atmospheric pressure enables open and laparoscopic surgery to be combined and reduces the risk of contaminating the incision sites, as the constant over-pressure will make it close to impossible for any particles to enter the sealed chamber C.

The embodiment shown in fig. 35 further comprises two gloves 2002a, 2002b integrated in the wall 2001 such that a surgeon is able to use the glove 2002a, 2002b from outside the chamber C to make manual manipulations inside the chamber C while maintaining sealed environment in the chamber C.

The sealing member 2005", may in any of the embodiments be assisted or replaced by an adhesive adapted to provide fixation to the skin of the patient, and/or sealing between the skin of the patient and at least one of the wall 2001 and enclosure.

Fig. 35 further shows a body port 2022 positioned in the knee region of the extremity of the patient. The body port 2022 may be adapted for an optical inspection device, such as a fiber optic camera, or one or more surgical instruments suitable for an arthroscopic or laparoscopic operation.

The medical device in fig. 35 further comprises an elongated tubular enclosure 2004 extending within the chamber C of the medical device. The tubular enclosure 2004 is contacting the skin of the patient and is adapted to separate the skin of the patient from the sealed environment of the chamber C of the medical device. The elongated tubular enclosure 2004 is for example a flexible or elastic tubular enclosure 2004 suitable for receiving a leg of a patient, and encircling the leg of the patient. The steps of the surgical procedure being performed within the chamber C thus includes cutting through the tubular enclosure 2004 for accessing the skin of the patient and thus being able to create an incision in the skin for accessing the knee of the patient. The tubular enclosure 2004 may comprise an adhesive on the side facing the skin of the patient such that the tubular enclosure 2004 can be fixated and sealed against the skin of the patient. By fixating the elongated tubular enclosure 2004 tightly against the skin of the patient, the risk of infections caused by bacteria present on the skin of the patient is reduced.

The embodiment of the medical device shown in fig. 35 further comprises a fluid system for circulating a fluid, in particular a liquid fluid, in the chamber C of the medical device. The fluid system comprises a fluid inlet 2030" placed in the wall 2001 and adapted for supplying fluid to the chamber C. The fluid inlet 2030" is by means of a fluid conduit 2029 connected to a fluid outlet 2031" positioned in an incision in the patients skin in the knee region of the patient, for evacuating fluid from the knee joint region.

When performing an arthroscopic operation, the joint region is expanded by means of a pressurized liquid. The pressurized liquid thus fills the area of the knee joint during the surgery. For enabling visual inspection in the joint, the liquid needs to be fully transparent. However, when the surgical procedure is started the fluid is contaminated by bodily fluids, mainly blood. The fluid thus needs to be rapidly circled and filtered or replaced by new fluid for maintaining the visibility within the joint throughout the surgical procedure. By filling the chamber C with the liquid, and evacuating the chamber C via the incision in the knee region, the main part of the contaminated liquid will be evacuated through the fluid outlet 2031" and recycled into the chamber C when filtered. For the purpose of filtering the fluid, a filtering unit 2026b" is provided on the fluid conduit, the filtering unit 2026b" being adapted to remove particles by a mechanical filter, such as a filter textile, or by means of a chemical filter such as activated carbon, or by a combination of a mechanical and chemical filter. Also provided on the fluid conduit 2029 is a sterilization unit 2026a" for sterilizing the circulating fluid. The sterilization unit 2026a" could for example be a sterilization device sterilizing the fluid by means of heat, a chemical sterilization device, or a sterilization device sterilizing by means of radiation, such as UV radiation. A fluid tempering unit 2026c" may also be provided on the fluid conduit 2029 for changing the temperature of the fluid passing fluid tempering device 2026c". The fluid is for example circled by means of a pump 2027", which for example could be a peristaltic pump, a gear pump, a membrane pump or a centrifugal pump.

The liquid in the chamber C may be an isotonic solution, such as a saline solution or boric acid, or an antibiotic liquid.

In alternative embodiments of the medical device, the medical device may comprise a second opening arranged such that the leg of a patient can exit the medical device.

The medical device shown in fig. 35 comprises a pressure sealing member 2005" adapted to seal by mechanically pressing against the skin of the patient, however it is equally conceivable that the pressure sealing member 2005" is assisted or replaced by a vacuum sealing member, such as the vacuum sealing member disclosed in any of the other embodiments herein, being adapted to hold a pressure below atmospheric pressure and thereby provide sealing.

The pressure sealing member 2005" may for example be in fluid connection with a pressure tank 2012 providing pressurized fluid for creating the pressure in the pressure sealing member 2005".

The medical device shown in fig. 35 further comprises an energy transferring coupling 2056 adapted to transfer energy from outside the chamber C into the sealed environment C, such that a tool 2016 within the chamber C can be energized. The coupling 2056 shown in fig 35 is a pneumatic coupling 2056 adapted to transfer pneumatic energy for energizing a pneumatic tool 2016 within the chamber C. However, in other embodiments, it is equally conceivable that the energy transferring coupling is adapted to transfer mechanical energy, in direct, hydraulic or magnetic form, and thus the energy consuming tool being a consumer of mechanic or hydraulic energy. It is furthermore conceivable that the energy transferring coupling is adapted to transfer electric energy such that a tool consuming electrical energy can be operated within the chamber C. The tool may be an orthopedic power tool, such as an orthopedic saw or orthopedic drill, or a surgical instrument for providing light or visual inspection, i.e. a camera.

The energy consuming tool 2016 could be pre-placed within the chamber C before the medical device is placed in connection with the patient, such that nothing needs to be transferred in and out of the chamber C after the chamber C has been positioned in contact with the skin of the patient, thus maintaining the chamber C sealed from the ambient environment.

The embodiment of fig. 35 further comprises a port 2022 positioned in the skin S of the patient for enabling manipulation within a cavity in the patient. In alternative embodiments (not shown) the port 2022 may comprise at least two ports such that multiple objects may be used simultaneously in the same port. For example, such a multiport may be adapted to receive two surgical instruments for manipulation and one camera for visual inspection of the relevant area. In alternative embodiments, a first port is adapted to handle a surgical instrument, and a second port is adapted to enable the transfer of an object from the chamber C to a cavity in the patient's body, an object may for example comprise a prosthetic part to be implanted in the patient, a part of the patients body to be removed, such as a tumor. One or more of the ports could comprise a an elastic membrane adapted to, in a non-expanded state, seal between the sealed environment and the environment outside of the sealed environment, and in an expanded state enable a hand to be inserted through the elastic membrane.

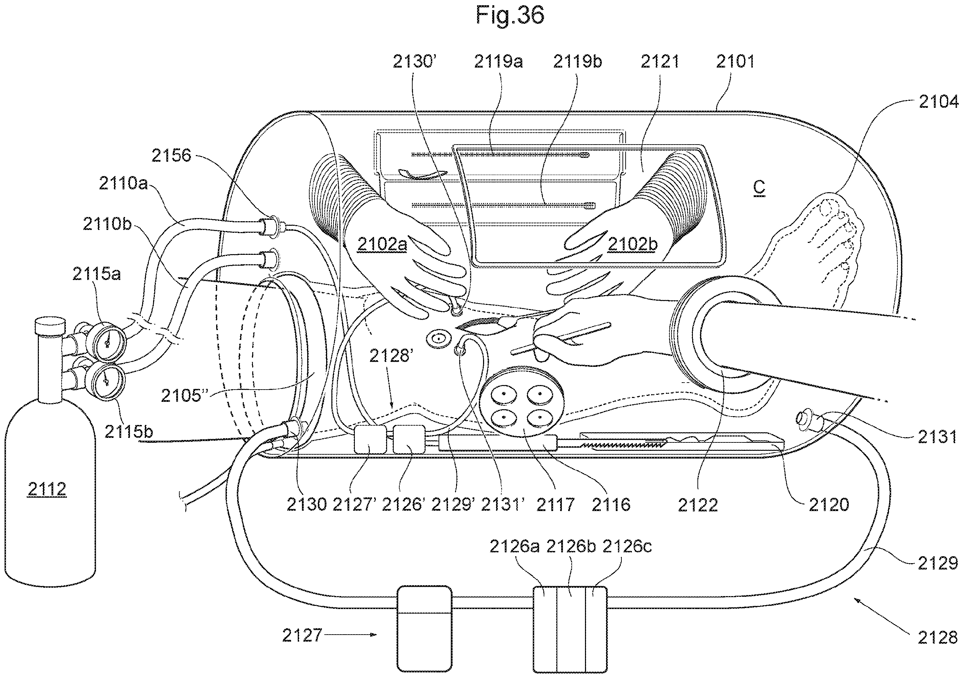

Fig. 36 shows the embodiment of the medical device similar to the embodiment disclosed under reference to fig. 35. The arm of the surgeon is inserted through a self sealing wall port 2122 in the partially collapsible wall 2101 for enabling manual manipulation therein. Manual manipulation through the self sealing wall port 2122 could for example be used as a last resort should complications occur during the procedure, or if additional hands are required for a specific step of the operation.

The embodiment of fig. 36 further shows two systems for fluid circulation. One internal system 2128' for fluid circulation within the joint of the patient, and one external system 2128 for fluid circulation in the chamber C. Both systems comprise fluid conduits 2129;2129'. In the external system 2128, the fluid conduits are connected at an inlet 2130 placed in the partially collapsible wall 2101 for supplying fluid to the chamber C, and an outlet 2131 for draining the fluid from the chamber C, and in the internal system 2128', the fluid conduits are connected to the inlet 2130' in the patient's body and an outlet 2131' in the patient's body for circulating fluid in a body cavity. The fluid is circulated by means pumping units 2127; 2127' and is circulated via a sterilizing/filtering/tempering unit 2126'. The external system 2128 disclosed contains a sterilizing 2126a, filtering 2126b, and tempering 2126c unit adapted to remove impurities, sterilize and temper the fluid. In embodiments where the fluid is a gaseous fluid, the filtering unit 2126b is adapted to remove particles that could be suspended in the gas. The filtering unit 2126a could further comprise an inlet for allowing the addition of gas from the surrounding environment. In cases where the circulating fluid is a liquid, the transparency of the liquid is of crucial importance for enabling the surgeon to have visual control of the procedure. The liquid is in this embodiment filtered for the removal of impurities and maintained transparency and sterilized. The filtering unit 2126a could for example comprise a filter containing activated carbon. The liquid circled could for example be an isotonic solution and an antibiotic liquid.

Sterilizing methods could comprises the use of heat, such as electrical or chemical heat, it is furthermore conceivable that the sterilizing is performed using irradiation, such as UV-light and/or though the addition of a chemical sterilizing agent.

In alternative embodiments, the medical device shown in fig. 36 may be altered such that the medical device comprises a second opening arranged such that the leg of a patient can exit the medical device. Such an embodiment would make the medical device smaller when for example being adapted for knee joint surgery.

The medical device in fig. 36 further comprises an elongated tubular enclosure 2104 extending within the chamber C of the medical device. The tubular enclosure 2104 is contacting the skin of the patient and is adapted to separate the skin of the patient from the chamber C of the medical device. The elongated tubular enclosure 2104 is for example a flexible or elastic tubular enclosure 2104 suitable for receiving the leg of a patient, and for encircling the leg of the patient. The steps of the surgical procedure being performed within the chamber C thus includes cutting through the tubular enclosure 2104 for accessing the skin of the patient and thus being able to create an incision in the skin for accessing the knee of the patient. The tubular enclosure 2104 may comprise an adhesive on the side facing the skin of the patient such that the tubular enclosure 2104 can be fixated and sealed against the skin of the patient. By fixating the elongated tubular enclosure 2104 tightly against the skin of the patient, the risk of infections caused by bacteria present on the skin of the patient is reduced.

The medical device shown in fig. 36 comprises a pressure sealing member 2105" adapted to seal by mechanically pressing against the skin of the patient, however it is equally conceivable that the pressure sealing member 2105" is assisted or replaced by a vacuum sealing member, such as the vacuum sealing member disclosed in any of the other embodiments herein, being adapted to hold a pressure below atmospheric pressure and thereby provide sealing.

The pressure sealing member 2105" may for example be in fluid connection with a pressure tank 2112 providing pressurized fluid for creating the pressure in the pressure sealing member 2105".

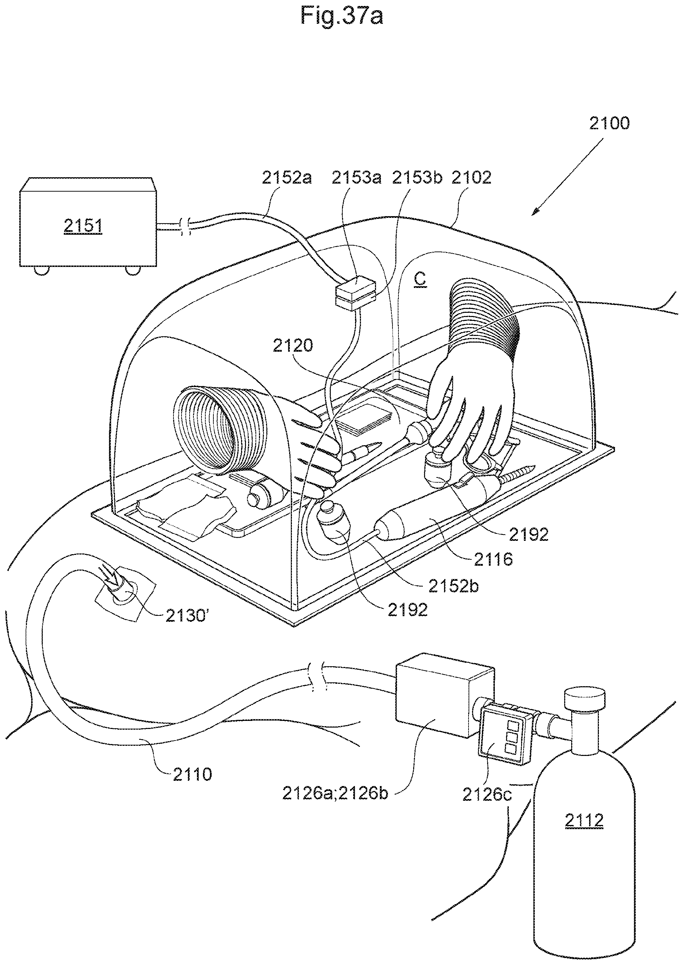

The medical device shown in fig. 36 further comprises an energy transferring coupling 2156 adapted to transfer energy from outside the chamber C into the sealed environment, such that a tool 2116 within the chamber C can be energized. The coupling 2156 shown in fig 211 is a pneumatic coupling 2156 adapted to transfer pneumatic energy for energizing a pneumatic tool 2116 within the chamber C. However, in other embodiments, it is equally conceivable that the energy transferring coupling is adapted to transfer mechanical energy, in direct, hydraulic or magnetic form, and thus the energy consuming tool being a consumer of mechanic or hydraulic energy. It is furthermore conceivable that the energy transferring coupling is adapted to transfer electric energy such that a tool consuming electrical energy can be operated within the chamber. The tool may be an orthopedic power tool, such as an orthopedic saw or orthopedic drill, or a surgical instrument for providing light or visual inspection, i.e. a camera. Couplings are further disclosed under reference to figs. 37a - 39.

The energy consuming tool 2116 could be pre-placed within the sealed environment before the medical device is placed in connection with the patient, such that nothing needs to be transferred in and out of the chamber C after the chamber C has been positioned in contact with the skin of the patient, thus maintaining the chamber C sealed from the ambient environment.

Figs. 37a - 39 shows a system for transferring energy and/or information from the ambient environment into the chamber C of the medical device 2100 whilst maintaining the sealed environment in the chamber C. The system could be used for transferring energy and/or information to the chamber C in any of the embodiments of the medical device disclosed herein. The energy could be provided to an energy consuming tool to be used within the chamber C, or an optical or imaging instrument needed inside the chamber C for supplying visual information. An information coupling could be needed to supply information (e.g. control information) to an energized tool inside the chamber, or for providing information from within the chamber to medical personnel on the outside. Information supplied from the chamber could for example include visual information for example provided by an endoscopic camera provided inside the chamber, or sensor information provided by a sensing probe within the chamber. Sensor information could for example be sensor information related to a physiological parameter of the patient, such as temperature, blood pressure, or blood flow, or a physical parameter of the medical device, such as the pressure in seals or in the chamber.

Fig. 37a shows an embodiment of the medical device 2100 similar to the embodiment disclosed under reference to fig, 214. The difference being that the fluid inlet is a fluid body inlet 2130' adapted to inflate a cavity in the patient's body. The fluid is then transferred to a sealed chamber C of the medical device 2100 through the trocars 2192 creating a fluid connection between the cavity in the patient and the chamber C of the medical device 2100. The medical device 2100 shown in fig. 37a comprises a wall 2101 adapted to be applied on the patient's body to form a sealed chamber C, in which a sealed environment can be maintained for encompassing part of the surgical procedure to be performed. The medical device 2100 further comprises a kinetic energy transferring member 2152a, 2152b for transferring energy from outside of the sealed chamber C to inside of the sealed chamber C. The first portion of the kinetic energy transferring member 2152a, positioned on the outside of the medical device 2100 is connected to an electric motor 2151 converting electrical energy to kinetic energy. The first portion of the kinetic energy transferring member 2152a is in the other end connected to a kinetic energy coupling 2153, comprising an external part 2153a adapted to be placed on the outside of the wall 2101 of the medical device 2100 and an internal part 2153b adapted to be placed on the inside of the wall 2101 of the medical device 2100. The kinetic energy coupling is adapted to wirelessly transfer kinetic energy from the inside of the chamber C to the outside of the chamber C wirelessly through the wall 2101 of the medical device 2100 by means of for example inductive or magnetic coupling. The internal part 2153b of the kinetic energy coupling is connected to a second portion of the kinetic energy transferring member 2152b which is connected to a kinetic energy consuming tool 2116, such as an orthopedic drill.

The fluid body inlet 2130' is connected to a fluid conduit 2110, which in turn is connected to a pressurized fluid tank 2112 via a tempering unit 2126c, a sterilizing unit 2126a and a filtering unit 2126b for filtering, sterilizing and tempering the fluid entering the cavity in the patient's body. The filtering could for example remove particles from the fluid and the tempering of the fluid could be a heating of the fluid for making the environment more suitable for the surgical procedure or the medical personnel, or a cooling of the fluid for example reducing the growth rate of bacteria in the chamber C.

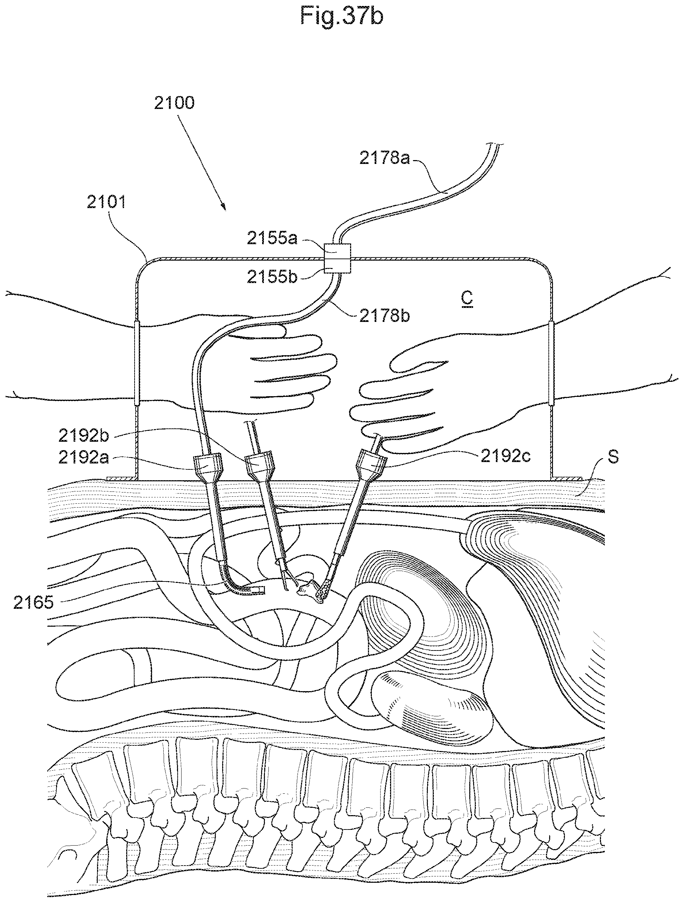

Fig. 37b shows an embodiment of the medical device 2100 placed on the abdomen of a patient. The medical device 2100 comprises three trocars 2192a, 2192b, 2192c positioned through the skin S of the patient such that a surgical procedure within the abdomen of the patient can be performed. The medical device 2100 comprises an information coupling 2155a, 2155b for transferring visual information from an endoscopic camera 2165 positioned in one of the trocars 2192a. The visual information is transferred via a lead 2178b to the interior part of the information transferring coupling 2155b to the exterior part of the information transferring coupling 2155a and further via another lead 2178a to a display device for displaying the visual information captured by the endoscopic camera 2165.

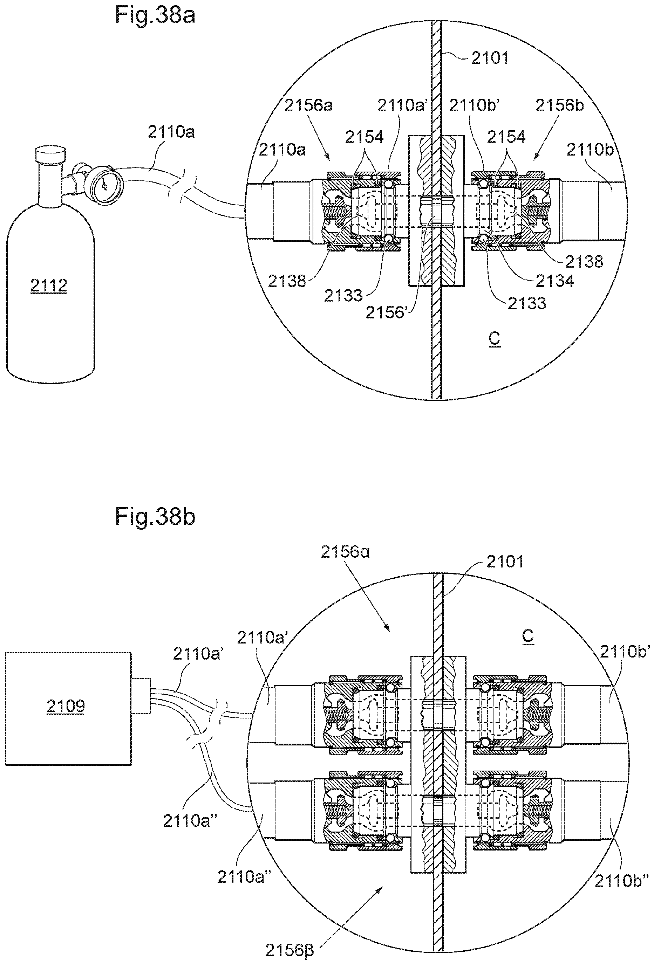

Fig. 38a shows a fluid energy transferring coupling 2156 in further detail. The fluid coupling 2156 enables the transfer of a pressurized fluid from a pressure source 2112 outside of the chamber C such as the pressurized fluid tank 2112 shown in fig. 38a connected via a fluid conduit 2110a to the external portion 2156a of the fluid coupling. The external portion 2156a of the fluid coupling comprising a valve 2138 adapted to open when the fluid conduit 2110 is connected to the fluid coupling tube 2156' penetrating the wall 2101 of the medical device. When the fluid coupling 2156a; 2156b is not coupled to the fluid conduit 2110a, the valve 2138 remains closed, hence, fluid can only be transferred from the fluid conduit 2110a on the outside of the chamber C to a fluid conduit 2110b on to inside of the chamber C and never to the environment in the chamber C, thus can the environment of the chamber C be kept sealed. The tube 2156' comprises a recess 2134 in the form of a groove adapted to receive protruding members 2133 of the fluid conduit 2110a for locking the fluid conduit 2110a to the tube 2156'. The protruding members 2133 is small spherical balls 2133 which are pressed into the recess 2134 by a slideable external portion 2110a' of the fluid conduit 2110a. The leftwards sliding of the external portion 2110a' causing the spherical balls 2133 to engage a recess of the external portion 2110a' and thus be removed from the recess 2133 of the tube 2156', thus releasing the fluid conduit 2110a from the tube 2156'. The inside of the connecting portion of the fluid conduit 2110a comprises a plurality of O-rings 2154 providing sealing between the tube 2156' and the connecting portion of the fluid conduit 2110a. The internal portion of the fluid coupling 2156b is identical to the external portion 2156a and thus connects an internal portion 21 10b of the fluid conduit to the tube 2156' enabling transfer of fluid in a corresponding way.

Fig. 38b shows an alternative embodiment of the fluid energy transferring coupling 2156 in detail. The alternative embodiment comprises two fluid energy transferring couplings 2156α, such that fluid energy can be circled. The fluid could enter the chamber C through a first fluid energy transferring coupling 2156β, and exit the chamber C through a second fluid energy coupling 2156, such that no fluid is added to the sealed environment of the chamber C.

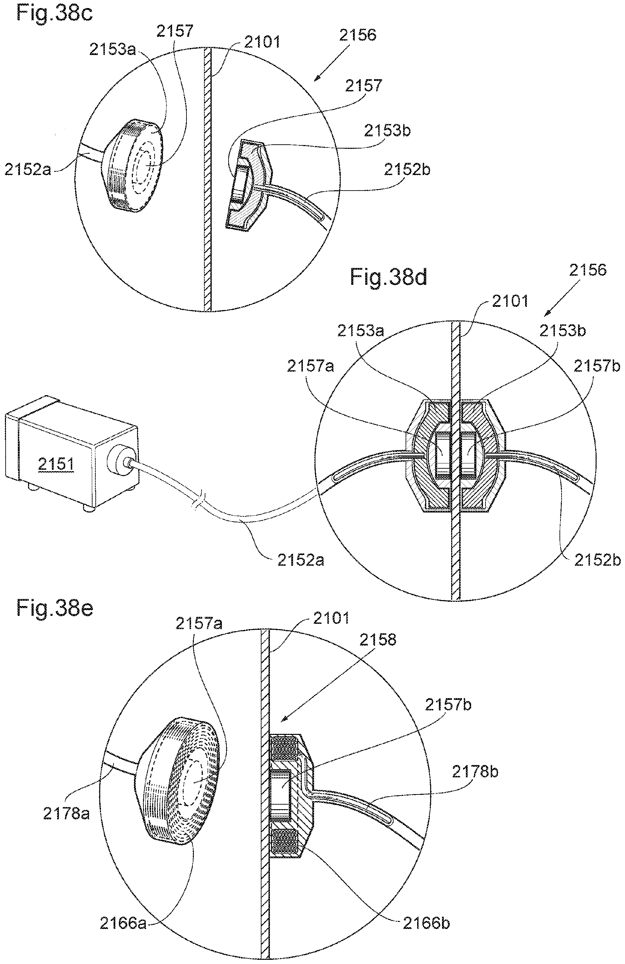

Fig. 38c shows a kinetic energy transferring coupling 2153 is detail. The coupling 2153 being adapted to transfer rotational kinetic energy, contactless through the wall 2101 of the medical device. The kinetic energy transferring coupling 2153 comprises an external part 2153a comprising a magnet connected to a kinetic energy transferring member 2152a such that the magnet of the external part 2153a rotates as the kinetic energy transferring member 2152a rotates. The kinetic energy transferring coupling 2153 further comprises an internal part 2153b connected to a kinetic energy transferring member 2152b, which in turn is connected to a tool inside of the chamber C consuming kinetic energy. The internal part 2153b comprises a magnet or magnetic material adapted to magnetically engage with the magnet of the external part 2153a, such that the magnet or magnetic material of the internal part 2153b rotates along with the magnet of the external part 2153a. The coupling further comprises a holding magnet 2157 placed centrally in the coupling 2153 and adapted to hold and align the external 2153a and internal 2153b parts of the coupling 2153, such that rotational energy can be transferred contactless from the outside of the chamber C to the inside of the chamber C, through the wall 2101 of the medical device, such that the chamber C is kept sealed while transferring kinetic energy from the outside of the chamber C to the inside of the chamber C.

Fig. 38d shows the kinetic energy transferring coupling 2153 of the embodiment shown in fig. 38c when connected by means of the holding magnets 2157a; 2157b magnetically holding and aligning the external 2153a and internal 2153b parts of the kinetic energy transferring coupling 2153, such that kinetic energy can be transferred from the outside of the chamber C to the inside of the chamber C. The external part 2153a of the kinetic energy transferring coupling 2153 is connected to a kinetic energy transferring member 2152a which in turn is connected to an electric motor 2151 transforming electrical energy to rotational kinetic energy. The internal part 2153b is connected to a kinetic energy transferring member 2152b, such as a wire adapted to transfer rotational force, which in turn is connected to a tool inside of the chamber C consuming kinetic energy, such as an orthopedic drill or an orthopedic reamer.

Fig. 38e shows an electric energy transferring coupling 2158 adapted to transfer energy from outside of the chamber C to the inside of the chamber C by means of induction. The energy transferring coupling 2158 comprises a first external part, comprising a first coil 2166a connected to a lead 2178a connected to some form of supply of electric energy. The second part comprises a second coil 2166b connected to a second lead 2178b, which in turn is connected to a tool within the chamber C consuming electric energy. Both the internal part and the external part further comprises magnets 2157a; 2157b adapted to hold and align the electric energy transferring coupling 2158 such that electric energy is efficiently transferred. Using the electric energy transferring coupling 2158 of fig. 38e, electric energy can be wirelessly transferred from the outside of the chamber C to the inside of the chamber C, such that the chamber C can be remained sealed during the transfer of the electric energy. The tool consuming electric energy could be a mechanical tool, such as an orthopedic drill, an optical instrument, such as a camera, or an electrical instrument, such as a diathermy instrument. The electric energy transferring coupling 2158 could furthermore be adapted to transfer information, e.g. in the form of binary electric pulses. The information could for example be optical information from a camera placed on the inside of the chamber C of the medical device to the outside of the chamber C of the medical device.

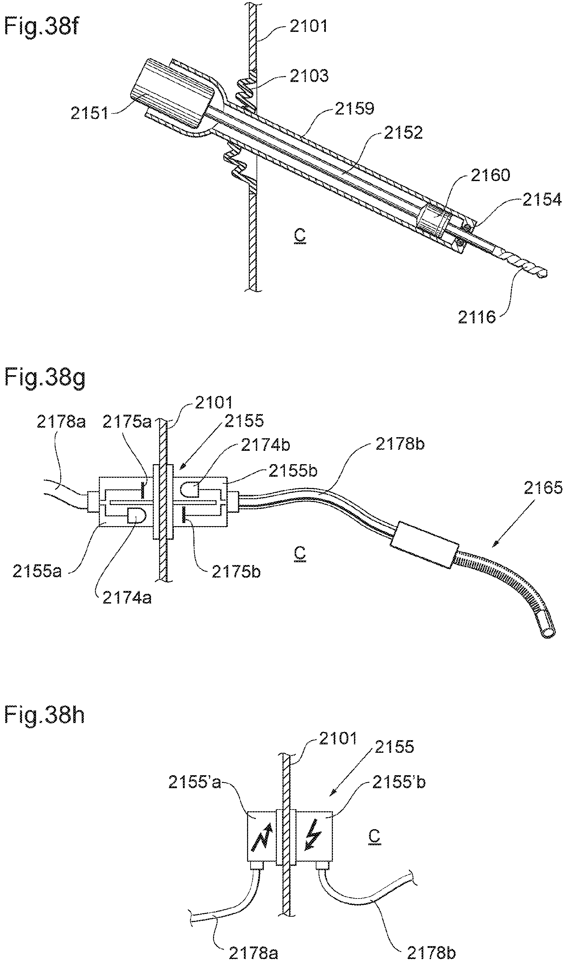

Fig. 38f shows an embodiment of a kinetic energy transferring member 2152 for transferring kinetic energy from outside of the chamber C to the inside of the chamber C. The device for kinetic energy transferring member comprises a sleeve 2159 adapted to be positioned through the wall 2110 of the medical device 2100. The sleeve 2159 is fixated to a pleated portion 2103 if the wall 2101 of the medical device, the pleated portion 2103 being adapted to enable movement of the sleeve 2159 in the chamber C, such that the position of the kinetic energy delivered inside the chamber C of the medical device 2100 can be changed. A motor 2151 is positioned in the sleeve 2159 and connected to an elongated kinetic energy transferring member 2152 adapted to transfer rotational kinetic energy. The elongated kinetic energy transferring member 2152 is in turn connected to a tool holder 2160 to which a tool 2116 consuming kinetic energy (such as the shown drill) can be fixated. The tool 2116 consuming kinetic energy is sealingly connected to the sleeve 2159 via an O-ring 2154 which acts as bearing and sealing between the sleeve 2159 and the tool 2116. As the O-ring 2154 provides sealing between the tool and the sleeve 2159 the hollow inside of the sleeve 2159 is sealed from the chamber C of the medical device, and thus is the chamber C sealed from the ambient environment when kinetic energy is transferred between the outside of the chamber C and the inside of the chamber C. The electric motor 2151 supplying rotational kinetic energy could be replaced by a motor supplying translating kinetic energy, in which case the kinetic energy transferring member 2152 transferring rotational kinetic energy is replaced by a kinetic energy transferring member adapted to transfer translating kinetic energy to for example a translating saw blade or a pivoting tool.

Fig. 38g shows an embodiment of an information transferring member 2155 adapted to wirelessly transfer information from inside the chamber C of the medical device to outside of the chamber C. The information transferring member 2155 comprises an external part 2155a positioned on the outside of the wall 2101 of the medical device and connected to the wall 2101. The external part 2155a comprises an Infra Red (IR) diode 2174a adapted to emit information in the form of IR light pulses, and an IR detector 2175a adapted to receive information in the form of IR light pulses. The information transferring member 2155 further comprises an internal part 2155b adapted to be placed on the inside of the wall 2101 of the medical device, connected to the wall 2101 of the medical device and aligned with the external part 2155a of the information transferring member 2155a such that the IR diode 2174a can emit information in the form of IR light pulses which can be received by the IR detector of the internal part 2155b such that information can be transferred from the external part 2155a to the internal part 2155b, through the wall 2101 of the medical device. The internal part 2155b further comprises an IR diode 2174b aligned with the IR detector 2175a of the external part 2155a, such that information can be transferred from the inside of the chamber C, through the wall 2101 of the medical device by means of IR light pulses detected by the IR detector 2175a.

Information transferred from the inside of the chamber C to the outside of the chamber C could for example be image data captured by a camera 2165 placed inside the chamber C of the medical device transferred to the internal part 2155b of the information transferring member 2155 by means of a lead 2178b. After the image data has been wirelessly transmitted through the wall 2101 of the medical device by means of the information transferring member 2155, the information is transferred to a display unit by means of a lead 2178a, such that medical personnel can see the images captured by the camera 2165 placed inside the chamber C of the medical device. Information required to be transferred into the chamber C for example includes settings for the camera 2165 placed inside the chamber C such as for example zoom level and/or resolution.

In alternative embodiments, the IR diode can be replaced by some other type of light emitting diode (LED) and a corresponding detector, without parting from the inventive concept. One advantage with having an information transferring member based on the transmission of light pulses is that no radio signals are emitted, which reduces the risk that the wireless transfer shall disturb other electronic equipment in the operating theater.

Fig. 38h shows an embodiment of the information transferring member 2155 in which the information transferring member 2155 comprises a first 2155a and second 2155b radio transceiver adapted to transmit and receive radio signals containing information from the inside of the chamber C to the outside of the chamber C, and vice versa. The first 2155a and second 2155b radio transceiver respectively being coupled to units transmitting and/or receiving information by means of leads 2178a; 2178b. Units that transmits and/or receives information could for example be control units or monitoring equipment on the outside of the chamber C and optical instruments or sensors on the inside of the chamber C.

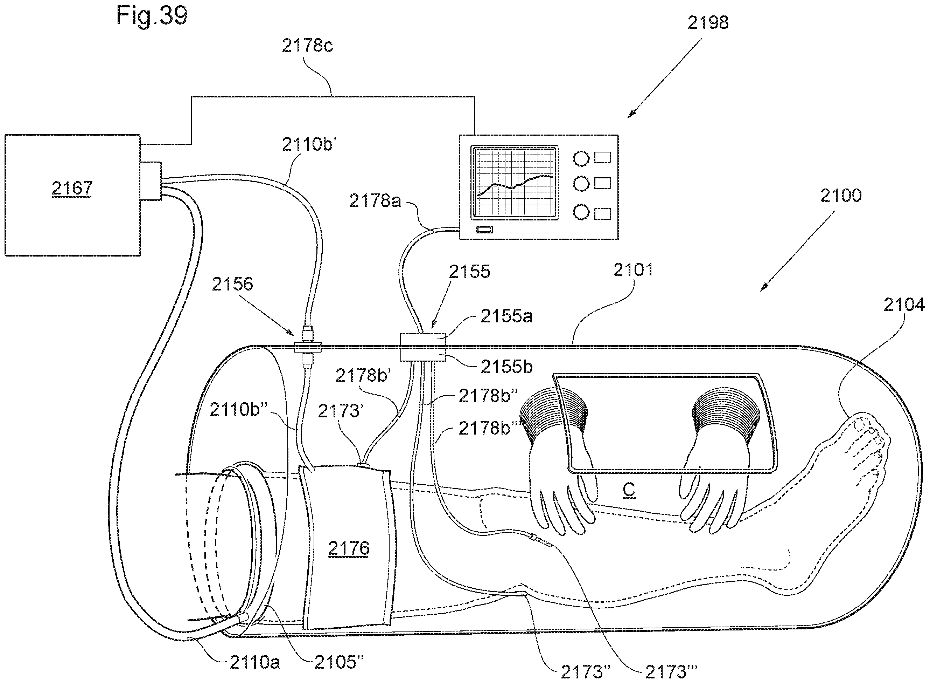

Fig. 39 shows an embodiment of the medical device 2100 in which a wall 2101 of the medical device 2100 forms a chamber C, in which a sealed environment can be maintained for encompassing part of a surgical procedure. The medical device 2100 further comprises a monitoring unit 2198 comprising a blood pressure monitoring unit operated as a sphygmomanometer. The blood pressure monitoring unit is connected to a cuff 2176 which is snugly placed around the leg of the patient. The pressure monitoring unit is operated by the cuff 2176 being inflated to a pressure initially in excess of the systolic arterial pressure and then reduced to below diastolic pressure over a period of about 30 seconds. When no blood is flowing passed the cuff 2176, the cuff 2176 pressure will be essentially constant. When blood flow is present, but restricted, the cuff 2176 pressure, which is monitored by a pressure sensing probe 2173' connected to the blood pressure monitoring unit, will vary periodically in synchrony with the cyclic expansion and contraction of the arteries. The values of the systolic and diastolic pressure can thus be computed. The flow is measured using an ultra sonic flow meter 2173" connected to a lead 2178b' and being adapted to measure the blood flow at a relevant place of the patient's leg, such as the lateral side of the knee. The blood pressure monitoring unit may be adapted to measure the blood pressure of the patient periodically, such as for example with an interval of: 1 - 5 minutes, 5 - 10 minutes, 10 - 20 minutes, or 20 - 30 minutes. The medical device 2100 further comprises a fluid energy transferring coupling 2156 (such as the fluid energy transferring coupling disclosed under reference to fig. 38a and 38b). The fluid energy transferring coupling 2156 connects a first portion of a fluid conduit 2110b' to a second portion of the fluid conduit 2110b". The first portion 2110b' of the fluid conduit is connected to a pressure creating member 2167 for supplying the fluid conduit 2110b' with a pressurized fluid. The second portion 2110b" of the fluid conduit is connected to the inflatable cuff 2176. The medical device further comprises a wireless information transferring member 2155 (such as the wireless information transferring members shown in figs. 38g and 38h, for wirelessly transferring values of the sensed blood flow through the wall 2101 of the medical device 2100 to the monitoring unit 2198. The monitoring unit is in turn connected via a lead 2178c to the pressure creating member 2167 which creates the pressure inflating the cuff 2176. By providing feedback to the pressure creating member inflating the cuff 2176 the blood flow in the leg of the patient can be regulated.

The embodiment shown in fig. 39 further comprises an invasive sensing probe 2173‴ adapted to sense a physiological parameter of the patient's body, such as saturation of the blood of the patient, or an ischemia marker such as lactate. The invasive sensor 2173‴ is connected to a lead 2178b‴ which in turn is connected to an internal part 2155b of the information transferring member 2155 such that the information from the invasive sensor 2173‴ is wirelessly transmitted to an external part 2155a of the information transferring member 2155 and further via a lead 2178a to a monitoring unit 2198 for monitoring the sensed physiological parameter. The monitoring unit 2198 is connected to the pressure creating member 2167 for providing feedback to the pressure creating member 2167 such that the pressure in the sealing member 2105" and/or the pressure in the cuff 2176 can be regulated on the basis of input from one or more of the pressure sensor 2173' sensing the pressure in the cuff 2176, the ultra sonic blood flow sensor 2173" sensing the blood flow in a blood vessel in the leg of the patient, and the invasive sensor 2173‴ sensing a physical parameter of the patient. By the described monitoring, it is possible to mediate between establishing that the leg of the patient is sufficiently saturated and that the pressure seal provides sufficient sealing between the elongated tubular enclosure 2104 and the ambient environment.

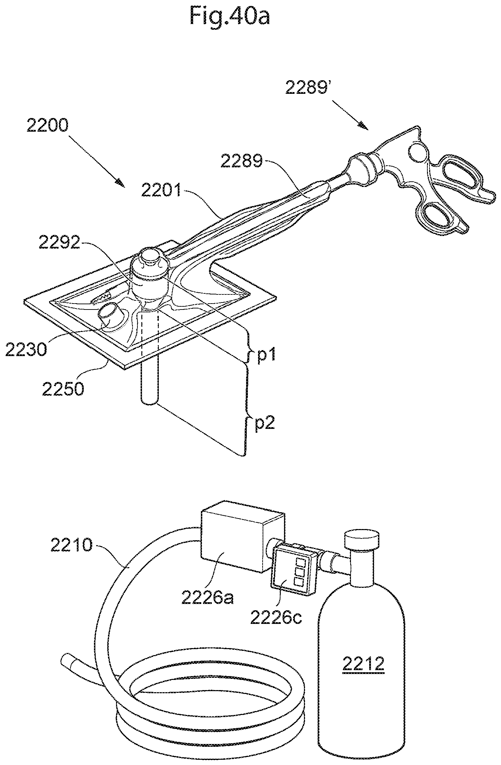

Fig. 40a - 40q"" shows a medical device for performing endoscopic surgery in a way such that the laparoscopic surgery can remain completely encapsulated during the entire operation. During normal laparoscopic surgery, leakage occurs in the incisions in which the trocars are placed, in the sealing membranes within the trocars and in connection with the inflation device penetrating the skin of the patient. The advantages of keeping the operation completely encapsulated includes less use of laparoscopic gas, further reduced risk of infections and reduced risk that the laparoscopic gas containing fumes from e.g. diathermy instruments used in the operation pollutes the environment of the operating theater. The advantage that the device enables a surgery which consumes less laparoscopic gas is particularly important when the medical device is used for field surgery, in which case the supply of laparoscopic gas may be limited.

In the medical device 2200 of figs. 40a - 40d, a trocar is placed through the bottom wall of the medical device 2200 such that a first portion p1 of the trocar 2292 is placed inside of a chamber formed by the wall 2201 and a second portion p2 of the trocar 2292 is placed outside of the chamber such that the second portion p2 of the trocar can be inserted through the skin of the patient and into a cavity of the patient. The second portion p2 of the trocar 2292 is protected by a protective sheet that is removed prior to the insertion of the trocar 2292 into the patient. The inside of the trocar 2292 preferably comprises a membrane which separates the chamber enclosed by the wall 2201 form the ambient environment and the environment within the cavity of the patient when the trocar 2292 is inserted through the skin of the patient. The endoscopic instrument 2289 is entirely encapsulated by the wall 2201 and thus completely placed in the chamber when not inserted through the membrane of the trocar 2292. The endoscopic instrument 2289 can thus be positioned inside of the chamber until the trocar 2292 is positioned through the skin of the patient, thus the endoscopic instrument 2289 is positioned either inside of the chamber, or inside of the cavity formed within the patient.

Fig. 40a shows a medical device for performing endoscopic surgery. The medical device comprises a wall 2201 adapted to form a chamber in which a step of the surgical procedure can be performed. A portion of the wall 2201 comprising an adhesive portion 2250 is adapted to be fixated to the skin of the patient. The medical device 2200 comprises an endoscopic instrument 2289 enclosed within the chamber. The medical device further comprises a trocar 2292 positioned through the wall 2201 of the medical device 2200 such that a fluid connection can be established between the chamber enclosed by the wall 2201 of the medical device and a cavity in the patient. A first portion p1 of the trocar 2292 being placed inside of the chamber of the medical device 2200, and a second portion p2 of the trocar 2292 being positioned on the outside of the chamber. The medical device comprises a fluid inlet 2230 positioned in the wall 2201 adapted to be connected to a system for inflating the chamber of the medical device 2200. The system comprising a fluid conduit connected to a tank 2212 comprising pressurized fluid. The system further comprising a tempering unit 2226c for tempering the fluid entering the chamber, and a sterilizing 2226a and/o filtering system for sterilizing and/or filtering the fluid entering the chamber. The surgical instrument 2289 comprises a handle portion 2289' operably encapsulated by the wall 2201 such that the handle portion 2289' can be manipulated from outside the chamber.

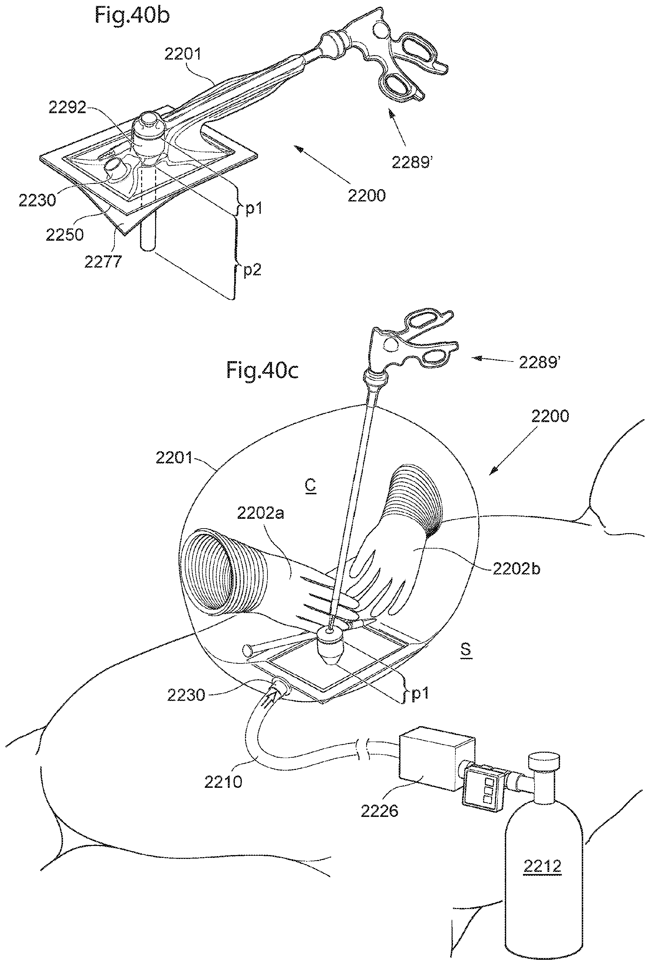

Fig. 40b shows the medical device 2200 according to the embodiment shown in fig. 40a, when a covering sheet 2277 is being removed from the adhesive surface 2250 of the medical device 2200, such that the second portion p2 of the trocar 2292 and the adhesive surface 2250 of the wall 2201 of the medical device 2200 is exposed such that the medical device 2200 can be fixated to the skin S of the patient.

Fig. 40c shows the medical device according to the embodiment shown in fig. 40a and 40b, when the adhesive portion 2250 of the wall 2201 is fixated to the skin S of the patient and the wall 2201 is inflated such that a chamber C is created in which a part of the surgical procedure can be performed. The wall 2201 of the medical device 2200 is flexible or elastic and adapted to hold a pressure exceeding atmospheric pressure. The wall for example being made from a flexible polymer material, such as polyvinyl chloride (PVC) with a plasticizer additive, or an elastic polymer material, such as silicone. The wall 2201 of the medical device 2200 is inflated by means of the system for inflation disclosed with reference to fig 220a through the inlet 2230 in the wall 2201 of the medical device 2200. The medical device 2200 additionally comprises gloves 2202a; 2202b placed in the wall 2201 of the medical device 2200 and enabling manipulation within the chamber C of the medical device 2200. In alternative embodiments, the medical device shown in figs. 40a - 40c may comprise a vacuum sealing member assisting or replacing the adhesive portion 2250.

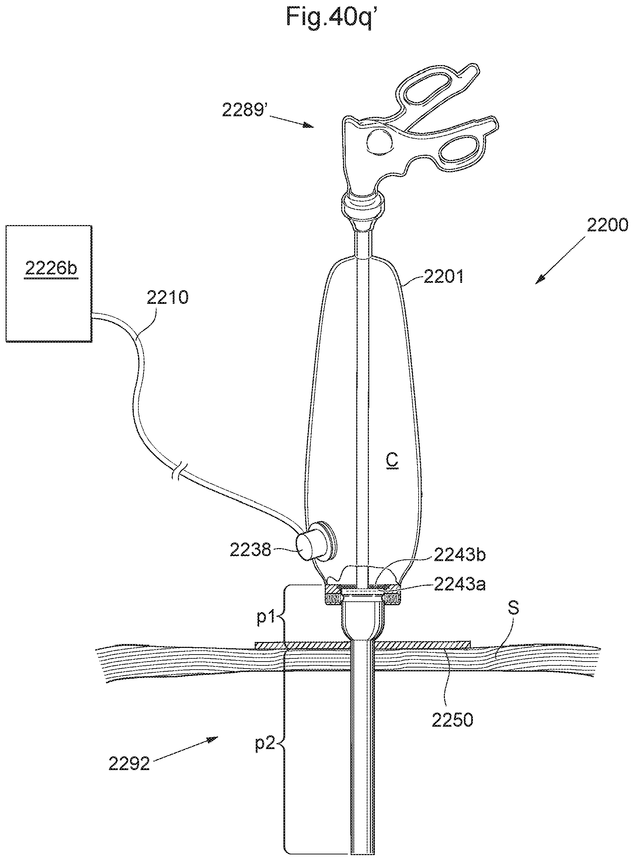

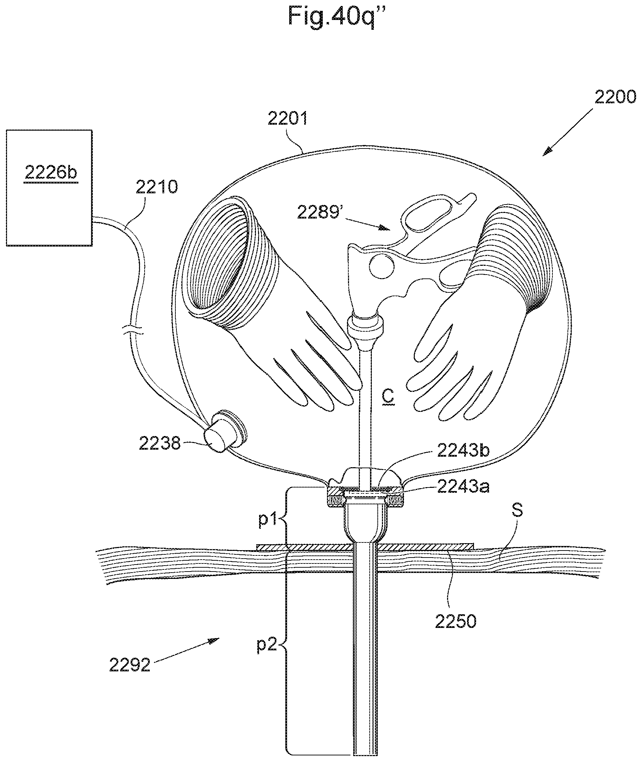

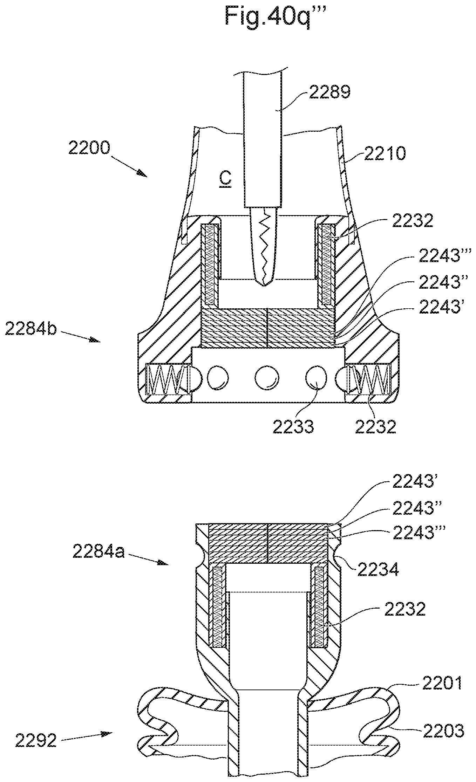

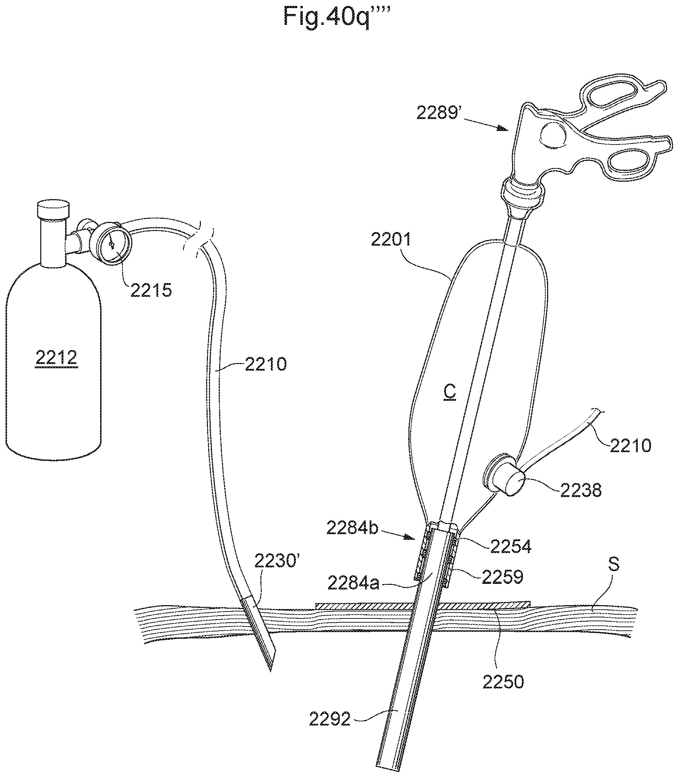

The medical device shown in figs. 40a - 40c may additionally comprise an evacuation valve for evacuating a fluid from the chamber, such as the evacuation valve and system shown in fig. 40q".

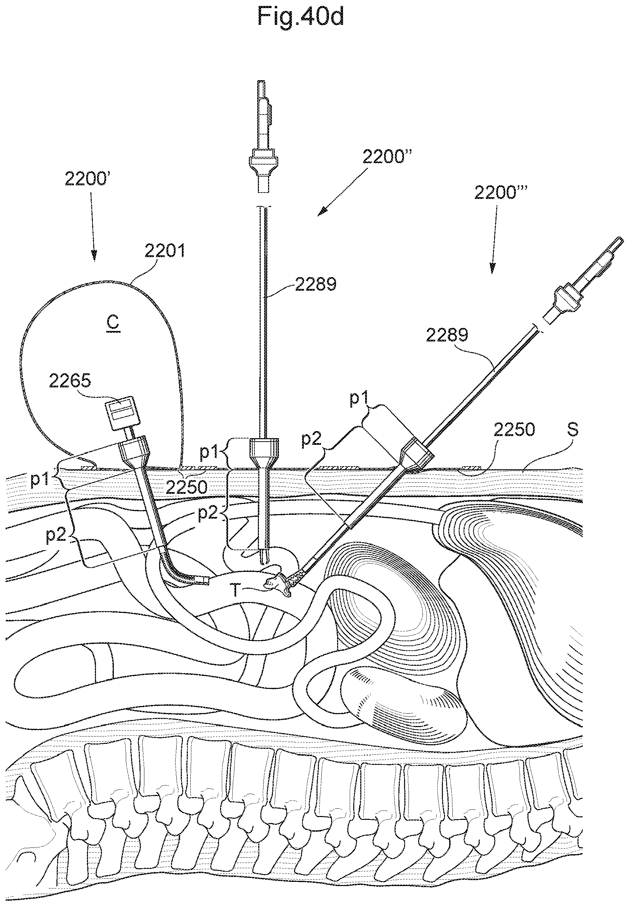

Fig. 40d shows and endoscopic system using the medical devices shown in figs. 40a - 40c, when used for performing an endoscopic operation. A first medical device 2200' comprises an endoscopic camera 2265 enclosed within the wall 2201 of the medical device 2200' is used for enabling visual inspection in a cavity within the patient, a second medical device 2200". Second 2200" and third 2200‴ medical devices are provided for inserting surgical instruments 2289 into the cavity in the patient for performing an endoscopic procedure, such as removing a tumor T on an intestine of the patient. Each medical device being fixated to the skin S of the patient by means of an adhesive portion 2250 of the wall 2201 of the medical device 2200. By each medical device being sealingly fixated to the skin S of the patient, a sealed endoscopic system is created which reduces the risk that bacteria will enter the

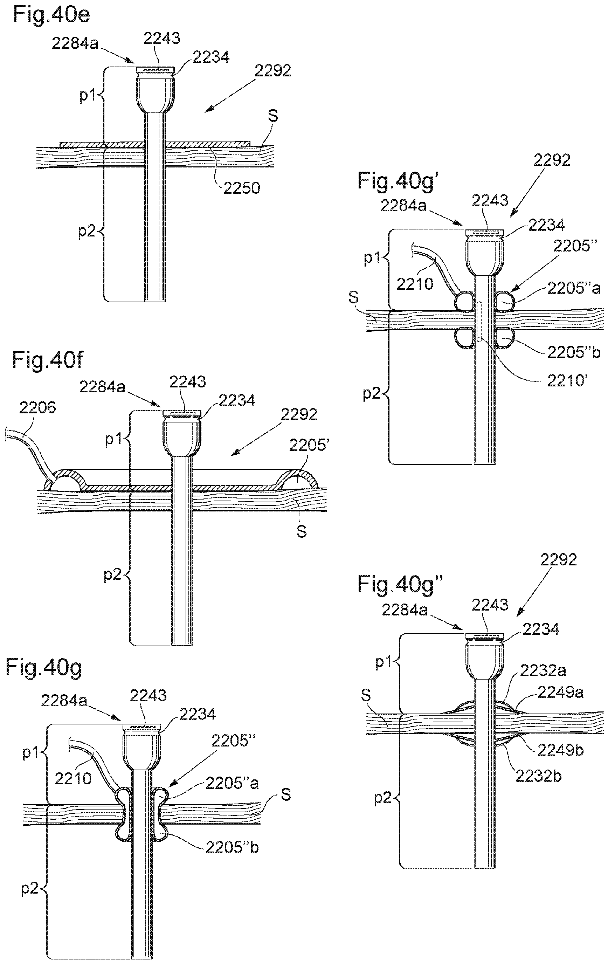

Figs. 40e - 40q shows a system for performing an endoscopic procedure in a sealed environment. The system comprises an endoscopic trocar adapted to be positioned through the skin of a patient, such that an endoscopic procedure can be performed inside a cavity of the patient, and a medical device adapted to encapsulate an instrument to be inserted through the trocar. By encapsulating the instrument, the instrument can be kept sterile even when being outside of the patient's body, such that instruments used in a particular trocar can be changed without the risk that the trocar is contaminated while being on the outside of the patient's body. Encapsulated endoscopic surgery could for example be used when the risk of infections is particularly large, such as in arthroscopic surgical procedures in a joint of a patient, or in environments that are difficult to keep sterile, such as e.g. in field hospitals and in hospitals in third world countries. The embodiments of figs. 40e - 40q shows alternative embodiments of the features needed to create a sealed endoscopic system. A further advantage with the sealed endoscopic system is that fluids used to inflate a cavity in the patient, does not leak outside of the sealed area of the endoscopic system, which keeps the consumption o the fluids at a minimum. Keeping the fluid consumption low could be of utmost importance in field applications where the fluids are a scarce resource. The features are however not limited to the particular embodiment in which it is shown and could be used in combination with any of the embodiments for creating a functioning medical device system.

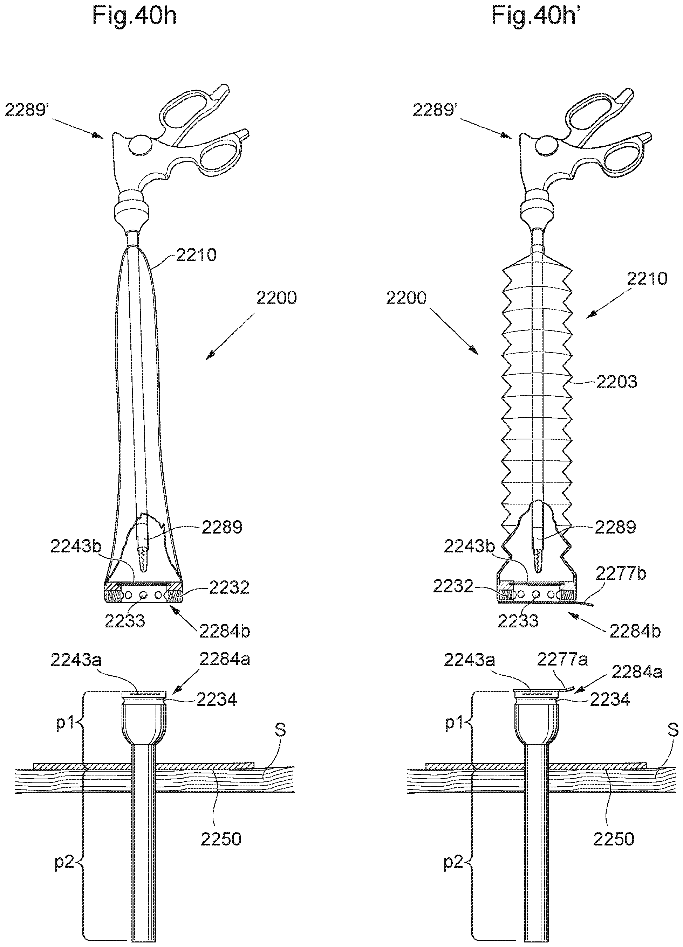

Fig. 40e shows a trocar 2292 having a first portion p1 adapted to be positioned on the outside of the patient's body, and a second portion p2 adapted to be positioned through the skin S of the patient and into a cavity in the patient's body. The trocar 2292 is adapted to be fixated to the skin S of the patient by means of an adhesive surface 2250 fixated to the trocar 2292 and being adapted to seal and fixate against the surface of the skin S of the patient. The trocar 2292 further comprises a seal 2243 within the trocar 2292, in the form of a self sealing membrane 2243 adapted to seal between the cavity in the body of the patient and the ambient environment. The self sealing membrane 2243 for example comprises a silicone self sealing membrane, or a gel type self sealing membrane, such as the gel port available from Applied medical inc. The trocar 2292 further comprises a connecting portion 2284a, for connecting the trocar 2292 to a medical device, such as the medical devices shown in figs. 40h, 40h', 40l - 40o. The connecting portion 2284a of the embodiment shown in fig. 40e comprises a recess 2234 in the form of a groove 2234 encircling the connecting portion 2284a of the trocar 2292. The recess 2234 is adapted to receive a protruding portion of a connecting portion of a medical device. However in alternative embodiments, the connecting portion 2284a could have a different configuration, the connecting portion could for example comprise a protruding portion and being adapted to be connected to a recess of a medical device.

Fig. 40f shows an embodiment in which the trocar 2292 comprises a vacuum sealing member 2205' adapted to hold a pressure below atmospheric pressure for providing a vacuum seal against the skin S of the patient. The vacuum sealing member 2205' is via a vacuum conduit 2206, in fluid connection with a vacuum source, such as the vacuum pump (shown as reference number xx08 in other embodiments disclosed in this application). The vacuum sealing member 2205' may be assisted by the surface contacting the skin S of the patient being adhesive.

Fig. 40g shows an alternative embodiment of the trocar, in which the trocar 2292 comprises a pressures seal 2205" connected via a fluid conduit 2210 to a pressure source (such as the pressurized fluid tank shown as reference number xx12 in other embodiments disclosed in this application, or the fluid pump shown as reference number xx09 in other embodiments disclosed in this application). When inflated, the pressure seal 2205" forms an inflated chamber having a first enlargement 2205"a on the outside of the skin S of the patient, and a second enlargement 2205"b on the inside of the skin S of the patient, such that the skin S (and tissue) is pressed from the outside by the first enlargement 2205"a and from the inside by the second enlargement 2205"b. The skin S (and tissue) is further pressed in a direction parallel to the skin S of the patient by the inflatable chamber 2205" being inflated, and sealing is thus provided both perpendicularly from the outside, against the skin S and tissue of the patient, and against the edges of the incision in the skin S, in a direction parallel to the skin S of the patient.

Fig. 40g' shows an embodiment similar to the embodiment shown in fig. 220g, the difference being that the pressure seal 2205" of the embodiment in fig. 220g' comprises two separate chambers 2205"a and 2205"b, wherein the first chamber 2205"a is adapted to press against the surface of the skin S of the patient, from the outside thereof. And the second chamber 2205"b of the pressure seal 2205" is adapted to press against the skin S and tissue of the patient from the inside thereof. The two separate chambers are adapted to be in fluid connection with each other by means of a fluid conduit 2210' running on the inside of the trocar 2292.

Fig. 40g" shows an embodiment in which the trocar 2292 comprises pivoting supports 2249a; 2249b pressing against the skin S and tissue by means of springs 2232; 2232b connected to the supports 2249a, 2249b. The external pivoting supports 2249a are adapted to press against the surface of the skin S of the patient, in a direction substantially perpendicular to the skin S of the patient and thus substantially parallel to the trocar 2292, by a spring 2232a being connected to the external support 2249a and exerting a pressing force on the external support 2249a. The internal pivoting supports 2249b are adapted to press against the inside of the skin S and tissue of the patient from the inside of the patient's body, in a direction substantially perpendicular to the skin S of the patient and thus substantially parallel to the trocar 2292, by a spring 2232b being connected to the internal pivoting support 2249b and exerting a pressing force on the internal support 2249b. The internal and external pivoting supports 2249a; 2249b can be folded against the trocar 2292, such that the trocar 2292 can be inserted through the incision performed in the skin S of the patient.

Fig. 40h shows an embodiment of a system for performing encapsulated endoscopic surgery. The system comprises the trocar 2292, further described under reference to fig. 40e, and a medical device 2200 adapted to be connected to the trocar 2292. The medical device 2200 comprises a flexible wall 2201 enclosing a chamber in which an endoscopic instrument 2289 is enclosed. The medical device 2200 comprises a connecting portion 2284b positioned in the flexible wall 2201 and adapted to connect to a connecting portion 2284a of the trocar 2292, such that a fluid connection is created between the chamber enclosed by the wall 2201, and a cavity within the patient. The connecting portion 2284b of the medical device 2200 comprises protruding members 2233 in the form of protruding balls pressed against the edge of an orifice by a spring 2232. The balls 2233 can thus be pushed into the cavity housing the spring 2232 by a force acting on the ball 2233 from the connecting portion 2284a of the trocar. When the recess 2234 of the connecting portion 2284a of the trocar 2292 is aligned with the cavities housing the springs 2232 and balls 2233, the balls 2233 are pushed into the recess 2234 by the springs 2232 and thus connects and fixates the medical device 2200 to the trocar 2292.

The connecting portion 2284b of the medical device 2200 comprises a self sealing membrane 2243b comprising a small self sealing hole in the center through which the endoscopic instrument 2289 can be inserted. The self sealing membrane 2243b is positioned at the connecting portion 2284a of the trocar, such that the self sealing membrane 2243b abuts the self sealing membrane 2243a of the trocar 2292 when the medical device 2200 and the trocar 2292 are connected. The two self sealing membranes 2243a; 2243b thus forms an integrated self sealing membrane enabling the insertion of the endoscopic instrument 2289 through the two self sealing membranes 2243a; 2243b acting as a single membrane, such that a fluid connection between the chamber of the medical device 2200 and the cavity in the patient can be formed while the chamber and cavity of the patient is kept completely sealed from the ambient environment.

The surgical instrument 2289 shown in fig. 40h comprises a handle portion 2289' for operating the surgical instrument. In the embodiment shown in fig. 40h for operating the small grasping portion in the end of the surgical instrument 2289. The handle portion is operably encapsulated by the wall 2201 such that the handle portion can be manipulated from outside the chamber.

Fig. 40h' shows an embodiment of the medical device similar to the embodiment shown in fig. 40h, the difference being that the wall 2201 of the medical device comprises a pleated 2203 portion such that it can be compressed when the handle portion 2289' is moved closer to the trocar 2292, such that the surgical instrument can perform manipulations within the cavity of the patient. The wall 2201 of the medical device is fixated to the surgical instrument just below the handle portion 2289' such that the handle portion is kept outside of the sealed environment in the chamber enclosed by the wall 2201, while the portion of the surgical instrument 2289 adapted to be inserted through the membranes 2243a; 2243b and into the cavity in the patient are encapsulated by the wall 2201 and thus kept in the sealed environment.

The embodiment of fig. 40h' further shows a first and second covering sheet 2277a; 2277b, the first covering sheet 2277a being adapted to cover the connecting portion 2284a of the trocar 2292 including the self sealing membrane 2243a. The second covering sheet is adapted to cover the connecting portion 2284b of the medical device 2200, such that the self sealing membrane 2243b is covered. The two covering sheets 2277a; 2277b are adapted to be removed before the medical device 2200 and the trocar 2292 are connected such that the self sealing membranes 2243a; 2243b are exposed. The covering sheets 2277a;b are preferably made from a thin polymer material covered with a non-stick surface, such as a PTFE surface.

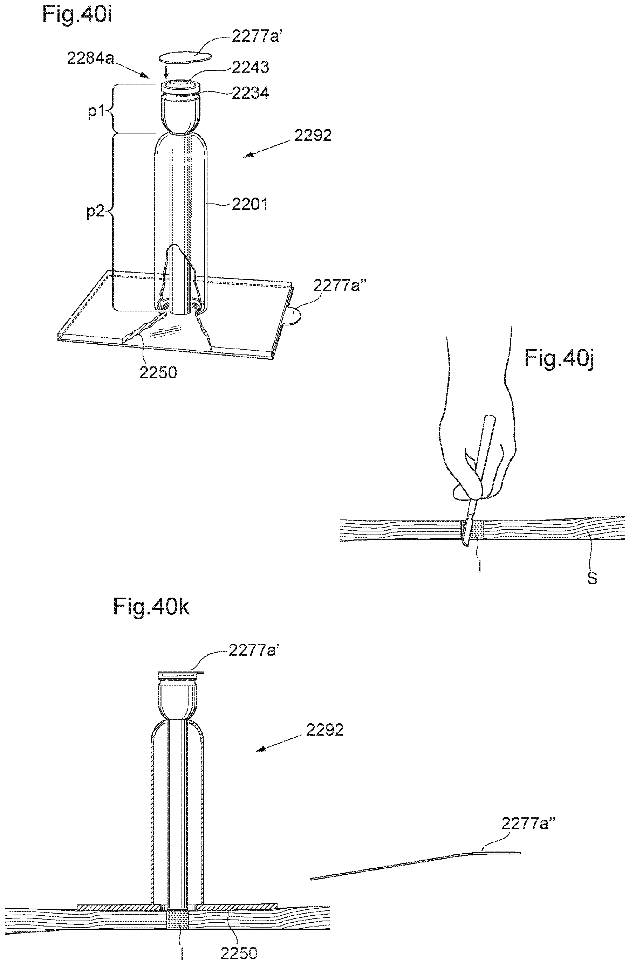

Fig. 40i shows an embodiment of the trocar 2292 similar to the embodiment shown in fig. 40e, the difference being that the embodiment of fig. 40i comprises a wall 2201 fixated to the trocar 2292 and being adapted to enclose the portion p2 of the trocar 2292 adapted to be inserted into the cavity of the patient, such that this portion p2 can be kept sterile. The embodiment shown in fig. 40i further comprises a covering sheet 2277a' covering the connecting portion 2284a of the trocar, including the membrane 2243a, and a covering portion 2277a" covering the adhesive surface 2250 of the trocar 2292 adapted to fixate the trocar 2292 to the skin of the patient. The adhesive portion 2250 being fixated to the wall 2201.

Fig. 40j shows the skin S of the patient when an incision I has been performed enabling the insertion of a trocar into a cavity in the patient.

Fig. 40k shows when the trocar 2292 according to the embodiment shown in fig. 40k when the covering sheet 2277a" covering the adhesive surface 2250 has been removed, thus exposing the adhesive surface 2250, and the adhesive surface 2250 has been fixated to the skin S of the patient by the adhesive surface 2250 contacting the skin S of the patient. The wall 2201 of the medical device 2200 could for example be made from a flexible polymer material, such as polyvinyl chloride (PVC) with a plasticizer additive, or an elastic polymer material, such as silicone.

Fig. 2201 shows the trocar 2292 when a portion p2 has been inserted through the skin S of the patient and into the cavity in the patient by the flexible wall 2201 of the trocar being compressed such that a pleated portion is formed. The protective cover 2277a' has been removed from the connecting portion 2284a of the trocar, exposing the self sealing membrane 2243a.

Fig. 40m shows the trocar 2292 shown in fig. 40l and a medical device 2200 comprising a connecting portion 2284b for connecting to the trocar 2292. The medical device being the medical device disclosed with reference to fig. 40h comprising a removable covering sheet 2277b for covering the connecting portion 2284b of the medical device 2200 and thus covering the self sealing membrane 2243b of the in the connecting portion 2284b of the medical device 2200.

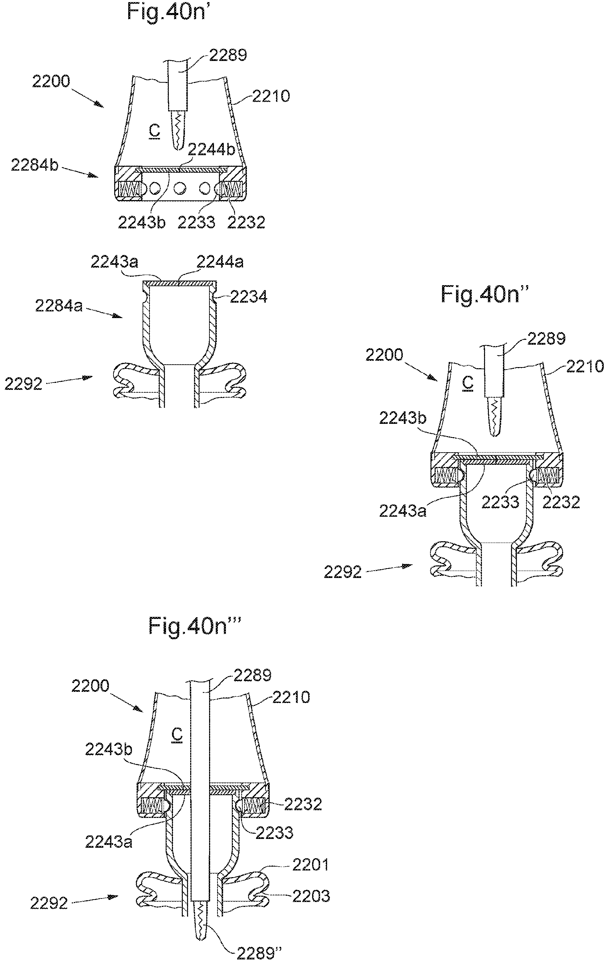

Fig. 40n' shows the connecting portions 2284a; 2284b of the trocar 2292 and the medical device 2200, respectively. The self sealing membrane 2243a of the trocar 2292 comprises a small self sealing hole 2244a placed centrally in the membrane 2243a and enabling the insertion of the instrument 2289 through the self sealing membrane 2243a by means of the elastic properties of the material of the self sealing membrane 2243a. The self sealing membrane 2243a could for example be the self sealing membrane of the gel port available from Applied medical inc.. Fig. 40n' further shows the connecting portion 2284b of the medical device 2200 in further detail. The medical device 2200 comprises a flexible wall 2201 enclosing a chamber C in which an endoscopic instrument 2289 is enclosed. The connecting portion 2284b positioned in the flexible wall 2201 and adapted to connect to the connecting portion 2284a of the trocar 2292, such that a fluid connection is created between the chamber C enclosed by the wall 2201, and a cavity within the patient. The connecting portion 2284b of the medical device 2200 comprises protruding members 2233 in the form of protruding balls pressed against the edge of an orifice by a spring 2232. The balls 2233 can thus be pushed into the cavity housing the spring 2232 by a force acting on the ball 2233 from the connecting portion 2284a of the trocar. When a recess 2234 of the connecting portion 2284a of the trocar 2292 is aligned with the cavities housing the springs 2232 and balls 2233, the balls 2233 are pushed into the recess 2234 by the springs 2232 and thus connects and fixates the medical device 2200 to the trocar 2292. The connecting portion 2284b of the medical device 2200 comprises a self sealing membrane 2243b comprising a small self sealing hole 2244b adapted to enable the insertion of an instrument 2289 through the self sealing membrane 2243b.

Fig. 40n" shows the medical device 2200 when the trocar 2292 and the medical device 2200 are connected. The balls 2233 have been pushed into the recess 2234 by the springs 2232 and thus connect and fixate the medical device 2200 to the trocar 2292. The self sealing membrane 2243a of the trocar abuts the self sealing membrane 2243b of the medical device 2200 such that an integrated self sealing membrane is formed. The small self sealing hole 2244a of the trocar 2292 is aligned with the small self sealing hole 2244b of the medical device 2200 such that the surgical instrument can be inserted through the integrated self sealing membrane by the elasticity of the material of the self sealing membranes 2243a; 2243b.

Fig. 40n‴ shows the trocar 2292 and medical device 2292 when connected and with the instrument 2289 inserter through the expanded orifices of the self sealing membranes 2243a; 2243b from the chamber C to the inside of the trocar 2292.

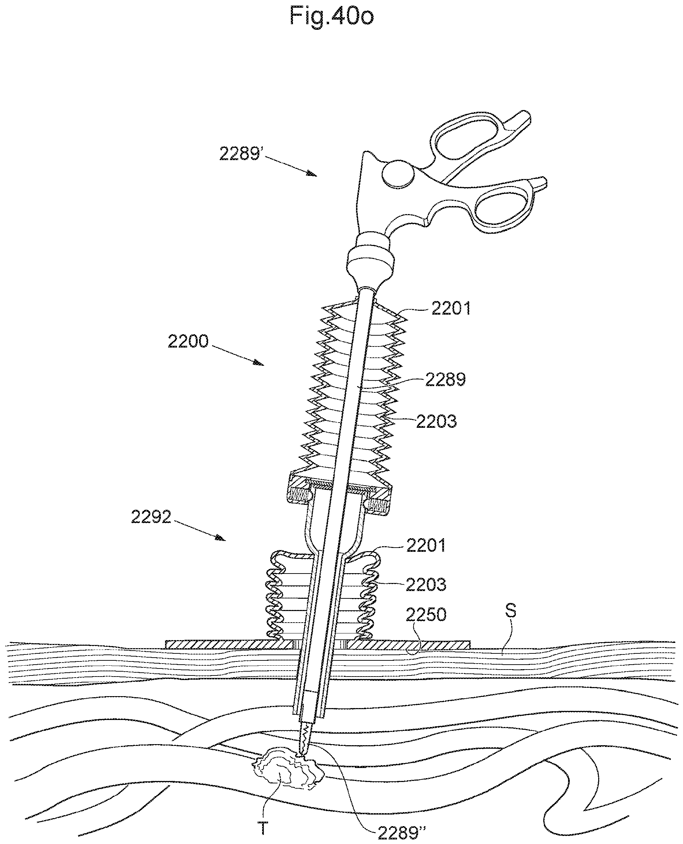

Fig. 40o shows the medical device system, comprising the connected trocar 2292 and medical device 2200, when the surgical instrument 2289 for manipulation in the cavity within the patient is inserted through the integrated self sealing membrane and through the trocar 2292 and into the cavity on the patient such that a grasping portion 2289" of the surgical instrument 2289 can perform a step of the surgical procedure within the cavity of the patient, such as assisting in the removal of a tumor T on the intestinal system of the patient. Both the wall 2201' of the medical device 2200 and the wall 2201" of the trocar have been compressed such that pleated portions 2203 have been formed. The walls 2201'; 2201" are additionally flexible such that the surgical instrument 2289 can be moved within the cavity of the patient such that different positions within the cavity can be reached.

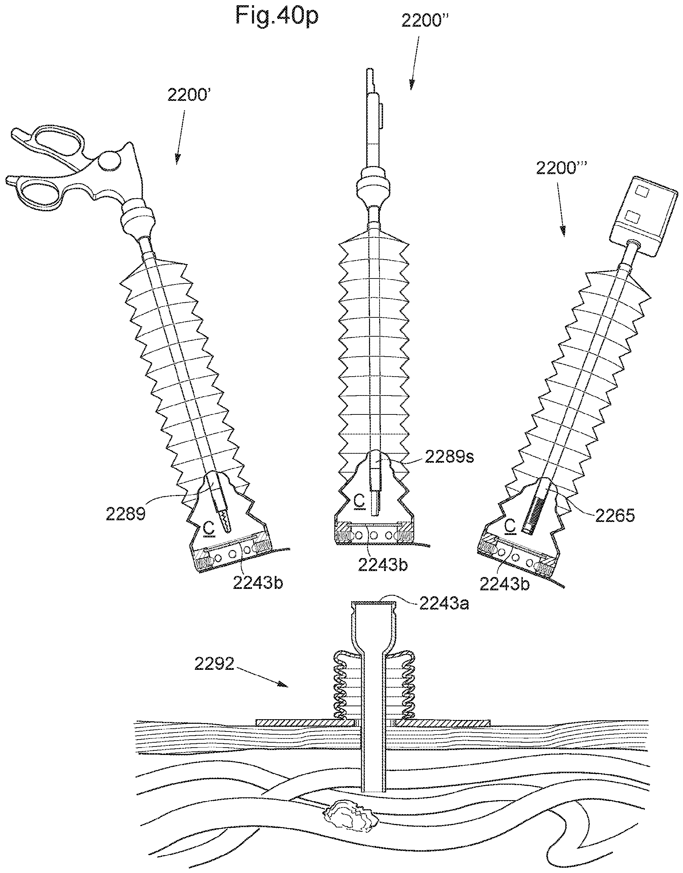

Fig. 40p shows the medical device system when a medical device 2200' is exchanged to a medical device 2200"; 2200" enclosing a different surgical instrument. The medical device 2200' enclosing a grasping surgical instrument 2289 could for example be exchanged to a medical device 2200‴ enclosing an endoscopic camera 2265 or a medical device enclosing a stapling instrument 2289s. As the surgical instrument/camera 2289; 2289s; 2265 is retracted from the cavity of the patient and the inside of the trocar 2292, through both self sealing membranes 2243a; 2243b before the medical device 2200 is disconnected from the trocar 2292, the chamber C of the surgical instrument 2200 and the inside of the trocar 2292 is sealed from the ambient environment A during all the steps of the procedure. Surgical instruments to be used in the trocar 2292 can thus be removed from the operation site when a different instrument is needed and brought back into use without the risk that the instrument will contaminate the operation site, making the system highly suitable for use when performing endoscopic procedures in less sterile environments.

Fig. 40q' shows the medical device system according to an embodiment where the trocar shown in fig. 40e is connected to a medical device 2200 comprising an evacuation valve 2238 for evacuating a fluid from the chamber C. The evacuated fluid could be a gas or liquid used to inflate the cavity in the patient for enabling visual inspection within the cavity. The gas or liquid could become contaminated by the steps of the surgical procedures. A laparoscopic gas, such as CO2 gas could become contaminated be fumes from diathermy instruments used inside the cavity during the surgical procedure, which could affect the possibility of obtaining good visibility within the chamber C and cavity. The evacuation valve 2238 is connected to a fluid conduit 2210 which in turn is connected to a filtering unit 2226b adapted to filter the evacuated fluid such that unpleasant fumes is not released into the environment of the operating theater during the surgical procedure, such as fumes from the use of a diathermy instrument. The filtering unit 2226b could furthermore be a control system for controlling the opening of the evacuation valve 2238. The control system could control the evacuation valve on the basis of for example the pressure in the chamber C, the visibility in the chamber C, detection of smoke in the chamber C, the temperature in the chamber C, and/or a time related parameter.

Fig. 40q" shows the medical device system according to an embodiment similar to the embodiment of fig. 40q', the difference being that the wall 2201 enclosing the chamber C is larger, such that the whole surgical instrument 2289, including the handle portion 2289', is enclosed in the chamber C. The medical device 2200 further comprises gloves 2202a; 2202b integrated in the wall 2201 of the medical device 2200 such that the handle portion 2289' of the surgical instrument 2289 can be manipulated within the chamber C by the a surgeon using the integrated gloves 2202a; 2202b. The wall 2201 of the medical device 2200 is adapted to hold a pressure exceeding atmospheric pressure which is inflated into the chamber C of the medical device 2200 by the fluid connection with an inflated cavity of the patient's body, when a surgical instrument is inserted through the seals.

Fig. 40q‴ shows an alternative embodiment in which the connecting portions 2284a;2284b comprises a plurality of self sealing membranes 2243';2243";2243‴ etc. which are removable such that a new sterile membrane can be exposed when the medical device 2200 is reconnected to the trocar 2292 after having been disconnected when the medical device is replaced for a different medical device for performing a specific step of the surgical procedure (the exchange of medical devices for example shown in fig. 40p). The stack of self sealing membranes are connected to each other by means of the first 2243' self sealing membrane adhering to the next 2243" and so forth. As the stack of self sealing membranes becomes thinner as self sealing membranes 2243';2243";2243‴ are removed the exposed self sealing membrane needs to be feed to the connecting position such that the exposed self sealing membrane is placed in connection with the exposed self sealing membrane of the trocar 2292 such that an interconnected self sealing membrane can be formed. The stack of self sealing membranes 2243';2243";2243‴ etc. are feed by the action of springs positioned in bores in the connecting portions 2284a;2284b perpendicular to the feeding direction of the self sealing membranes 2243';2243";2243‴ etc..

Fig. 40q"" shows an alternative embodiment of the medical device system where the connecting portion 2284b of the medical device 2200 comprises a sleeve 2259 adapted to connect to the connecting portion 2284a of the trocar 2292 by engaging the trocar 2292 on the outside thereof. The sleeve 2259 comprises sealing members in the form of O-rings 2254 for sealing between the sleeve 2259 and the trocar 2292. Fig. 40q‴ further shows a fluid system for inflating the cavity of the patient with a fluid. The fluid system comprises a fluid inlet 2230' placed through the skin S of the patient and connected to a fluid conduit 2210, which in turn is connected to a tank 2212 comprising pressurized fluid. The fluid flow into the cavity of the patient is controlled/monitored by means of a control/monitoring system 2215 placed on a portion of the fluid conduit 2210 and for example comprising a manometer and a restriction valve for adapting the flow and/or pressure of the fluid entering the cavity of the patient.

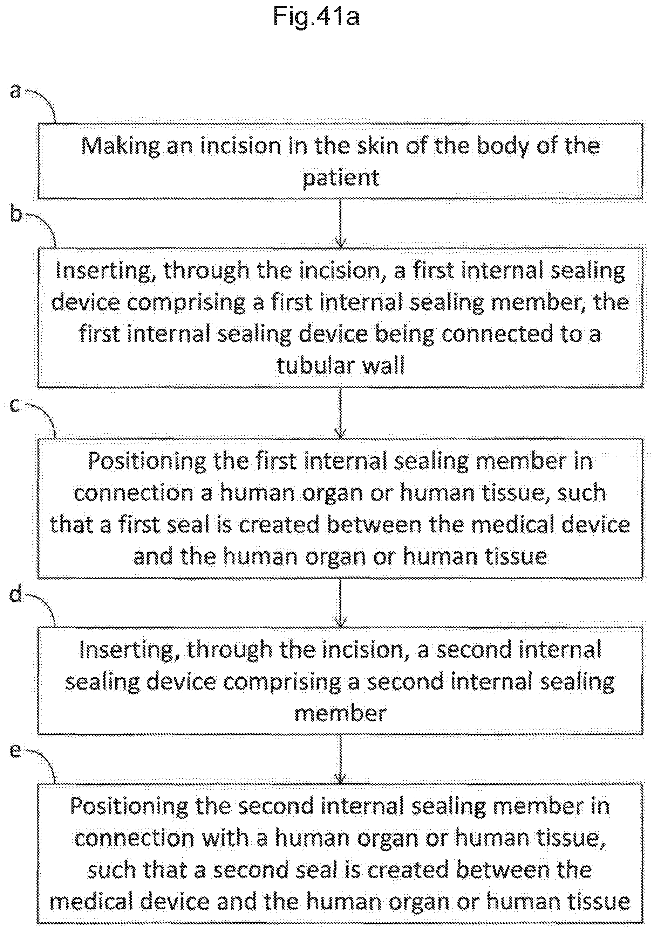

Fig. 41a is a flow chart describing a method of forming a sterile environment inside of the body of a patient using a medical device. The method comprises a making an incision in the skin of the body of the patient, which may be larger incision for more of an open type surgery, or a smaller incision more suitable for key-hole type surgery. The method then comprises the step of b inserting, through the incision, a first internal sealing device comprising a first internal sealing member, the first internal sealing device is connected to a tubular wall and c positioning the first internal sealing member in connection with at least a part of at least one human organ or human tissue, such that a first seal is created between the medical device and at least the part of the at least one human organ or human tissue, d inserting, through the incision, a second internal sealing device comprising a second internal sealing member, the second internal sealing device being connected to the tubular wall, and e positioning the second internal sealing member in connection with at least a part of at least one human organ or human tissue, such that a second seal is created between the medical device and at least the part of the at least one human organ or human tissue, such that a medical device chamber is created sealingly between the first and second internal sealing devices and the tubular wall.

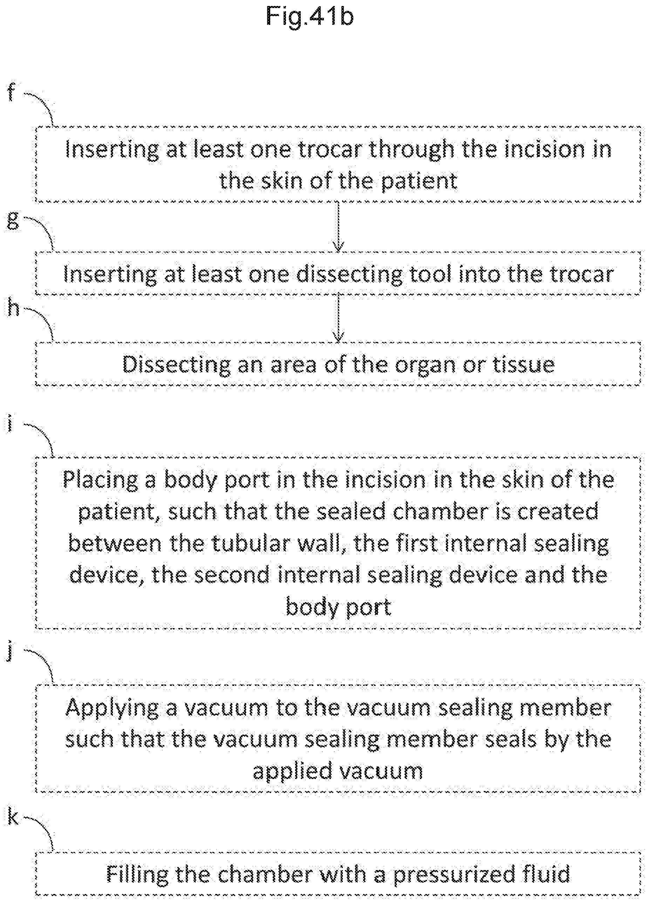

Fig. 41b is a flowchart showing the method of fig. 41a additionally, optionally may comprises at least one of the steps of **f** inserting at least one trocar through the incision in the skin of the patient, **g** inserting at least one dissecting tool into the trocar, and **h** dissecting an area of the organ or tissue. The method may further additionally optionally comprise the step of i placing a body port in the incision in the skin of the patient, wherein the tubular wall is connected to the body port, such that the sealed chamber is created between the tubular wall, the first internal sealing device, the second internal sealing device and the body port.

The step of positioning the first and second internal sealing devices may comprises positioning the first and second sealing device such that they each encircle a tubular organ, and the step of positioning the first and second internal sealing device may comprises the steps of positioning the first sealing device encircling an intestine of the patient and positioning the second internal sealing device encircling the esophagus of the patient, such that the wall encapsulates the stomach of the patient, such that the stomach of the patient is placed in a sealed chamber between the first sealing member, the second sealing member, and the wall.

The flowchart of fig. 41b further shows that the method additionally, optionally may comprise the step of **j** applying a vacuum to the vacuum sealing member such that the vacuum sealing member seals by the applied vacuum, in which case the internal sealing member is an internal vacuum sealing member.

The method may further comprise the step of **k** filling the chamber with a pressurized fluid, such as disclosed in relation to other embodiments herein.

In addition, the method may further comprise at least one of the following surgical procedures performed within the sealed chamber; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hrnia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open j oint surgery, combined open j oint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

The method may further comprise placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,

The method may further comprise placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

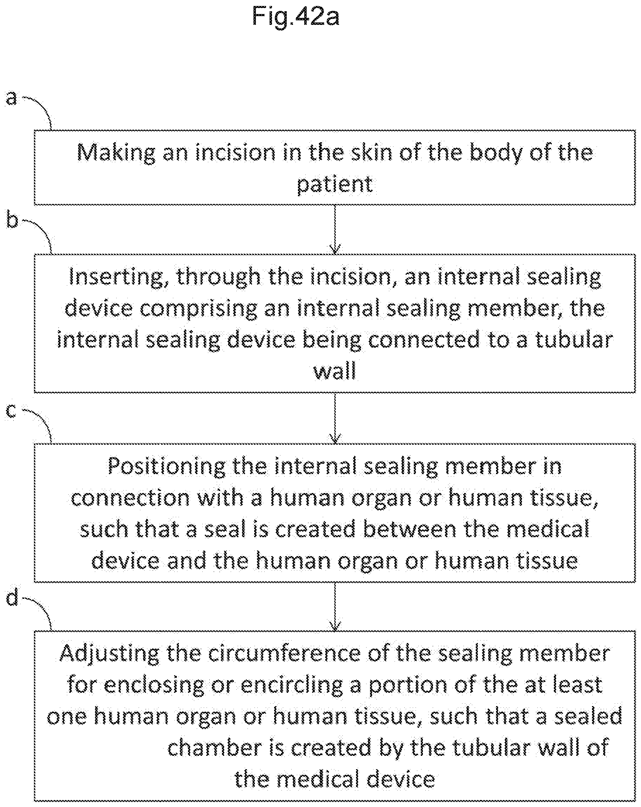

Fig. 42a is a flow chart of a method of creating a sealed chamber at least partially inside of the body of a patient. The method comprises the steps of a making an incision in the skin of the body of the patient, **b** inserting, through the incision, an internal sealing device comprising an internal sealing member, the internal sealing device being connected to a tubular wall, **c** positioning the internal sealing member in connection with at least a part of at least one human organ or human tissue, such that a seal is created between the medical device and at least the part of the at least one human organ or human tissue, and **d** adjusting the circumference of the sealing member for enclosing or encircling a portion of the at least one human organ or human tissue, such that a sealed chamber is created by the tubular wall of the medical device, the internal sealing device, and the organ or tissue.

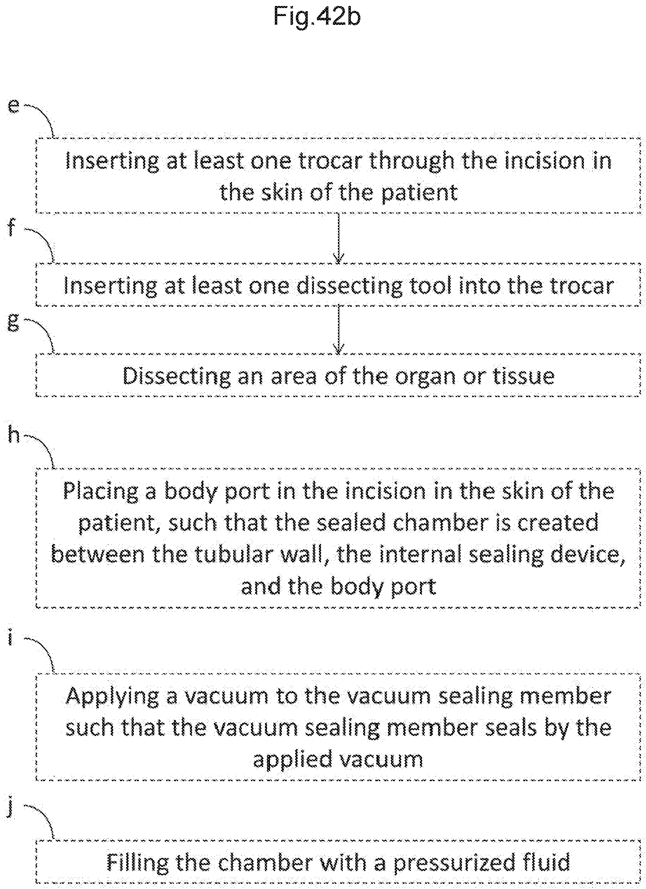

Fig. 42b is a flowchart showing the method of fig. 42a additionally, optionally may comprises at least one of the steps of **e** inserting at least one trocar through the incision in the skin of the patient, **f** inserting at least one dissecting tool into the trocar, and **g** dissecting an area of the organ or tissue.

The method may further comprise the step of **h** placing a body port in the incision in the skin of the patient, and wherein the tubular wall is connected to the body port, such that the sealed chamber is created between the tubular wall, the internal sealing device and the body port.

The flowchart of fig. 42b further shows that the method additionally, optionally may comprise the step of **i** applying a vacuum to the vacuum sealing member such that the vacuum sealing member seals by the applied vacuum, in which case the internal sealing member is an internal vacuum sealing member.

The method may further comprise the step of **j** filling the chamber with a pressurized fluid, such as disclosed in relation to other embodiments herein.

In one embodiment, the method may further comprise the steps of: inserting, through the incision, a second internal sealing device comprising a second internal sealing member, the second internal sealing device also being connected to the tubular wall, and positioning the second internal sealing member in connection with at least a second part of the at least one human organ or human tissue, such that a seal is created between the second medical device and at least the part of the at least one human organ or human tissue, such that a sealed chamber is created by the tubular wall of the medical device, the first and second internal sealing devices, and the organ or tissue. The method may comprise the step of adjusting the circumference of the second sealing member for enclosing or encircling a portion of the at least one human organ.

The internal sealing member may comprise at least one sealing surface adapted to connect to and seal against a tissue portion of the patient, which may be placed in the front of the internal sealing device or on the inner circumference of the sealing member. The step of positioning the internal sealing member in connection with at least a part of at least one human organ or human tissue may further comprise applying the internal sealing member axially against a surface of an organ or tissue, or radially against the outer circumference of a tubular organ or tissue.

In addition, the method may further comprise at least one of the following surgical procedures performed within the sealed chamber; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hrnia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open joint surgery, combined open joint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

The method may further comprise placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,

The method may further comprise placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

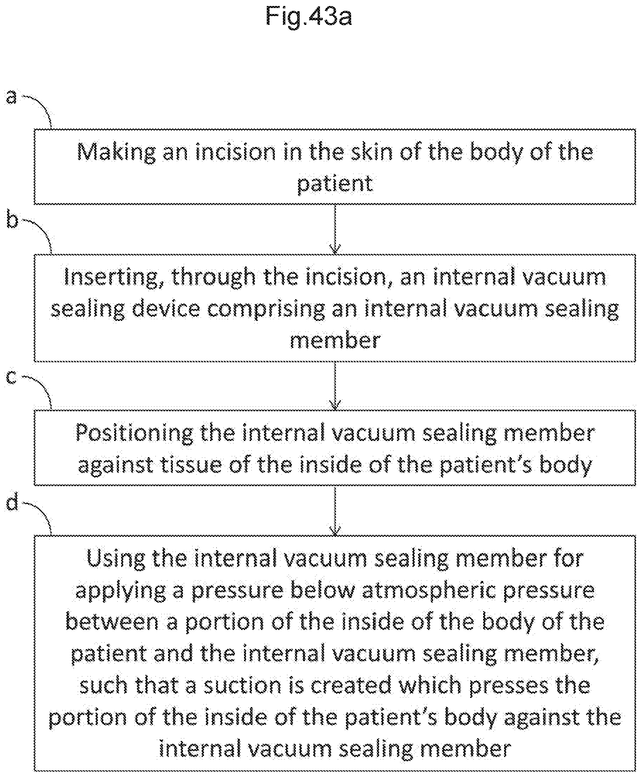

Fig. 43a is a flow chart of a method of creating a sealed chamber at least partially inside of the body of a patient. The method comprises the steps of a making an incision in the skin of the body of the patient, b inserting, through the incision, an internal vacuum sealing device comprising an internal vacuum sealing member, c positioning the internal vacuum sealing member against tissue of the inside of the patients body and d using the internal vacuum sealing member for applying a pressure below atmospheric pressure between a portion of the inside of the body of the patient and the internal vacuum sealing member, such that a suction is created which presses the portion of the inside of the patient's body against the internal vacuum sealing member, thereby creating a vacuum seal between the medical device and human tissue inside of the skin of the patient.

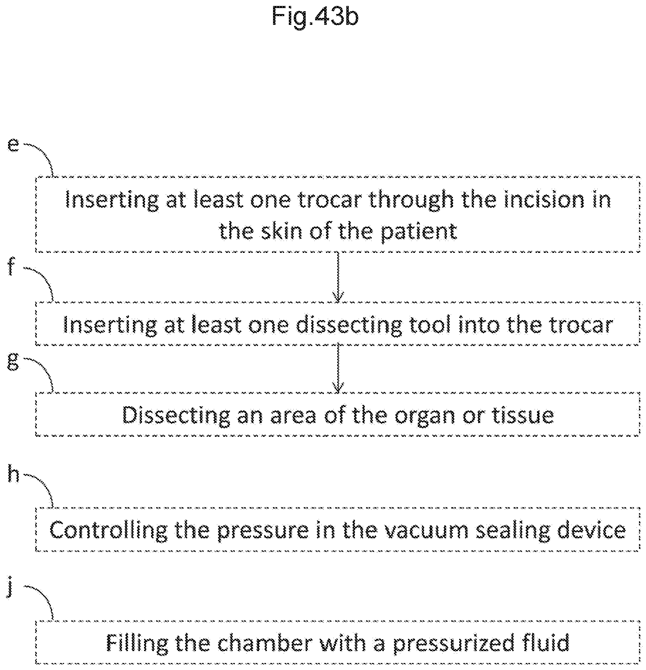

Fig. 43b is a flowchart showing that the method of fig. 42a additionally, optionally may comprises at least one of the steps of e inserting at least one trocar through the incision in the skin of the patient, f inserting at least one dissecting tool into the trocar, and g dissecting an area of the organ or tissue.

The method may further, optionally, comprise the step of h controlling the pressure of the vacuum sealing device such that the pressure of the vacuum sealing device does not substantially affect the blood flow of the patient.

The method may further, optionally, comprise the step of filling the sealed chamber with a pressurized fluid.

The step of positioning the internal vacuum sealing member against tissue of the inside of the patients body may comprise positioning the internal vacuum sealing member against at least one of; muscle fascia, muscle fascia of the rectus abdominis in the abdominal wall, the peritoneum, fat tissue, fibrotic tissue, muscle tissue, and a bodily organ.

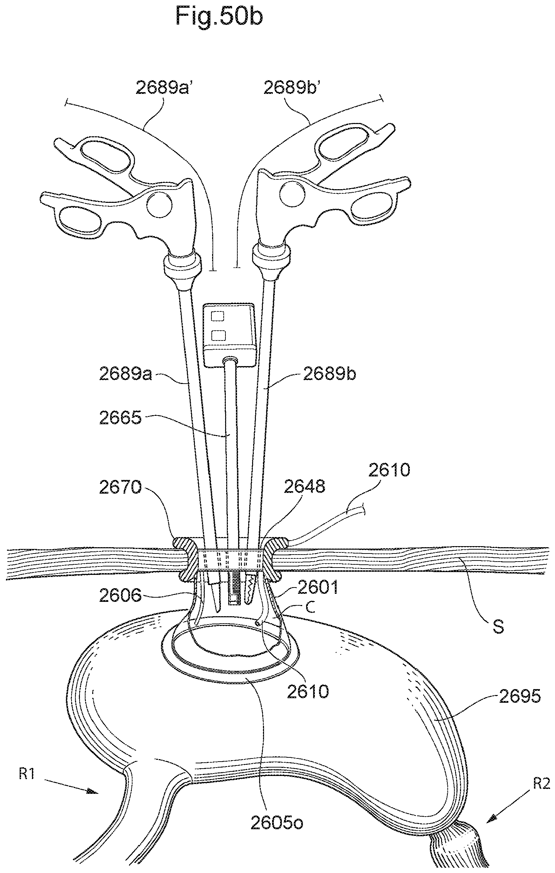

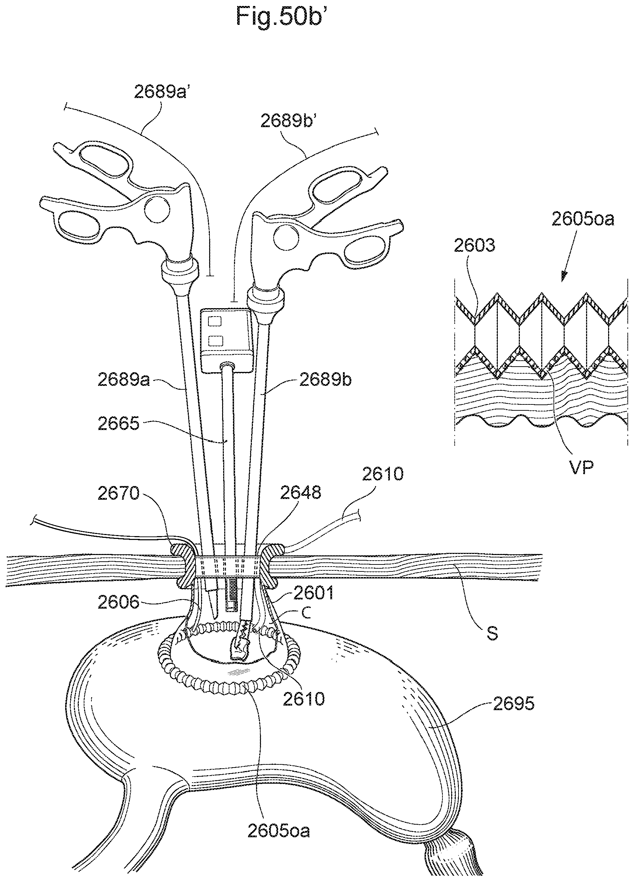

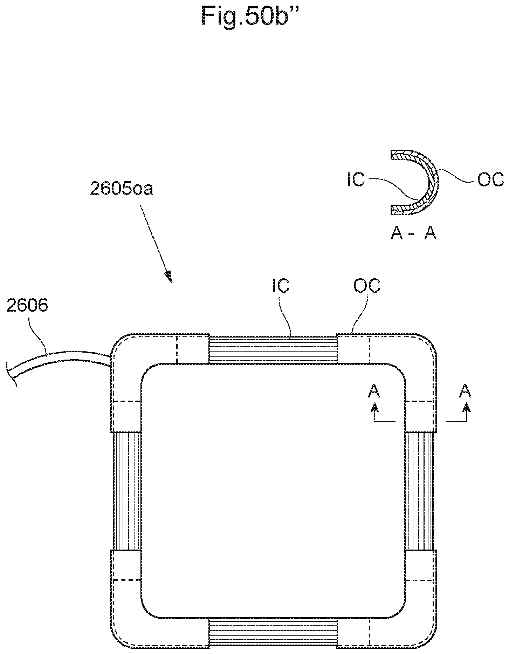

The internal vacuum sealing member may have an adjustable circumference, such that the size of the area enclosed or encircled can be adjusted. Examples of embodiments of vacuum sealing members with adjustable circumference are for example described with reference to figs. 50b', 50b", 51b' and 52b'.

In addition, the method may further comprise at least one of the following surgical procedures performed within the sealed chamber; the opening of an organ with bodily matter containing bacteria, the extirpation of at least a part of the at least one organ or human tissue related to and contact with said organ, extirpation of any bodily matter from inside or outside the at least one organ, extirpation of any bodily tissue from inside or outside the at least one organ or relating to the at least one organ, connecting different parts of the at least one organ, connecting different parts of different organs, placing at least one medical device in relation to the at least one organ, replacing at least one bodily function, placing at least one medical device in relation to the at least one organ and replacing at least one bodily function by placing at least one medical device in relation to the at least one organ, appendectomy, cholecystectomy, nephrectomy, hysterectomy, oophorectomy, adrenalectomy, gastric bypass, Nissen fundoplication, intestinal shunts, hrnia repair, splenectomy, colon or small intestine resection, liver resection, cecostomy, colostomy, duodenostomy, ileostomy, appendicostomy, esophagostomy, gastrostomy, urostomy, nephrostomy, ureterostomy, vesicostomy, endoscopic surgery, open surgery, combined open and endoscopic surgery, arthroscopic surgery, open j oint surgery, combined open j oint and arthroscopic surgery, cryoablation, ultrasound knife surgery, removal of separated bone or cartilage tissue, the insertion of ligaments, the insertion of fixation elements such as screws, nails, and plates, prosthetic joint replacement, lubrication procedures, cancer surgery, insertion of another medical device, insertion of foreign parts, and insertion of a medical device and other foreign parts using combined open and laparoscopic or arthroscopic surgery.

The method may further comprise placing at least one of the following other implants into the medical device chamber; an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,

The method may further comprise placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

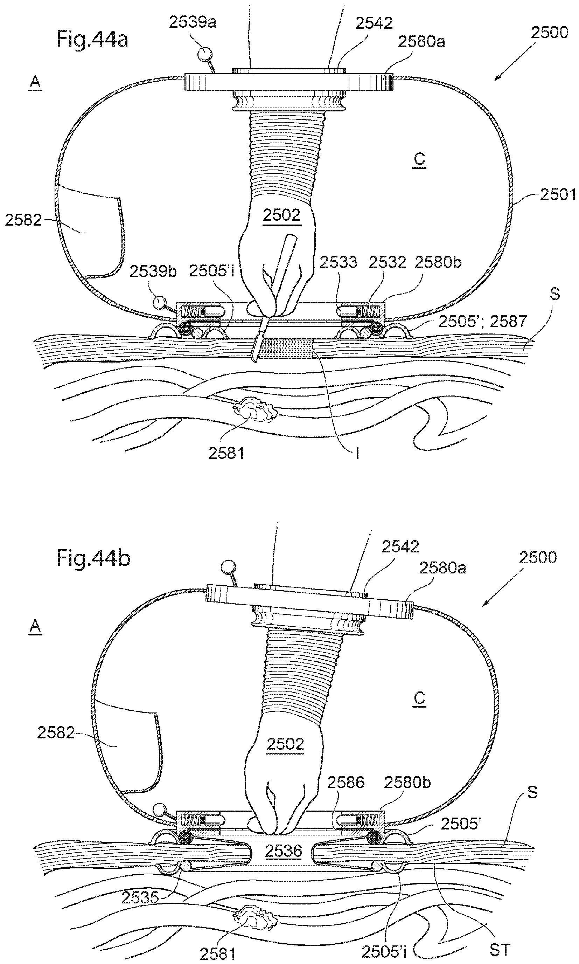

Figs. 44a - 49c shows embodiments of a medical device in which the medical device comprises an internal sealing device enabling the sealing against a tissue on the inside of the patient's, such that the surface of the skin remains unaffected by the sealing device, such that no hematomas or marks appear on the patient's skin S. The internal sealing device could for example seal against muscle fascia, the peritoneum, fat tissue, fibrotic tissue, muscle tissue or against the inside of the patient's skin. Further advantages of applying the internal sealing device inside the body of the patient is that further areas of the body can be isolated or enclosed by the internal sealing device or a connecting member, connecting the internal sealing device to the medical device external to the body of the patient. In the embodiments shown in figs. 44a - 49c the internal sealing device is shown comprising an internal vacuum sealing member, however, in all embodiments it is equally conceivable that the internal vacuum sealing member is exchanged to an internal sealing member adapted to seal by means of pressure or an adhesive, such as described in relation to sealing device in other embodiments herein. The method and structures for sealing against the tissue on the inside of the patient's body could be used in combination with any of the embodiments of the medical devices herein and is to be seen as a principle for supplying sealing between the body of the patient and a medical device. In all of the embodiments, the chamber of the medical device could be adapted to hold a fluid having a pressure exceeding the atmospheric pressure, such that a laparoscopic surgical procedure with an inflated abdominal cavity can be performed. The fluid filling the chamber having a pressure exceeding atmospheric pressure can be supplied by a fluid pump through a fluid inlet positioned in the wall of the medical device (not shown in all embodiments).

Fig. 44a shows an embodiment of the medical device 2500 adapted to be positioned in relation to an incision I made in the skin S of the patient. The medical device 2500 comprises a vacuum sealing device comprising an internal vacuum sealing member 2505'i adapted to be positioned inside the body of the patient, for creating a vacuum seal between the medical device 2500 and human tissue inside of the skin S of the patient. In the state shown in fig. 44a, the incision I in the patient's skin S is being created by the surgeon by means of an inset 2542 latched to a first coupling 2580a comprising a glove 2502 enabling manual manipulation within the chamber C. In the embodiment shown in fig. 44a, the medical device is fixated to the skin S of the patient, during the process of creating the incision I, by means of a vacuum sealing member 2505' adapted to be connected to the surface of the skin S of the patient, the vacuum sealing member 2505' thus functions as a holding member 2587 for holding the medical device 2500 in place after the internal vacuum sealing member 2505'i has been positioned. As the internal vacuum sealing member 2505'i is to be connected to a portion of the patient's body on the inside of the patient's body, the internal vacuum sealing member cannot be applied until the incision I has been created in the skin S.

In the embodiment shown in fig. 44a, the medical device 2500 is a medical device comprising a wall 2501 enclosing a chamber C in which a sterile environment can be maintained. When the incision I in the skin S of the patient is created, a fluid connection between the chamber C and a cavity in the patient's body is created, which may be used for transferring objects or medical devices from the chamber C to a cavity in the patient's body, or the other way around. The wall 2501 is connected to an upper and lower coupling 2580a, 2580b and made from a transparent collapsible polymer material, such as polyvinyl chloride (PVC) with a plasticizer additive.

Fig. 44b shows the medical device 2500 when the incision I has been concluded and the internal vacuum sealing member 2505'i has been inserted through the skin S of the patient and placed in connection with the subcutaneous tissue ST on the inside of the skin of the patient. The internal vacuum sealing member 2505'i is connected to an intra body ring, which together with the internal vacuum sealing member 2505'i forms the internal sealing device. The intra-body ring is in turn connected to a connecting member 2536 connecting the external portion of the medical device to the internal sealing device and retracting the incision such that the fluid connection between the cavity in the body of the patient and the chamber C of the medical device is kept open. The wall 2501 enclosing the chamber C is elastic or flexible which enables the surgeon move the upper coupling 2580a in relation to the lower coupling 2580b for getting better access to different angles within the patient's body, for example for removing a specimen 2581 such as a tumor. The embodiment of fig. 44b further comprises an iris valve 2586 placed in the second coupling 2580b for closing the hole in the second lower coupling 2580b such that the chamber C is sealed from at least one of the ambient environment A and a cavity in the patient. The closing of the iris valve 2586 enables the maintaining of a cavity within the chamber C without maintaining a pressure in the chamber C, at the same time as a pressure and/or sealed environment can be maintained within the body of the patient.

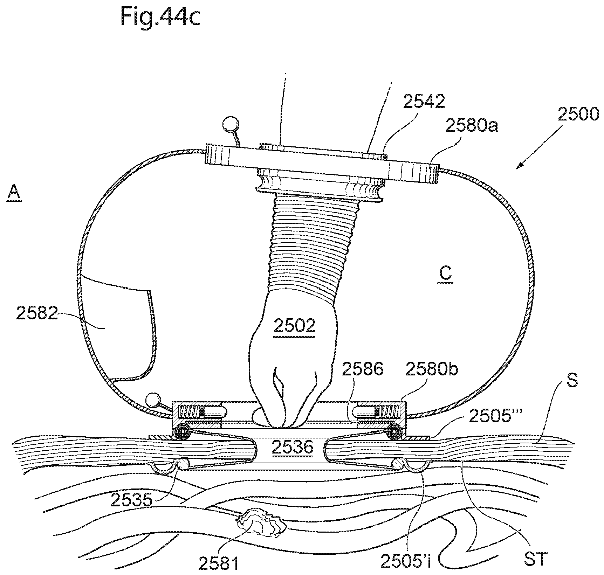

Fig. 44c shows an embodiment of the medical device identical to the embodiment shown with reference to fig. 44b, the only difference being that the vacuum sealing member 2505' is replaced by adhesive sealing members 2505‴ sealing and are holding the medical device by means of adhesive force.

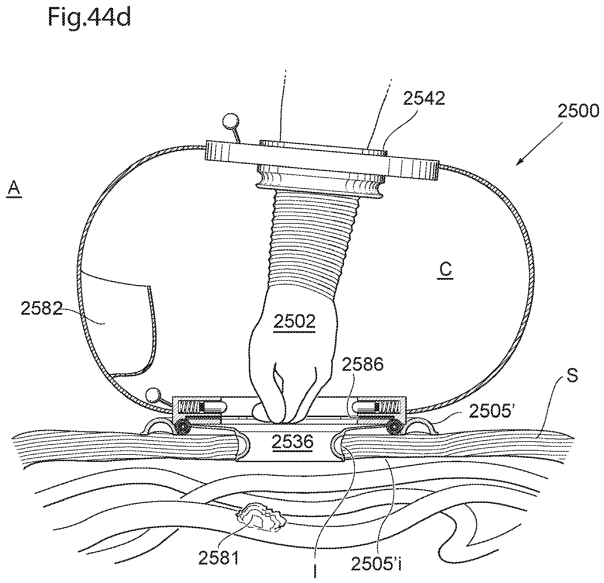

Fig. 44d shows an embodiment of the medical device 2500 very similar to the embodiment shown with reference to figs. 44a, 44b, the difference being that the internal vacuum sealing member 2505'i is adapted to engage to surface of the incision I made in the skin S. The internal vacuum sealing member 2505'i thus engages the cut surface which is advantageous as it is easily accessible from outside the body of the patient and no hematomas or marks are formed on the surface of the skin S. The engaged cut surface could include only the skin of the patient and/or the subcutaneous fat tissue, and/or the muscle layer and or fascia layer and/or a cut surface of the peritoneum.

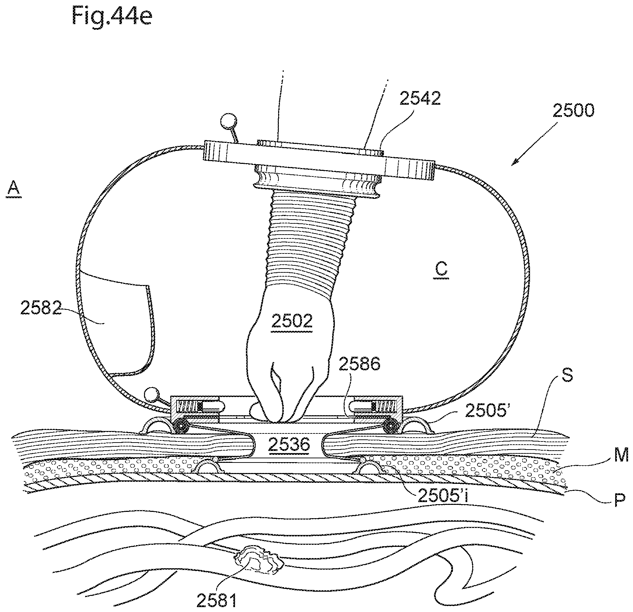

Fig. 44e shows an embodiment of the medical device 2500 in which the internal vacuum sealing member 2505'i is adapted to engage the peritoneum P of the patient, on the outside thereof. The peritoneum P is a serous membrane that forms the lining of the abdominal cavity, and the vacuum sealing member 2505'i is positioned on the outside thereof and thus placed in the fat tissue between the abdominal muscles M and the peritoneum P. By engaging the peritoneum P the connecting member 2536 (or retractor) covers the cut surface of the skin S and muscle layer M and thus encloses bodily fluids from the cut surfaces, such as blood from small bleedings that may occur when creating the incision.

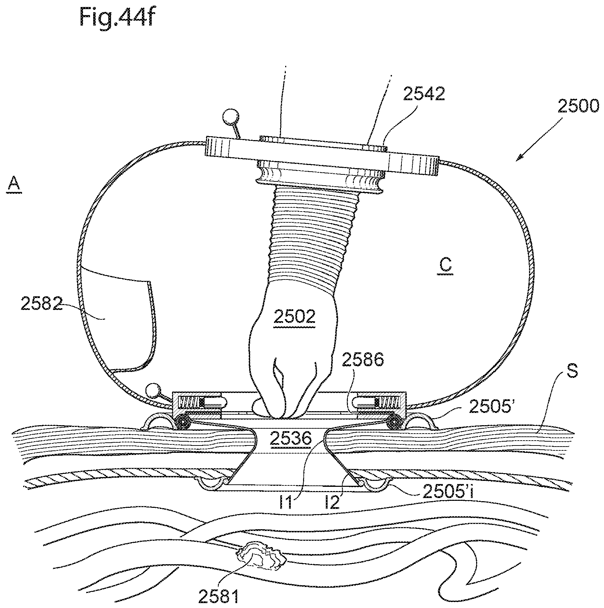

Fig. 44f shows an embodiment of the medical device 2500 in which the internal vacuum sealing member 2505'i is adapted to engage the peritoneum P of the patient, on the inside thereof. The vacuum sealing member 2505'i is positioned on the inside thereof and thus placed between the abdominal cavity and the peritoneum P. By engaging the peritoneum P on the inside, the connecting member 2536 (or retractor) covers the cut surface of the skin S, the muscle layer M, and the peritoneum P and thus encloses bodily fluids from the cut surfaces, such as blood from small bleedings that may occur when creating the incision. The internal vacuum sealing member 2505'i engaging the inside of the peritoneum P also enables the connecting member 2536 to retract the peritoneum P such that a tunnel through which the surgical procedure can be performed can be maintained from outside of the body of the patient to the abdominal cavity.

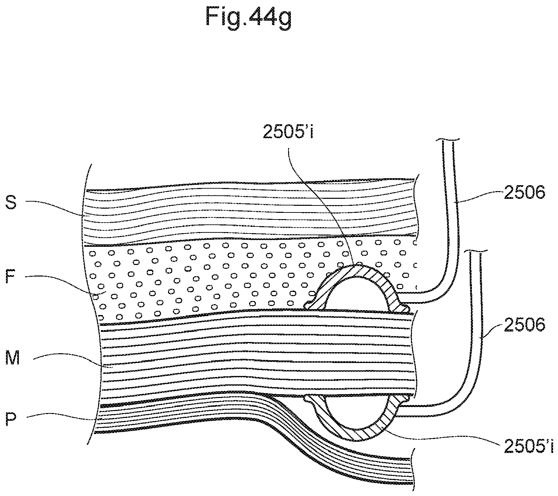

Fig 44g shows the skin S, the fat tissue F, the muscle layer M and the peritoneum P of a patient detailed sectional view. In the embodiment shown in fig. 44g, the internal sealing member 2505'i is positioned on two alternative positions, either on the outside of the muscular layer M in a position where some of the fat has been dissected or on the inside of the muscular layer M in a position where the peritoneum P has been separated from the muscular layer M.

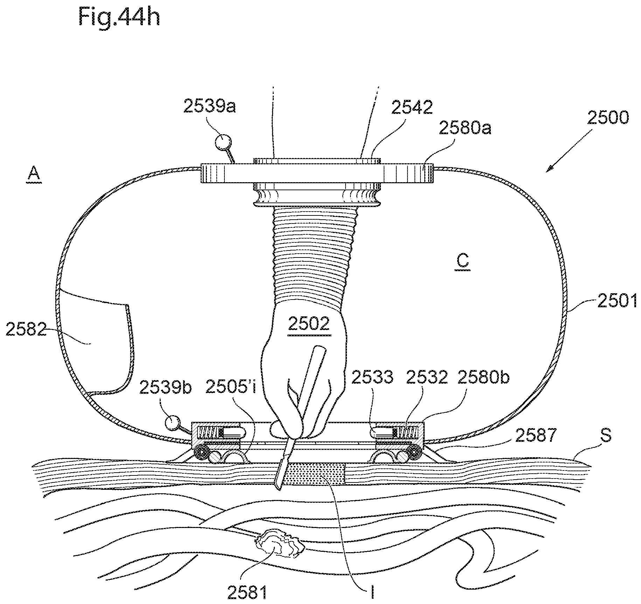

Fig. 44h shows the medical device according to an embodiment in which the external vacuum sealing members shown as 2505' in figs 44a - 44f has been replaced by mechanical holding members 2587 not providing a vacuum against the skin S of the patient. When the medical device has been positioned and the vacuum sealing members 2505'i positioned inside the patient's body, the connecting member or retractor is tightened, which presses the holding members 2587 on the surface of the skin S against the skin S and the internal vacuum sealing member 2505'i placed inside the body of the patient, thereby the medical device 2500 is fixated to the body of the patient by the vacuum sealing member 2505'i and the mechanical holding member 2587 squeezes the skin S of the patient.

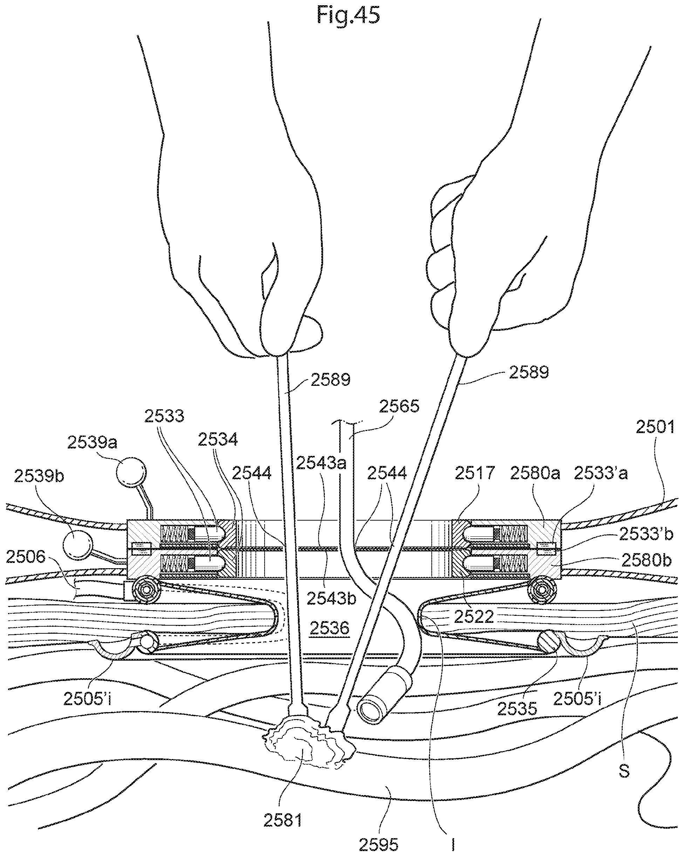

Fig. 45 shows a portion of the medical device of the embodiments shown in figs. 44a - 44f and 46a - 49c, when a specimen in form or a tumor 2581 on the small intestine 2595 of the patient is being removed. The medical device comprises an upper coupling 2580a to which an inset 2542 in form of a wall port 2517 is latched by means of the protruding members 2533 engaging a recess of the inset 2542. The medical device further comprises a lower coupling 2380b to which an inset 2542 comprising a body port 2522 is latched. Both the wall port 2517 and the body port 2522 comprise disc-shaped self sealing membranes 2543a, 2543b. In the body port 2522, the self sealing membrane 2543b is positioned in the top portion of the body port 2522, whereas in the wall port 2517, the membrane 2543a is positioned in the bottom part of the wall port 2517. The self sealing membranes 2543a, 2543b have small self sealing holes 2544 such that instruments 2589 or a camera 2565 can be inserted through the self sealing membranes 2543a, 2543b. In alternative embodiments, the self sealing membranes 2543a, 2543b are made from a gel-type material which enables the insertion of the instruments anywhere in the membranes 2543a, 2543b.

Fig. 45 shows a portion of the medical device of the embodiments shown in figs. 44a - 44g and 46a - 49c, when a specimen in form or a tumor 2581 on the small intestine 2595 of the patient is being removed. This embodiment continue to build on Fig. 44h, however, in this case two ports for laparoscopic surgery is connected. When connected, the wall port 2517 and body port 2522 forms an interconnected port. In the interconnected port, the membranes 2543a, 2543b are contacting each other such that the instruments 2589 engage an interconnected sealing membrane (made up of the first and second membrane 2543a, 2543b) in the interconnected port. If the ports 2517;2522 were to comprise two spaced-apart membranes, the two membranes would reduce the ability to move the instruments far more than the two integrated membranes acting as a single membrane. The sealing membranes 2543a, 2543b are adapted to, in a non-expanded state, provide a seal, and in an expanded state, enable an instrument 2589 to be inserted through the membranes 2543a, 2543b.

A roll-up system for rolling the connecting member 2536 is connected to the seat of the lower coupling 2580b is, such that the connecting member 2536 can be tighten for functioning as a retractor keeping the incision I formed in the skin S of the patient open. The connecting member 2536 connects the roll-up system to an intra-body ring 2535adapted to be placed on the inside of the skin S of the patient. The intra-body ring 2535 is in turn connected to the internal vacuum sealing member 2505'i, and the intra-body ring and the vacuum sealing member 2505'i together forms the sealing device. The internal vacuum sealing member 2505'i is connected to a vacuum creating member (not shown), such as a vacuum pump, via a vacuum conduit 2506. A monitoring unit adapted to sense the pressure in the vacuum sealing member 2505'i and/or the blood flow, and/or the blood pressure of the patient may further be incorporated in the medical device. The vacuum creating member may be connected to the monitoring unit for adjusting the pressure in the vacuum sealing member 2505'i in response to the input from the monitoring unit, for mediating between sealing and keeping the blood flow of the patient unaffected. The vacuum creating member may be adapted to adjust the pressure in the vacuum sealing member 2505'i for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.

The upper 2580a and lower 2580b couplings are connected by means of magnets 2533'a, 2533'b provided in the seat of the upper and lower coupling 2580a, 2580b, and the insets 2542 in the upper and lower couplings 2580a, 2580b, respectively, can be released and exchanged by means of the releasing levers 2539a, 2539b retracting the protruding members 2533 against the action of the springs 2532.

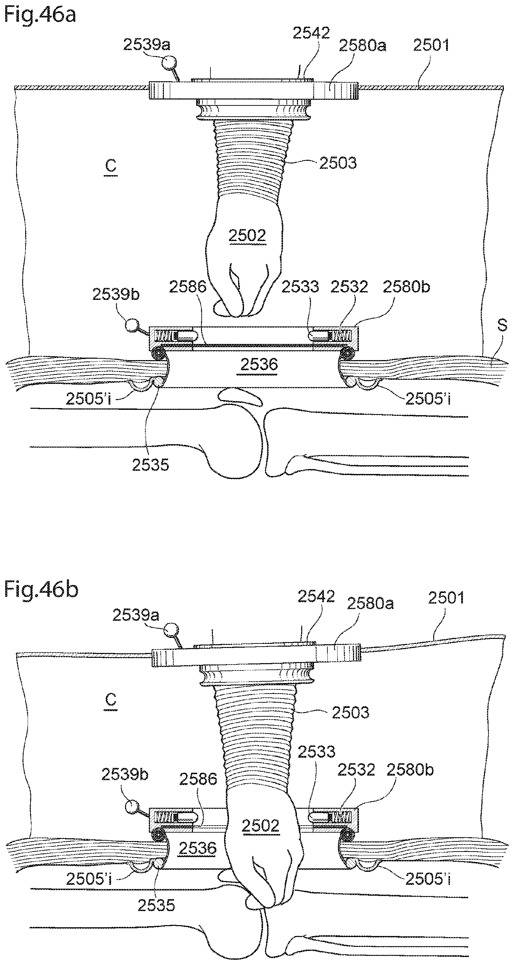

Fig. 46a shows an alternative embodiment of the medical device including the features as for example described with reference to fig. 45, with the difference that the embodiment of fig. 46a is adapted to be used in knee joint surgery. The medical device is provided with an internal vacuum sealing member 2505'i adapted to engage the inside of the skin S of the patient and seal against the inside of the skin S of the patient. An inset 2542 comprising a glove member 2502 is latched in the upper coupling 2580a, for enabling manual manipulation within the knee joint of the patient or the chamber C of the medical device. Please note that the relative size of the incision is way too large compared to the bone parts forming the knee and also the device is in relation to the hand too large, however, describing the principle.

Fig. 46b shows the embodiment of the medical device shown with reference to fig. 46a, when the surgeon is performing manual manipulation within the knee joint of the patient. The wall 2501 of the medical device is flexible or elastic such that the upper coupling 2580a (to which the glove member 2502 is latched) can move in relation to the lower coupling 2580b, and thus in relation to the body of the patient.

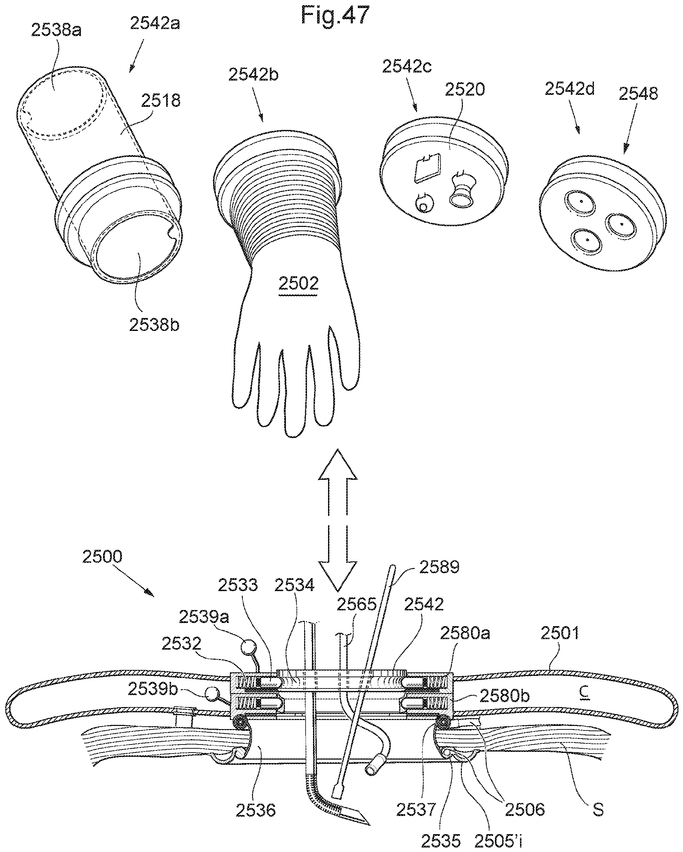

Fig. 47 shows the medical device according to an embodiment similar to the embodiment described with reference to figs. 44a - 46b, when multiport insets 2542 are locked in the upper and lower couplings 2580a, 2580b such that a procedure using laparoscopic instruments 2589 can be performed inside of the body of the patient. The multiport insets 2542 of the upper and lower coupling 2580a, 2580b can however be exchanged for another inset as shown in fig. 47. The insets for which the multiport inset can be exchanged could for example an inset 2542a comprising an airlock sluice 2518 enabling the transfer of objects through the couplings 2580a, 2580b while maintaining the sealed environment of the chamber C. The airlock sluice 2518 comprises a first valve member 2538a separating the area of the airlock 2518 from the ambient environment, and a second valve member 2538b separating the area of the airlock 2518 from the sealed environment of the chamber C. Alternatively, the multiport inset may be exchanged to an inset 2542b comprising a glove 2502 which can be placed in the upper coupling 2580a and enable the manipulation within the chamber C, and/or within the cavity in the body of the patient. An additional alternative is an inset 2542c comprising a device or instrument mount 2520 which could comprise holding portions for holding different instruments of objects which may be used within the chamber C for performing the surgical procedure. The multiport inset 2548 is also shown as an inset alternative 2542d. The exchange of the insets 2542 in the upper and/or lower coupling 2580a, 2580b can be performed when the upper and lower couplings 2850a, 2580b are connected (as shown in fig. 47) or separated (such as for example shown in fig. 44a - 44f).

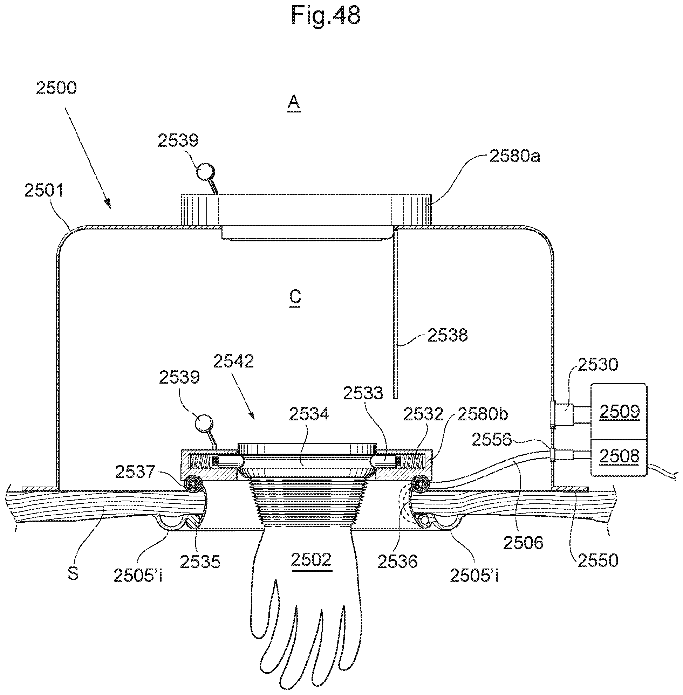

Fig. 48 shows an embodiment of the medical device 2500 in which the medical device 2500 comprises a wall 2501 enclosing a chamber C which is sealed from the ambient environment A. The wall 2501 is fixated and sealed to the skin S of the patient by means of an adhesive surface 2550. The medical device 2500 comprises a lower coupling 2580b adapted to be fixated in an incision made in the skin S by means of an intra-body ring 2535 and an internal vacuum sealing member 2505'i together constituting a sealing device sealing between the medical device 2500 and the body of the patient. The internal vacuum sealing member 2505'i is connected to a vacuum creating member in form of a vacuum pump 2508 placed on the outside of the wall 2501 of the medical device 2500. The connection between the vacuum creating member 2508 and the vacuum sealing member 2505 is made by means of a vacuum conduit 2506 which is transferred through the wall 2501 of the medical device 2500 by means of a fluid coupling 2556. The chamber C of the medical device is according to the embodiment described adapted to hold a fluid having a pressure exceeding the atmospheric pressure, such that a laparoscopic surgical procedure with an inflated abdominal cavity can be performed. The fluid filling the chamber C having a pressure exceeding atmospheric pressure is supplied by a fluid pump 2509 through a fluid inlet 2530 positioned in the wall 2501 of the medical device 2500. A monitoring unit adapted to sense the pressure in the vacuum sealing member 2505'i and/or the blood flow, and/or the blood pressure of the patient may further be incorporated in the medical device 2500.

The vacuum creating member 2508 may further be connected to the monitoring unit (not shown) for adjusting the pressure in the internal vacuum sealing member 2505'i in response to the input from the monitoring unit, for mediating between sealing and keeping the blood flow of the patient unaffected. The vacuum creating member 2508 may be adapted to adjust the pressure in the vacuum sealing member 2505'i for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, - 10 mmHg and 0 mmHg, and -5 mmHg and 0 mmHg and thus keeping the blood flow of the patient substantially unaffected. An example of the operation of a monitoring unit is further described with reference to figs. 34a - 34j.

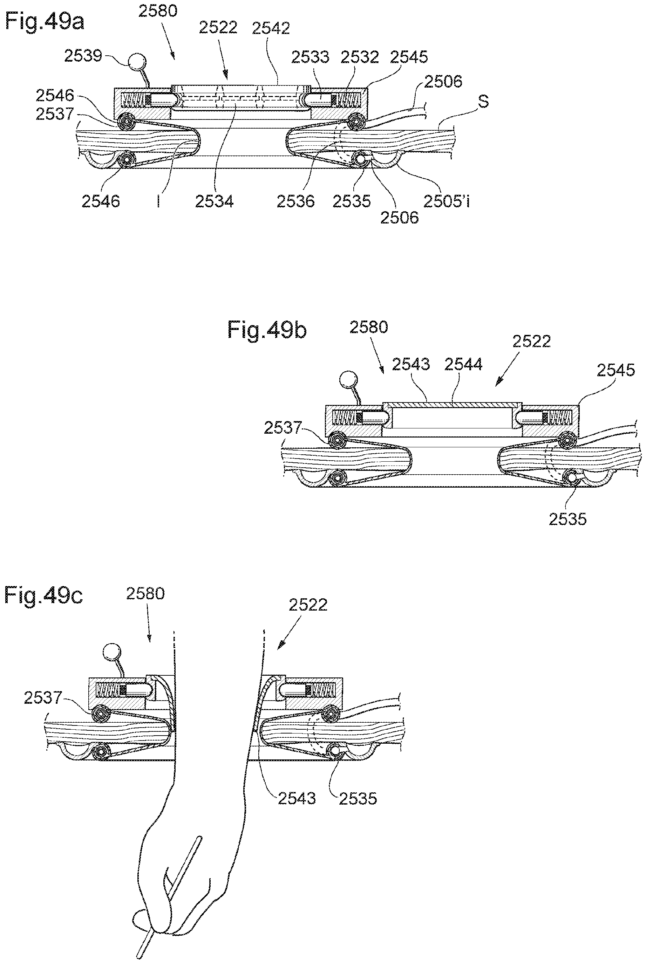

Fig. 49a shows an embodiment of the medical device in which the medical device does not enclose a chamber outside the body of the patient, but rather only provides a coupling 2580 connected in the skin S of the patient. In the coupling 2580, an inset 2542 comprising a body multiport 2542 is provided, latched in the coupling 2580. A roll-up system 2537 for rolling the connecting member 2536 is connected to the seat of the lower coupling 2580b, such that the connecting member 2536 can be tightened for functioning as a retractor keeping the incision I created in the skin S of the patient open. The connecting member 2536 connects the roll-up system to an intra-body ring 2535 adapted to be placed on the inside of the skin S of the patient. The intra-body ring 2535 is in turn connected to the internal vacuum sealing member 2505'i, and the intra-body ring 2535 and the vacuum sealing member 2505'i together forms the sealing device. The internal vacuum sealing member 2505' is connected to a vacuum creating member (not shown), such as a vacuum pump, via a vacuum conduit 2506.

Fig. 49b shows an embodiment of the medical device identical to the medical device shown in fig. 49a, the only difference being that the inset 2542 latched in the coupling 2580 is exchanged to an inset comprising a body port with a self sealing membrane 2543 comprising a small self sealing hole 2544. In fig. 49c the hand of the surgeon is placed through the hole 2544 in the membrane 2543 enabling manual manipulation within the body of the patient.

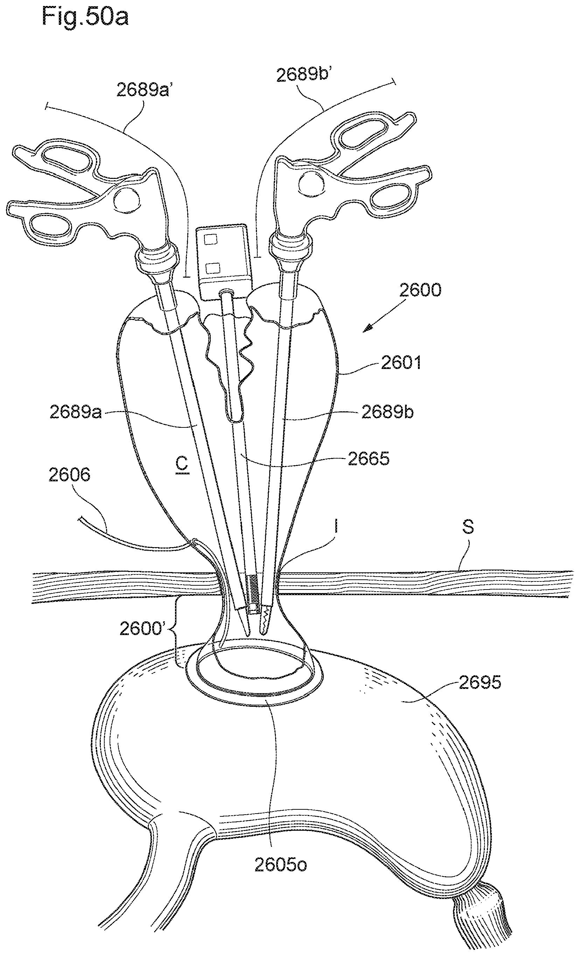

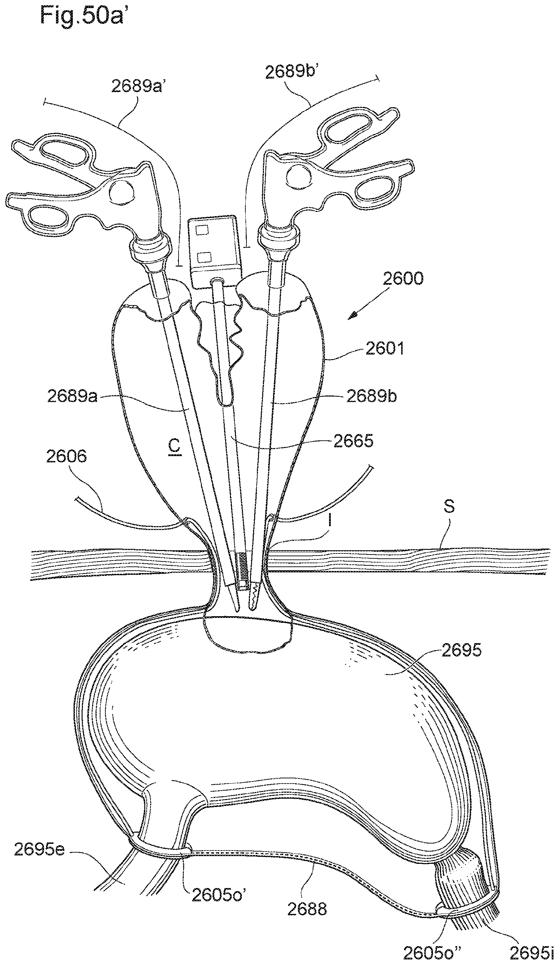

Figs. 50a - 53 show embodiments of a medical device for sealing against an organ or tissue inside the body of the patient, such that a sealed tunnel in which a portion of the surgical procedure can be performed is created between an area inside of the body of the patient and an incision in the skin S, a chamber external to the body of the patient, or the ambient environment. The created sealed tunnel for example makes it possible to contain contents of the opened organ or tissue within the sealed environment, such that the rest of the patient's body is free from contamination from the surgical area. The medical device comprises a sealing device for sealing against the tissue or organ, which could comprise a vacuum sealing member for sealing against the tissue or organ by means of negative pressure, or a pressure sealing member adapted to seal against the tissue or organ by applying pressure thereon, or an adhesive sealing member adapted to seal against the tissue or organ by adhering to the surface of the tissue or organ. The sealing device is sealingly connected to a wall of the medical device, which is adapted to enclose the sealed chamber in which the surgical procedure is partially performed. The medical device could be adapted to be used in laparoscopic surgery, in which case at least one laparoscopic trocar seals the enclosed environment, or in open surgery, in which case the wall of the medical device is sealed around the skin of the patient such that a fluid connection is established between the ambient environment and the chamber of the medical device in connection with the organ or tissue of the patient. The medical device could in different embodiments be adapted to engage human tissue or an organ, which for example could be one of: the esophagus, the stomach, the small intestine, the large intestine, the anus, the gallbladder, the bile duct, the kidney, the renal pelvis, the urethra, the urine bladder, the urethra, the blood vessel, the heart, the lung, the bronchus, an orifice of the body, muscle fascia, the peritoneum, fat tissue, fibrotic tissue or muscle tissue. In the embodiments shown in figs. 50a - 53 the internal sealing device is shown comprising an internal vacuum sealing member, however, in all embodiments it is equally conceivable that the internal vacuum sealing member is exchanged to an internal sealing member adapted to seal by means of pressure or an adhesive, such as described in relation to sealing devices in other embodiments herein. The medical device in any of the embodiments may have the chamber connected to a pressurized fluid supply such that the medical device can receive pressurized fluid and at least one of; hold the same pressure as a pressurized fluid filling a body cavity outside the chamber, hold a lower pressure than a pressurized fluid filling a body cavity outside the chamber, and hold a higher pressure than a pressurized fluid filling a body cavity outside the chamber. The medical device may further comprise at least one of; a nipple for fluid supply placed outside the body and a nipple for fluid supply placed outside the body in combination with a second nipple adapted to receive flow of fluid leaving the chamber to allow circulation of fluid. The method and structures for sealing against an organ or tissue on the inside of the patient's body could be used in combination with any of the embodiments of the medical devices herein and is to be seen as a principle for supplying sealing between the body of the patient and a medical device.

Fig. 50a shows a medical device 2600 having an intra-body portion 2600' being adapted to be positioned inside a patient's body via an incision I made in the skin S of the patient. The medical device 2600 comprises a sealing device 2605o comprising an internal or organ sealing member 2605o adapted to be positioned in connection with the stomach 2695 of the patient and seal against the stomach 2695 of the patient. The medical device 2600 further comprises a flexible wall 2601 adapted to create a medical device chamber C sealingly connected to the first sealing device 2505o, such that the medical device chamber C is placed in connection with the stomach 2695. By the first sealing device and the wall 2601 of the medical device, a sealed space is created during the surgical procedure, such that part of the surgical procedure can be performed within the sealed space and thus isolated from the ambient environment, by which the risk of infection is reduced as well as the risk that staff members present in the ambient environment is contaminated by particles from the body of the patient. The internal vacuum sealing member 2605o comprises a vacuum groove adapted to create a vacuum chamber together with the stomach 2695. The sealing device shown in fig. 50a comprises an internal vacuum sealing member 2606o, however, in alternative embodiments, it is equally conceivable that the internal sealing device seals against the stomach 2695 by means of a biocompatible adhesive contacting the surface of the stomach. In yet alternative embodiments it is furthermore conceivable that the sealing device comprises a pressure sealing member adapted to seal by exerting a pressure on the stomach of the patient. The pressure sealing member could for example be pneumatic pressure sealing member comprising an inflatable portion adapted to be inflated and thereby press against the stomach of the patient.

The laparoscopic instruments 2689a, 2689b shown in fig. 50a comprises handle portions 2689a', 2689b' operably encapsulated by the flexible wall 2601 such that the handle portions 2689a', 2689b' can be manipulated from outside the chamber C.

Fig. 50a' shows an alternative embodiment of the medical device described with reference to fig. 50a. In the alternative embodiment of fig. 50a', the medical device comprises a first internal sealing device comprising a first internal sealing member 2605o' adapted to be placed encircling the esophagus 2695e of the patient for creating a seal between the medical device and the esophagus 2695e. The medical device further comprises a second internal sealing device, comprising a second internal sealing member 2605o"adapted to be positioned encircling the intestine 2695i of the patient for creating a seal between the medical device and the intestine 2695i of the patient. The first and second sealing members 2605o', 2605o" each comprises at least one sealing surface adapted to connect to and seal against the esophagus and intestine respectively. The sealing surface is placed on the inner circumference of the internal sealing devices 2605o', 2605o", such that the internal sealing devices 2605o', 2605o" can seal radially and thus against the outer circumference of the esophagus 2695e and intestine 2695i respectively.

As the sealing members 2605o' and 2605o" arte placed encircling the intestine 2695i and esophagus 2695e, the wall 2601 of the medical device is flexible and encloses the stomach of the patient, such that a surgical procedure on the stomach can be performed inside of the sealed chamber of the medical device, inside of the body of the patient. The wall further comprises a closing portion 2688 adapted to be opened and closed for such that the wall can be extended around the stomach of the patient, for enclosing the stomach of the patient in the chamber C.

The sealing devices 2605o' and 2605o" could be sealing devices such as the sealing devices described with reference to other embodiments in herein. The sealing device 2605o', 2605o" may be vacuum sealing devices, such as shown in fig. 50a', sealing by using a vacuum towards esophagus 2695e and intestine 2695i respectively. The vacuum is supplied by vacuum conduits 2606 attached to a vacuum creating member outside of the body of the patient. A monitoring unit adapted to sense the pressure in the vacuum sealing member 2605o may further be incorporated in the medical device, and/or a monitoring unit for monitoring the pressure in the chamber C, and/or the blood flow, and/or the blood pressure of the patient. The vacuum creating member may be connected to the monitoring unit for adjusting the pressure in the vacuum sealing members 2605o', 2605o" in response to the input from both the monitoring unit, for mediating between keeping the chamber C sealed and keeping the blood flow of the patient unaffected. The vacuum creating member may be adapted to adjust the pressure in the vacuum sealing member 2605o for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.

In alternative embodiments, the sealing devices 2605o', 2605o" may be pressure sealing devices, sealing using a pressure towards the esophagus 2695e and intestine 2695i respectively, or adhesive sealing devices, sealing by adhesively contacting the esophagus 2695e and intestine 2695i respectively.

One example of a surgical procedure suitable to be performed in the sealed chamber is a gastric bypass Roux en-Y procedure, as it involves the opening of the stomach and intestines which is advantageous to perform isolated from the rest of the abdominal cavity of the patient.

The wall forming the chamber may be adapted to hold a pressure within the chamber exceeding atmospheric pressure.

Fig. 50b shows an alternative embodiment of the medical device in which the wall 2601 fixated the sealing member 2605o is further fixated to an incision retractor 2670 placed in the incision in the skin S. The incision retractor 2670 seals against the cut surface of the incision in the skin S by the incision being somewhat smaller than the incision cut in the patient's skin S. The elasticity of the skin S thus constricts the retractor member 2670 creating a seal between the retractor member 2670 and the skin S. In the retractor member 2670, a laparoscopic multiport 2648 is positioned, enabling the simultaneous use of a plurality of laparoscopic instruments 2689a, 2689b, 2665. The laparoscopic multiport 2648 comprises a plurality of ports or holes through which laparoscopic instruments 2689a, 2689b or a laparoscopic camera 2665 can be inserted and operated. Each of the port comprises a membrane seal comprising an elastic polymer sheet material sealing between the laparoscopic instrument 2689 or camera 2665 and the multiport 2648. The internal portion of the wall 2601 of the medical device forming a chamber C inside the body of the patient is adapted to have a predefined shape, such that the wall 2601 assumes the predefined shape after having been inserted through the incision in the skin S. The predefined shape could for example be a predefined shape adapted suitable for connecting to a specific organ in the body of the patient. For retaining its shape, the wall may comprise one or more ring or loop shaped reinforcements placed in the wall perpendicular to the axis from the incision to the sealing member placed on the organ or tissue. The embodiment shown on fig. 50b additionally comprises a fluid conduit 2610 for providing a rinsing fluid to the tissue of the organ of the patient for rinsing and/or sucking in the surgical area. The fluid provided through the fluid conduit could for example be a sterilization fluid, such as chlorhexidine or hydrgenperoxide. In other embodiments, medical device may comprise one, two or more fluid conduits leading from outside the body to inside the body, inside the chamber, adapted to allow sucking and injecting fluid into the chamber from outside the body, wherein the one or two or more fluid conduits may be adapted to be at least one of; integrated in at least one of; in the medical device, the wall and a body port placed in close proximity to the incision, placed as a separate tube and having the two ends of the fluid conduit displaced as a separate tubes and there in between adapted to be integrated in at least one of; in the medical device, the wall and the body port placed in close proximity to the incision.

The sealing device shown in fig. 50b comprises an internal vacuum sealing member 2605o, just as in the embodiment shown in fig. 50a and 50a'. The vacuum sealing member 2505o is connected to a vacuum source or vacuum creating member (not shown), such as a vacuum pump, by means of a vacuum conduit 2606. A monitoring unit adapted to sense the pressure in the vacuum sealing member 2605o may further be incorporated in the medical device, and/or a monitoring unit for monitoring the pressure in the chamber C, and/or the blood flow, and/or the blood pressure of the patient. The vacuum creating member may be connected to the monitoring unit for adjusting the pressure in the vacuum sealing member 2605o in response to the input from both the monitoring unit, for mediating between keeping the chamber C sealed and keeping the blood flow of the patient unaffected. The vacuum creating member may be adapted to adjust the pressure in the vacuum sealing member 2605o for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.

The sealing provided by the incision retracing member 2670 may be assisted or replaced by an additional sealing device, such as the additional sealing devices comprises vacuum sealing members (2505'), as for example described with reference to for example figs.267c and 267d or any other example described herein.

Fig. 50b' shows an embodiment of the medical device similar to the embodiment described with reference to fig. 50b, the difference being that the internal sealing device comprising the internal sealing member comprises an internal sealing member 2605oa having an adjustable circumference, such that the size of the encloses area of the organ, in this case the stomach, can be adjusted. In the embodiment of fig. 50b' the vacuum sealing member 2605oa is an extendable pleated vacuum conduit comprising pleated portions 2603 such that the circumference can be made shorter by shortening the vacuum conduit. The extendible vacuum conduit comprises vacuum perforations VP which are adapted to exert a suction force against the tissue of the patient, such that the tissue adapted to the pleated portions of the extendible vacuum conduit, such that a sealing is created between the sealing member 2605oa and the tissue of the patient. By the circumference of the sealing member 2605oa being adjustable, the enclosed area may be adapted to the surgical procedure to be performed, or the sealing member 2605oa may be made smaller after engaging the tissue of the patient, such to better enclose a portion of tissue of the patient in the chamber.

The extendible vacuum conduit 2605oa is connected to a vacuum conduit 2606 connecting the extendible vacuum conduit to a vacuum creating member placed outside of the body of the patient.

In the embodiment described with reference to fig. 50b', vacuum perforations VP are placed in the front end of the internal vacuum sealing member device, such that the sealing member can seal against a planar surface of a tissue.

Fig. 50b" is an alternative embodiment of the adjustable vacuum sealing member 2605oa in which the adjustable vacuum sealing member comprises vacuum channels IC, OC placed displaceably in each other such that the inner channel IC can be displaced in relation to the outer channel OC, forming a vacuum groove, such that the circumference of the vacuum sealing member can be adjusted and thus also the area enclosed by the vacuum sealing member 2605oa. The vacuum channels IC, OC are preferably made from a semi-flexible or semi-elastic material such as a hard silicone, polyurethane or a rubber material.

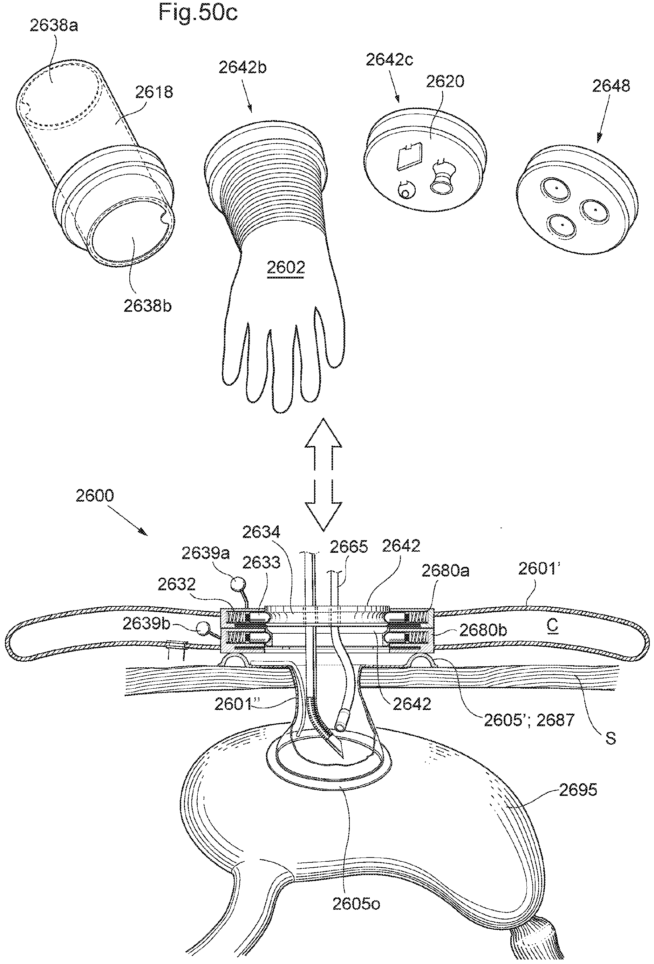

Fig. 50c shows an alternative embodiment of the medical device 2600 similar to the embodiment shown with reference to figs. 50a, 50b and 50b', the difference being that the medical device of fig. 50c comprises an upper coupling 2680a fixated to the wall 2601 of the medical device 2600 to which an inset 2642 can be fixated, such as further described with reference to other embodiments herein. The upper coupling 2680a is in the state shown in fig. 50c connected to a lower coupling 2680b which in turn is connected to the skin S of the patient by means of a vacuum sealing member 2605'. The vacuum sealing member 2605' fixates and seals the medical device to the skin S of the patient and furthermore connected the lower wall portion 2601" to the upper wall portion 2601', by the lower wall portion being sealingly connected to the vacuum sealing member 2605', which in turn is connected to the lower coupling, which in turn is connected to the upper wall portion 2601'. The vacuum sealing member 2605' thus functions as a holding member 2687 holding the medical device in place when having been placed in the operational position. This embodiment could of course use all the different sub-embodiments or part and pieces of the other different embodiments in this application in forms of both descriptions and drawings. For example could the vacuum sealing member 2605' be placed in or inside the incision as described in other places herein. Furthermore, the vacuum sealing does not need to be involved in holding the device in place. A retractor fixated to the wall and placed in the skin would be an attractive option.

The lower wall portion 2601" travels from the vacuum sealing member 2605', through the incision made in the skin S of the patient and connects with the internal sealing device comprising the internal vacuum sealing member 2605o which connects to and seals against the stomach 2695 of the patient. A tunnel is thereby created from the interconnected couplings 2680a, 2680b to the stomach 2695 portion in connection with the internal vacuum sealing member 2605o. The couplings 2680a; 2680b each comprises a releasing lever 2639a, 2639b for releasing the insets 2642. The levers 2639a, 2639b are connected to protruding members 2633 which are spring 2632 loaded from the rear in order to secure the recess 2634 of the inset.

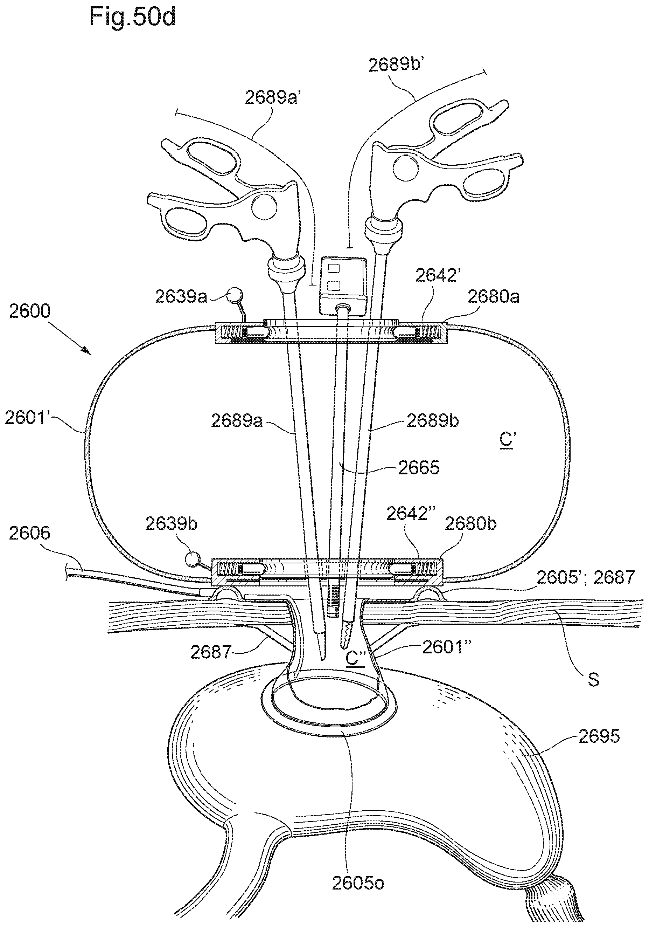

The multiport insets 2642 latched in the upper and lower couplings 2680a, 2680b can be exchanged to another inset as shown in fig. 50c. The insets 2642 for which the multiport inset can be exchanged could for example an inset 2642a comprising an airlock sluice 2618 enabling the transfer of objects through the couplings 2680a;b while maintaining the sealed environment of the chamber C. The airlock sluice 2618 comprises a first valve member 2638a separating the area of the airlock 2618 from the ambient environment, and a second valve member 2638b separating the area of the airlock 2618 from the sealed environment of the chamber C. Alternatively, the multiport inset 2642 may be exchanged to an inset 2642b comprising a glove 2602 which can be placed in the upper coupling 2680a and enable the manipulation within the chamber C, and/or within the cavity in the body of the patient. An additional alternative is an inset 2642c comprising a device or instrument mount 2620 which could comprise holding portions for holding different instruments of objects which may be used within the chamber C for performing the surgical procedure. The multiport inset 2648 is also shown as an inset alternative 2642. The exchange of the insets 2642 in the upper and/or lower coupling 2680a, 2680b can be performed when the upper and lower couplings are connected (as shown in fig. 50c) or separated (such as for example shown in fig. 50d). The features described in fig. 50c may be combined with any of the medical device embodiment of figs. 50a, 50b and 50b'.

Fig. 50d shows the embodiment of the medical device shown in fig. 50c in a state when the upper coupling 2680a is disconnected from the lower coupling 2680b such that a larger external chamber C' is formed by the external wall portion 2601'. The external wall portion 2601' is made from a flexible polymer material such that the upper coupling 2680a can be moved in relation to the lower coupling 2680b, enabling the laparoscopic tools 2689a, 2689b and camera 2665 to be moved inside of the body of the patient. The inset 2642" in the lower coupling 2680b may be omitted such that a single chamber is formed by the external and internal wall portions 2601', 2601". Omitting the lower inset 2642" (in the embodiments when the inset comprises a multiport) enhances the motion range of the laparoscopic instruments. The embodiment shown on fig. 50d additionally comprises mechanical holding members 2687 for fixating the medical device from the inside of the body of the patient, against the inside of the skin S. In alternative embodiments, the holding member may comprise any one of: a ring shape, a circular bendable shape, a flexible structure, two circular bendable shaped structures, whereof at least one is adapted to hold inside the incision, one, two or more ring shaped structures adapted to have a size larger than the incision in the body, at least one ring shaped structure adapted to be bendable for introducing through the incision, adapted to hold inside the incision, two or more holding member parts adapted to be placed on at least one of; the inside of the incision, outside the incision, and against the tissue related to the incisional cut, wherein two or more parts is adapted to be placed a s separate parts in the selected places described, at least two parts connected to each other, at least two parts connected to each other, the connection being elongated and being substantially longer than the closest distance between the parts, a construction adapted to allow an expansion of the incisional area or circumference of the incision to allow a hand or other parts to pass through the incision.

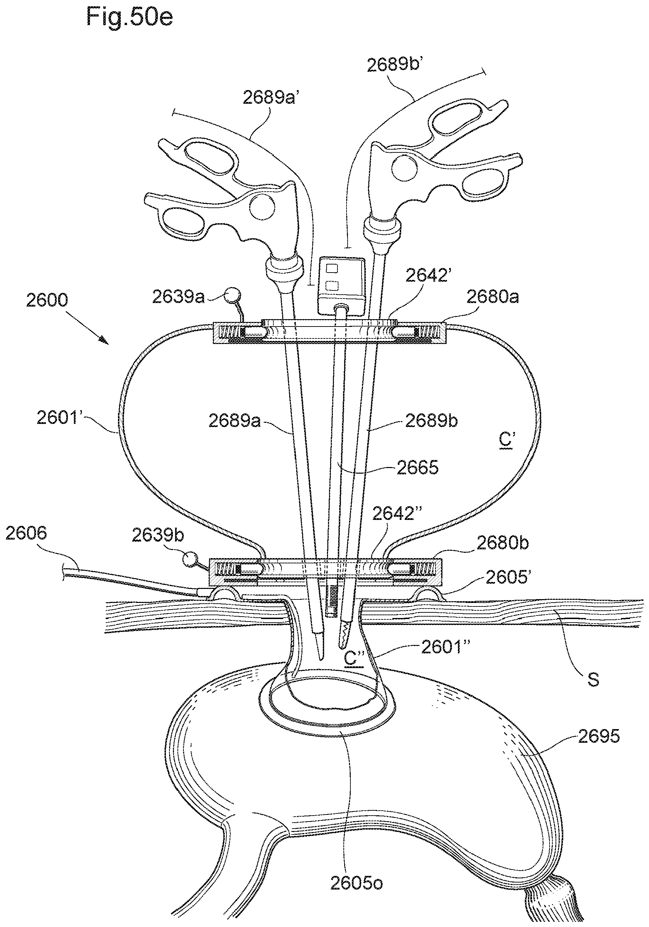

Fig. 50e shows an alternative embodiment similar to the embodiment shown in fig. 50d, the difference being that the external wall portion 2601' is in the bottom connected to an inset 2642" latched in the lower coupling 2680b, such that the external wall portion 2601' and thus the external portion of the chamber C' can be disconnected from the coupling enabling direct laparoscopic or open surgery in the lower coupling 2680b. When the inset 2642' latched in the upper coupling has been removed, an inset comprising a multiport, or an inset comprising a glove member (such as described in other embodiments herein) can be latched in the lower coupling enabling laparoscopic surgery or manual manipulation within the internal chamber C" of the medical device 2600.

Fig. 51a shows an embodiment of the medical device in which the medical device 2600 is adapted for surgery performed on the small or large intestine 2695 of the patient, such as for example the removal of a tumor 2681 on the intestine 2695. The medical device 2600, as in the embodiments disclosed with reference to figs. 50a - 50d' comprises a sealing device comprising a vacuum sealing member 2605o sealing against the intestine 2695 of the patient. The medical device comprises a wall 2601 enclosing the chamber C. Two laparoscopic gripping instruments 2689a, 2689b and a laparoscopic camera 2665 are integrated in the wall 2601 of the medical device 2600, enclosed by the wall 2601, such that the instruments 2689a, 2689b and camera 2665 are hermetically enclosed and thus fully placed within the chamber C enclosed by the wall 2601 and sealed from the ambient environment. The wall 2601 of the medical device 2600 passes through the incision I made in the skin S of the patient and thus forming a tunnel from the portion of the chamber C on the outside of the body of the patient to the portion of the chamber C placed inside the body of the patient, such that surgical steps can be performed inside of the sealed environment of the chamber C on the outside of the patient's body, as well as on the inside of the patient's body.

The wall 2601 is made from a transparent flexible polymer material, such as PVC with a plasticizer additive. The lower portion if the wall 2601 is connected to an internal vacuum sealing member 2605o which engages the organ (intestine) 2695 of the patient for sealing there-against. The internal vacuum sealing member 2605o is connected to a vacuum creating member (not shown, but further described in relation to for example fig. 48) via a vacuum conduit 2606.

Fig. 51b shows an embodiment of the medical device in which the medical device is adapted for surgery performed on the small or large intestine 2695 of the patient, such as for example the removal of a portion of the intestine 2695. The medical device comprises a first and second internal vacuum sealing member 2605o', 2605o", each adapted to encircle the intestine 2695 of the patient such that a portion of the intestine 2695 of the patient is enclosed in a chamber C enclosed by the lower wall portion 2601" of the medical device 2600. The first and second vacuum sealing members 2605o', 2605o" are connected to a first and second vacuum conduit 2606', 2606" that travels through the wall 2601' of the medical device through a first and second fluid coupling 2656', 2656" positioned in the wall 2601 of the medical device 2600. The first and second vacuum conduits 2606', 2606" are each connected to a vacuum creating member (not shown), such as a vacuum pump. A monitoring unit adapted to sense the pressure in the vacuum sealing member 2605o and/or the blood flow, and/or the blood pressure of the patient may further be incorporated in the medical device. The vacuum creating member may be connected to the monitoring unit for adjusting the pressure in the vacuum sealing member 2605o in response to the input from the monitoring unit, for mediating between keeping the chamber C sealed and keeping the blood flow of the patient unaffected. The vacuum creating member may be adapted to adjust the pressure in the vacuum sealing member 2605o for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.

The portion of the chamber C placed in the body of the patient is further isolated by a closing sealing device in the form of a penetratable self sealing membrane 2643, for example comprising a self sealing gel (further described with reference to other embodiments herein). The penetratable self sealing membrane is adapted to be penetrated by the laparoscopic instruments 2689a, 2689b and camera 2665 such that a laparoscopic procedure can be performed within the lower chamber C" of the medical device. In alternative embodiments the closing sealing device could be operated in alternative ways, such as for example by means of magnetic force or vacuum. The external portion of the wall 2601' of the medical device enclosing the external portion of the chamber C' penetrates the skin S of the patient through an incision retractor 2670 position in the incision I. The incision retractor 2670 could for example be positioned in the navel of the patient, such that the procedure can be performed without leaving any scarring. The laparoscopic instruments 2689a, 2689b and camera 2665 are integrated in the flexible wall 2601' of the medical device such that the instruments 2689a, 2689b, can be moved in relation to the intestine 2695 for performing the laparoscopic procedure in the intestine 2695.

Turning to the portion of the medical device inside the body of the patient, the portion of the wall 2601" inside the body of the patient is adapted to encircle the portion of the intestine 2695 by the wall 2601 comprising a closing portion 2688, which is also operated by means of the vacuum supplied by the vacuum creating members through the vacuum conduits 2606', 2606". In alternative embodiments the closing portion could be operated in alternative ways, such as for example by means of magnetic force. The internal portion of the wall 2601" is positioned inside the body of the patient encircling the small or large intestine of the patient, vacuum is applied to the first and second vacuum sealing member 2605o', 2605o", such that sealing is created between the sealing members 2605o', 2605o" and the wall 2601", thereby enclosing the lower portion of the chamber C" inside the body of the patient.

In surgical procedures where a portion of the intestine 2695 is removed, the operation can be performed partially in the lower portion of the chamber C". The intestine 2695 is cut at suitable portions and the end portions created are connected using sutures or staplers. The removed portion is retracted through the self sealing membrane and positioned in the upper portion of the chamber C', the lower portion of the chamber C" can be properly rinsed, such that no intestinal contents is transferred into the abdomen of the patient. When the lower chamber C' is properly rinsed, the internal vacuum sealing members 2605o', 2605o" are released and the closing portion 2688 is opened such that the medical device can be removed from the intestine 2695.

The principle in Fig 51b could easily be slightly adapted to enclose more organs and larger space. For example a roux-en-y procedure or gastric bypass could performed enclosing and sealing the intestine and esophagus including the stomach inside the sealed space, or alternatively sealing part of the stomach in relation to seal of intestine or esophagus.

Fig. 51b'shows and embodiment of the medical device similar to the embodiment shown in fig. 51b', the difference being that the first and second vacuum sealing members 2605oa', 2605oa" are adjustable, such that the circumference of the sealing members can be adjusted. The adjustable vacuum sealing members 2605oa', 2605oa" are very similar to the vacuum sealing member (2605oa) disclosed with reference to figs. 50b', with the difference that the vacuum perforations are placed on the inner circumference of the pleated vacuum conduit, such that the internal sealing device can seal radially and thus against the outer circumference of a tubular organ or tissue, such as the intestine shown in fig. 51b'.

In the embodiment shown in fig. 51b', the first and second adjustable vacuum sealing members are adjusted differently, such that the wall forming the chamber C can enclose the portion of the intestine 2695 in the overpass from the small intestine to the large intestine, by the first sealing member 2605oa' being adjusted to a smaller circumference than the second sealing member 2605oa".

In the embodiment disclosed in fig. 51b', the adjustable sealing members are adjustable vacuum sealing members 2605oa', 2605oa", made up of adjustable vacuum conduits connected to vacuum conduits 2606', 2606" which are connected to a vacuum creating member placed on the outside of the body of the patient. The vacuum creating member may be connected to the monitoring unit for adjusting the pressure in the vacuum sealing member 2605o in response to the input from the monitoring unit, for mediating between keeping the chamber C sealed and keeping the blood flow of the patient unaffected. The vacuum creating member may be adapted to adjust the pressure in the adjustable vacuum sealing members 2605oa', 2605oa", for keeping the negative pressure between at least one of: -135 mmHg and 0 mmHg, -115 mmHg and 0 mmHg, -100 mmHg and 0 mmHg, -50 mmHg and 0 mmHg, -30 mmHg and 0 mmHg, -20 mmHg and 0 mmHg, -10 mmHg and 0 mmHg, and -5 mmHg and 0 mmHg and thus keeping the blood flow of the patient substantially unaffected.. However, in alternative embodiments, it is equally conceivable that the adjustable sealing members are adjustable pressure sealing members and/or adjustable adhesive sealing members.

It is obvious that all the other solutions to seal around the incision described herein, different solutions of sealing using a direct port and/or retractor in the skin to connect to the wall both with a retractor, port, multiport or inset in the skin, and/or combining with an external reservoir, as well as all the other pieces of embodiments disclosed herein could be used together with this embodiment. The combination of an external and internal chamber with its closable port in between could then be used as a sluice taken body parts out from the body as well as introducing medical implants into the body and still continuing the laparoscopic procedure and combining with changing port to a glove inset and allowing hand-assistant surgery still keeping the pressure needed inside the body to continue the key whole surgery procedure. The same principle could of course apply in arthroscopic surgery.

Fig. 51c shows an embodiment of the medical device similar to the embodiment shown with reference to fig. 51c, the difference being that there is no sealed chamber on the outside of the body of the patient. An incision retractor 2670 is positioned in the incision I made in the skin S of the patient which provides sealing against the skin S of the patient by means of the elastic skin S constricting the incision retractor 2670. In the incision retractor 2670, a body multiport 2648" is positioned for allowing the laparoscopic instruments 2689a, 2689b and camera 2665 to operate in the cavity in the body of the patient for performing the surgical procedure on the intestine 2695 of the patient. The embodiment shown in fig. 51c comprises a penetratable self sealing membrane 2643, such as a penetratable self sealing gel, which provides a sealing between a first sealed chamber C' between the body multiport 2648" positioned in the incision retractor 2670, and the penetratable self sealing membrane 2643. The penetratable self sealing membrane 2643 makes it possible to retract e.g. portions of the intestine to the upper chamber C' while removing the medical device from the intestine of the patient, which requires the opening of the closing portion 2688, and thus connecting the lower chamber C" with the abdominal cavity of the patient.

The incision retractor 2670 could, as all embodiment of the incision retractor, for example be positioned in the navel of the patient, such that the procedure can be performed without leaving any scarring in the skin of the patient.

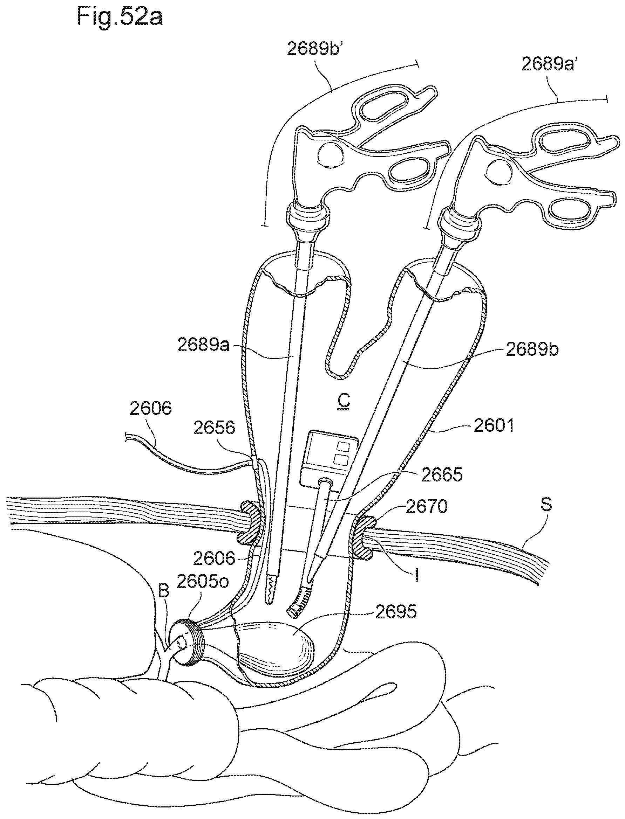

Fig. 52a shows an embodiment of the medical device adapted for cholecystectomy, i.e. surgical removal of the gallbladder 2695. The medical device comprises an internal sealing device comprising an internal vacuum sealing member 2605o adapted to encircle the bile duct B and seal against the bile duct B by means of vacuum. The sealing device is sealingly connected to a flexible wall 2601 of the medical device and adapted to enclose a chamber C in which a portion of the surgical procedure can be performed sealed from the ambient environment. By the sealing device being connected to the wall 2601, the gallbladder 2695 becomes positioned inside the chamber C, which enables the removal of the gallbladder 2695 within the chamber C, separated from the rest of the abdomen, such that the incisions and open portions of the gallbladder 2695 can remain sealed from the rest of the abdominal cavity during the entire procedure. When the gallbladder 2695 has been removed, the bile duct B is closed with sutures or staplers and the internal vacuum sealing member 2605o further constricts the bile duct B as the medical device is being retracted, such that the chamber C remains sealed at all times during the removal of the medical device. When the bile duct B finally leaves the internal vacuum sealing member 2605o, it closes entirely and the chamber C is thus a completely sealed environment within the body of the patient, which can be removed through the incision in the skin S without the removed gallbladder 2695 ever being exposed to the environment of the abdomen.

All other solutions to seal around the incision described herein, different solutions sealing using a direct port and/or retractor in the skin to connect to the wall both with a retractor, port, multiport or inset in the skin, and/or combining with an external reservoir, as well as all the other pieces of embodiments disclosed herein could be used together with this embodiment. The combination of an external and internal chamber with its closable port in between could then be used as a sluice taken the gall bladder out from the body as well as introducing any part into the body and still continuing the laparoscopic procedure and combining with changing port to a glove inset and allowing hand-assistant surgery still keeping the pressure needed inside the body to continue the key whole surgery procedure. The infection rate will be reduced. The number of open surgical gallbladder operations will be reduced because of the hand assistance. In such a case the camera will be introduced in an alternative position to combine the hand with a good view. A tube from outside the skin could also be used for disinfection inside the sealed space in the operation area. This tube may form an integrated part of the device.

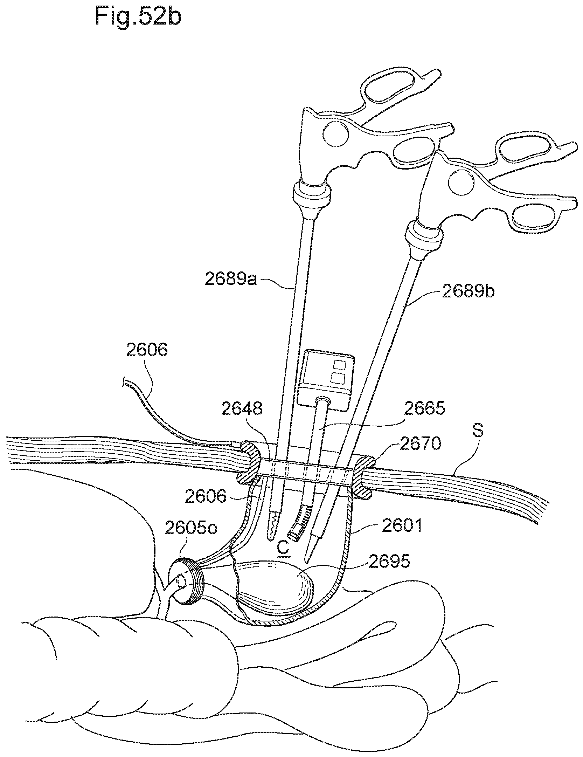

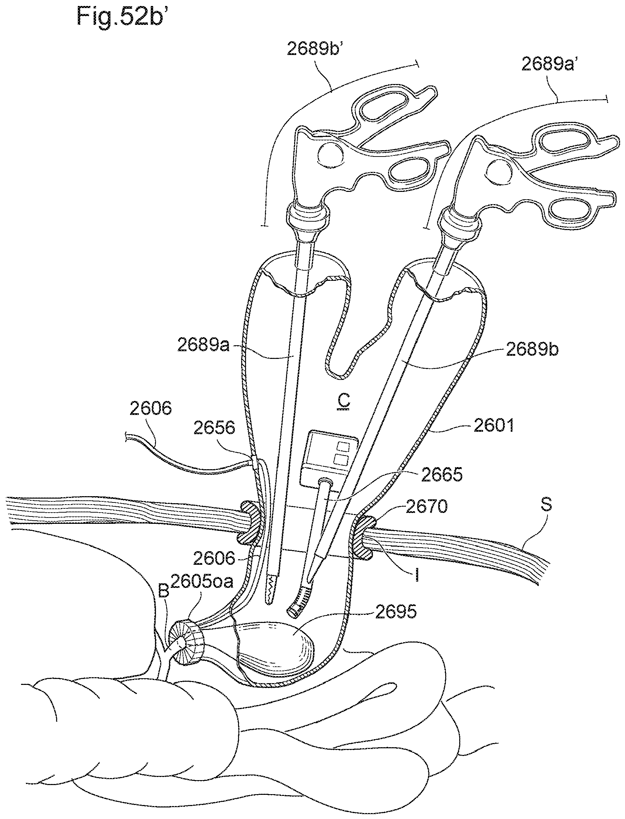

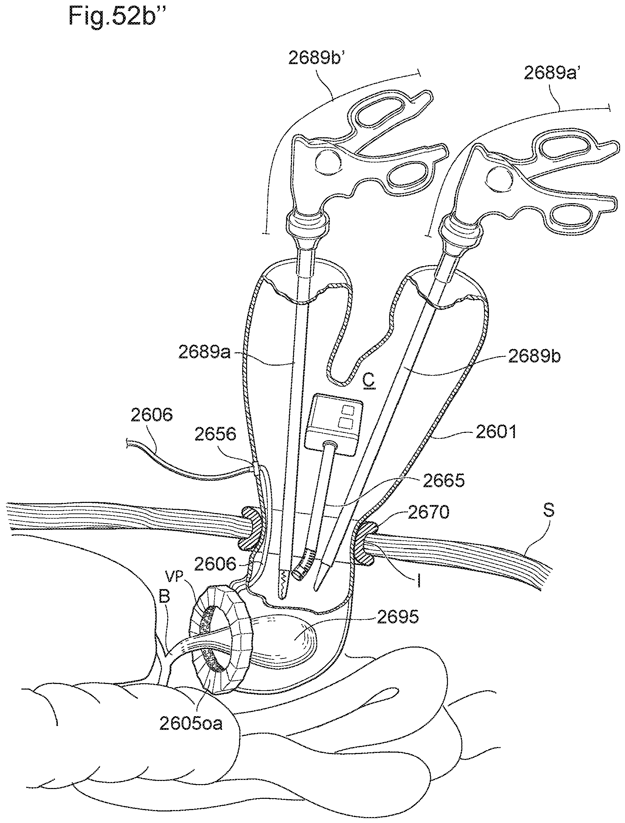

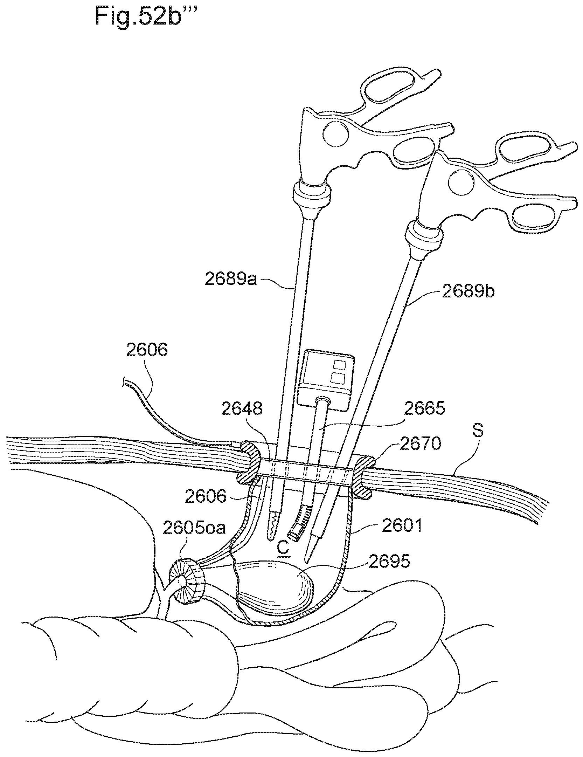

Fig. 52b shows an embodiment of the medical device similar to the embodiment shown with reference to fig. 52a, the difference being that there is no sealed chamber on the outside of the body of the patient. An incision retractor 2670 is positioned in the incision I made in the skin S of the patient which provides sealing against the skin S of the patient by means of the elastic skin S constricting the incision retractor 2670. In the incision retractor 2670, a body multiport 2648 is positioned for allowing the laparoscopic instruments 2689a, 2689b and camera 2665 to operate in the cavity in the body of the patient for performing the cholecystectomy. The wall of the medical device is in its upper portion sealingly connected to the incision retractor 2670, such that a sealed chamber C is created between vacuum sealing member 2605o and the incision retractor 2670 enclosed by the wall 2601 in which the gallbladder is removed. When the gallbladder 2695 has been removed, the bile duct B is closed with sutures or staplers and the internal vacuum sealing member 2605o further constricts the bile duct B as the medical device is being retracted, such that the chamber C remains sealed at all times during the removal of the medical device. When the bile duct B finally leaves the vacuum sealing member 2605o, it closes entirely and the chamber C is thus a completely sealed environment within the body of the patient, which can be removed through the incision in the skin S without the removed gallbladder 2695 ever being exposed to the environment of the abdomen. The incision retractor 2670 could for example be positioned in the navel of the patient, such that the procedure can be performed without leaving any scarring.

Fig. 52b' shows an embodiment of the medical device similar to the embodiment shown in fig. 52b, the difference being that the sealing member is an adjustable sealing member 2605oa having an adjustable circumference, such that the adjustable sealing member can be expanded to travel over the gallbladder 2695 and retracted to enclose the gallbladder inside of the chamber C of the medical device, such that a surgical procedure involving the gallbladder can be performed inside of the sealed chamber C. The sealing member 2605oa is in the embodiment disclosed in fig. 52a' an adjustable vacuum sealing member comprising a sealing surface comprising vacuum perforations placed on the inner circumference of the vacuum sealing member, such that the sealing surface seals radially against the tubular bile duct for enclosing the gallbladder in the sealed chamber formed by the wall of the medical device.

Fig. 52b" shows the embodiment of the medical device shown in fig. 52b', when the adjustable vacuum sealing member 2605oa is in its expanded state, such that the gallbladder 2695 can pass through the adjustable vacuum sealing member 2605oa and thus enclose the gallbladder in the chamber of the medical device. The vacuum perforations VP are placed radially along the surface of the inner circumference of the vacuum sealing member 2605oa, such that the adjustable vacuum sealing member performs a sucking action against the outer circumference of the bile duct.

Fig. 52b‴ shows an embodiment of the medical device similar to the embodiment shown with reference to fig. 52b', the difference being that there is no sealed chamber on the outside of the body of the patient. An incision retractor 2670 is positioned in the incision I made in the skin S of the patient which provides sealing against the skin S of the patient by means of the elastic skin S constricting the incision retractor 2670. In the incision retractor 2670, a body multiport 2648 is positioned for allowing the laparoscopic instruments 2689a, 2689b and camera 2665 to operate in the cavity in the body of the patient for performing the cholecystectomy. The wall of the medical device is in its upper portion sealingly connected to the incision retractor 2670, such that a sealed chamber C is created between vacuum sealing member 2605o and the incision retractor 2670 enclosed by the wall 2601 in which the gallbladder 2695 is removed. When the gallbladder 2695 has been removed, the bile duct B is closed with sutures or staplers and the internal vacuum sealing member 2605o further constricts the bile duct B as the medical device is being retracted, such that the chamber C remains sealed at all times during the removal of the medical device. When the bile duct B finally leaves the vacuum sealing member 2605o, it closes entirely and the chamber C is thus a completely sealed environment within the body of the patient, which can be removed through the incision in the skin S without the removed gallbladder 2695 ever being exposed to the environment of the abdomen. The incision retractor 2670 could for example be positioned in the navel of the patient, such that the procedure can be performed without leaving any scarring.

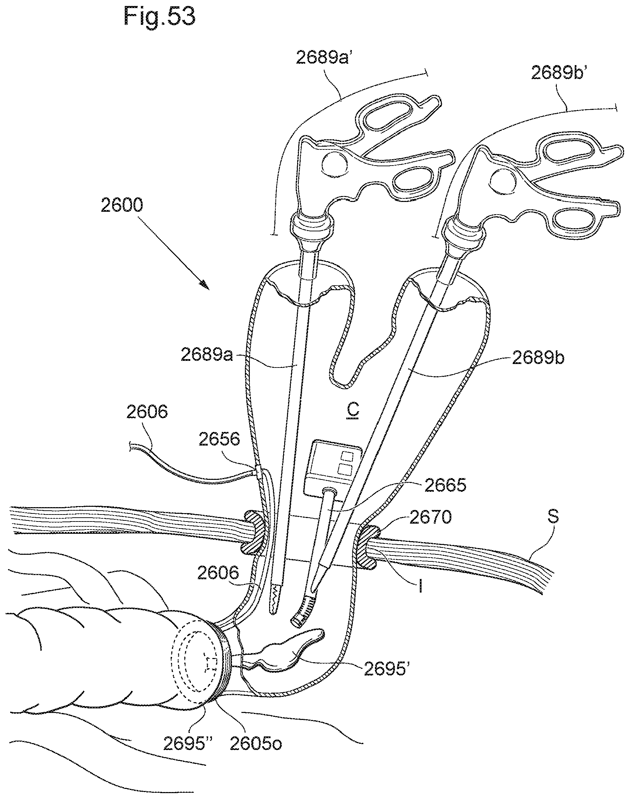

Fig. 53 shows an embodiment of the medical device adapted for appendectomy, i.e. the removal of the appendix 2695'. The medical device 2600 comprises an internal sealing device comprising a vacuum sealing member 2605o adapted to encircle the distal portion of the large intestine 2695" (to which the appendix is connected) and seal against the large intestine 2695" by means of vacuum. The sealing device is sealingly connected to a flexible wall 2601 of the medical device 2600 and adapted to enclose a chamber C in which a portion of the surgical procedure can be performed sealed from the ambient environment. By the sealing device being connected to the wall 2601, the appendix 2695' becomes positioned inside the chamber C, which enables the removal of the appendix 2695' within the chamber C, separated from the rest of the abdomen, such that the incisions and open portions of the appendix and large intestine 2695" can remain sealed from the rest of the abdominal cavity during the entire procedure. When the appendix 2695' has been removed, the internal vacuum sealing member 2605o further constricts the large intestine 2695" as the medical device is being retracted, such that the chamber C remains sealed at all times during the removal of the medical device 2600. When the large intestine 2695" finally leaves the internal vacuum sealing member 2605o, the internal vacuum sealing member 2605o closes entirely and the chamber C is thus a completely sealed environment within the body of the patient. The medical device 2600 with the appendix in the chamber C can thus be removed through the incision I in the skin S without the appendix 2695' ever being exposed to the environment of the abdomen. Analogly to the embodiments disclosed for cholecystectomy and removal of a portion of the intestine (figs. 51b, 51b', 51c, 52a, 52a', 52b, 52b', 52b"), the embodiment of fig. 53 could be adjusted such that the portion of the chamber outside the body of the patient is omitted, and a body multiport is positioned in the incision retractor 2670.

It is obvious that all the other solutions to seal around the incision described herein, different solutions sealing using a direct port and/or retractor in the skin to connect to the wall both with a retractor, port, multiport or inset in the skin, and/or combining with an external reservoir, as well as all the other pieces of embodiments disclosed herein could be used together with this embodiment. The combination of an external and internal chamber with its closable port in between could then be used as a sluice taken the appendix out from the body, and the whole sealed space could be taken out including the appendix. It is possible to combine with changing port to a glove inset and allowing hand-assisted surgery still keeping the pressure needed inside the body to continue the key whole surgery procedure.

The different aspects or any part of an aspect or different embodiments or any part of an embodiment may all be combined in any possible way. Any method or any step of method may be seen also as an apparatus description, as well as, any apparatus embodiment, aspect or part of aspect or part of embodiment may be seen as a method description and all may be combined in any possible way down to the smallest detail. Any detailed description should be interpreted in its broadest outline as a general summary description, and please note that any embodiment or part of embodiment as well as any method or part of method could be combined in any way. All examples herein should be seen as part of the general description and therefore possible to combine in any way in general terms.

### NUMBERED EMBODIMENTS

In the following, exemplifying numbered embodiments are provided in groups A - D and numbered within that group. The numbered embodiments are not to be seen as limiting the scope of the invention, which is defined by the appended claims. The reference numerals in the different numbered embodiments are to be seen only as examples of elements in the appended drawings which correspond to elements described in the numbered embodiments.

### Numbered embodiment A 1 - 70:

1. A medical device adapted to be positioned inside a patient's body via an incision made in the skin of the patient, the medical device comprising;
   - a first internal sealing device, comprising a first internal sealing member adapted to be positioned in connection to and seal against at least a part of at least one human organ or human tissue for creating a seal between the medical device and at least the part of the at least one human organ or human tissue,
   - a second internal sealing device, comprising a second internal sealing member adapted to be positioned in connection to and seal against at least a part of at least one human organ or human tissue, for creating a seal between the medical device and at least the part of the at least one human organ or human tissue, and
   - a wall adapted to create a medical device chamber sealingly connected to the first and second internal sealing device, such that a portion of at least one human organ is placed inside of the medical device chamber between the first and second internal sealing members.
2. The medical device according to embodiment 1, further comprising a body port placed in an incision in the skin of the patient, and wherein the wall is connected to the port, such that the medical device chamber is created between the first internal sealing member, the second internal sealing member and the body port.
3. The medical device according to any one of the preceding embodiments, wherein at least one of the first and second sealing members is adapted to encircle a tubular organ.
4. The medical device according to any one of the preceding embodiments, wherein the wall comprises a closing portion adapted to be opened and closed for enclosing a human organ or human tissue in the medical device chamber.
5. The medical device according to embodiment 4, wherein the closing portion comprises a zipper.
6. The medical device according to any one of embodiments 4 and 5, wherein the closing portion is placed between the first and second internal sealing device.
7. The medical device according to embodiment 6, wherein the first internal sealing device is adapted to encircle and seal against an intestine of the patient and the second internal sealing device is adapted to encircle and seal against the esophagus of the patient, and wherein the wall is adapted to encapsulate the stomach of the patient, such that the stomach of the patient is placed in the medical device chamber between the first sealing member, the second sealing member, and the wall.
8. The medical device according to any one of the preceding embodiments, wherein the first sealing member has a smaller circumference than the second sealing member.
9. The medical device according to embodiment 1, wherein the internal sealing device comprises at least one of;
   a. an internal vacuum sealing device, sealing by using a vacuum towards the human organ or human tissue related to and in contact with said human organ,
   b. an internal pressure sealing device, sealing using a pressure towards the human organ or human tissue related to and in contact with said human organ, and
   c. an internal adhesive sealing device, sealing by adhesively contacting the human organ or human tissue related to and in contact with said human organ.
10. The medical device according to any one of the preceding embodiments, wherein the internal sealing device is adapted to be positioned, at least one of;
   a. on the human organ,
   b. on human tissue related to and in contact with said human organ,
   c. in an incision made in the at least one human organ of the patient,
   d. on the inside of the at least one human organ or human tissue related to and in contact with the inside of said human organ, and
   e. on the outside of the at least one human organ or human tissue related to and in contact with the outside of said human organ.
11. The medical device according to any one of the preceding embodiments, wherein the medical device chamber is adapted to communicate with at least one of;
   - the cavity inside the organ in the patient's body,
   - a portion of the medical chamber placed outside the body, and
   - a portion of the medical chamber placed outside the body, wherein a port placed in the incision of the body and connected to the wall, is adapted to be opened or closed to allow communication to the portion of the chamber placed outside the body when said port being open.
   wherein the internal sealing device is adapted to be positioned inside the body to seal the total chamber formed by the alternatives above, towards the outside thereof.
12. The medical device according to any one of the preceding embodiments, wherein the medical device is adapted to seal fluid or other bodily matter from the inside of the at least one human organ, when an incision is made in the human organ, to avoid the spreading of such fluid or other bodily matter outside the sealed space.
13. The medical device according to any one of the preceding embodiments, wherein the medical device is adapted to seal the sealed space during a surgical procedure involving at least one of;
   a. the opening of an organ with bodily matter containing bacteria,
   b. the extirpation of at least a part of the at least one organ or human tissue related to and contact with said organ,
   c. extirpation of any bodily matter from inside or outside the at least one organ,
   d. extirpation of any bodily tissue from inside or outside the at least one organ or relating to the at least one organ,
   e. connecting different parts of the at least one organ,
   f. connecting different parts of different organs,
   g. placing at least one medical device in relation to the at least one organ,
   h. replacing at least one bodily function,
   i. placing at least one medical device in relation to the at least one organ, and
   j. replacing at least one bodily function by placing at least one medical device in relation to the at least one organ.
14. The medical device according to any one of the preceding embodiments,
   wherein the medical device is adapted to receive within the medical device chamber,
   at least one of;
      a. at least one trocar,
      b. at least one inset,
      c. at least one port,
      d. at least one camera,
      e. at least one tube or channel for injecting fluid into the chamber
      f. at least one suction instrument,
      g. at least one diathermy instrument, and
      h. at least one instrument or tool, to be able to perform the surgical procedure in the sealed space.
15. The medical device according to any one of embodiments 9 - 14, wherein the internal vacuum sealing member comprises a vacuum groove adapted to create a vacuum chamber together with the human organ or human tissue.
16. The medical device according to any one of embodiments 9 - 15, wherein the internal vacuum sealing device comprises a second or more vacuum sealing members comprising at least a second vacuum groove creating a second vacuum chamber together with another part of the human body.
17. The medical device according to any one of embodiments 9 - 16, wherein at least one of the internal vacuum sealing members is a loop shaped vacuum sealing member adapted to at least one of; encircle the incision made in the at least one organ of the patient, and encircling the human organ, wherein at least one of the vacuum sealing members is adapted to be placed to seal by means of vacuum towards the at least one human organ, to create the sealed space for performing a surgical procedure when an incision has been made in the skin of the patient.
18. The medical device according to any one of embodiments 1 - 17, further comprising at least one holding member adapted to hold the medical device positioned in relation to at least one of; the incision, and the at least one organ of the patient, at least one the holding member further be adapted to at least one of;
   - be holding the medical device from inside the patient's body,
   - be holding the medical device from both the inside of the patient's body and the skin on the outside of the body,
   - be holding by clamping on both sides of at least one of; the abdominal wall, the thoraxial wall and the wall of a created body cavity, and
   - be integrated with the vacuum sealing device,
   - be holding the medical device connected to a body port placed in relation the incision in the body, and
   - be holding in different separate positions.
19. The medical device according to any one of embodiments 9 - 18, wherein the at least one internal vacuum sealing member is a circular vacuum sealing member.
20. The medical device according to any one of embodiments 9 - 19, further comprising an additional vacuum sealing member adapted to seal the incision in the body, adapted to seal against at least one of; the inside of the incision, and tissue connected to the cut part of the incision.
21. The medical device according to any one of embodiments 9 - 20, further comprising an additional vacuum sealing member adapted to seal the incision in the skin of the patient, on the outside thereof.
22. The medical device according to any one of embodiments 9 - 21, further comprises at least one of; at least one holding member, and at least one a pressure sealing member, adapted to be placed on the inside, the outside or both inside and outside of the incision in the body, the medical device further comprising an additional vacuum sealing member, adapted to seal the incision in the patient, adapted to be placed on, the inside, the outside or both inside and outside of the incision in the body.
23. The medical device according to any one of embodiments 20 - 22, wherein the l additional vacuum sealing member is a loop shaped vacuum sealing member adapted to encircle the incision made in the skin of the patient, and wherein the additional vacuum sealing member is further adapted to connect to at least one of;
   - the skin of the patient on the outside thereof, and
   - human tissue of the patient inside the body, sealing towards at least one of;
      a. a muscle fascia,
      b. the outside of a muscle fascia,
      c. the inside of a muscle fascia,
      d. the muscle fascia of the rectus abdominis in the abdominal wall,
      e. the peritoneum,
      f. fat tissue,
      g. fibrotic tissue,
      h. muscle tissue, and
      i. a body organ,
   all positioned inside the body, wherein the at least one additional vacuum sealing member is adapted to be placed to seal with vacuum towards the human tissue in close relation to the incision, when an incision has been made in the skin of the patient.
24. The medical device according to any one of the preceding embodiments, wherein the medical device comprises at least one of; a closable body port adapted to enable transfer from the outside of the patient's body to the inside of the patient's body through the incision made in the skin of the patient, and a closable wall port placed in another portion of the wall, adapted to form a portion of the chamber placed outside the incision, and wherein the closable wall port is adapted to enable transfer from the outside of the medical device to the inside of the medical device chamber through the wall of the medical device in which the wall port is mounted.
25. The medical device according to any one of the preceding embodiments, further comprising a multiport, and wherein the medical device comprises a portion of the chamber formed outside the incision adapted to change from a first to a second shape during the surgical procedure, and wherein:
   a. the medical device in the first shape allows the multiport to be positioned in close relation to the incision in the skin of the patient, to allow key hole surgery through the multiport placed in close relation to the incision in the skin,
   b. the medical device in the second shape allows the multiport to be positioned further from the incision in the skin of the patient, such that the portion of the chamber placed outside the skin is adapted to be formed with enough space to receive at least one of; a hand placed inside an inset comprising a glove, an apparatus, a non-body part, a body part, an instrument and a part used for the surgical procedure.
26. The medical device according to any one of embodiments 1 - 25, wherein the medical device is adapted to create a sealed tunnel between; the tissue in proximity to the incision in the skin of the patient in accordance with embodiments 20 - 23, and the human organ or human tissue related to and in contact with said human organ sealed towards the medical device by the internal sealing device, thereby adapted to contain operational matter inside the sealed space.
27. The medical device according to any one of the preceding embodiments, wherein the medical device is adapted to create a sealed tunnel between; a body port connected to the wall of the medical device, the body port adapted to be placed in close proximity to the incision in the skin of the patient, and the human organ or human tissue related to and in contact with said human organ sealed towards the medical device by the internal sealing device, thereby allowing the chamber to contain operational matter inside the sealed space.
28. The medical device according to any one of the preceding embodiments, wherein the multiport and medical device according to embodiment 25 comprises a two part port adapted to be disconnectably connected allowing the medical device to take the first and second shape, wherein the two parts is connected to the wall to create a portion of the sealed chamber placed outside the body.
29. The medical device according to any one of the preceding embodiments, wherein the medical device comprises at least one of; at least two closable body ports, and at least two closable multiports comprising two, three or more ports, adapted to enable transfer from the outside of the patient's body to the inside of the patient's body, through the incision made in the skin of the patient.
30. The medical device according to any one of the preceding embodiments, wherein the medical device wall is adapted to enclose a chamber in which a portion of the surgical procedure can be performed, and wherein the chamber is adapted to be in fluid connection with a cavity having both a first external portion adapted to be placed on the outside of the body and a second internal portion being adapted to be placed inside the body of the patient, wherein the medical device is adapted to have the external and internal portions of the chamber adapted to be at least one of; forming together one chamber, and allowing a closable body port adapted to be placed in close proximity to the incision of the body between the external and internal portion of the chamber, to have the chamber to be divided in two separate closed parts of the chamber.
31. The medical device according to any one of the preceding embodiments, wherein the medical device comprises a tube or channel adapted to be in fluid connection with and reaching from the outside to the inside of the body for allowing injection of an disinfection agent or injecting fluid to clean or disinfect remaining body parts inside the sealed space, when the surgical procedure is performed.
32. The medical device according to any one of the preceding embodiments, wherein the medical device is adapted to at least one of; loosen the at least one internal sealing device from the human organ or human tissue related to and in contact with said human organ, and using the at least one internal sealing device to keep the sealed space closed after the internal sealing device has been loosened.
33. The medical device according to any one of the preceding embodiments, further comprising a closing sealing device, adapted to keep the sealed space closed when finishing the surgical procedure to avoid leakage of bodily matter from the sealed space.
34. The medical device according to any one of the preceding embodiments, wherein the medical device chamber has a volume larger than 500 000 mm3.
35. The medical device according to any one of the preceding embodiments, wherein the wall comprises a closable wall port adapted to enable transfer from the ambient environment to the inside of the chamber.
36. The medical device according to any one of the preceding embodiments, wherein the wall is flexible and/or elastic.
37. The medical device according to any one of the preceding embodiments, wherein the wall comprises at least one integrated glove or inset comprising an integrated glove enabling manual manipulation within the chamber of the medical device and/or inside the cavity of the patient.
38. The medical device according to any one of the preceding embodiments, further comprising a coupling adapted to connect an inset or port to the medical device.
39. The medical device according to embodiment 38, wherein the coupling is placed in at least one of: the wall of the medical device, and the incision made in the patient's skin.
40. The medical device according to any one of the preceding embodiments, further comprising an inset selected from:
   a. a glove inset,
   b. a hand access inset,
   c. a port inset,
   d. a multiport inset, and
   e. a gel port inset.
41. The medical device according to any one of the preceding embodiments, comprising at least one of; a closable wall port or wall multiport, placed in the wall, and a closable body port or body multiport adapted to be placed in or in close relation to the incision of the patient, wherein the closable wall port or wall multiport and the closable body port or body multiport comprises connections adapted to be connecting the closable wall port or wall multiport to the closable body port or body multiport, and wherein the multiport comprise two, three, four or more or more ports placed in one multiport casing.
42. The medical device according to embodiment 41, wherein the closable body port comprises a first penetrable self sealing gel and the closable wall port comprises a second penetrable self sealing gel, and wherein the first and second penetrable self sealing gels are adapted to be placed tightly together by the wall and body ports being connected, such that they act as a single penetrable self sealing gel adapted to, in a non-compressed state, provide sealing, and in a compressed state, enable a hand or an object to be inserted through the self sealing gel while maintaining the seal.
43. The medical device according to any one of the preceding embodiments, wherein the wall forming the chamber is adapted to hold a pressure within the chamber exceeding atmospheric pressure.
44. The medical device according to any one of the preceding embodiments, wherein the first and second internal sealing device comprises loop shaped vacuum sealing members adapted to at least one of; encircle the incision made, encircle two or more positions on or around the at least one organ of the patient, and encircling two or more human organs to enclose a larger surgical area in the sealed space,
   wherein the at least one vacuum sealing member is adapted to be placed to seal by means of vacuum towards the at least one human organ or related tissue, when an incision has been made in the organ of the patient.
45. The medical device according to embodiment 33, wherein the closing sealing device, is adapted to seal with vacuum to keep the sealed space closed when finishing the surgical procedure to avoid leakage of bodily matter from the sealed space.
46. The medical device according to any one of the preceding embodiments, comprising at least one fluid conduit leading from outside the body to inside the body, inside the chamber, adapted to allow sucking and injecting fluid into the chamber from outside the body, wherein the at least one fluid conduit is adapted to be at least one of;
   integrated in at least one of the medical device and the wall or body port placed in close proximity to the incision, and
   placed as a separate tube having the two ends of the fluid conduit displaced as a separate tube and there in between adapted to be integrated in at least one of the medical device and the wall or body port placed in close proximity to the incision.
47. The medical device according to any one of the preceding embodiments, wherein the medical device chamber is adapted to encompass part of the surgical procedure to be performed, wherein the medical device further comprises;
   - a closeable wall multiport (17) placed in a portion of the wall,
   - a closeable body multiport (22) placed in a second portion of the wall and adapted to be placed in or closely related to an incision in the patient's body, and
   - a sealing device (05) is adapted to be at least one of;
      ∘ sealingly connected to at least one of; the wall and the closable body port, and
      ∘ an integrated part of at least one of; the wall and the closable body port,

   wherein the sealing device further being adapted to seal against at least one of the skin and tissue of the patient,
   wherein the wall, wall multiport and body multiport are adapted to together form an airlock sluice (18) between a cavity in the body of the patient and the ambient environment (18), when the surgical procedure is performed.
48. The medical device according to any one of embodiments 46 - 47, wherein the wall port is displaceable between a first position and a second position, wherein the second position is situated more remote from the body port.
49. The medical device according to any one of the embodiments 46 - 48, wherein the wall multiport is adapted to be at least one of:
   a. connected to the body multiport,
   b. abutting the body multiport,
   c. locked to the body multiport,
   d. formed into an integrated port together with the body multiport.
50. The medical device according to any one of the embodiments 46 - 49, wherein at least one of; the closable wall multiport and closable body multiport comprises a penetratable self sealing gel, allowing an object or hand to be inserted through the self sealing gel.
51. The medical device according to any one of the preceding embodiments, further comprising at least one of; an additional body port, an additional body multiport, an additional body inset, an additional wall port, an additional wall multiport and an additional wall inset, wherein at least one of a wall port, a wall multiport, a body port, a body multiport, a wall inset and a body inset is exchangeable by at least one of; the additional body port, the additional body multiport, the additional body inset, the additional wall port, the additional wall multiport and the additional wall inset.
52. The medical device according to any one of the preceding embodiments, comprising a sealing member adapted to seal the wall towards human tissue according to embodiment 23, comprising at least one of; a vacuum sealing member (05') adapted to hold a pressure less than atmospheric pressure, and a pressure sealing member (05") adapted to hold a pressure above atmospheric pressure.
53. The medical device according to any one of the preceding embodiments, including the closable body port and closable multiport according to embodiment 24, wherein at least one of;
   - the closable body port comprises a closable body multi-port adapted to be placed from inside the chamber in an incision to be performed in the skin of the patient, and wherein the body multiport is adapted to be connected to at least one of;
      ∘ the wall port, and
      ∘ a wall multiport, when the medical device is applied on the patient's body for performing a surgical procedure, and
   - the closable wall port comprises a closable wall multiport, wherein the wall multiport is adapted to be connected to at least one of;
      ∘ the body port, and
      ∘ a body multiport placed in an incision in the patient's body, when the medical device is applied on the patient's body for performing a surgical procedure.
54. The medical device according to any one of the preceding embodiments, wherein the wall is a flexible wall and the medical device comprises a first inset (42;42';42"), or an interconnectable port (47) placed in the wall, the interconnectable port comprising;
   - a first port or multiport(17;22),
   - a second coupling adapted be interconnected with at least one of; the first port to form the interconnectable port, and the first inset, wherein the second coupling is disconnectable from the first port or first inset, and

   wherein at least one of:
      the first port or multiport is connected to a first portion of the flexible wall (01), and
      the first inset is connected to a third portion of the flexible wall (01),
   wherein the second coupling is connected to a second portion of the flexible wall,
   the flexible wall is adapted to form a chamber (C) together with the second coupling and at least one of; the first port, and first inset,
   the second coupling adapted to allow a closable opening,
   the chamber is adapted to allow communication through the coupling into a cavity inside the patient's body, after an incision has been made in the skin of the patient, when a surgical procedure is performed,
   at least one of; the first port or multiport and first inset, in a first position, is adapted to connect to the second coupling in close relation to the patients skin, wherein the chamber having a first smaller volume, and
   at least one of; the first port or multiport and first inset, in a second position is adapted to be disconnected from the second coupling, being separated from the coupling in a more remote position in relation to the patients skin, wherein the chamber having a second larger volume.
55. The medical device according to embodiment 54, wherein the first port (17) forms a closable wall port or closable multiport when disconnected from the second coupling.
56. The medical device according to any one of embodiments 54 and 55, wherein the second coupling forms a closable body port by itself when the first port or multiport has been disconnected from the second coupling.
57. The medical device according to any one of embodiments 54 - 56, wherein the first port comprises a multiport, comprising two, three, four or more individual ports.
58. The medical device according to any one of embodiments 54 - 57 wherein the first inset (42;42';42") comprises at least one of: a surgical glove (02) and a device or instrument mount (20), for assisting in the surgical procedure.
59. The medical device according to any one of embodiments 54 - 58, comprising at least one first coupling (80a), and wherein at least one of; the first port or multiport and first inset is connected to the first coupling, and at least one of; the first port or multiport and first inset is indirectly connected to the second coupling, being adapted to be directly connected to the first coupling, wherein the first coupling is adapted to be reversibly directly connected to the second coupling.
60. The medical device according to embodiment 59, wherein: at least one of the first and second coupling comprises a protruding member (33), and at least one of the first and second coupling comprises a recess (34), and wherein, the protruding member is adapted to engage the recess for locking the first coupling to the second coupling.
61. The medical device according to embodiment 60, wherein the recess is a groove in at least one of the first and second coupling.
62. The medical device according to any one of the embodiments 59 - 61, wherein the interconnectable port is adapted to:
   a. in a first state, when the first port or the first coupling is adapted to be interconnected with the second coupling, enable a surgeon to operate using endoscopic surgery through the interconnected interconnectable port, and
   b. in a second state, when the second coupling is disconnected from the first port or first coupling enable the surgeon to perform steps of the procedure within the chamber formed by the flexible wall between the first port and second coupling of the interconnectable port.
63. The medical device according to embodiment 62, wherein the interconnectable port is adapted to, in the second state, enable the surgeon to remove a specimen (81) from the body of the patient through the second coupling and into the chamber, and to perform one of: placing the specimen within the chamber, and removing the specimen from the chamber through the first port.
64. The medical device according to any one of embodiments 59 - 63, comprising a second body port adapted to also be placed in the second coupling, wherein at least one of the first port, the second body port and the first coupling are adapted to be reconnected to the second coupling after having been disconnected from the second coupling, such that the surgeon can continue operating through the interconnected port after the specimen has been removed.
65. The medical device according to any one of the preceding embodiments, adapted to have the chamber connected to a pressurized fluid supply such that the medical device can receive pressurized fluid and at least one of; hold the same pressure as a pressurized fluid filling a body cavity outside the chamber, hold a lower pressure than a pressurized fluid filling a body cavity outside the chamber, and hold a higher pressure than a pressurized fluid filling a body cavity outside the chamber.
66. The medical device according to embodiment 65, comprising at least one of; a nipple for fluid supply placed outside the body and a nipple for fluid supply placed outside the body in combination with a second nipple adapted to receive flow of fluid leaving the chamber to allow circulation of fluid.
67. The medical device according to any one of the preceding embodiments, wherein the medical device comprises at least one of:
   a. a filtering unit (26b) for filtering fluid supplied to the chamber,
   b. a sterilization unit (26a) for directly or indirectly sterilizing fluid supplied to the chamber,
   c. a fluid tempering unit (26c) adapted change the temperature of fluid supplied to the chamber,
   d. a filtering unit (26b) for filtering fluid and a sterilization unit (26a) for directly or indirectly sterilizing fluid supplied to the chamber,
   e. a filtering unit (26b) for filtering fluid supplied to the chamber and a fluid tempering unit (26c) adapted change the temperature of fluid supplied to the chamber,
   f. a sterilization unit (26a) for directly or indirectly sterilizing fluid supplied to the chamber and a fluid tempering unit (26c) adapted change the temperature of fluid supplied to the chamber, and
   g. a filtering unit (26b) for filtering fluid supplied to the chamber, a sterilization unit (26a) for directly or indirectly sterilizing fluid supplied to the chamber and a fluid tempering unit (26c) adapted change the temperature of fluid supplied to the chamber.
68. The medical device according to any one of the preceding embodiments, wherein, when the medical device is applied on the patient's body for performing a surgical procedure, the medical device further comprises at least one of:
   a. at least one inlet (30) provided in at least one of:
      i. the wall of the medical device, and
      ii. the skin of the patient for supplying a fluid to at least one of
         - the chamber, and
         - the cavity in the patient's body, and
   b. at least one outlet (31) provided in at least one of:
      iii. the wall of the medical device, and
      iv. the skin of the patient for discharging the fluid from at least one of:
         - the chamber, and
         - the body cavity.
69. The medical device according to embodiment 68, further comprising a pump (27) adapted to circulate the fluid from the outlet to the inlet.
70. The medical device according to any one of the preceding embodiments, wherein the wall comprises a pleated portion such that the flexible wall can be expanded.
71. The medical device according to any one of the preceding embodiments, further comprising a sensor.
72. The medical device according to embodiment 71, wherein the sensor is adapted to sense at least one of:
   a. the blood flow of the patient,
   b. the saturation of the blood of the patient,
   c. an ischemia marker of the patient,
   d. the temperature at the skin of the patient, and
   e. the patient's skin tone,
   f. a pressure,
   g. a force,
   h. a time,
   i. a movement,
   j. a stretching,
   k. a distance, and
   l. a volume.
73. The medical device according to embodiment 71, wherein the sensor is adapted to sense at least one of:
   a. the pressure in the sealing device,
   b. the pressure in the chamber, and
   c. the direct or indirect leakage of fluid from the chamber
   d. a force,
   e. a time,
   f. a movement,
   g. a stretching,
   h. a distance,
   i. a volume,
   j. an electrical parameter,
   k. energy,
   l. energy balance,
   m. voltage,
   n. current, and
   o. temperature.

### Numbered embodiment B 1-13:

1. A method of creating a sealed chamber at least partially inside of the body of a patient, the method comprising:
   - making an incision in the skin of the body of the patient,
   - inserting, through the incision, a first internal sealing device comprising a first internal sealing member, the first internal sealing device being connected to a tubular wall,
   - positioning the first internal sealing member in connection with at least a part of at least one human organ or human tissue, such that a first seal is created between the medical device and at least the part of the at least one human organ or human tissue,
   - inserting, through the incision, a second internal sealing device comprising a second internal sealing member, the second internal sealing device being connected to a tubular wall,
   - positioning the second internal sealing member in connection with at least a part of at least one human organ or human tissue, such that a second seal is created between the medical device and at least the part of the at least one human organ or human tissue, such that a medical device chamber is created sealingly between the first and second internal sealing devices and the tubular wall.
2. The method according to embodiment 1, further comprising at least one of the steps of:
   - inserting at least one trocar through the incision in the skin of the patient,
   - inserting at least one dissecting tool into the trocar, and
   - dissecting an area of the organ or tissue.
3. The method according to any one of embodiments 1 and 2, further comprising the step of placing a body port in the incision in the skin of the patient, and wherein the tubular wall is connected to the body port, such that the sealed chamber is created between the tubular wall, the first internal sealing device, the second internal sealing device and the body port.
4. The method according to any one of embodiments 1-3, wherein the step of positioning the first and second internal sealing devices comprises positioning the first and second sealing device such that they each encircle a tubular organ.
5. The method according to embodiment 4, wherein the step of positioning the first and second internal sealing devices comprises the steps of positioning the first sealing device encircling an intestine of the patient and positioning the second internal sealing device encircling the esophagus of the patient, such that the wall encapsulates the stomach of the patient, such that the stomach of the patient is placed in a sealed chamber between the first sealing member, the second sealing member, and the wall.
6. The method according to any one of embodiments 1 - 5, wherein the wall comprises a closing portion adapted to be opened and closed for enclosing a human organ or human tissue in the medical device chamber, and wherein the method comprises the steps of:
   - opening the wall the encircle an organ of the patient, and
   - closing the wall to encapsulate the organ.
7. The method according to any one of embodiments 1-6, wherein the internal sealing member is an internal vacuum sealing member, and wherein the method further comprises the step of applying a vacuum to the vacuum sealing member such that the vacuum sealing member seals by the applied vacuum.
8. The method according to any one of embodiments 1-6, wherein the internal sealing member is an internal pressure sealing member, and wherein the method further comprises the step of applying a pressure to the pressure sealing member such that the pressure sealing member seals by means of the applied pressure.
9. The method according to embodiment 6, wherein the closing portion comprises a zipper, and wherein the step of closing the wall to encapsulate the organ comprises closing the zipper.
10. The method according to any one of embodiments 1 - 9, further comprising the step of filling the chamber with a pressurized fluid.
11. The method according to any one of embodiments 1 - 10, further comprising at least one of the following surgical procedures performed within the sealed chamber;
   a. the opening of an organ with bodily matter containing bacteria,
   b. the extirpation of at least a part of the at least one organ or human tissue related to and contact with said organ,
   c. extirpation of any bodily matter from inside or outside the at least one organ,
   d. extirpation of any bodily tissue from inside or outside the at least one organ or relating to the at least one organ,
   e. connecting different parts of the at least one organ, connecting different parts of different organs,
   f. placing at least one medical device in relation to the at least one organ, replacing at least one bodily function,
   g. placing at least one medical device in relation to the at least one organ and replacing at least one bodily function by placing at least one medical device in relation to the at least one organ,
   h. appendectomy,
   i. cholecystectomy,
   j. nephrectomy,
   k. hysterectomy,
   l. oophorectomy,
   m. adrenalectomy,
   n. gastric bypass,
   o. Nissen fundoplication,
   p. intestinal shunts,
   q. hernia repair,
   r. splenectomy,
   s. colon or small intestine resection,
   t. liver resection,
   u. cecostomy,
   v. colostomy,
   w. duodenostomy,
   x. ileostomy,
   y. appendicostomy,
   z. esophagostomy,
   aa. gastrostomy,
   bb. urostomy,
   cc. nephrostomy,
   dd. ureterostomy,
   ee. vesicostomy,
   ff. endoscopic surgery,
   gg. open surgery,
   hh. combined open and endoscopic surgery,
   ii. arthroscopic surgery,
   jj. open joint surgery,
   kk. combined open joint and arthroscopic surgery,
   ll. cryoablation,
   mm. ultrasound knife surgery,
   nn. removal of separated bone or cartilage tissue,
   oo. the insertion of ligaments,
   pp. the insertion of fixation elements such as screws, nails, and plates,
   qq. prosthetic joint replacement,
   rr. lubrication procedures,
   ss. cancer surgery,
   tt. insertion of another medical device,
   uu. insertion of foreign parts, and
   vv. insertion of a medical device and other foreign parts using combined open and laparoscopic or arthroscopic surgery.
12. The method according to any one of embodiments 1 - 11, wherein the method comprises placing at least one of the following other implants into the medical device chamber;
   an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,
13. The method according to any one of embodiments 1 - 12, wherein the method includes placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

### Numbered embodiment C 1 - 43:

1. A medical device adapted to be positioned inside a patient's body via an incision made in the skin of the patient, the medical device comprising;
   - an internal sealing device, comprising an internal sealing member adapted to be positioned in connection to and seal against at least a part of at least one human organ or human tissue related to and in contact with said human organ, for creating a seal between the medical device and at least the part of the at least one human organ or human tissue related to and in contact with said human organ, and
   - a tubular wall adapted to create a medical device chamber sealingly connected to the internal sealing device, such that a portion of at least one human organ or tissue is placed inside of the medical device chamber, wherein
   - the internal sealing member has an adjustable circumference, for enclosing or encircling a portion of the at least one human organ or human tissue.
2. The medical device according to embodiment 1, further comprising a body port placed in an incision in the skin of the patient, and wherein the wall is connected to the body port, such that a portion of at least one human organ is placed inside of the medical device chamber created between the first internal sealing member, the second internal sealing member and the body port.
3. The medical device according to embodiment 1, further comprising a second internal sealing device comprising a second internal sealing member adapted to be positioned in connection to and seal against at least a part of at least one human organ or human tissue, for creating a seal between the medical device and at least the part of the at least one human organ or human tissue, wherein the wall is further connected to the second internal sealing member, such that a portion of at least one human organ is placed inside of the medical device chamber between the first and second internal sealing members.
4. The medical device according to embodiment 3, wherein the second sealing member has an adjustable circumference for enclosing or encircling a portion of the at least one human organ or tissue.
5. The medical device according to any one of embodiments 1-4, wherein at least one of the first and second internal sealing members is circular and has an adjustable diameter, such that the circumference is adjusted by adjustment of the diameter.
6. The medical device according to embodiment 1, wherein the internal sealing device comprises at least one of;
   a. an internal vacuum sealing device, sealing by using a vacuum towards the human organ or human tissue related to and in contact with said human organ,
   b. an internal pressure sealing device, sealing using a pressure towards the human organ or human tissue related to and in contact with said human organ, and
   c. an internal adhesive sealing device, sealing by adhesively contacting the human organ or human tissue related to and in contact with said human organ.
7. The medical device according to embodiment 6, wherein the internal sealing member comprises at least one sealing surface adapted to connect to and seal against a tissue portion of the patient, wherein the sealing surface is placed in the front of the internal sealing device, such that the internal sealing device can seal axially against a surface of a tissue.
8. The medical device according to embodiment 6, wherein the internal sealing member comprises at least one sealing surface adapted to connect to and seal against a tissue portion of the patient, wherein the sealing surface is placed on the inner circumference of the internal sealing device, such that the internal sealing device can seal radially and thus against the outer circumference of a tubular organ or tissue.
9. The medical device according to any one of embodiments 1 - 8, wherein at least one of the internal sealing members comprises a pleated portion, such that the circumference is adjustable by the adjustment of the pleated portion.
10. The medical device according to embodiment 6, wherein the internal sealing device is an internal vacuum sealing device comprising a pleated vacuum conduit comprising perforations adapted to generate suction against the tissue of the patient, such that a sealing is created between the pleated vacuum conduit and the organ or tissue of the patient.
11. The medical device according to embodiment 10, further comprising a vacuum conduit adapted to connect the pleated vacuum conduit to a vacuum source outside the body of the patient.
12. The medical device according to any one of embodiments 10 and 11, wherein the perforations are placed in the front end of the internal sealing device, such that the internal sealing device can seal against a planar surface of a tissue.
13. The medical device according to any one of embodiments 10 and 11, wherein the perforations are placed on the inner circumference of the pleated vacuum conduit, such that the internal sealing device can seal radially and thus against the outer circumference of a tubular organ or tissue.
14. The medical device according to any one of embodiments 1 - 13, wherein the internal sealing device is adapted to be positioned, at least one of;
   a. on the human organ,
   b. on human tissue related to and in contact with said human organ,
   c. in an incision made in the at least one human organ of the patient,
   d. on the inside of the at least one human organ or human tissue related to and in contact with the inside of said human organ, and
   e. on the outside of the at least one human organ or human tissue related to and in contact with the outside of said human organ.
15. The medical device according to any one of embodiments 1 - 14, wherein the medical device chamber is adapted to communicate with at least one of;
   - the cavity inside the organ in the patient's body,
   - a portion of the medical chamber placed outside the body, and
   - a portion of the medical chamber placed outside the body, wherein a port placed in the incision of the body and connected to the wall, is adapted to be opened or closed to allow communication to the portion of the chamber placed outside the body when said port being open.
   wherein the internal sealing device is adapted to be positioned inside the body to seal the total chamber formed by the alternatives above, towards the outside thereof.
16. The medical device according to any one of embodiments 1 - 15, wherein the medical device is adapted to seal fluid or other bodily matter from the inside of the at least one human organ, when an incision is made in the human organ, to avoid the spreading of such fluid or other bodily matter outside the sealed space.
17. The medical device according to any one of embodiments 6 - 16, wherein the internal vacuum sealing member comprises a vacuum groove adapted to create a vacuum chamber together with the human organ or human tissue.
18. The medical device according to any one of embodiments 6 - 17, wherein the internal vacuum sealing device comprises a second or more vacuum sealing members comprising at least a second vacuum groove creating a second vacuum chamber together with another part of the human body.
19. The medical device according to any one of embodiments 6 - 18, further comprising an additional vacuum sealing member adapted to seal against at least one of; the inside of the incision, and tissue connected to the cut part of the incision.
20. The medical device according to any one of embodiments 6 - 19, further comprising an additional vacuum sealing member adapted to seal the incision in the skin of the patient, on the outside thereof.
21. The medical device according to embodiment 20, wherein the additional vacuum sealing member is a loop shaped vacuum sealing member adapted to encircle the incision made in the skin of the patient, and wherein the additional vacuum sealing member is further adapted to connect to at least one of;
   - the skin of the patient on the outside thereof, and
   - human tissue of the patient inside the body, sealing towards at least one of;
      a. a muscle fascia,
      b. the outside of a muscle fascia,
      c. the inside of a muscle fascia,
      d. the muscle fascia of the rectus abdominis in the abdominal wall,
      e. the peritoneum,
      f. fat tissue,
      g. fibrotic tissue,
      h. muscle tissue, and
      i. a body organ,
   all positioned inside the body, wherein the at least one additional vacuum sealing member is adapted to be placed to seal with vacuum towards the human tissue in close relation to the incision, when an incision has been made in the skin of the patient.
22. The medical device according to any one of embodiments 1 - 21, wherein the medical device comprises at least one of; a closable body port adapted to enable transfer from the outside of the patient's body to the inside of the patient's body through the incision made in the skin of the patient, and a closable wall port placed in another portion of the wall, adapted to form a portion of the chamber placed outside the incision, and wherein the closable wall port is adapted to enable transfer from the outside of the medical device to the inside of the medical device chamber through the wall of the medical device in which the wall port is mounted.
23. The medical device according to any one of embodiment 22, wherein the closeable wall or body port comprises a two part port adapted to be disconnectably connected allowing the medical device to take the first and second shape, wherein the two parts are connected to the wall for creating a portion of the sealed chamber outside of the body.
24. The medical device according to any one of the preceding embodiments, wherein the wall is adapted to create a medical device chamber having a volume larger than 500 000 mm3.
25. The medical device according to any one of the preceding embodiments, wherein the wall is flexible and/or elastic.
26. The medical device according to any one of the preceding embodiments, wherein the wall comprises at least one integrated glove or inset comprising an integrated glove enabling manual manipulation within the chamber of the medical device and/or inside the cavity of the patient.
27. The medical device according to any one of the preceding embodiments, further comprising a coupling adapted to connect an inset or port to the medical device.
28. The medical device according to embodiment 27, wherein the coupling is placed in at least one of: the wall of the medical device, and the incision made in the patient's skin.
29. The medical device according to embodiment 26, further comprising an inset selected from:
   a. a glove inset,
   b. a hand access inset,
   c. a port inset,
   d. a multiport inset, and
   e. a gel port inset.
30. The medical device according to any one of the preceding embodiments, further comprising a closable body port comprising a first penetrable self sealing gel and the closable wall port comprising a second penetrable self sealing gel, and wherein the first and second penetrable self sealing gels are adapted to be placed tightly together by the wall and body ports being connected, such that they act as a single penetrable self sealing gel adapted to, in a non-compressed state, provide sealing, and in a compressed state enable a hand or an object to be inserted through the self sealing gel while maintaining the seal.
31. The medical device according to any one of the preceding embodiments, wherein the wall forming the chamber is adapted to hold a pressure within the chamber exceeding atmospheric pressure.
32. The medical device according to any one of embodiments 22 - 31, wherein the wall port is displaceable between a first position and a second position, wherein the second position is situated more remote from the body port.
33. The medical device according to any one of embodiments 22 - 31, wherein at least one of; the closable wall port and closable body port comprises at least one of an elastic membrane and a flexible membrane, and wherein the membrane is adapted to, in a non-compressed state, seal between the chamber and the environment outside the chamber, and in a compressed state enable a hand or an object to be inserted through the membrane.
34. The medical device according to any one of the preceding embodiments, wherein the body port comprises a first sealing member (35) adapted to be positioned at the inside of the patient's skin around an incision to be performed in the skin and a second sealing member (37) adapted to be positioned at outside of the patient's skin around the incision, and a spring-loaded or elastic connecting member (36) sealingly interconnecting the first and second sealing members, whereby the spring-loaded or elastic connecting member is adapted to seal between at least one of: (a) the second sealing member and the skin of the patient, (b) the first sealing member and tissue of the patient, and c) the connecting member and tissue in relation to the incision.
35. The medical device according to any one of the preceding embodiments, wherein the medical device further comprises at least one of:
   a. a filtering unit (26b) for filtering fluid supplied to the chamber,
   b. a sterilization unit (26a) for directly or indirectly sterilizing fluid supplied to the chamber,
   c. a fluid tempering unit (26c) adapted change the temperature of fluid supplied to the chamber,
   d. a filtering unit (26b) for filtering fluid and a sterilization unit (26a) for directly or indirectly sterilizing fluid supplied to the chamber,
   e. a filtering unit (26b) for filtering fluid supplied to the chamber and a fluid tempering unit (26c) adapted change the temperature of fluid supplied to the chamber,
   f. a sterilization unit (26a) for directly or indirectly sterilizing fluid supplied to the chamber and a fluid tempering unit (26c) adapted change the temperature of fluid supplied to the chamber, and
   g. a filtering unit (26b) for filtering fluid supplied to the chamber, a sterilization unit (26a) for directly or indirectly sterilizing fluid supplied to the chamber and a fluid tempering unit (26c) adapted change the temperature of fluid supplied to the chamber.
36. The medical device according to any one of the preceding embodiments, wherein, when the medical device is applied on the patient's body for performing a surgical procedure, the medical device further comprises at least one of:
   a. at least one inlet (30) provided in at least one of:
      i. the wall of the medical device, and
      ii. the skin of the patient for supplying a fluid to at least one of
         - the chamber, and
         - the cavity in the patient's body, and
   b. at least one outlet (31) provided in at least one of:
      i. the wall of the medical device, and
      ii. the skin of the patient for discharging the fluid from at least one of:
         - the chamber, and
         - the body cavity.
37. The medical device according to embodiment 36, further comprising a pump (27) adapted to circulate the fluid from the outlet to the inlet.
38. The medical device according to any one of embodiments 22 - 37, wherein:
   a. at least one of the wall and body port comprises a protruding member, and
   b. at least one of the wall and body port comprises a recess, and wherein,
   the protruding member is adapted to engage the recess for locking the wall port to the body port.
39. The medical device according to embodiment 38, wherein the recess is a groove in at least one of the wall port and body port.
40. The medical device according to any one of embodiments 22 - 39, wherein the wall port is adapted to be fixated to a first coupling (80a) and the body port is adapted to be fixated to a second coupling (80a), and wherein the first and second couplings are adapted to be connected for connecting the wall port and body port.
41. The medical device according to any one of embodiments 25 - 40, wherein the flexible wall comprises a pleated portion (03) such that the flexible wall can be expanded.
42. The medical device according to any one of embodiment 1 - 41, further comprising at least one sensor adapted to sense at least one of; a pressure, flow and a physiological or physical parameter of the patient.
43. The medical device according to embodiment 42, wherein the physiological or physical parameter is a parameter selected from:
   a. the blood flow of the patient,
   b. the saturation of the blood of the patient,
   c. an ischemia marker of the patient,
   d. the temperature at the skin of the patient, and
   e. the patient's skin tone,
   f. a pressure,
   g. a force,
   h. a time,
   i. a movement,
   j. a stretching,
   k. a distance, and
   l. a volume.

### Numbered embodiment D 1 - 14:

1. A method of creating a sealed chamber at least partially inside of the body of a patient, the method comprising:
   - making an incision in the skin of the body of the patient,
   - inserting, through the incision, an internal sealing device comprising an internal sealing member, the internal sealing device being connected to a tubular wall,
   - positioning the internal sealing member in connection with at least a part of at least one human organ or human tissue, such that a seal is created between the medical device and at least the part of the at least one human organ or human tissue, and
   - adjusting the circumference of the sealing member for enclosing or encircling a portion of the at least one human organ or human tissue, such that a sealed chamber is created by the tubular wall of the medical device, the internal sealing device, and the organ or tissue.
2. The method according to embodiment 1, further comprising at least one of the steps of:
   - inserting at least one trocar through the incision in the skin of the patient,
   - inserting at least one dissecting tool into the trocar, and
   - dissecting an area of the organ or tissue.
3. The method according to any one of embodiments 1 and 2, further comprising the step of placing a body port in the incision in the skin of the patient, and wherein the tubular wall is connected to the body port, such that the sealed chamber is created between the tubular wall, the internal sealing device and the body port.
4. The method according to any one of embodiments 1-3, further comprising the steps of:
   - inserting, through the incision, a second internal sealing device comprising a second internal sealing member, the second internal sealing device also being connected to the tubular wall, and
   - positioning the second internal sealing member in connection with at least a second part of the at least one human organ or human tissue, such that a seal is created between the second medical device and at least the part of the at least one human organ or human tissue, such that a sealed chamber is created by the tubular wall of the medical device, the first and second internal sealing devices, and the organ or tissue.
5. The method according to embodiment 4, further comprising the step of adjusting the circumference of the second sealing member for enclosing or encircling a portion of the at least one human organ.
6. The method according to any one of embodiments 1 - 5, wherein at least one of the first and second internal sealing members is circular and has an adjustable diameter, such that the circumference is adjusted by adjustment of the diameter.
7. The method according to any one of embodiments 1-6, wherein the internal sealing member is an internal vacuum sealing member, and wherein the method further comprises the step of applying a vacuum to the vacuum sealing member such that the vacuum sealing member seals by the applied vacuum.
8. The method according to any one of embodiments 1-6, wherein the internal sealing member is an internal pressure sealing member, and wherein the method further comprises the step of applying a pressure to the pressure sealing member such that the pressure sealing member seals by means of the applied pressure.
9. The method according to any one of embodiments 1 - 8, wherein the internal sealing member comprises at least one sealing surface adapted to connect to and seal against a tissue portion of the patient, wherein the sealing surface is placed in the front of the internal sealing device, and wherein the step of positioning the internal sealing member in connection with at least a part of at least one human organ or human tissue further comprises applying the internal sealing member axially against a surface of an organ or tissue.
10. The method according to any one of embodiments 1 - 8, wherein the internal sealing member comprises at least one sealing surface adapted to connect to and seal against a tissue portion of the patient, wherein the sealing surface is placed on the inner circumference of the internal sealing device, and wherein the step of positioning the internal sealing member in connection with at least a part of at least one human organ or human tissue further comprises applying the internal sealing member radially axially against the outer circumference of a tubular organ or tissue.
11. The method according to any one of embodiments 1 - 10, further comprising the step of filling the chamber with a pressurized fluid.
12. The method according to any one of embodiments 1 - 11, further comprising at least one of the following surgical procedures performed within the sealed chamber;
   a. the opening of an organ with bodily matter containing bacteria,
   b. the extirpation of at least a part of the at least one organ or human tissue related to and contact with said organ,
   c. extirpation of any bodily matter from inside or outside the at least one organ,
   d. extirpation of any bodily tissue from inside or outside the at least one organ or relating to the at least one organ,
   e. connecting different parts of the at least one organ, connecting different parts of different organs,
   f. placing at least one medical device in relation to the at least one organ, replacing at least one bodily function,
   g. placing at least one medical device in relation to the at least one organ and replacing at least one bodily function by placing at least one medical device in relation to the at least one organ,
   h. appendectomy,
   i. cholecystectomy,
   j. nephrectomy,
   k. hysterectomy,
   l. oophorectomy,
   m. adrenalectomy,
   n. gastric bypass,
   o. Nissen fundoplication,
   p. intestinal shunts,
   q. hernia repair,
   r. splenectomy,
   s. colon or small intestine resection,
   t. liver resection,
   u. cecostomy,
   v. colostomy,
   w. duodenostomy,
   x. ileostomy,
   y. appendicostomy,
   z. esophagostomy,
   aa. gastrostomy,
   bb. urostomy,
   cc. nephrostomy,
   dd. ureterostomy,
   ee. vesicostomy,
   ff. endoscopic surgery,
   gg. open surgery,
   hh. combined open and endoscopic surgery,
   ii. arthroscopic surgery,
   jj. open joint surgery,
   kk. combined open joint and arthroscopic surgery,
   ll. cryoablation,
   mm. ultrasound knife surgery,
   nn. removal of separated bone or cartilage tissue,
   oo. the insertion of ligaments,
   pp. the insertion of fixation elements such as screws, nails, and plates,
   qq. prosthetic joint replacement,
   rr. lubrication procedures,
   ss. cancer surgery,
   tt. insertion of another medical device,
   uu. insertion of foreign parts, and
   vv. insertion of a medical device and other foreign parts using combined open and laparoscopic or arthroscopic surgery.
13. The method according to any one of embodiments 1 - 12, wherein the method comprises placing at least one of the following other implants into the medical device chamber;
   an arthroplastic prosthesis, a heart assisting device, an energized implant, a control logic, a filling esthetical implant, an implantable medicament dispenser, a powering unit, a vascular implant, an urological implant, an abdominal implant, a drug-releasing implant, a gynecological implant, an active and a passive medical implant,
14. The method according to any one of embodiments 1 - 13, wherein the method includes placing at least one of the following instruments into the medical device chamber; a scalpel, a trocar, a tweezer, a suturing instrument, a scissor, a camera, a clamping instrument, a dissecting instrument, a gripping instrument, a bonding instrument, a suturing or stapling instrument, a severing instrument, an electromagnetic or ultrasonic diathermy, a laparoscopic grasper, a laparoscopic claw grasper, a laparoscopic stone grasper, a laparoscopic needle holder, a laparoscopic hook, a laparoscopic dissector, a laparoscopic diathermy instrument, an arthroscopy instrument, a joint replacement instrument, an orthopedic type of instrument, an undefined instrument, a surgical instrument, an endoscopic instrument, and a machine.

The different aspects or any part of an aspect of the different numbered embodiments or any part of an embodiment may all be combined in any possible way. Any method embodiment or any step of any method embodiment may be seen also as an apparatus description, as well as, any apparatus embodiment, aspect or part of aspect or part of embodiment may be seen as a method description and all may be combined in any possible way down to the smallest detail. Any detailed description should be interpreted in its broadest outline as a general summary description.

## Claims

1. A medical device adapted to be positioned inside a patient's body via an incision made in the skin of the patient, the medical device comprising;
- an internal sealing device, comprising an internal sealing member (2605oa) adapted to be positioned in connection to and seal against at least a part of at least one human organ or human tissue related to and in contact with said human organ, for creating a seal between the medical device and at least the part of the at least one human organ or human tissue related to and in contact with said human organ, and
- a tubular wall (2601) adapted to create a medical device chamber (C) sealingly connected to the internal sealing device, such that a portion of at least one human organ or tissue is placed inside of the medical device chamber (C), wherein
- the internal sealing member (2605oa) has an adjustable circumference, for enclosing or encircling a portion of the at least one human organ or human tissue.

2. The medical device according to claim 1, further comprising a body port (2648) placed in an incision in the skin (S) of the patient, and wherein the wall is connected to the body port, such that a portion of at least one human organ is placed inside of the medical device chamber created between the first internal sealing member and the body port.

3. The medical device according to claim 1, further comprising a second internal sealing device comprising a second internal sealing member (2605oa") adapted to be positioned in connection to and seal against at least a part of at least one human organ or human tissue, for creating a seal between the medical device and at least the part of the at least one human organ or human tissue, wherein the wall (2601') is further connected to the second internal sealing member, such that a portion of at least one human organ is placed inside of the medical device chamber between the first and second internal sealing members.

4. The medical device according to claim 3, wherein the second sealing member (2605oa") has an adjustable circumference for enclosing or encircling a portion of the at least one human organ or tissue.

5. The medical device according to any one of claims 1-4, wherein at least one of the first and second internal sealing members (2605oa', 2605oa") is circular and has an adjustable diameter, such that the circumference is adjusted by adjustment of the diameter.

6. The medical device according to claim 1, wherein the internal sealing device comprises at least one of;
a. an internal vacuum sealing device, sealing by using a vacuum towards the human organ or human tissue related to and in contact with said human organ,
b. an internal pressure sealing device, sealing using a pressure towards the human organ or human tissue related to and in contact with said human organ, and
c. an internal adhesive sealing device, sealing by adhesively contacting the human organ or human tissue related to and in contact with said human organ.

7. The medical device according to claim 6, wherein the internal sealing member comprises at least one sealing surface adapted to connect to and seal against a tissue portion of the patient, wherein the sealing surface is placed in the front of the internal sealing device, such that the internal sealing device can seal axially against a surface of a tissue.

8. The medical device according to claim 6, wherein the internal sealing member comprises at least one sealing surface adapted to connect to and seal against a tissue portion of the patient, wherein the sealing surface is placed on the inner circumference of the internal sealing device, such that the internal sealing device can seal radially and thus against the outer circumference of a tubular organ or tissue.

9. The medical device according to any one of claims 1 - 8, wherein at least one of the internal sealing members comprises a pleated portion, such that the circumference is adjustable by the adjustment of the pleated portion.

10. The medical device according to claim 6, wherein the internal sealing device is an internal vacuum sealing device comprising a pleated vacuum conduit comprising perforations adapted to generate suction against the tissue of the patient, such that a sealing is created between the pleated vacuum conduit and the organ or tissue of the patient.

11. The medical device according to claim 10, further comprising a vacuum conduit adapted to connect the pleated vacuum conduit to a vacuum source outside the body of the patient.

12. The medical device according to any one of claims 10 and 11, wherein the perforations are placed in the front end of the internal sealing device, such that the internal sealing device can seal against a planar surface of a tissue.

13. The medical device according to any one of claims 10 and 11, wherein the perforations are placed on the inner circumference of the pleated vacuum conduit, such that the internal sealing device can seal radially and thus against the outer circumference of a tubular organ or tissue.

14. The medical device according to any one of claims 1 - 13, wherein the internal sealing device is adapted to be positioned, at least one of;
a. on the human organ,
b. on human tissue related to and in contact with said human organ,
c. in an incision made in the at least one human organ of the patient,
d. on the inside of the at least one human organ or human tissue related to and in contact with the inside of said human organ, and
e. on the outside of the at least one human organ or human tissue related to and in contact with the outside of said human organ.

15. The medical device according to any one of claims 1 - 14, wherein the medical device chamber is adapted to communicate with at least one of;
- the cavity inside the organ in the patient's body,
- a portion of the medical chamber placed outside the body, and
- a portion of the medical chamber placed outside the body, wherein a port placed in the incision of the body and connected to the wall, is adapted to be opened or closed to allow communication to the portion of the chamber placed outside the body when said port being open.
wherein the internal sealing device is adapted to be positioned inside the body to seal the total chamber formed by the alternatives above, towards the outside thereof.
